(19) Europäisches Patentamt
European Patent Office
Office européen des brevets

(11) Veröffentlichungsnummer: **0 092 830**
**A2**

# (12) EUROPÄISCHE PATENTANMELDUNG

(21) Anmeldenummer: 83104037.3

(22) Anmeldetag: 25.04.83

(51) Int. Cl.³: **C 07 D 501/20**
A 61 K 31/545, C 07 D 277/48
C 07 D 417/12, C 07 D 285/08

(30) Priorität: 27.04.82 CH 2568/82
09.11.82 CH 6504/82

(43) Veröffentlichungstag der Anmeldung: 02.11.83 Patentblatt 83/44

(84) Benannte Vertragsstaaten: AT BE CH DE FR IT LI LU NL SE

(71) Anmelder: **CIBA-GEIGY AG**
**Patentabteilung Postfach**
**CH-4002 Basel(CH)**

(72) Erfinder: **Kocsis, Karoly, Dr.**
**Austrasse 54/3**
**CH-4051 Basel(CH)**

(72) Erfinder: **Wiederkehr, René, Dr.**
**Grenzweg 9**
**CH-4148 Pfeffingen(CH)**

(72) Erfinder: **Wehrli, Hansuli, Dr.**
**Binningerstrasse 41**
**CH-4153 Reinach(CH)**

(74) Vertreter: **Zumstein, Fritz, Dr. et al,**
**Bräuhausstrasse 4**
**D-8000 München 2(DE)**

## (54) 7 beta-Acylamido-3-cephem-4-carbonsäureverbindungen, Verfahren zu ihrer Herstellung, pharmazeutische Präparate welche diese Verbindungen enthalten, und Verwendung von letzteren.

(57) 7β-Acylamido-3-cephem-4-carbonsäure-Verbindungen der Formel

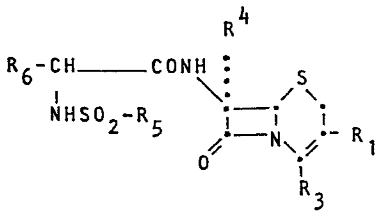

(I) ,

worin

$R_1$ Wasserstoff, Niederalkyl, Niederalkoxy, Halogen oder eine Gruppe der Formel $-CH_2-R_2$, worin
$R_2$ eine freie, veresterte oder verätherte Hydroxyl- oder Mercaptogruppe oder eine Ammoniogruppe darstellt,
$R_3$ Carboxyl oder geschütztes Carboxyl,
$R_4$ Wasserstoff,
$R_5$ einen organischen Rest, welcher mit einem Kohlenstoffatom an die Sulfonylgruppe gebunden ist, und
$R_6$ einen heterocyclischen Rest darstellen,

Hydrate und Salze von diesen Verbindungen besitzen antibiotische Eigenschaften und sind gegen grampositive und gramnegative Mikroorganismen wirksam. Die neuen Verbindungen können z.B. in Form von antibiotisch wirksamen Präparaten zur Behandlung von Infektionen verwendet werden. Die neuen Verbindungen werden in an sich bekannter Weise hergestellt. Ebenfalls umfasst sind Zwischenprodukte.

0092830



CIBA-GEIGY AG
Basel (Schweiz)

4-13895/1+2 /+

7β-Acylamido-3-cephem-4-carbonsäureverbindungen, Verfahren zu ihrer Herstellung, pharmazeutische Präparate, welche diese Verbindungen enthalten, und Verwendung von letzteren

Die vorliegende Erfindung betrifft neue 7β-Acylamido-3-cephem-4-carbonsäure-Verbindungen, Verfahren zu ihrer Herstellung, pharmazeutische Präparate, welche solche Verbindungen enthalten, und ihre Verwendung zur Herstellung von pharmazeutischen Präparaten oder als pharmakologisch wirksame Verbindungen, sowie neue Zwischenprodukte und Verfahren zu ihrer Herstellung.

Die vorliegende Erfindung betrifft 7β-Acylamido-3-cephem-4-carbonsäure-Verbindungen der Formel

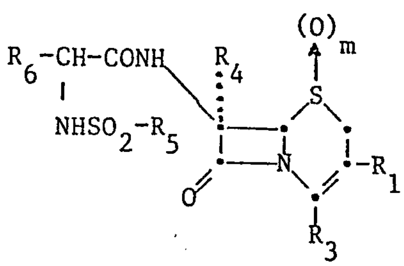

(I),

worin m eine ganze Zahl von 0 bis 2,

$R_1$ Wasserstoff, Niederalkyl, Niederalkenyl, Niederalkoxy, Halogen, eine Gruppe der Formel $-CH_2-R_2$, worin $R_2$ eine freie, veresterte oder verätherte Hydroxy- oder Mercaptogruppe oder eine Ammoniogruppe darstellt, oder eine Gruppe der Formel $-CH=CHR_2$, worin $R_2$ eine verätherte Mercaptogruppe darstellt,

$R_3$ Carboxy oder geschütztes Carboxy,

$R_4$ Wasserstoff,

$R_5$ einen organischen Rest, welcher mit einem Kohlenstoffatom an die Sulfonylgruppe gebunden ist, und

$R_6$ einen heterocyclischen Rest darstellen.

Stereoisomere, Mischungen von diesen Stereoisomeren, Hydrate und Salze von Verbindungen der Formel I, Verfahren zur Herstellung von Verbindungen der Formel I, pharmazeutische Präparate, welche Verbindungen der Formel I enthalten und die Verwendung von Verbindungen der Formel I zur Herstellung von pharmazeutischen Präparaten oder als pharmakologisch wirksame Verbindungen.

In der Beschreibung der vorliegenden Erfindung bedeutet der im Zusammenhang mit Gruppen oder Resten, z.B. Niederalkyl, Niederalkylen, Niederalkoxy, Niederalkanoyl etc., verwendete Ausdruck "Nieder", dass die so bezeichneten Gruppen oder Reste, falls nicht ausdrücklich anders definiert, bis zu 7 und bevorzugt bis zu 4 Kohlenstoffatome enthalten.

In der Formel I bedeutet m in erster Linie null. Falls m den Wert 1 hat, kann die 1-Oxidogruppe in α- oder β-Stellung stehen. Es kann auch ein Gemisch von Verbindungen der Formel I mit der 1-Oxidogruppe in beiden Stellungen vorliegen.

Das Kohlenstoffatom mit der substituierten Aminogruppe der Teilformel $-NSO_2-R_5$ ist R- oder S-konfiguriert. Es kann auch ein Gemisch von Verbindungen der Formel I mit der substituierten Aminogruppe der Teilformel $-NHSO_2-R_5$ in beiden Stellungen vorliegen.

Niederalkyl $R_1$ enthält 1-4 Kohlenstoffatome und ist beispielsweise Aethyl, Propyl, Butyl oder insbesondere Methyl.

Niederalkenyl $R_1$ enthält 1-4 Kohlenstoffatome und ist beispielsweise Vinyl oder Allyl.

Niederalkoxy $R_1$ enthält 1-4 Kohlenstoffatome und ist beispielsweise Aethoxy, Propoxy, Butoxy oder insbesondere Methoxy.

Halogen $R_1$ ist Fluor, Brom, Jod oder bevorzugt Chlor.

Verestertes Hydroxy oder Mercapto $R_2$ ist eine Hydroxy- oder Mercaptogruppe, welche durch eine aliphatische Carbonsäure, eine durch Acyl, z.B. Niederalkanoyl, z.B. Acetyl, substituierte, aliphatische Carbonsäure, die Carbaminsäure oder eine substituierte Carbaminsäure verestert ist, beispielsweise Niederalkanoyloxy, z.B. Acetoxy, Niederalkanoylniederalkanoyloxy, z.B. Acetacetoxy, oder Carbamoyloxy bzw. Niederalkanoylthio, z.B. Acetylthio oder Formylthio, oder Carbamoylthio.

Substituenten der Carbaminsäure sind beispielsweise Niederalkyl, z.B. Methyl oder Aethyl, oder durch Halogen, z.B. Chlor, oder Niederalkanoyloxy, z.B. Acetoxy, substituiertes Niederalkyl, z.B. 2-Chloräthyl oder 2-Acetoxyäthyl.

Durch eine substituierte Carbaminsäure verestertes Hydroxy oder Mercapto $R_2$ ist beispielsweise Methylcarbamoyloxy, Aethylcarbamoyloxy, 2-Chloräthylcarbamoyloxy, 2-Acetoxyäthylcarbamoyloxy oder Methylcarbamoylthio.

Veräthertes Hydroxy oder Mercapto $R_2$ ist eine Hydroxy- oder Mercaptogruppe, welche durch einen aliphatischen Kohlenwasserstoffrest veräthert ist, beispielsweise Niederalkoxy mit 1-4 C-Atomen, z.B. Methoxy oder Aethoxy, oder Niederalkylthio mit 1-4 C-Atomen, z.B. Methylthio.

Veräthertes Mercapto $R_2$ ist vorzugsweise durch einen Heterocyclus veräthert, welcher über ein Ringkohlenstoffatom mit der Mercaptogruppe verbunden ist, z.B. durch einen monocyclischen Heterocyclus, welcher 1 bis 4 Stickstoffheteroatome und gegebenfalls ein zusätzliches Sauerstoff- oder Schwefelatom besitzt oder durch einen bicyclischen Heterocyclus mit 1 bis 5 Stickstoffheteroatomen.Eine solche verätherte Mercaptogruppe wird im folgenden "Heterocyclylthiogruppe $R_2$" genannt.

Heterocyclyl in einer Heterocycylylthiogruppe $R_2$ ist insbesondere aromatisches, monocyclisches, fünf- oder sechsgliedriges Diaza-, Triaza-, Tetraaza-, Thiaza-, Thiadiaza-, Thia-, Oxaza- oder Oxadiazacyclyl oder ist aromatisches oder partiell gesättigtes, bicyclisches, fünf- oder sechs Ringatome pro Ring enthaltendes Aza-, Diaza-, Triaza-, Tetraaza- oder Pentaazabicyclyl.

Substituenten des genannten Heterocyclylrestes in einer Heterocyclylthiogruppe $R_2$ sind beispielsweise unsubstituiertes Niederalkyl, z.B. Aethyl, n-Propyl, Isopropyl, n-Butyl, Isobutyl oder tert.-Butyl, insbesondere Methyl oder substituiertes Niederalkyl, z.B. Methyl oder Aethyl,welches durch folgende funktionelle, abgewandelte funktionelle, sowie heterocyclische Gruppen substituiert ist:

Hydroxy, verestertes Hydroxy, z.B. Niederalkanoyloxy, z.B. Acetoxy, oder Halogen, z.B. Fluor oder Chlor, gegebenenfalls in Salzform, z.B. als Alkalimetall-, z.B. Natriumsalz, vorliegendes Niederalkylphosphonyl, z.B. Natriummethyl- oder Natriumäthylphosphonyl, Diniederalkylphosphonyl, z.B. Dimethyl- oder Diäthylphosphonyl, Carboxy, Sulfo, in Salzform, z.B. als Alkalimetall- oder Ammoniumsalz, z.B. als Natriumsalz, vorliegendes Carboxy oder Sulfo, z.B. Natriumcarboxylat oder Natriumsulfonat, verestertes Carboxy, z.B. Niederalkoxycarbonyl, z.B. Aethoxycarbonyl, Sulfamino, in Salzform, z.B. als Alkalimetall-, z.B. Natriumsalz, vorliegendes Sulfoamino, z.B. Natriumsulfonatoamino, Sulfamoyl, Amino, Niederalkylamino, z.B. Methyl- oder Aethylamino, Diniederalkylamino, z.B. Dimethylamino oder Diäthylamino, Acylamino, z.B. Niederalkanoylamino, z.B. Acetylamino, oder durch Carboxy oder Halogen, z.B. Chlor, substituiertes Niederalkanoylamino, z.B. Carboxyacetylamino oder Chloracetylamino sowie Tetrazolyl, z.B. Tetrazol-1H-5-yl.

Ein Niederalkylrest, welcher durch diese Gruppen substituiert ist, ist beispielsweise:

Hydroxyniederalkyl, z.B. Hydroxymethyl oder 2-Hydroxyäthyl, Acetoxyniederalkyl, z.B. Acetoxymethyl oder 2-Acetoxyäthyl, Halogenniederalkyl, z.B. Chlormethyl, 2-Chloräthyl, 2,2,2-Trichloräthyl oder Trifluormethyl, Niederalkylphosphononiederalkyl, z.B. Aethylphosphonomethyl, Diniederalkylphosphononiederalkyl, z.B. Diäthylphosphonomethyl, Carboxyniederalkyl, z.B. Carboxymethyl oder 2-Carboxyäthyl, Sulfoniederalkyl, z.B. Sulfomethyl oder 2-Sulfoäthyl, Niederalkoxycarbonylniederalkyl, z.B. Aethoxycarbonylmethyl oder 2-Aethoxycarbonyläthyl, Sulfamoylniederalkyl, z.B. Sulfamoylmethyl oder 2-Sulfamoyläthyl, Natriumsulfonatoaminoniederalkyl, z.B. Natriumsulfonatoaminomethyl oder 2-Natriumsulfonatoaminoäthyl, Aminoniederalkyl, z.B. Aminomethyl oder 2-Aminoäthyl, Niederalkylaminoniederalkyl, z.B. Methylaminomethyl oder 2-Methylaminoäthyl, Diniederalkylaminoniederalkyl, z.B. Dimethylaminomethyl oder 2-Dimethylaminoäthyl, Niederalkanoylaminoniederalkyl, z.B. 2-Acetylaminoäthyl, Carboxyniederalkanoylaminoniederalkyl, z.B. 3-Carboxypropionylaminoäthyl oder 2-Carboxyacetylaminoäthyl, oder Halogenniederalkanoylaminoniederalkyl, z.B. 3-Chlorpropionylaminoäthyl oder 2-Chloracetylaminoäthyl, sowie Tetrazolylniederalkyl, z.B. Tetrazol-1H-5-ylmethyl oder 2-(Tetrazol-1H-5-yl)-äthyl,

Niederalkenyl, z.B. Vinyl oder Allyl, funktionelle Gruppen oder abgewandelte, z.B. geschützte, funktionelle Gruppen, z.B. Halogen, z.B. Fluor, Chlor oder Brom, Amino oder substituiertes Amino, z.B. durch Niederalkyl, z.B. Methyl oder Aethyl, mono- oder disubstituiertes Amino, z.B. Methylamino oder Dimethylamino, Acylamino, z.B. Niederalkanoylamino, z.B. Acetylamino, oder Niederalkylsulfonylamino, z.B. Mesylamino, oder durch Halogen, z.B. Chlor, oder Carboxy substituiertes Niederalkanoylamino, z.B. 3-Chlorpropionylamino oder 3-Carboxypropionylamino, Nitro, Hydroxy, Niederalkoxy, z.B. Methoxy oder Aethoxy, Carboxy, verestertes Carboxy, z.B. Niederalkoxycarbonyl, z.B. Methoxycarbonyl oder Aethoxycarbonyl, amidiertes Carbonyl, z.B. Carbamoyl, mono- oder diniederalkyliertes Carbamoyl, z.B. Methylcarbamoyl oder Dimethylcarbamoyl, oder Cyan, sowie Oxo oder Oxido sind ebenfalls Substituenten des Heterocyclylrestes in einer Heterocyclylthiogruppe $R_2$.

Eine Heterocyclylthiogruppe $R_2$, worin Heterocyclyl aromatisches, monocyclisches, fünfgliedriges Heterocyclyl darstellt, ist vorzugsweise Imidazolylthio, z.B. 2-Imidazolylthio, Triazolylthio oder durch Niederalkyl, z.B. Methyl, und/oder Phenyl substituiertes Triazolylthio, z.B. 1H-1,2,3-Triazol-5-yl-thio, 1-Methyl-1H-1,2,3-triazol-4-yl-thio, 1H-1,2,4-Triazol-3-ylthio, 5-Methyl-1H-1,2,4-triazol-4-ylthio, 1H-1,2,4-Triazol-3-ylthio, 5-Methyl-1H-1,2,4-triazol-3-ylthio oder 4,5-Dimethyl-1,2,4-triazol-3-ylthio, Tetrazolylthio, z.B. 1H-Tetrazol-5-ylthio, durch Niederalkyl, z.B. Methyl oder Aethyl, oder substituiertes Niederalkyl, z.B. Aethyl- oder Diäthylphosphonomethyl, 2-Carboxyäthyl, Sulfomethyl, 2-Sulfoäthyl, 2-Natriumsulfonatoäthyl, 2-Dimethylaminoäthyl, Cyanomethyl oder Tetrazolylmethyl substituiertes Tetrazolylthio, z.B.1-Methyl-1H-tetrazol-5-ylthio, 1-Aethyl- oder 1-Diäthylphosphonylmethyl-1H-tetrazol-5-ylthio, 1-Carboxymethyl-1H-tetrazol-5-ylthio, 1-(2-Carboxyäthyl)-1H-tetrazol-5-ylthio, 1-Sulfomethyl-1H-tetrazol-5-ylthio, 1-(2-Sulfoäthyl)-1H-tetrazol-5-ylthio, 1-(2-Natriumsulfonatoäthyl)-1H-tetrazol-5-ylthio, 1-(2-Dimethylaminoäthyl)-1H-tetrazol-5-ylthio, 1-Cyanomethyl-1H-tetrazol-5-ylthio, 1-(Tetrazol-1H-5-ylmethyl)-1H-tetrazol-5-ylthio, Thiazolylthio oder durch Carboxyniederalkyl, z.B. Carboxymethyl, und/oder Niederalkyl, z.B. Methyl, substituiertes Thiazolylthio, z.B. 2-Thiazolylthio, 4-Methyl-5-carboxymethylthiazol-2-ylthio oder 4,5-Dimethyl-2-thiazclylthio, Isothiazolylthio, z.B. 3-Isothiazolylthio, 4-Isothiazolylthio oder 5-Isothiazolylthio, Thiadiazolylthio oder durch Niederalkyl, z.B. Methyl, substituiertes Thiadiazolylthio, z.B. 1,2,3-Thiadiazol-4-ylthio, 1,2,3-Thiadiazol-5-ylthio, 1,3,4-Thiadiazol-2-ylthio, 2-Methyl-1,3,4-thiadiazol-5-ylthio, 1,2,4-Thiadiazolyl-5-ylthio oder 1,2,5-Thiadiazol-3-ylthio, Thiatriazolylthio, z.B. 1,2,3,4-Thiatriazol-5-ylthio, Oxazolylthio oder durch Niederalkyl, z.B. Methyl, substituiertes Oxazolylthio, z.B. 2- oder 5-Oxazolylthio, oder 4-Methyl-5-oxazolylthio, durch Niederalkyl, z.B. Methyl, substituiertes Isooxazolylthio, z.B. 3-Methyl-5-isoxazolylthio, Oxadiazolylthio oder durch Niederalkyl, z.B. Methyl, substituiertes

Oxadiazolylthio, z.B. 1,2,4-Oxadiazol-5-ylthio oder 2-Methyl-1,3,4-oxadiazol-5-ylthio.

Eine Heterocyclylthiogruppe $R_2$, worin Heterocyclyl aromatisches, monocyclisches, sechsgliedrigs Heterocyclyl darstellt, enthält 1-3 Stickstoffatome und ist vorzugsweise 5,6-Dioxotetrahydro-as-triazinylthio oder durch Niederalkyl, z.B. Methyl, Carboxyniederalkyl, z.B. Carboxymethyl, oder durch Sulfoniederalkyl, z.B. Sulfomethyl, substituiertes 5,6-Dioxotetrahydro-as-triazinylthio, z.B. 1- oder 2-Methyl-5,6-dioxo-1,2,5,6-tetrahydro-as-triazin-3-ylthio, 4-Methyl-5,6-dioxo-1,4,5,6-tetrahydro-as-triazin-3-ylthio, 1- oder 2-Carboxymethyl-5,6-dioxo-1,2,5,6-tetrahydro-as-triazin-3-ylthio, 4-Carboxymethyl-5,6-dioxo-1,4,5,6-tetrahydro-as-triazin-3-ylthio, 1- oder 2-Sulfomethyl-5,6-dioxo-1,2,5,6-tetrahydro-as-triazin-3-ylthio oder 4-Sulfomethyl-5,6-dioxo-1,4,5,6-tetrahydro-as-triazin-3-ylthio.

Eine Heterocyclylthiogruppe $R_2$, worin Heterocyclyl aromatisches oder partiell gesättigtes, bicyclisches, fünf- oder sechs Ringatome pro Ring enthaltendes Heterocyclyl darstellt, ist vorzugsweise Indolylthio, durch Niederalkyl, z.B. Methyl, substituiertes Indolylthio, z.B. Indol-2-ylthio oder N-Methylindol-2-ylthio, Isoindolylthio, z.B. Isoindol-2-ylthio, Chinolylthio, z.B. 2-, 4- oder 8-Chinolylthio, Benzimidazolylthio, durch Niederalkyl, z.B. Methyl, oder Carboxyniederalkyl, z.B. Carboxymethyl, substituiertes Benzimidazolylthio, z.B. 1-Methyl-, 1-Carboxymethyl- oder 1-(2-Carboxyäthyl)-benzimidazol-2-ylthio, Benztriazolylthio, durch Niederalkyl, z.B. Methyl, oder Carboxyniederalkyl, z.B. Carboxymethyl, substituiertes Benzotriazolylthio, z.B. 1-Methyl- oder 1-Carboxymethyl-1H-benzo[d]-triazol-5-ylthio, Tetrazolopyridazinylthio oder durch Niederalkyl, z.B. Methyl oder Aethyl, Carboxy, Carboxyniederalkyl, z.B. Carboxymethyl, Carbamoyl, Niederalkylcarbamoyl, z.B. Methylcarbamoyl, Diniederalkylcarbamoyl, z.B. Dimethylcarbamoyl, Amino, Niederalkylamino, z.B. Methylamino, Diniederalkylamino, z.B. Dimethylamino oder Diäthylamino, substituiertes Tetrazolopyridazinylthio, z.B. 8-Methyl-, 8-Aethyl-, 8-Carboxy-, 8-Carboxymethyl-,

8-(2-Carboxyäthyl)-, 8-Carbamoyl-, 8-Methylcarbamoyl-, 8-Dimethylcarbamoyl-, 8-Amino-, 8-Dimethylamino- oder 8-Diäthylaminotetrazolo-[1,5-b]pyridazin-6-ylthio.

Eine Ammoniogruppe $R_2$ ist von einer organischen, tertiären, stickstoffhaltigen Base, beispielsweise von einem tertiären, aliphatischen Amin oder vorzugsweise von einer tertiären, heterocyclischen, aromatischen Stickstoffbase, abgeleitet, indem die betreffende Base mit ihrem Stickstoffatom an die in 3-Stellung des Cephemgerüsts befindliche Methylengruppe gebunden ist. Die positive Ladung am quaternären Stickstoffatom der Ammoniogruppe wird beispielsweise durch die negativ geladene Carboxylatgruppe kompensiert, welche sich statt der undissoziierten Carboxylgruppe in 4-Stellung des Cephemgerüstes befindet.

Eine Ammoniogruppe $R_2$, welche von einem tertiären, aliphatischen Amin abgeleitet ist, ist beispielsweise Triniederalkylammonio, z.B. Trimethyl- oder Triäthylammonio.

Eine quaternäre Ammoniogruppe $R_2$, welche von einer tertiären, heterocyclischen, aromatischen Stickstoffbase abgeleitet ist, ist beispielsweise 1-Pyrazolio oder durch Niederalkyl, z.B. Methyl oder Aethyl, Niederalkenyl, z.B. Vinyl oder Allyl, Carboxyniederalkyl, z.B. Carboxymethyl, Niederalkoxycarbonylniederalkyl, z.B. Methoxycarbonylmethyl, Sulfoniederalkyl, z.B. Sulfomethyl, Aminoniederalkyl, z.B. 2-Aminoäthyl, oder Diniederalkylaminoniederalkyl, z.B. 2-Dimethylaminoäthyl, in 2-Stellung substituiertes 1-Pyrazolio, z.B. 2-Methyl- oder 2-Aethyl-1-pyrazolio, 2-Allyl- oder 2-Vinyl-1-pyrazolio, 2-Sulfomethyl-1-pyrazolio, 2-(2-Aminoäthyl)-1-pyrazolio oder 2-(2-Dimethylaminoäthyl)-1-pyrazolio, 1-Triazolio oder durch Niederalkyl, z.B. Methyl oder Aethyl, Carboxyniederalkyl, z.B. Carboxymethyl oder Diniederalkylaminoniederalkyl, z.B. 2-Dimethylaminoäthyl, in 3-Stellung substituiertes 1-Triazolio, z.B. 3-Methyl-1-triazolio, 3-Carboxymethyl-1-triazolio oder 3-(2-Dimethylaminoäthyl)-1-triazolio.

Eine Ammoniogruppe $R_2$, welche von einer tertiären, heterocyclischen, aromatischen Stickstoffbase abgeleitet ist, ist vorzugsweise Pyridinio oder durch Niederalkyl, z.B. Methyl, Carbamoyl, Niederalkylcarbamoyl, z.B. Methylcarbamoyl, Hydroxyniederalkyl, z.B. Hydroxymethyl, Niederalkoxyniederalkyl, z.B. Methoxymethyl, Cyanoniederalkyl, z.B. Cyanomethyl, Carboxyniederalkyl, z.B. Carboxymethyl, Sulfoniederalkyl, z.B. 2-Sulfoäthyl, Carboxyniederalkenyl, z.B. 2-Carboxyvinyl, Carboxyniederalkylthio, z.B. Carboxymethylthio, Thiocarbamoyl, Halogen, z.B. Brom oder Chlor, Carboxy, Sulfo oder Cyan mono- oder disubstituiertes Pyridinio, z.B. Niederalkylpyridinio, z.B. 2-, 3- oder 4-Methylpyridinio oder 2-, 3- oder 4-Aethylpyridinio, Carbamoylpyridinio, z.B. 3- oder 4-Carbamoylpyridinio, Niederalkylcarbamoylpyridinio, z.B. 3- oder 4-Methylcarbamoylpyridinio, Diniederalkylcarbamoylpyridinio, z.B. 3- oder 4-Dimethylcarbamoylpyridinio, Hydroxyniederalkylpyridinio, z.B. 3- oder 4-Hydroxymethylpyridinio, Niederalkoxyniederalkylpyridinio, z.B. 4-Methoxymethylpyridinio, Cyanniederalkylpyridinio, z.B. 3-Cyanmethylpyridinio, Carboxyniederalkylpyridinio, z.B. 3-Carboxymethylpyridinio, Sulfoniederalkylpyridinio, z.B. 4-(2-Sulfoäthylpyridinio), Carboxyniederalkenylpyridinio, z.B. 3-(2-Carboxyvinyl)-pyridinio, Carboxyniederalkylthiopyridinio, z.B. 4-Carboxymethylthiopyridinio, Thiocarbamoylpyridinio, z.B. 4-Thiocarbamoylpyridinio, Halogenpyridinio, z.B. 3-Brom- oder 4-Brompyridinio, Carboxypyridinio, z.B. 3- oder 4-Carboxypyridinio, Sulfopyridinio, z.B. 3- oder 4-Sulfopyridinio, Cyanpyridinio, z.B. 3-Cyanpyridinio, Carboxyniederalkyl-carbamoylpyridinio, z.B. 3-Carboxymethyl-4-carbamoylpyridinio, Aminocarbamoylpyridinio, z.B. 2-Amino-5-carbamoylpyridinio, Carboxycarbamoylpyridinio, z.B. 3-Carboxy-4-carbamoylpyridinio, Cyanohalogenmethylpyridinio, z.B. 3-Cyano-4-trifluormethylpyridinio oder Amino-carboxypyridinio, z.B. 2-Amino-3-carboxypyridinio.

Eine Ammoniogruppe $R_2$ ist bevorzugt Pyridinio oder durch Hydroxyniederalkyl, z.B. Hydroxymethyl, Carboxy, Carboxyniederalkyl, z.B.

Carboxymethyl, Halogen, z.B. Chlor oder Brom,oder Carbamoyl substituiertes Pyridinio, z.B. 3- oder 4-Hydroxymethylpyridinio, 4-Carboxypyridinio, 3- oder 4-Carboxymethylpyridinio, 3- oder 4-Chlorpyridinio, 3- oder 4-Brompyridinio oder 3- oder 4-Carbamoylpyridinio.

In einer Gruppe der Formel $-CH=CH-R_2$ hat die verätherte Mercaptogruppe $R_2$ die weiter vorn genannten Bedeutungen, z.B. Heterocyclylthio. $R_2$ bedeutet bevorzugt durch Niederalkoxy, z.B. Methoxy, substituiertes 5,6-Dioxotetrahydro-as-triazinylthio, z.B. 4-Methoxy-5,6-dioxo-1,4,5,6-tetrahydro-as-triazin-3-ylthio.

Geschütztes Carboxyl $R_3$ ist durch eine der im folgenden beschriebenen Carboxylschutzgruppen verestertes Carboxyl, insbesondere unter physiologischen Bedingungen spaltbares, verestertes Carboxyl.

Eine unter physiologischen Bedingungen spaltbare, veresterte Carboxylgruppe $R_3$ ist in erster Linie eine Acyloxyniederalkoxycarbonylgruppe, worin Acyl, z.B. die Acylgruppe einer organischen Carbonsäure, in erster Linie einer gegebenenfalls substituierten Niederalkancarbonsäure bedeutet, oder worin Acyloxymethyl den Rest eines Lactons bildet.

Eine solche veresterte Carboxylgruppe $R_3$ ist vorzugsweise Niederalkanoyloxyniederalkoxycarbonyl, z.B. Niederalkanoyloxymethoxycarbonyl oder Niederalkanoyloxyäthoxycarbonyl, z.B.Acetoxymethoxycarbonyl, Pivaloyloxymethoxycarbonyl oder 2-Propionyloxyäthoxycarbonyl, Niederalkoxycarbonyloxyniederalkoxycarbonyl, z.B. 1-Aethoxycarbonyloxyäthoxycarbonyl oder tert.-Butoxycarbonyloxymethoxycarbonyl, Aminoniederalkanoyloxymethoxycarbonyl, insbesondere α-Amino-niederalkanoyloxymethoxycarbonyl, z.B. Glycyloxymethoxycarbonyl, L-Valyloxymethoxycarbonyl oder L-Leucyloxymethoxycarbonyl, ferner Phthalidyloxycarbonyl, z.B. 2-Phthalidyloxycarbonyl, oder Indanyloxycarbonyl, z.B. 5-Indanyloxycarbonyl.

Ein organischer Rest $R_5$, welcher mit einem Kohlenstoffatom an die Sulfonylgruppe gebunden ist, hat bis zu 18 Kohlenstoffatome und ist ein unsubstituierter oder substituierter, gesättigter oder ungesättigter, aliphatischer, cycloaliphatischer oder cycloaliphatisch-aliphatischer Kohlenwasserstoffrest, ein unsubstituierter oder substituierter aromatischer oder aromatisch-aliphatischer Kohlenwasserstoffrest oder ist ein unsubstituierter oder substituierter Heterocyclyl- oder Heterocyclyl-aliphatischer Rest.

Ein gesättigter, aliphatischer Kohlenwasserstoffrest $R_5$ ist beispielsweise Niederalkyl mit 1-7, bevorzugt 1-4, Kohlenstoffatomen, z.B. Methyl, Aethyl, n-Propyl, Isopropyl oder n-Butyl oder ist Niederalkyl, welches durch eine, zwei oder mehrere funktionelle oder abgewandelte funktionelle Gruppen substituiert ist, beispielsweise durch Hydroxy, veräthertes Hydroxy, beispielsweise Niederalkoxy, z.B. Methoxy, Aethoxy oder tert.-Butyloxy, oder Niederalkenyloxy, z.B. Vinyloxy oder Allyloxy, verestertes Hydroxy, z.B. Niederalkanoyloxy, z.B. Acetoxy, oder Halogen, z.B. Chlor, veräthertes Mercapto, z.B. Niederalkylthio, z.B. Methylthio oder Aethylthio, oder Niederalkylthio, worin Niederalkyl durch Amino und Carboxy substituiert ist, z.B. 2-Amino-2-carboxyäthylthio, oder Heterocyclylthio, wobei Heterocyclyl wie weiter vorn der Heterocyclylrest in einer Heterocyclylgruppe $R_2$ definiert ist, Niederalkanoyl, z.B. Acetyl, Aroyl, z.B. Benzoyl, Carboxy, verestertes Carboxy, z.B. Niederalkoxycarbonyl, amidiertes Carboxy, z.B. Carbamoyl, Niederalkylcarbamoyl, z.B. Methylcarbamoyl, Diniederalkylcarbamoyl, z.B. Dimethylcarbamoyl, Cyano, Sulfo, Niederalkansulfonyl, z.B. Methansulfonyl, Sulfamoyl, Niederalkylsulfamoyl, z.B. Methylsulfamoyl, Diniederalkylsulfamoyl, z.B. Dimethylsulfamoyl, Amidino, Guanidino, oder Amino zusammen mit einer oder zwei der genannten funktionellen Gruppen, wobei, falls möglich, die Substituenten in einer höheren als der 1-Stellung des Niederalkylrests stehen.

Niederalkyl, welches durch eine, zwei oder mehrere funktionelle oder abgewandelte funktionelle Gruppen substituiert ist, ist beispielsweise

Hydroxyniederalkyl, z.B. Hydroxymethyl oder 2-Hydroxyäthyl, Niederalkoxyniederalkyl, z.B. Methoxymethyl, 2-Methoxyäthyl oder 2-Aethoxyäthyl, Niederalkenyloxyniederalkyl, z.B. 2-Vinyloxyäthyl, Niederalkanoyloxyniederalkyl, z.B. 2-Acetoxyäthyl, Halogeniederalkyl, z.B. Chloromethyl, 2-Chloroäthyl, 3-Chloropropyl, 4-Chlorobutyl oder 2-Bromoäthyl, Niederalkylthioniederalkyl, z.B. 2-Methylthioäthyl oder 2-Aethylthioäthyl, Aminocarboxyniederalkylthioniederalkyl, z.B. 2-(2-Amino-2-carboxyäthylthio)-äthyl, Benzoylniederalkyl, z.B. Benzoylmethyl, Carboxyniederalkyl, z.B. Carboxymethyl oder 2-Carboxyäthyl, Niederalkoxycarbonylniederalkyl, z.B. Aethoxycarbonylmethyl oder 2-Aethoxycarbonyläthyl, Carbamoylniederalkyl, z.B. Carbamoylmethyl, Cyanoniederalkyl, z.B. Cyanomethyl oder 1-Cyano-oder 2-Cyanoäthyl, Sulfoniederalkyl, z.B. Sulfomethyl oder 2-Sulfoäthyl, Sulfamoylniederalkyl, z.B. Sulfamoylmethyl oder 2-Sulfamoyläthyl, oder ist Aminocarboxyniederalkyl, z.B. 2-Amino-2-carboxyäthyl.

Niederalkyl, welches durch eine funktionelle oder abgewandelte funktionelle Gruppe substituiert ist, hat vorzugsweise die Teilformel

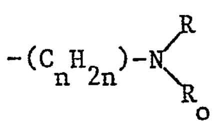

(A),

worin n eine ganze Zahl von 1 bis 4, R Wasserstoff, Niederalkyl, Sulfo, in Salzform vorliegendes Sulfo, oder eine Acylgruppe und $R_o$ Wasserstoff oder Niederalkyl bedeuten, oder worin der Stickstoff Bestandteil eines Heterocyclus ist und R und $R_o$ zusammen Alkylen darstellen, welches gegebenenfalls durch Sauerstoff, Schwefel, >NH oder durch Niederalkyl, z.B. Methyl, substituierten Stickstoff unterbrochen ist.

Die Gruppe $-(C_nH_{2n})-$ ist eine unverzweigte oder verzweigte Alkylenkette und ist beispielsweise Methylen, 1,2-Aethylen, 1,3-Propylen oder 1,4-Butylen, ferner 1,1-Aethylen, 1,1-Propylen, 1,2-Propylen, 1,1-Butylen oder 1,1-Isobutylen.

Niederalkyl R hat 1-7 Kohlenstoffatome und ist beispielsweise Methyl, Aethyl, Isopropyl, n-Propyl, Isobutyl, tert.-Butyl, n-Pentyl, Neopentyl, n-Hexyl oder n-Heptyl.

In Salzform vorliegendes Sulfo R ist beispielsweise als Alkalimetallsalz, z.B. Natriumsalz, oder als Ammoniumsalz vorliegendes Sulfo.

Eine Acylgruppe R hat bis zu 19 Kohlenstoffatome und ist die Acylgruppe R einer Carbonsäure, eines Halbesters der Kohlensäure, der Carbaminsäure, einer substituierten Carbaminsäure, der Thiocarbaminsäure, einer substituierten Thiocarbaminsäure, einer Sulfonsäure, der Amidosulfonsäure, einer substituierten Amidosulfonsäure oder ist eine Acylcarbamoyl- oder Acylthiocarbamoylgruppe.

Eine solche Acylgruppe R hat beispielsweise die Teilformeln:

$R^a$-CO-, $R^a$-O-CO-, $(R^a)R^b$N-CO-, $(R^a)R^b$N-CS-, $R^a$-$SO_2$-, $(R^a)R^b$N-$SO_2$-, $(R^a$-CO$)$-$R^b$N-CO-, $(R^a$-CO$)$-$R^b$N-CS- oder

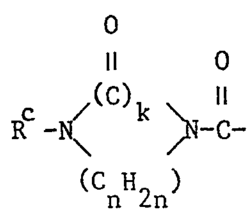

(B),

worin n eine ganze Zahl von 1 bis 4, bevorzugt 2, k 1 oder 2, $R^a$ oder $R^b$ unabhängig voneinander Wasserstoff, einen unsubstituierten oder substituierten,gesättigten oder ungesättigten, aliphatischen, cycloaliphatischen, cycloaliphatisch-aliphatischen Kohlenwasserstoffrest mit bis zu 18, bevorzugt bis zu 10, Kohlenstoffatomen, einen unsubstituierten oder substituierten, aromatischen oder aromatisch-aliphatischen Kohlenwasserstoffrest mit bis zu 18, bevorzugt bis zu 10, Kohlenstoffatomen oder einen unsubstituierten oder substituierten Heterocyclyl- oder Heterocyclylniederalkylrest und $R^c$ Wasserstoff, Niederalkyl, Niederalkyl substituiert durch Hydroxy, Halogen, Carboxy, Niederalkoxy oder Amino, Niederalkenyl, Niederalkanoyl, Niederalkansulfonyl oder Sulfamoyl bedeuten.

Ein gesättigter oder ungesättigter, aliphatischer, cycloaliphatischer oder cycloaliphatisch-aliphatischer Kohlenwasserstoffrest $R^a$ oder $R^b$ ist beispielsweise Niederalkyl, Niederalkenyl, Niederalkinyl, Cycloalkyl, Cycloalkenyl, Cycloalkylniederalkyl, Cycloalkylniederalkenyl oder Cycloalkenylniederalkyl.

Substituenten eines solches Rests $R^a$ oder $R^b$ sind beispielsweise Hydroxy, veräthertes oder verestertes Hydroxy, z.B. Niederalkoxy, z.B. Methoxy oder Aethoxy, Niederalkanoyloxy, z.B. Acetoxy, in Salzform vorliegendes Hydroxysulfonyloxy oder Halogen, z,B. Chlor, veräthertes Mercapto, z.B. Niederalkylthio, z.B. Methylthio, Carboxy, verestertes Carboxy, z.B. Niederalkoxycarbonyl, z.B. Methoxycarbonyl oder Aethoxycarbonyl, amidiertes Carboxy, z.B. Carbamoyl, Cyano, Nitro, in Salzform vorliegendes Sulfo, Amino, Niederalkanoylamino, z.B. Acetylamino, Niederalkylamino, z.B. Methyl- oder Aethylamino, oder Diniederalkylamino, z.B. Dimethylamino.

Niederalkyl $R^a$ oder $R^b$ enthält bis zu 7 Kohlenstoffatome und ist z.B. Methyl, Aethyl, n-Propyl, Isopropyl, n-Butyl, Isobutyl, tert.-Butyl, n-Pentyl, n-Hexyl oder n-Heptyl.

Substituiertes Niederalkyl $R^a$ oder $R^b$ ist in erster Linie substituiertes Methyl oder ist Aethyl oder Propyl, wobei die Substituenten, falls möglich, vorzugsweise in einer höheren als der 1-Stellung des Niederalkylrests stehen.

Substituiertes Niederalkyl $R^a$ oder $R^b$ ist beispielsweise Hydroxyniederalkyl, z.B. Hydroxymethyl, 2-Hydroxyäthyl oder 3-Hydroxypropyl, Niederalkoxyniederalkyl, z.B. Niederalkoxymethyl, Niederalkoxyäthyl oder Niederalkoxypropyl, z.B. Methoxymethyl, 2-Methoxyäthyl oder 3-Methoxypropyl, Niederalkanoyloxyniederalkyl, z.B. Niederalkanoyloxymethyl, Niederalkanoyloxyäthyl oder Niederalkanoyloxypropyl, z.B. Acetoxymethyl, Propionyloxymethyl, 2-Acetoxyäthyl oder 3-Acetoxypropyl, Halogenniederalkyl, z.B. Halogenmethyl, Halogenäthyl oder

Halogenpropyl, z.B. 2-Chlor- oder 2-Bromäthyl oder 3-Chlor- oder 3-Brompropyl, in Salzform, z.B. als Alkalimetallsalz, z.B. als Natriumsalz, oder als Ammoniumsalz, vorliegendes Hydroxysulfonyloxyniederalkyl, z.B. Hydroxysulfonyloxymethyl, 2-Hydroxysulfonyloxyäthyl oder 3-Hydroxysulfonyloxypropyl, Niederalkylthioniederalkyl, z.B. Methylthiomethyl, 2-Methylthioäthyl, 2-Methylthiopropyl oder tert.-Butylthiomethyl, Carboxyniederalkyl, z.B. Carboxymethyl oder 2-Carboxyäthyl, Niederalkoxycarbonylniederalkyl, z.B. Niederalkoxycarbonylmethyl oder Niederalkoxycarbonyläthyl, z.B. Methoxycarbonylmethyl, 2-Methoxycarbonyläthyl, Aethoxycarbonylmethyl oder 2-Aethoxycarbonyläthyl, Carbamoylniederalkyl, z.B. Carbamoylmethyl oder 2-Carbamoyläthyl, Niederalkylcarbamoylniederalkyl, z.B. Methylcarbamoylmethyl, Diniederalkylcarbamoylniederalkyl, z.B. Dimethylcarbamoylmethyl, Cyanoniederalkyl, z.B. Cyanomethyl oder 2-Cyanoäthyl, in Salzform, z.B. als Alkalimetall-, z.B. als Natriumsalz, oder als Ammoniumsalz, vorliegendes Sulfoniederalkyl, z.B. Sulfomethyl, 2-Sulfoäthyl oder 3-Sulfopropyl, Aminoniederalkyl, z.B. Aminomethyl oder 2-Aminoäthyl, Niederalkanoylaminoniederalkyl, z.B. Acetylaminomethyl oder 2-Acetylaminoäthyl, Niederalkylaminoniederalkyl, z.B. Methylaminomethyl oder 2-Methylaminoäthyl, oder Diniederalkylaminoniederalkyl, z.B. Dimethylaminomethyl oder 2-Dimethylaminoäthyl.

Niederalkenyl $R^a$ oder $R^b$ enthält 2 bis 7, insbesondere 2 bis 4, Kohlenstoffatome, und ist z.B. Vinyl, Allyl oder 2- oder 3-Butenyl. Substituiertes Niederalkenyl $R^a$ oder $R^b$ kann durch die gleichen Substituenten substituiert sein, wie substituiertes Niederalkyl.

Niederalkinyl $R^a$ oder $R^b$ enthält 2 bis 7, insbesondere 2 bis 4, Kohlenstoffatome und ist beispielsweise Aethinyl, 1-Propinyl oder 2-Propinyl.

Cycloalkyl $R^a$ oder $R^b$ enthält 3 bis 7 Kohlenstoffatome und ist z.B. Cyclopropyl, Cyclobutyl, Cyclopentyl oder Cyclohexyl.

Cycloalkenyl $R^a$ oder $R^b$ enthält 3 bis 7 Kohlenstoffatome und ist beispielsweise Cyclohexenyl, z.B. 1-Cyclohexenyl, oder Cyclohexadienyl, z.B. 1,4-Cyclohexadienyl.

Cycloalkylniederalkyl $R^a$ oder $R^b$ enthält 4-9 Kohlenstoffatome und ist beispielsweise Cyclopropylmethyl, Cyclobutylmethyl, Cyclopentylmethyl oder Cyclohexylmethyl.

Cycloalkylniederalkenyl $R^a$ oder $R^b$ enthält 4-9 Kohlenstoffatome und ist beispielsweise Cyclohexylvinyl oder Cyclohexylallyl.

Cycloalkenylniederalkyl $R^a$ oder $R^b$ enthält 4-9 Kohlenstoffatome und ist beispielsweise 1-Cyclohexemylmethyl oder 1,4-Cyclohexadienylmethyl.

Ein aromatischer oder aromatisch-aliphatischer Kohlenwasserstoffrest $R^a$ oder $R^b$ ist beispielsweise Phenyl, Phenylniederalkyl, z.B. Benzyl, 2-Phenyläthyl, Diphenylmethyl oder Trityl, oder Phenylniederalkenyl, z.B. 3-Phenylallyl.

Phenyl, sowie Phenylniederalkyl oder Phenylniederalkenyl kann am Phenylrest beispielsweise durch Niederalkyl, z.B. Methyl oder Aethyl, Niederalkoxy, z.B. Methoxy oder Halogen, z.B. Fluor oder Chlor, ferner durch Nitro oder Amino substituiert sein. In einem substituierten Phenylniederalkyl- oder Phenylniederalkenylrest $R^a$ oder $R^b$ kann Niederalkyl in α-Stellung zum Phenylrest, z.B. durch Hydroxy, Hydroxysulfonyloxy, Carboxy, Sulfo oder Amino substituiert sein.

Heterocyclyl $R^a$ oder $R^b$ hat bis zu 10 Kohlenstoffatome und bis zu 4 Heteroatome aus der Gruppe Stickstoff, Sauerstoff oder Schwefel und ist beispielsweise aromatisches, monocyclisches, fünf- oder sechsgliedriges Aza-, Thia-, Oxa-, Oxaaza-, Thiaaza-, Diaza-, Thiadiaza-, Triaza- oder Tetraazacyclyl. Heterocyclyl $R^a$ oder $R^b$ ist

beispielsweise Pyridyl, z.B. 2- oder 4-Pyridyl, Thienyl, z.B. 2- oder 3-Thienyl, Furyl, z.B. 2- oder 3-Furyl, Oxazolyl, z.B. 2-Oxazolyl, Thiazolyl, z.B. 2-Thiazolyl, Isothiazolyl, z.B. 2- oder 4-Isothiazolyl, Pyrimidyl, z.B. 4- oder 5-Pyrimidyl, Thiadiazolyl, z.B. 1,2,4-Thiadiazolyl-3-yl, 1,2,5-Thiadiazol-3-yl oder 1,2,4-Thiadiazol-3-yl, Triazolyl, z.B. 3-Triazolyl, oder Tetrazolyl, z.B. 1- oder 5-Tetrazolyl.

Substituenten des Heterocyclylrests $R^a$ oder $R^b$ sind die gleichen weiter vorn für die Heterocyclylgruppe $R_2$ genannten Substituenten.

Heterocyclyl $R^a$ oder $R^b$ ist bevorzugt Pyridyl, z.B. 3- oder 4-Pyridyl, Thienyl, z.B. 2- oder 3-Thienyl, Furyl, z.B. 2- oder 3-Furyl, Aminooxazolyl, z.B. 2-Amino-4-oxazolyl, Aminothiazolyl, z.B. 2-Amino-4-thiazolyl, Hydroxypyrimidyl, z.B. 2,6-Dihydroxy-1,3-pyrimid-4-yl, Aminothiadiazolyl, z.B. 5-Amino-1,2,4-thiadiazolyl-3-yl, Hydroxythiadiazolyl, z.B. 4-Hydroxy-1,2,5-thiadiazolyl-3-yl, und Aminotriazolyl, z.B. 5-Amino-1,2,4-triazol-3-yl.

Heterocyclylniederalkyl $R^a$ oder $R^b$ ist beispielsweise Niederalkyl, z.B. Methyl, das durch weiter vorn genanntes Heterocyclyl $R^a$ oder $R^b$ substituiert ist, z.B. Tetrazolylniederalkyl, z.B. 1H-Tetrazol-5-ylmethyl, oder Aminothiazolylniederalkyl, z.B. 2-Amino-1,3-thiazol-4-ylmethyl.

$R^a$ oder $R^b$ ist in erster Linie Wasserstoff, Niederalkyl, z.B. Methyl oder Aethyl, Niederalkyl substituiert durch Hydroxy, Niederalkoxy, z.B. Methoxy, Halogen, z.B. Fluor, Chlor oder Brom, Carboxy, Cyano oder Amino, z.B. 1-Hydroxyäthyl, Methoxymethyl, Cyanomethyl oder Aminomethyl, Niederalkenyl, z.B. Vinyl, Niederalkinyl, z.B. Aethinyl, Cycloalkyl, z.B. Cyclopropyl, Phenyl, Phenyl substituiert durch Amino oder Nitro, z.B. 4-Aminophenyl, 4-Nitrophenyl,2,4 -Dinitrophenyl, Phenylniederalkyl, z.B. Benzyl, Phenylniederalkyl, worin Niederalkyl in α-Stellung zum Phenylrest durch Hydroxy oder Amino substituiert

ist, z.B. Hydroxy- oder Aminobenzyl, Pyridyl, z.B. 4-Pyridyl, Thienyl, z.B. 2-Thienyl, Furyl, z.B. 2-Furyl, Hydroxypyrimidyl, z.B. 2,6-Dihydroxy-1,3-pyrimid-4-yl, Hydroxythiadiazolyl, z.B. 4-Hydroxy-1,2,5-thiadizaol-3-yl, Tetrazolylniederalkyl, z.B. Tetrazolyl-5-ylmethyl, oder Aminothiazolylniederalkyl, z.B. 2-Amino-1,3-thiazol-4-ylmethyl.

Eine Acylgruppe R ist bevorzugt die Acylgruppe einer Carbonsäure, beispielsweise Niederalkanoyl, z.B. Formyl oder Acetyl, Niederalkanoyl substituiert durch Hydroxy, Niederalkoxy, z.B. Methoxy, Halogen, z.B. Brom, Carboxy, Cyano oder Amino, z.B. α-Hydroxypropionyl, Methoxyacetyl, Bromacetyl, Carboxyacetyl, Cyanacetyl oder Glycyl, Niederalkenoyl, z.B. Acryloyl, Niederalkinoyl,z.B. Propioloyl, Cycloalkylcarbonyl, z.B. Cyclopropylcarbonyl, Benzoyl, 4-Aminobenzoyl, 4-Niederalkanoylaminobenzoyl, z.B. 4-Acetylaminobenzoyl, 4-Cyanobenzoyl, 4-Nitrobenzoyl oder 3,4-Dinitrobenzoyl, Pyridylcarbonyl, z.B. Nicotinoyl oder Isonicotinoyl, Furoyl, z.B. 2-Furoyl, Thienylcarbonyl, z.B. 2-Thienylcarbonyl, Hydroxypyrimidylcarbonyl, z.B. 2,6-Dihydroxy-1,3-pyrimid-4-ylcarbonyl, Hydroxythiadiazolylcarbonyl, z.B. 4-Hydroxy-1,2,5-thiadiazol-3-ylcarbonyl, Tetrazolylniederalkanoyl, z.B. 2-Tetrazol-5-ylacetyl oder Aminothiazolylniederalkanoyl, z.B. 2-(2-Amino-1,3-thiazol-4-yl)-acetyl, die Acylgruppe eines Halbesters der Kohlensäure, beispielsweise Niederalkoxycarbonyl, z.B. Methoxycarbonyl oder Isopropoxycarbonyl, Niederalkanoyloxy substituiert durch Carboxy und Amino, z.B. 2-Amino-2-carboxyäthoxycarbonyl, oder Benzoyloxycarbonyl, die Acylgruppe einer substituierten Carbaminsäure, beispielsweise Niederalkylcarbamoyl, z.B. Methylcarbamoyl oder Anilinocarbonyl, die Acylgruppe einer substituierten Thiocarbaminsäure, beispielsweise Niederalkylthiocarbamoyl, z.B. Methylthiocarbamoyl, die Acylgruppe einer substituierten Sulfonsäure, beispielsweise Niederalkansulfonyl, z.B. Methansulfonyl, durch Halogen, z.B. Fluor substituiertes Niederalkansulfonyl, z.B. Difluormethansulfonyl, Benzolsulfonyl, 4-Nitrobenzolsulfonyl, 2,4-Dinitrobenzolsulfonyl, Aminobenzolsulfonyl, z.B. 4-Aminobenzolsulfonyl, eine Acylcarbamoylgruppe, beispielsweise

Benzoylcarbamoyl oder Furoylcarbamoyl, eine Acylthiocarbamoylgruppe, beispielsweise Benzoylthiocarbamoyl oder Furoylthiocarbamoyl, 2-Oxo-1-imidazolidinocarbonyl, 4-Niederalkyl-2,3-dioxo-1-piperazinocarbonyl, z.B. 4-Aethyl-2,3-dioxo-1-piperazinocarbonyl, und 4-Niederalkylsulfonyl-1-piperazino-carbonyl, z.B. 4-Methylsulfonyl-1-piperazinocarbonyl. Niederalkyl $R^c$ ist beispielsweise Methyl oder Aethyl.

Niederalkyl $R^c$ substituiert durch Hydroxy, Halogen, Carboxy, Niederalkoxy oder Amino ist beispielsweise Hydroxymethyl, 1- oder 2-Hydroxyäthyl, Chloräthyl, Trichloräthyl, Carboxymethyl, Methoxymethyl, Aminomethyl oder 2-Aminoäthyl.

Niederalkenyl $R^c$ ist beispielsweise Vinyl, Allyl oder 1-Isobutenyl.

Niederalkanoyl $R^c$ ist beispielsweise Acetyl.

Niederalkansulfonyl $R^c$ ist beispielsweise Methansulfonyl oder Aethansulfonyl.

$R^c$ ist bevorzugt Wasserstoff, Niederalkyl, z.B. Aethyl, oder Niederalkansulfonyl, z.B. Methansulfonyl.

Niederalkyl $R_o$ ist beispielsweise Methyl, Aethyl, n-Propyl, Isopropyl, n-Butyl oder tert.-Butyl.

In einer Gruppe der Teilformel A, worin der Stickstoff Bestandteil eines Heterocyclus ist, stellen R und $R_o$ Alkylen dar, z.B. Aethylen, Propylen, Butylen, Pentylen oder Hexylen,welches gegebenenfalls durch Sauerstoff, Schwefel, $>NH$, oder durch Niederalkyl, z.B. Methyl, substituierten Stickstoff unterbrochen ist. Ein solcher Heterocyclus kann durch ein oder zwei Hydroxy- oder Oxogruppen an den Kohlenstoffatomen substituiert sein und ist beispielsweise 1-Aziridinyl, 1-Pyrrolidinyl, 1-Piperidyl, 1H-2,3,4,5,6,7-Hexahydroazepinyl, 4-Morpholinyl, 4-Thiomorpholinyl und bevorzugt 1-Piperazinyl oder 4-Methyl-1-piperazinyl.

Eine bevorzugte Gruppe der Teilformel A ist beispielsweise 2-Aminoäthyl, 2-Niederalkylaminoäthyl, z.B. 2-Methylaminoäthyl oder 2-n-Hexylaminoäthyl, 2-Diniederalkylaminoäthyl, z.B. 2-Dimethylaminoäthyl oder 2-Di-n-hexylaminoäthyl, 2-Sulfoaminoäthyl, Niederalkanoylaminoäthyl, z.B. 2-Formylaminoäthyl oder 2-Acetylaminoäthyl, 2-Niederalkoxyniederalkanoylaminoäthyl, z.B. 2-Methoxyacetylaminoäthyl, 2-Halogenniederalkanoylaminoäthyl, z.B. 2-Bromacetylaminoäthyl-2-(α-hydroxypropionylamino)-äthyl, 2-Glycylaminoäthyl, 2-(3-Amino-3 carboxypropionylamino)-äthyl, 2-(α-Hydroxypropionylamino)-äthyl, 2-Glycylaminoäthyl, 2-(3-Amino-3-carboxypropionylamino)-äthyl, 2-Acryloylaminoäthyl, 2-Propioloylaminoäthyl, 2-Cyclopropylcarbonylaminoäthyl, 2-Benzoylaminoäthyl, 2-(4-Aminobenzoylamino)-äthyl, 2-(4-Acetylaminobenzoylamino)-äthyl, 2-(4-Cyanobenzoylamino)-äthyl, 2-(4-Nitrobenzoylamino)-äthyl, 2-(3,4-Dinitrobenzoylamino)-äthyl, 2-Mandeloylaminoäthyl, 2-Phenylglycylaminoäthyl, 2-Nicotinoylaminoäthyl, 2-Isonicotinoylaminoäthyl, 2-(2-Furoylamino)-äthyl, 2-(2-Thienylcarbonylamino)-äthyl, 2-(2,6-Dihydroxy-1,3-pyrimid-4-ylcarbonylamino)-äthyl, 2-(4-Hydroxy-1,2,5-thiadiazol-3-ylcarbonylamino)-äthyl, 2-(2-Tetrazol-1-ylacetylamino)-äthyl, 2-[2-(2-Amino-1,4-thiazol-4-yl)-acetylamino]-äthyl, 2-Niederalkoxycarbonylaminoäthyl, z.B. 2-Methoxycarbonylaminoäthyl oder 2-Isopropoxycarbonylaminoäthyl, 2-(2-Amino-2-carboxyäthoxycarbonylamino)-äthyl, 2-Benzoyloxycarbonylaminoäthyl, 2-Niederalkylcarbamoylaminoäthyl, z.B. 2-Methylcarbamoylaminoäthyl, 2-Anilinocarbonylaminoäthyl, 2-Niederalkylthiocarbamoylaminoäthyl, z.B. 2-Methylthiocarbamoylaminoäthyl, 2-Niederalkansulfonylaminoäthyl, z.B. 2-Methansulfonylaminoäthyl, 2-Halogenmethansulfonylaminoäthyl, z.B. 2-Difluormethansulfonylaminoäthyl, 2-Cyanomethansulfonylaminoäthyl, 2-Benzolsulfonylaminoäthyl, 2-(4-Nitrobenzolsulfonylamino)-äthyl, 2-(3,4-Dinitrobenzolsulfonylamino)-äthyl, 2-Benzoylcarbamoylaminoäthyl, 2-(2-Furoylcarbamoylamino)-äthyl, 2-(2-Oxo-1-imidazolidinocarbonylamino)-äthyl, 2-(4-Aethyl-2,3-dioxo-1-piperazinocarbonylamino)-äthyl und 2-(4-Methylsulfonylpiperazinocarbonylamino)-äthyl.

Ein ungesättigter, aliphatischer Kohlenwasserstoffrest $R_5$ ist beispielsweise Niederalkenyl mit 2-5 Kohlenstoffatomen, z.B. Vinyl oder Allyl.

Ein gesättigter, cycloaliphatischer Kohlenwasserstoffrest $R_5$ ist beispielsweise Cycloalkyl mit 3-8, insbesondere 3-6, Kohlenstoffatomen, z.B. Cyclopropyl, Cyclopentyl oder Cyclohexyl.

Ein ungesättigter, cycloaliphatischer Kohlenwasserstoffrest $R_5$ ist beispielsweise Cycloalkenyl mit 5 oder 6 Kohlenstoffatomen, z.B. 1-Cyclohexenyl oder 1,4-Cyclohexadienyl.

Ein aromatischer Kohlenwasserstoffrest $R_5$ ist beispielsweise Phenyl oder Naphthyl, welches durch folgende Substituenten substituiert sein kann: Alkyl, z.B. Dodecyl oder Niederalkyl, z.B. Methyl oder Aethyl, Halogenniederalkyl, z.B. Trifluormethyl, Hydroxy, veräthertes Hydroxy, z.B. Niederalkoxy, z.B. Methoxy oder Aethoxy, verestertes Hydroxy, z.B. Niederalkanoyloxy, z.B. Acetoxy, oder Halogen, z.B. Chlor, oder Niederalkoxycarbonyloxy, z.B. Aethoxycarbonyloxy, Amino, Diniederalkylamino, z.B. Dimethylamino, Niederalkylamino, z.B. Methylamino, Niederalkanoylamino, z.B. Formylamino oder Acetylamino, Diniederalkylaminomethylenamino, z.B. Dimethylaminomethylenamino, Hydrazino, Carbazo, Thiocarbamoylhydrazino, Niederalkoxycarbonylamino, z.B. Aethoxycarbonylamino, Cyano, Nitro, Carboxyl oder verestertes Carboxyl, z.B. Methoxycarbonyl oder Aethoxycarbonyl.

Ein aromatischer Kohlenwasserstoffrest $R_5$ ist bevorzugt Phenyl, Naphthyl, 4-Alkylphenyl, z.B. 4-Dodecylphenyl, 4-Niederalkylphenyl, z.B. 4-Methylphenyl, 3-Halogenniederalkylphenyl, z.B. 3-Trifluormethylphenyl, 4-Aminophenyl, 4-Niederalkanoylaminophenyl, z.B. 4-Formylaminophenyl oder 4-Acetylaminophenyl, 4-Diniederalkylaminomethylenaminophenyl, z.B. 4-Dimethylaminomethylenaminophenyl, 4-Hydrazinophenyl, 4-Carbazophenyl, 4-Thiocarbamoylhydrazino, 4-Niederalkoxycarbonylaminophenyl, z.B. 4-Aethoxycarbonylaminophenyl, 4-Nitrophenyl, 4-Cyanophenyl, 4-Carboxyphenyl, 5-Carboxy-6-hydroxy-2-naphthyl, 6-Niederalkoxycarbonyloxy-2-naphthyl, z.B. 6-Aethoxycarbonyloxy-2-naphthyl, 5- oder 6-Alkanoylamino-1-naphthyl, z.B. 5- oder 6-Acetylamino-1-naphthyl, 6-Niederalkoxycarbonylamino-1-naphthyl, z.B. 6-Aethoxycarbonylamino-1-naphthyl, oder 4-Niederalkoxycarbonyloxy-6-niederalkoxycarbonylamino-1-naphthyl, z.B. 4-Aethoxycarbonyloxy-6-äthoxycarbonylamino-1-naphthyl.

Ein aromatisch-aliphatischer Kohlenwasserstoffrest $R_5$ ist beispielsweise einer der genannten aliphatischen Reste, z.B. Niederalkyl, z.B. Methyl oder Aethyl, der durch einen der genannten aromatischen Reste, z.B. Phenyl, substituiert ist, beispielsweise Benzyl oder Phenäthyl.

Heterocyclyl $R_5$ ist beispielsweise aromatisches oder hydriertes, monocyclisches oder benzokondensiertes Heterocyclyl, beispielsweise monocyclisches, aromatisches, fünf- oder sechsgliedriges Aza-, Thia- oder Oxacyclyl, z. B. Pyridyl, z.B. 2-, 3- oder 4-Pyridyl, Thienyl, z.B. 2- oder 3-Thienyl, oder Furyl, z.B. 2- oder 3-Furyl, monocyclisches, aromatisches fünf- oder sechsgliedriges Diazacyclyl, z.B. Imidazolyl, z.B. 2-Imidazolyl oder 5-Imidazolyl, Pyrimidyl, z.B. 4- oder 5-Pyrimidyl, monocyclisches, aromatisches, fünfgliedriges Thiadiazacyclyl, z.B. Thiadiazolyl, z.B. 1,3,4-Thiadiazol-5-yl, monocyclisches, hydriertes, fünfgliedriges Oxacyclyl, z.B. Tetrahydrofuryl, z.B. 3-Tetrahydrofuryl, benzokondensiertes Azacyclyl, z.B. Indolyl, z.B. 5-Indolyl, benzokondensiertes Diazacyclyl, z.B. Chinoxalinyl, z.B. 7-Chinoxalinyl, oder Indazolyl, z.B. 5-Indazolyl, benzokondensiertes Oxaazacyclyl, z.B. Benzooxazolyl, z.B. 5-Benzoxazolyl, oder ist benzokondensiertes Thiaazacyclyl, z.B. Benzthiazolyl, z.B. 2-, 5- oder 6-Benzthiazolyl.

Substituenten des Heterocyclylrests $R_5$ sind beispielsweise Oxo, Hydroxy, Halogen, z.B. Chlor, Niederalkyl, z.B. Methyl, Niederalkoxycarbonyl, z.B. Aethoxycarbonyl, Niederalkanoylamino, z.B. Acetylamono oder N-Niederalkylureido, z.B. N-Methylureido.

Substituiertes Heterocyclyl $R_5$ ist beispielsweise Niederalkanoylaminopyridyl, z.B. 5-Acetylaminopyrid-2-yl, Niederalkyl-niederalkanoylamino-niederalkoxycarbonyl-thienyl, z.B. 3-Methyl-4-äthoxycarbonyl-5-acetylaminothien-2-yl, Niederalkylimidazolyl, z.B. 1-Methylimidazolyl-5-yl, Dihydroxypyrimidyl, z.B. 2,4-Dihydroxypyrimid-5-yl, Niederalkanoylaminothiadiazolyl, z.B. 2-Acetylamino-1,3,4-thiadiazol-5-yl, Niederalkylureidothiadiazolyl, z.B. 2-Methylaminocarbonylamino-1,3,4-thiadiazol-5-yl, Niederalkylindolyl, z.B. 2-Methylindol-5-yl, Dihydroxychinoxalinyl, z.B. 2,3-Dihydroxychinoxalin-7-yl, Hydroxyindazolyl, z.B. 3-Hydroxyindazol-5-yl, Hydroxychlorobenzoxazolyl, z.B. 2-Hydroxy-6-chlorobenzoxazol-5-yl, oder Aminobenzthiazolyl, z.B. 2-Aminobenzthiazol-6-yl.

Heterocyclyl $R_6$ ist beispielsweise aromatisches, monocyclisches, fünf- oder sechsgliedriges Aza-, Thia-, Oxa-, Thiaza-, Diaza-, Thiadiaza-, Triaza- oder Tetraazacyclyl, z.B. Pyridyl, z.B. 3- oder 4-Pyridyl, Thienyl, z.B. 2- oder 3-Thienyl, Furyl, z.B. 2- oder 3-Furyl, Thiazolyl, z.B. 2-Thiazolyl, Isothiazolyl, z.B. 2- oder 4-Isothiazolyl, Pyrimidyl, z.B. 4- oder 5-Pyrimidyl, Thiadiazolyl, z.B. 1,2,4-Thiadiazol-3-yl, 1,2,5-Thiadiazol-3-yl, oder 1,3,4-Thiadiazol-3-yl, Triazolyl, z.B. 3-Triazolyl, oder Tetrazolyl, z.B. 1- oder 5-Tetrazolyl.

Substituenten des Heterocyclylrests $R_6$ sind die gleichen weiter vorn für die Heterocyclylthiogruppe $R_2$ genannten Substituenten.

Heterocyclyl $R_6$ ist bevorzugt Pyridyl, z.B. 3- oder 4-Pyridyl, Thienyl, z.B. 2- oder 3-Thienyl, Furyl, z.B. 2- oder 3-Furyl, Aminothiazolyl, z.B. 2-Amino-4-thiazolyl, Hydroxypyrimidyl, z.B. 2,6-Dihydroxy-1,3-pyrimid-4-yl, Aminothiadiazolyl, z.B. 5-Amino-1,2,4-thiadiazolyl-3-yl, Hydroxythiadiazolyl, z.B. 4-Hydroxy-1,2,5-thiadiazol-3-yl, oder Aminotriazolyl, z.B. 5-Amino-1,2,4-triazol-3-yl.

Ein Heterocyclyl-aliphatischer Rest $R_6$ ist beispielsweise Niederalkyl, z.B. Methyl, das durch weiter vorn genanntes Heterocyclyl $R_6$ substituiert ist, z.B. Tetrazolylniederalkyl, z.B. Tetrazol-5-ylmethyl, oder Aminothiazolylniederalkyl, z.B. 2-Amino-1,3-thiazol-4-ylmethyl.

Die in Verbindungen der Formel I vorhandenen funktionellen Gruppen, insbesondere die Carboxyl-, Amino-, Hydroxy- und Sulfogruppen, sind gegebenenfalls durch solche Schutzgruppen (conventionyl protecting groups) geschützt, die üblicherweise in der Penicillin-, Cephalosproin- und Peptidchemie verwendet werden.

Der Schutz von funktionellen Gruppen mit solchen Schutzgruppen, die Schutzgruppen selbst, sowie ihre Abspaltungsreaktionen, sind beispielsweise in "Protective Groups in Organic Chemistry", Plenum Press, London und New York 1973, in "Protective Groups in Organic Chemistry", Wiley, New York 1974, in "The Peptides", Vol. I, Schröder and Lubke, Academic Press, London und New York 1965, sowie in "Methoden der organischen Chemie", Houben-Weyl, 4. Auflage, Bd.15/I Georg Thieme Verlag, Stuttgart 1974, beschrieben.

Solche Schutzgruppen lassen sich unter schonenden Bedingungen, das heisst, dass unerwünschte Nebenreaktionen kaum oder gar nicht stattfinden, beispielsweise solvolytisch, reduktiv, photolytisch oder auch unter physiologischen Bedingungen, leicht abspalten.

Eine Carboxylgruppe, z.B. die Carboxylgruppe $R_3$, ferner eine in $R_2$, $R_5$ und $R_6$ vorhandene Carboxylgruppe, ist üblicherweise in veresterter Form geschützt, wobei die Estergruppe unter schonenden Bedingungen leicht spaltbar ist. Eine in veresterter Form geschützte Carboxylgruppe ist in erster Linie durch eine Niederalkylgruppe verestert, welche in 1-Stellung der Niederalkylgruppe verzweigt oder in 1- oder 2-Stellung der Niederalkylgruppe durch geeignete Substituenten substituiert ist.

Eine geschützte Carboxylgruppe, welche durch eine Niederalkylgruppe verestert ist, welche in 1-Stellung der Niederalkylgruppe verzweigt ist, ist beispielsweise tert.-Niederalkoxycarbonyl, z.B. tert.-Butoxycarbonyl, Arylmethoxycarbonyl mit einem oder zwei Arylresten, worin Aryl vorzugsweise unsubstituiertes oder z.B. durch Niederalkyl, z.B. tert.-Niederalkyl, z.B. tert.-Butyl, Niederalkoxy, z.B. Methoxy, Hydroxy, Halogen, z.B. Chlor, und/oder Nitro, mono-, di- oder trisubstituiertes Phenyl bedeutet, beispielsweise Benzyloxycarbonyl, durch die genannten Substituenten substituiertes Benzyloxycarbonyl, z.B. 4-Nitrobenzyloxycarbonyl oder 4-Methoxybenzyloxycarbonyl, Diphenylmethoxycarbonyl oder durch die genannten Substituenten substituiertes Diphenylmethoxycarbonyl, z.B. Di-(4-methoxyphenyl)-methoxycarbonyl.

Eine geschützte Carboxylgruppe, welche durch eine Niederalkylgruppe verestert ist, welche in 1- oder 2-Stellung der Niederalkylgruppe durch geeignete Substituenten substituiert ist, ist beispielsweise 1-Niederalkoxyniederalkoxycarbonyl, z.B. Methoxymethoxycarbonyl, 1-Methoxyäthoxycarbonyl oder 1-Aethoxymethoxycarbonyl, 1-Niederalkylthioniederalkoxycarbonyl, z.B. 1-Methylthiomethoxycarbonyl oder 1-Aethylthioäthoxycarbonyl, Aroylmethoxycarbonyl, z.B. Phenacyloxycarbonyl, sowie 2-Halogenniederalkoxycarbonyl, z.B. 2,2,2-Trichloräthoxycarbonyl, 2-Bromäthoxycarbonyl oder 2-Jodäthoxycarbonyl.

Eine Carboxylgruppe kann auch als organische Silyloxycarbonylgruppe geschützt sein. Eine organische Silyloxycarbonylgruppe ist beispielsweise eine Triniederalkylsilyloxycarbonylgruppe, z.B. Trimethylsilyloxycarbonyl. Das Siliciumatom der Silyloxycarbonylgruppe kann auch durch zwei Niederalkylgruppen, z.B. Methylgruppen, und die Carboxylgruppe oder Aminogrippe eines zweiten Moleküls der Formel I substituiert sein. Verbindungen mit solchen Schutzgruppen lassen sich z.B. bei Verwendung von Dimethyldichlorsilan als Silylierungsmittel herstellen.

Eine geschützte Carboxylgruppe ist bevorzugt tert.-Niederalkoxycarbonyl, z.B. tert.-Butoxycarbonyl, Benzyloxycarbonyl, 4-Nitrobenzyloxycarbonyl und Diphenylmethoxycarbonyl.

Eine Aminogruppe, z.B. eine in $R_2$, $R_5$ und $R_6$ vorhandene Aminogruppe, kann z.B. in Form einer leicht spaltbaren Acylamino-, Arylmethylamino-, verätherten Mercaptoamino-, 1-Acylniederalk-1-en-2-ylamino- oder Silylaminogruppe geschützt sein.

In einer leicht spaltbaren Acylaminogruppe ist Acyl beispielsweise die Acylgruppe von einer organischen Carbonsäure mit bis zu 10 Kohlenstoffatomen, insbesondere einer unsubstituierten oder einer, z.B. durch Halogen oder Aryl, substituierten Niederalkancarbonsäure oder der unsubstituierten oder, z.B. durch Halogen, Niederalkoxy oder Nitro, substituierten Benzoesäure oder eines Kohlensäurehalbesters. Eine solche Acylgruppe ist beispielsweise Niederalkanoyl, z.B. Formyl, Acetyl oder Propionyl, Halogenniederalkanoyl, z.B. 2-Halogenacetyl, insbesondere 2-Chlor-, 2-Brom-, 2-Jod-, 2,2,2-Trifluor- oder 2,2,2-Trichloracetyl, Benzoyl oder z.B. durch Halogen, z.B. Chlor, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung durch geeignete Substituenten substituiertes Niederalkoxycarbonyl.

In 1-Stellung des Niederalkylrests verzweigtes Niederalkoxycarbonyl ist beispielsweise tert.-Niederalkoxycarbonyl, z.B. tert.-Butoxycarbonyl, Arylmethoxycarbonyl mit einem oder zwei Arylresten, worin Aryl vorzugsweise unsubstituiertes oder z.B. durch Niederalkyl, insbesondere tert.-Niederalkyl, z.B. tert.-Butyl, Niederalkoxy, z.B. Methoxy, Hydroxy, Halogen, z.B. Chlor, und/oder Nitro, mono-, di- oder trisubstituiertes Phenyl bedeutet, beispielsweise Diphenylmethoxycarbonyl oder Di-(4-methoxyphenyl)-methoxycarbonyl.

In 1- oder 2-Stellung durch geeignete Substituenten substituiertes Niederalkoxycarbonyl ist beispielsweise Aroylmethoxycarbonyl, z.B. Phenacyloxycarbonyl, 2-Halogenniederalkoxycarbonyl, z.B. 2,2,2-Trichloräthoxycarbonyl, 2-Bromäthoxycarbonyl oder 2-Jodäthoxycarbonyl, oder 2-(trisubstituiertes Silyl)-äthoxycarbonyl, worin die Silylgruppe durch organische Reste, z.B. Niederalkyl, Phenylniederalkyl oder Phenyl substituiert ist, beispielsweise 2-Triniederalkylsilyläthoxycarbonyl, z.B. 2-Trimethylsilyläthoxycarbonyl oder 2-(Di-n-butylmethylsilyl)-äthoxycarbonyl, oder 2-Triphenylsilyläthoxycarbonyl.

Arylmethylamino ist beispielsweise Mono-, Di- oder insbesondere Triphenylmethylamino. Arylmethylamino ist beispielsweise Benzyl-, Diphenylmethyl- und insbesondere Tritylamino.

In einer verätherten Mercaptoaminogruppe ist die verätherte Mercaptogruppe in erster Linie Arylthio, z.B. 4-Nitrophenylthio.

In einer 1-Acylniederalk-1-en-2-aminogruppe ist Acyl z.B. die Acylgruppe einer Niederalkancarbonsäure oder eines Kohlensäureniederalkylhalbesters. Solche Aminoschutzgruppen sind in erster Linie 1-Niederalkanoylprop-1-en-2-yl, z.B. 1-Acetylprop-1-en-2-yl, oder 1-Niederalkoxycarbonylprop-1-en-2-yl, z.B. 1-Aethoxycarbonylprop-1-en-2-yl.

Eine Silylaminogruppe ist beispielsweise eine Triniederalkylsilylaminogruppe, z.B. Trimethylsilylamino. Das Siliciumatom der Silylaminogruppe kann auch nur durch zwei Niederalkylgruppen, z.B. Methylgruppen, und die Aminogruppe oder Carboxylgruppe eines zweiten Moleküls der Formel I substituiert sein. Verbindungen mit solchen Schutzgruppen lassen sich z.B. bei Verwendung von Dimethyldichlorsilan als Silylierungsmittel herstellen.

Eine Aminogruppe kann auch in protonierter Form geschützt sein. Als Anionen sind in erster Linie die Anionen von starken anorganischen Säuren, wie von Halogenwasserstoffsäuren, z.B. das Chlor- oder Bromanion, oder von organischen Sulfonsäuren, wie p-Toluolsulfonsäure, geeignet.

Eine geschützte Aminogruppe ist bevorzugt tert.-Butoxycarbonylamino (BOC), 4-Nitrobenzyloxycarbonylamino, Diphenylmethoxycarbonylamino, 2-Halogenniederalkoxycarbonylamino, z.B. 2,2,2-Trichloräthoxycarbonylamino, Tritylamino und Formylamino-

Eine Hydroxygruppe, z.B. eine in $R_2$, $R_5$ und $R_6$ vorhandene Hydroxygruppe, kann beispielsweise durch eine Acylgruppe, z.B. durch Halogen substituiertes Niederalkanoyl, z.B. 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2,2,2-Trichloräthoxycarbonyl, 4-Nitrobenzyloxycarbonyl, ein organischer Silylrest mit den weiter vorn genannten Substituenten, z.B. Trimethylsilyl oder Dimethyl-n-butylsilyl, ferner eine leicht abspaltbare, veräthernde Gruppe, wie tert.-Niederalkyl, z.B. tert.-Butyl, ein 2-Oxa- oder ein 2-thiaaliphatischer oder -cycloaliphatischer Kohlenwasserstoffrest, beispielsweise 1-Niederalkoxyniederalkyl oder 1-Niederalkylthioniederalkyl, z.B. Methoxymethyl, 1-Methoxyäthyl, 1-Aethoxyäthyl, 1-Methylthiomethyl, 1-Methylthipäthyl oder 1-Aethylthioäthyl, oder 2-Oxa- oder 2-Thiacycloalkyl mit 5-7 Ringatomen, z.B. 2-Tetrahydrofuryl oder 2-Tetrahydropyranyl, oder ein entsprechendes Thiaanaloges, sowie 1-Phenylniederalkyl, z.B. Benzyl oder Diphenylmethyl, wobei die Phenylreste beispielsweise durch Halogen, z.B. Chlor, Niederalkoxy, z.B. Methoxy, und/oder Nitro substituiert sein können.

Eine Sulfogruppe, z.B. eine in $R_2$, $R_5$ und $R_6$ vorhandene Sulfogruppe, ist vorzugsweise durch eine tert.-Niederalkylgruppe, z.B. tert.-Butyl,oder durch eine Silylgruppe, z.B. durch Triniederalkylsilyl,

geschützt. Eine Sulfogruppe kann z.B. durch Carboxyschutzgruppen geschützt sein.

Salze sind in erster Linie die pharmazeutisch annehmbaren oder verwendbaren, nicht-toxischen Salze von Verbindungen der Formel I.

Solche Salze werden beispielsweise von den in Verbindungen der Formel I vorhandenen sauren Gruppen, z.B. Carboxy- oder Sulfogruppen gebildet und sind in erster Linie Metall- oder Ammoniumsalze, beispielsweise Alkalimetall- und Erdalkalimetall-, z.B. Natrium-, Kalium-, Magnesium- oder Calciumsalze, sowie Ammoniumsalze, welche mit Ammoniak oder geeigneten organischen Aminen gebildet werden, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen. Solche Basen sind beispielsweise Niederalkylamine, z.B. Triäthylamin, Hydroxyniederalkylamine, z.B. 2-Hydroxyäthylamin, Bis-(2-hydroxyäthyl)-amin oder Tris-(2-hydroxyäthyl)-amin, basische aliphatische Ester von Carbonsäuren, z.B. 4-Aminobenzoesäure-2-diäthyldiaminoäthylester, Niederalkylenamine, z.B. 1-Aethylpiperidin, Cycloalkylamine, z.B. Dicyclohexylamin, oder Benzylamine, z.B. N,N'-Dibenzyläthylendiamin, ferner Basen vom Pyridintyp, z.B. Pyridin, Collidin oder Chinolin.

Die in Verbindungen der Formel I vorhandenen basischen Gruppen, z.B. Aminogruppen, können Säureadditionssalze, z.B. mit anorganischen Säuren, wie Salzsäure, Schwefelsäure oder Phosphorsäure, oder mit geeigneten organischen Carbon- oder Sulfonsäuren, z.B. Trifluoressigsäure, sowie mit Aminosäuren, wie Arginin und Lysin, bilden.

Liegen in Verbindungen der Formel I mehrere saure Gruppen, z.B. zwei Carboxylgruppen, oder mehrere basische Gruppen, z.B. zwei Aminogruppen, vor, können Mono- oder Polysalze gebildet werden. Wenn die

Verbindungen der Formel I mindestens eine saure Gruppe, z.B. die Carboxylgruppe $R_3$, und mindestens eine basische Gruppe, z.B. eine Aminogruppe in $R_5$ oder $R_6$ besitzen, können diese in Form von inneren Salzen, d.h. in zwitterionischer Form, vorliegen. In Verbindungen der Formel I können eine saure und eine basische Gruppe in Form eines inneren Salzes und zusätzlich saure und/oder basiche Gruppen beispielsweise als Säureadditions- und/oder Baseadditionssalz vorliegen.

Zur Isolierung oder Reinigung können auch pharmazeutisch ungeeignete Salze Verwendung finden. Zur therapeutischen Anwendung gelangen nur pharmazeutisch verwendbare, nicht toxische Salze, die deshalb bevorzugt sind.

Die Verbindungen der Formel I, worin die funktionellen Gruppen in freier Form und die Carboxylgruppen gegebenenfalls in physiologisch spaltbarer veresterter Form vorliegen, und ihre pharmazeutisch verwendbaren, nichttoxischen Salze sind wertvolle, antibiotische Wirkstoffe, die insbesondere als antibakterielle Antibiotika verwendet werden können.

Beispielsweise sind sie in vitro gegen grampositive und gramnegative Mikroorganismen, inklusive β-Lactamase produzierende Stämme, z.B. gegen Kokken wie Staphylococcus aureus, Streptococcus pneumoniae, Streptococcus pyogenes und Neisseria gonorrhoeae in Minimalkonzentrationen von ca. 0,001 bis ca. 32 µg/ml, gegen Enterobakterien, z.B. gegen Escherichia coli, Salmonella typhimurium, Klebsiella pneumoniae, Proteus spp., Enterobacter cloacae, Serratia marcescens, Haemophilus influenzae und Pseudomonas aeruginosa, und gegen anaerobe grampositive und gramnegative Bakterien, z.B. Bacteroides fragilis oder Clostridium perfringens, in Minimalkonzentrationen von ca. 0,001 bis ca. 64 µg/ml wirksam. In vivo, bei subkutaner Applikation an der Maus, sind sie beispielsweise gegen systemische Infektionen mit Kokken, z.B.

Staphylococcus aureus, in einem Dosisbereich von ca. 3 mg/kg bis ca. 100 mg/kg und gegen systemische Infektionen mit Enterobakterien, z.B. Escherichia coli, Proteus morganii oder mit Pseudomonas aeruginosa, in einem Dosisbereich von ca. 0,1 mg/kg bis ca. 100 mg/kg wirksam.

Im folgenden Versuchsbericht wird anhand von ausgewählten Verbindungen die Wirksamkeit von Verbindungen der Formel I gezeigt.

## Versuchsbericht

### I. Getestete Verbindungen:

Für die folgenden Verbindungen wurde die antibiotische Wirksamkeit getestet:

1. 3-Methoxy-7β-[(2R,S)-2-(2-aminoäthansulfonylamino)-2-(2-aminothiazol-4-yl)-acetamido]-3-cephem-4-carbonsäure-natriumsalz (Beispiel 16a).

2. 3-Acetoxymethyl-7β-[(2R,S)-2-(2-aminoäthansulfonylamino)-2-(2-aminothiazol-4-yl)-acetamido]-3-cephem-4-carbonsäure-natriumsalz (Beispiel 13a).

3. 7β-[(2R,S)-2-(2-Aminoäthansulfonylamino)-2-(2-aminothiazol-4-yl)-acetamido]-3-cephem-4-carbonsäure-natriumsalz (Beispiel 14a).

4. 3-(1-Methyl-1H-tetrazol-5-ylthiomethyl)-7β-[(2R,S)-2-(2-aminoäthansulfonylamino)-2-(2-aminothiazol-4-yl)-acetamido]-3-cephem-4-carbonsäure-natriumsalz (Beispiel 15a).

5. 3-(1-Carboxymethyl-1H-tetrazol-5-ylthiomethyl)-7β-[(2R,S)-2-(2-aminoäthansulfonylamino)-2-(2-aminothiazol-4-yl)-acetamido]-3-cephem-4-carbonsäure-natriumsalz (Beispiel 18).

6. 3-(1-Sulfomethyl-1H-tetrazol-5-ylthiomethyl)-7β-[(2R,S)-2-(2-aminoäthansulfonylamino)-2-(2-aminothiazol-4-yl)-acetamido]-3-cephem-4-carbonsäurematriumsalz (Beispiel 19).

7. 7β-[(2R,S)-2-(2-Methansulfonylaminoäthansulfonylamino)-2-(2-aminothiazol-4-yl)-acetamido]-3-cephem-4-carbonsäurenatriumsalz (Beispiel 21a).

8. 2-(1-Methyl-1H-tetrazol-5-ylthiomethyl)-7β-[(2R,S)-2-(2-aminothiazol-4-yl)-2-methansulfonylaminoacetamido]-3-cephem-4-carbonsäurenatriumsalz (Beispiel 22a).

9. 3-(4-Carbamoylpyridiniomethyl)-7β-[(2R,S)-2-(2-aminothiazol-4-yl)-2-methansulfonylaminoacetamido]-3-cephem-4-carbonsäure (Beispiel 23a).

10. 3-Acetoxymethyl-7β-[(2R,S)-2-(2-aminothiazol-4-yl)-2-(2-acetylaminoäthansulfonylamino)-acetamido]-3-cephem-4-carbonsäurenatriumsalz (Beispiel 25a).

11. 7β-[(2R,S)-2-(2-Aminothiazol-4-yl)-2-(2-formylaminoäthansulfonylamino)-acetamido]-3-cephem-4-carbonsäurenatriumsalz (Beispiel 27 a).

12. 3-(1-Methyl-1H-tetrazol-5-ylthiomethyl)-7β-[(2R)-2-(2-aminothiazol-4-yl)-2-formylaminoäthansulfonylamino)-acetamido]-3-cephem-4-carbonsäurenatriumsalz (Beispiel 28a)

13. 3-(1-Methyl-1H-tetrazol-5-ylthiomethyl)-7β-[(2S)-2-(2-aminothiazol-4-yl)-2-(2-formylaminoäthansulfonylamino)-acetamido]-3-cephem-4-carbonsäurenatriumsalz (Beispiel 28b).

14. 7β-[(2R,S)-2-(2-(2-Aminothiazol-4-ylacetamido)-äthansulfonylamino)-2-(2-aminothiazol-4-yl)-acetamido]-3-cephem-4-carbonsäurenatriumsalz (Beispiel 29a).

15. 7β-[(2R,S)-2-(2-Aminothiazol-4-yl)-2-(2-acryloylaminoäthansulfonylamino)-acetamido]-3-cephem-4-carbonsäurenatriumsalz (Beispiel 31a).

16. 7β-[(2R,S)-2-(2-Aminothiazol-4-yl)-2-(2-methoxyacetylaminoäthansulfonylamino)-acetamido]-3-cephem-4-carbonsäurenatriumsalz (Beispiel 34a).

17. 7β-[(2R,S)-2-(2-(4-Nitrobenzolsulfonylamino)-äthansulfonylamino)-2-(2-aminothiazol-4-yl)-acetamido]-3-cephem-4-carbonsäurenatriumsalz (Beispiel 38a).

18. 7β-[(2R,S)-2-(2-Methylaminoäthansulfonylamino)-2-(2-aminothiazol-4-yl)-acetamido]-3-cephem-4-carbonsäure (Beispiel 42a).

19. 7β-[(2R,S)-2-(2-Methoxyäthansulfonylamino)-2-(2-aminothiazol-4-yl)-acetamido]-3-cephem-4-carbonsäure (Beispiel 43a).

20. 7β-[(2R,S)-2-(2-Aminothiazol-4-yl)-2-cyanomethansulfonylamino-acetamido]-3-cephem-4-carbonsäurenatriumsalz (Beispiel 44a).

21. 7β-[(2R,S)-2-(2-Aminothiazol-4-yl)-2-(2-methoxymalonylaminoäthansulfonylamino)-acetamido]-3-cephem-4-carbonsäurenatriumsalz (Beispiel 50a).

22. 7β-[(2R,S)-2-(2-Aminothiazol-4-yl)-2-(2-bromacetylaminoäthansulfonylamino)-acetamido]-3-cephem-4-carbonsäurenatriumsalz (Beispiel 51a).

23. 7β-[(2R,S)-2-(2-Aminothiazol-4-yl)-2-(2-(4-nitrobenzoylamino)-äthansulfonylamino)-acetamido]-3-cephem-4-carbonsäurenatriumsalz (Beispiel 55a).

24. 7β-[(2R,S)-2-(2-Aminothiazol-4-yl)-2-(2-(4-acetamidobenzolsulfonylamino)-äthansulfonylamino)-acetamido]-3-cephem-4-carbonsäurenatriumsalz (Beispiel 56a).

25. 7β-[(2R,S)-2-(2-Aminothiazol-4-yl)-2-(2-(4-äthyl-2,3-dioxopiperazin-1-ylcarbonylamino)-äthansulfonylamino)-acetamido]-3-cephem-4-carbonsäurenatriumsalz (Beispiel 58a).

26. 7β-[(2R,S)-2-(2-Aminothiazol-4-yl)-2-(2-acetylaminoäthansulfonylamino)-acetamido]-3-cephem-4-carbonsäurenatriumsalz (Beispiel 24a).

27. 7β-[(2R,S)-2-(2-Aminothiazol-4-yl)-2-(2-butyrylamino)-äthansulfonylamino)-acetamido]-3-cephem-4-carbonsäure (Beispiel 30a).

28. 7β-[(2S)-2-(2-Aminothiazol-4-yl)-2-(2-cyclopropylcarbonylaminoäthansulfonylamino)-acetamido]-3-cephem-4-carbonsäurenatriumsalz (Beispiel 32a).

29. 7β-[(2R,S)-2-(2-Aminothiazol-4-yl)-2-(2-propioloylaminoäthansulfonylamino)-acetamido]-3-cephem-4-carbonsäurenatriumsalz (Beispiel 35a).

30. 7β-[(2S)-2-(2-Aminothiazol-4-yl)-2-methansulfonylaminoacetamido]-3-cephem-4-carbonsäurenatriumsalz (Beispiel 8a).

31. 7β-[(2R,S)-2-(2-Aminothiazol-4-yl)-2-(2-cyanacetylaminoäthansulfonylamino)-acetamido]-3-cephem-4-carbonsäurenatriumsalz (Beispiel 33a).

32. 3-Acetoxymethyl-7β-[(2R,S)-2-(2-(4-nitrobenzolsulfonylamino)-äthansulfonylamino)-2-(2-aminothiazol-4-yl)-acetamido]-3-cephem-4-carbonsäurenatriumsalz (Beispiel 39a).

33. 7β-[(2R,S)-2-(2-(2,4-Dinitrobenzolsulfonylamino)-äthansulfonylamino)-2-(2-aminothiazol-4-yl)-acetamido]-3-cephem-4-carbonsäurenatriumsalz (Beispiel 40a).

34. 7β-[(2R,S)-2-(2-Aminothiazol-4-yl)-2-(5-imidazolsulfonylamino)-acetamido]-3-cephem-4-carbonsäurenatriumsalz (Beispiel 65a).

35. 3-Carbamoyloxymethyl-7β-[(2R,S)-2-(2-aminothiazol-4-yl)-2-(2-(4-nitrobenzolsulfonylamino)-äthansulfonylamino)-acetamido]-3-cephem-4-carbonsäurenatriumsalz (Beispiel 67a).

36. 7β-[(2R,S)-2-(2-Aminothiazol-4-yl)-2-(2-(4-aminobenzolsulfonylamino)-äthansulfonylamino)-acetamido]-3-cephem-4-carbonsäurenatriumsalz (Beispiel 68a).

37. 3-(1-Methyl-1H-tetrazol-5-ylthiomethyl)-7β-[(2R,S)-2-(2-aminothiazol-4-yl)-2-cyanomethansulfonylaminoacetamido]-3-cephem-4-carbonsäurenatriumsalz (Beispiel 71a).

38. 3-Carbamoyloxymethyl-7β-[(2R,S)-2-(2-aminothiazol-4-yl)-2-(2-formylaminoäthansulfonylamino)-acetamido]-3-cephem-4-carbonsäurediphenylmethylester (Beispiel 72a).

39. 3-Acetoxymethyl-7β-[(2R,S)-2-(2-aminothiazol-4-yl)-2-(2-formylaminoäthansulfonylamino)-acetamido]-3-cephem-4-carbonsäurenatriumsalz (Beispiel 73a).

40. 3-Carbamoyloxymethyl-7β-[(2S)-2-(2-aminothiazol-4-yl)-2-methansulfonylaminoacetamido]-3-cephem-4-carbonsäurenatriumsalz (Beispiel 75a).

41. 3-(1-Methyl-1H-tetrazol-5-ylthiomethyl)-7β-[(2R,S)-2-(2-aminothiazol-4-yl)-2-(2-acryloylaminoäthansulfonylamino)-acetamido]-3-cephem-4-carbonsäurenatriumsalz (Beispiel 85 a).

42. 3-Carbamoyloxymethyl-7β-[(2S)-2-(2-aminothiazol-4-yl)-2-(2-acryloylaminoäthansulfonylamino)-acetamido]-3-cephem-4-carbonsäurenatriumsalz (Beispiel 87b).

43. 3-(1-Methyl-1H-tetrazol-5-ylthiomethyl)-7β-[(2R,S)-2-(2-aminothiazol-4-yl)-2-(2-acetylaminoäthansulfonylamino)-acetamido]-3-cephem-4-carbonsäurenatriumsalz (Beispiel 90a).

44. 3-(1-Sulfomethyl-1H-tetrazol-5-ylthiomethyl)-7β-[(2R,S)-2-(2-aminothiazol-4-yl)-2-methansulfonylaminoacetamido]-3-cephem-4-carbonsäuredinatriumsalz (Beispiel 95).

45. 3-Carbamoyloxymethyl-7β-[(2R,S)-2-(2-aminothiazol-4-yl)-2-(2-methansulfonylamino)-äthansulfonylamino)-acetamido]-3-cephem-4-carbonsäurenatriumsalz (Beispiel 103a).

46. 3-Carbamoyloxymethyl-7β-[(2S)-2-(2-aminothiazol-4-yl)-2-(2-(4-aminobenzolsulfonylamino)-äthansulfonylamino)-acetamido]-3-cephem-4-carbonsäurenatriumsalz (Beispiel 105a).

47. 3-Acetoxymethyl-7β-[(2R,S)-2-(2-aminothiazol-4-yl)-2-(2-(4-aminobenzolsulfonylamino)-äthansulfonylamino)-acetamido]-3-cephem-4-carbonsäurenatriumsalz (Beispiel 106).

## II. Experimentelles:

A. Die antibiotische Aktivität der Testverbindungen in vitro wurde durch die Agarverdünnungsmethode nach Ericsson, H.M., und Sherris, S.C., 1971, Acta Path. Microb. Scand. Section B, Suppl. No. 217, Bd. 1-90, in DST Agar ermittelt. Die gefundenen, das Wachstum der Testorganismen noch hemmenden Minimalkonzentrationen (MIC = minimum inhibitory concentration) werden in Mikrogramm pro Mililiter (μg/ml) für die geprüften Verbindungen in der Tabelle 1 angegeben.

B. Die chemotherapeutische Wirksamkeit in vivo gegen systemische Infektionen in weiblichen SPF, $MF_2$ Mäusen wurde nach der Methode von Zak, O. et al., 1979, Drugs Exptl. Clin. Res. 5, 45-59, ermittelt. Die gefundenen $ED_{50}$-Werte in Milligramm Substanz pro Kilogramm Maus (mg/kg) für eine Anzahl von Mikroorganismen werden für die oral (p.o.) oder subcutan (s.c.) applizierten Testverbindungen in der Tabelle 2 angegeben.

III. Versuchsergebnisse:

Tabelle 1: Antibiotische Aktivität (in vitro)

| Testverbindung | MIC [µg/ml] | | | |
|---|---|---|---|---|
| | Escherichia coli 205 | Klebsiella pneumoniae 327 | Salmonella typhimurium 277 | Neisseria gonorrhoeae 1317/4 |
| 1 | 0,5 | 0,5 | 0,5 | 0,1 |
| 2 | 0,02 | 0,02 | 0,02 | 0,005 |
| 3 | 0,02 | 0,1 | 0,02 | 0,02 |
| 4 | 0,02 | 0,1 | 0,02 | 0,005 |
| 5 | 0,1 | 0,1 | 0,1 | 0,001 |
| 6 | 0,1 | 0,1 | 0,05 | 0,005 |
| 7 | 0,02 | 0,02 | 0,02 | 0,02 |
| 8 | 0,01 | 0,01 | 0,01 | 0,001 |
| 9 | 0,1 | 0,2 | 0,1 | 0,001 |
| 10 | 0,05 | 0,05 | 0,05 | n.g. |
| 11 | 0,01 | 0,01 | 0,01 | 0,005 |
| 12 | 0,05 | 0,05 | 0,05 | 0,002 |

Tabelle 1: (Fortsetzung)

| Testverbindung | MIC [µg/ml] | | | |
|---|---|---|---|---|
| | Escherichia coli 205 | Klebsiella pneumoniae 327 | Salmonella typhimurium 277 | Neisseria gonorrhoeae 1317/4 |
| 13 | 0,005 | 0,01 | 0,01 | 0,001 |
| 14 | 0,02 | 0,05 | 0,02 | 0,001 |
| 15 | 0,01 | 0,05 | 0,01 | 0,01 |
| 16 | 0,05 | 0,05 | 0,02 | 0,01 |
| 17 | 0,5 | 0,5 | 0,2 | 0,002 |
| 18 | 0,01 | 0,01 | 0,01 | 0,01 |
| 19 | 0,05 | 0,05 | 0,02 | n.g. |
| 20 | 0,02 | 0,05 | 0,01 | 0,01 |
| 21 | 0,05 | 0,05 | 0,02 | 0,01 |
| 22 | 0,05 | 0,05 | 0,05 | 0,02 |
| 23 | 1,0 | 1,0 | 1,0 | 0,05 |
| 24 | 0,05 | 0,2 | 0,1 | n.g. |
| 25 | 0,05 | 0,1 | 0,05 | 0,001 |
| 26 | 0,01 | 0,02 | 0,01 | n.g. |
| 27 | 0,05 | 0,05 | 0,05 | n.g. |
| 28 | 0,05 | 0,05 | 0,02 | n.g. |
| 29 | 0,05 | 0,05 | 0,05 | 0,01 |
| 30 | 0,02 | 0,02 | 0,01 | 0,005 |
| 31 | 0,01 | 0,01 | 0,01 | n.g. |
| 32 | 1 | 1 | 1 | 0,01 |
| 33 | 0,02 | 0,05 | 0,02 | 0,02 |
| 34 | 0,05 | 0,2 | 0,05 | n.g. |
| 35 | 0,5 | 1 | 0,5 | 0,01 |
| 36 | 0,05 | 0,1 | 0,05 | 0,01 |
| 37 | 0,02 | 0,05 | 0,05 | 0,002 |
| 38 | 0,01 | 0,02 | 0,01 | 0,002 |
| 39 | 0,01 | 0,02 | 0,01 | 0,002 |
| 40 | 0,005 | 0,01 | 0,005 | 0,001 |

Tabelle 1: (Fortsetzung)

| Testverbindung | MIC [µg/ml] | | | |
|---|---|---|---|---|
| | Escherichia coli 205 | Klebsiella pneumoniae 327 | Salmonella typhimurium 277 | Neisseria gonorrhoeae 1317/4 |
| 41 | 0,05 | 0,05 | 0,05 | 0,002 |
| 42 | 0,01 | 0,01 | 0,01 | 0,002 |
| 43 | 0,01 | 0,05 | 0,05 | 0,002 |
| 44 | 0,1 | 0,05 | 0,1 | 0,001 |
| 45 | 0,02 | 0,02 | 0.01 | 0,002 |
| 46 | 0,01 | 0,05 | 0,02 | 0,002 |
| 47 | 0,05 | 0,1 | 0,05 | 0,005 |

(n.g. = nicht geprüft)

Tabelle 2: Chemotherapeutische Wirksamkeit (in vivo)

| Testverbindung | $ED_{50}$ [mg/kg, s.c./p.o.] oder [mg/kg, s.c.] | |
|---|---|---|
| | Staphylococcus aureus 10 B | Escherichia coli 205 |
| 1 | >30 | 25/80 |
| 2 | 18 | 0,3/4,0 |
| 3 | 18 | 0,9/2,2 |
| 4 | 20 | 0,35/5,5 |
| 5 | >30 | 0,2/3,0 |
| 6 | >30 | <1 |
| 7 | 30 | 0,9/3 |
| 8 | 13 | 1,5/6 |

Tabelle 2: Fortsetzung

| Testverbindung | $ED_{50}$ [mg/kg, s.c./p.o.] oder [mg/kg, s.c.] | |
|---|---|---|
| | Staphylococcus aureus 10 B | Escherichia coli 205 |
| 9 | 7 | <1/10 |
| 10 | n.g. | <1,0/6 |
| 11 | >30 | 0,35 /1,6 |
| 12 | 15 | 0,87/7 |
| 13 | 14 | 0,35 /3,5 |
| 14 | 30 | 1,8 /10 |
| 15 | 85 | 2,0 /7 |
| 16 | 10 | 1/4,8 |
| 17 | 43 | 18/ >30 |
| 18 | 78 | 0,45/2 |
| 19 | >30 | 6,0 /10 |
| 20 | >30 | 0,8 /8 |
| 21 | >100 | 2,0 /9 |
| 23 | 55 | 25 |
| 24 | 90 | 2,5 |
| 25 | >100 | 1,0 |
| 26 | > 30 | 0,5/3 |
| 27 | > 30 | 7/25 |
| 28 | 60 | 2,2 |
| 29 | 44 | <1 |
| 30 | 30 | 0,6/2,3 |
| 31 | 22 | 1,5/8 |
| 32 | 10 | 3 |
| 33 | 20 | <1 |
| 34 | >30 | 10 |
| 35 | 10 | 2 |
| 36 | 30 | n.g. |
| 37 | 15 | 2/13 |
| 38 | 15 | 0,9/2,8 |

Tabelle 2: (Forsetzung)

| Testverbindung | $ED_{50}$ [mg/kg, s.c./p.o.] oder [mg/kg, s.c.] | |
|---|---|---|
| | Staphylococcus aureus 10 B | Escherichia coli 205 |
| 39 | 10 | 0,8/5,5 |
| 40 | 3 | 0,1/0,9 |
| 41 | 24 | 2/10 |
| 42 | 15 | 0,1/2,5 |
| 43 | 50 | 1/8 |
| 44 | 85 | $<1$ |
| 45 | 55/90 | n.g. |
| 46 | 9 | $<1/14$ |
| 47 | 5/100 | 2,8/$>$30 |

(n.g.: nicht geprüft)

Verbindungen der Formel I, worin die funktionellen Gruppen geschützt sind, werden als Ausgangsmaterialien zur Herstellung von Verbindungen der Formel I verwendet, worin funktionelle Gruppen in freier Form oder in physiologisch spaltbarer Form voliegen.

Die vorliegende Erfindung betrifft bevorzugt solche Verbindungen der Formel I, worin funktionelle Gruppen in freier Form oder in physiologisch spaltbarer geschützter Form vorliegen, und deren pharmazeutisch annehmbare Salze, da hauptsächlich diese Verbindungen die angegebene Wirksamkeit besitzen und für den angegebenen Zweck verwendet werden können.

Die Erfindung betrifft insbesondere Verbindungen der Formel I, worin m eine ganze Zahl von 0 bis 2, $R_1$ Wasserstoff, Niederalkyl, z.B. Methyl, Niederalkoxy, z.B. Methoxy oder Aethoxy, Halogen, z.B. Chlor, oder eine Gruppe der Formel $-CH_2-R_2$, worin $R_2$ Niederalkanoyloxy, z.B. Aceoxy, Carbamoyloxy, Niederalkylcarbamoyloxy, aromatisches. monocyclisches, fünf- oder sechsgliedriges Heterocyclylthio, z.B. Diaza-, Triaza-, Tetraaza-, Thiaza, Thiadiaza-, Oxaza- oder Oxadiazacyclylthio, z.B. Imidazolylthio, Triazolylthio, z.B. 1H-1,2,3-Triazol-5-ylthio, Tetrazolylthio, z.B. 1H-Tetrazol-5-ylthio, Thiazolylthio, Thiadiazolylthio, z.B. 1,3,4-Thiadiazol-5-ylthio, Oxazolylthio, Oxadiazolylthio oder 5,6-Dioxotetrahydro-as-triazin-3-ylthio, z.B. 5,6-Dioxo-1,2,5,6-tetrahydro-as-triazin-3-ylthio oder 5,6-Dioxo-1,4,5,6-tetrahydro-as-triazin-3-ylthio, welche durch Niederalkyl, z.B. Methyl, Diniederalkylaminoniederalkyl, z.B. Dimethylaminomethyl oder 2-Dimethylaminoäthyl, Sulfoniederalkyl, z.B. Sulfomethyl oder Sulfoäthyl, Carboxyniederalkyl, z.B. Carboxymethyl, Amino, Carboxyniederalkylamino, z.B. 2-Carboxyäthylamino, Carbamoyl oder durch Tetrazolylniederalkyl, z.B. Tetrazol-1H-5-ylmethyl, substituiert sein können, oder eine Ammoniogruppe, z.B. 2-Niederalkyl-1-pyrazolio, z.B. 2-Methyl-1-pyrazolio, 2-Carboxyniederalkyl-1-pyrazolio, z.B. 2-Carboxymethyl-1-pyrazolio, 3-Niederalkyl-1-triazolio, z.B. 3-Methyl-1-triazolio, Pyridinio, durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinio, z.B. 3- oder 4-Hydroxymethylpyridinio, 4-Carboxypyridinio, 3- oder 4-Carboxymethylpyridinio, 3- oder 4-Chlorpyridinio, 3- oder 4-Brompyridinio oder 3- oder 4-Carbamoylpyridinio, $R_3$ Carboxy oder unter physiologischen Bedingungen spaltbares Carboxy, z.B. Acyloxyniederalkoxycarbonyl, z.B. Niederalkanoyloxyniederalkoxycarbonyl, z.B. Niederalkanoyloxymethoxycarbonyl oder Niederalkanoyloxyäthoxycarbonyl, z.B. Pivaloyloxymethoxycarbonyl oder 2-Propionyloxyäthoxycarbonyl oder Niederalkoxycarbonyloxyniederalkoxycarbonyl, z.B. 1-Aethoxycarbonyloxyäthoxycarbonyl oder tert.-Butyloxycarbonyloxymethoxycarbonyl, $R_4$ Wasserstoff,

$R_5$ Niederalkyl, z.B. Methyl oder Aethyl, Hydroxyniederalkyl, z.B. Hydroxymethyl oder 2-Hydroxyäthyl, Niederalkoxyniederalkyl, z.B. Methoxymethyl, 2-Methoxyäthyl oder 2-Aethoxyäthyl, Niederalkenyloxyniederalkyl, z.B. 2-Vinyloxyäthyl, Niederalkanoyloxyniederalkyl, z.B. 2-Acetoxyäthyl, Halogenniederalkyl, z.B. Chloromethyl, 2-Chloroäthyl, 3-Chloropropyl, 4-Chlorobutyl oder 2-Bromäthyl, Niederalkylthioniederalkyl, z.B. 2-Methylthioäthyl oder 2-Aethylthioäthyl, Aminocarboxyniederalkylthioniederalkyl, z.B. 2-(2-Amino-2-carboxyäthylthio)-äthyl, Benzoylniederalkyl, z.B. Benzoylmethyl, Carboxyniederalkyl, z.B. Carboxymethyl oder 2-Carboxyäthyl, Niederalkoxycarbonylniederalkyl, z.B. Aethoxycarbonylmethyl oder 2-Aethoxycarbonyläthyl, Carbamoylniederalkyl, z.B. Carbamoylmethyl, Cyanoniederalkyl, z.B. Cyanomethyl oder 1-Cyano- oder 2-Cyanoäthyl, Sulfoniederalkyl, z.B. Sulfomethyl oder 2-Sulfoäthyl, Sulfamoylniederalkyl, z.B. Sulfamoylmethyl oder 2-Sulfamoyläthyl, Aminocarboxyniederalkyl, z.B. 2-Amino-2-carboxyäthyl oder eine Gruppe der Teilformel A, worin die Gruppe $-(C_nH_{2n})-$ Aethylen oder Propylen, $R_o$ Wasserstoff oder Niederalkyl, z.B. Methyl, und R Wasserstoff, Niederalkyl, z.B. Methyl oder Aethyl, Niederalkanoyl, z.B. Formyl oder Acetyl, Niederalkanoyl substituiert durch Hydroxy, Niederalkoxy, z.B. Methoxy, Halogen, z.B. Brom, Carboxy, Cyano oder Amino, z.B. α-Hydroxypropionyl, Methoxyacetyl, Bromacetyl, Carboxyacetyl, Cyanacetyl oder Glycyl, Niederalkenoyl, z.B. Acryloyl, Niederalkinoyl, z.B. Propioloyl, Cycloalkylcarbonyl, z.B. Cyclopropylcarbonyl, Benzoyl, 4-Aminobenzoyl, 4-Niederalkanoylaminobenzoyl, z.B. 4-Acetylaminobenzoyl, 4-Cyanobenzoyl, 4-Nitrobenzoyl oder 2,4-Dinitrobenzoyl, Pyridylcarbonyl, z.B. Nicotinoyl oder Isonicotinoyl, Furoyl, z.B. 2-Furoyl, Thienylcarbonyl, z.B. 2-Thienylcarbonyl, Hydroxypyrimidylcarbonyl, z.B. 2,6-Dihydroxy-1,3-pyrimid-4-ylcarbonyl, Hydroxythiadiazolylcarbonyl, z.B. 4-Hydroxy-1,2,5-thiadiazol-3-ylcarbonyl, Tetrazolylniederalkanoyl, z.B. 2-Tetrazol-5-ylcetyl oder Aminothiazolylniederalkanoyl, z.B. 2-(2-Amino-1,3-thiazol-4-yl)-acetyl, die Acylgruppe eines Halbesters der Kohlensäure, beispielsweise Niederalkoxycarbonyl, z.B. Methoxycarbonyl oder Isopropoxycarbonyl, Niederalkanoyloxy substituiert durch Carboxy und

Amino, z.B. 2-Amino-2-carboxyäthoxycarbonyl, oder Benzoyloxycarbonyl, die Acylgruppe einer substituierten Carbaminsäure, beispielsweise Niederalkylcarbamoyl, z.B. Methylcarbamoyl oder Anilinocarbonyl, die Acylgruppe einer substituierten Thiocarbaminsäure, beispielsweise Niederalkylthiocarbamoyl, z.B. Methylthiocarbamoyl, die Acylgruppe einer substituierten Sulfonsäure, beispielsweise Niederalkansulfonyl, z.B. Methansulfonyl, Benzolsulfonyl, 4-Nitrobenzolsulfonyl, 2,4-Dinitrobenzolsulfonyl, Aminobenzolsulfonyl, z.B. 4-Aminobenzolsulfonyl, eine Acylcarbamoylgruppe, beispielsweise Benzoylcarbamoyl oder Furoylcarbamoyl, eine Acylthiocarbamoylgruppe, beispielsweise Benzoylthiocarbamoyl oder Furoylthiocarbamoyl, 2-Oxo-1-imidazolidinocarbonyl, 4-Niederalkyl-2,3-dioxo-1-piperazinocarbonyl, z.B. 4-Aethyl-2,3-dioxo-1-piperazinocarbonyl, und 4-Niederalkansulfonyl-1-piperazinocarbonyl, z.B. 4-Methansulfonyl-1-piperazinocarbonyl, bedeuten, und $R_6$ Pyridyl, z.B. 3- oder 4-Pyridyl, Thienyl, z.B. 2- oder 3-Thienyl, Furyl, z.B. 2- oder 3-Furyl, Aminothiazolyl, z.B. 2-Amino-4-thiazolyl, Hydroxypyrimidyl, z.B. 2,6-Dihydroxy-1,3-pyrimid-4-yl, Aminothiadiazolyl, z.B. 5-Amino-1,2,4-thiadiazol-3-yl, Hydroxythiadiazolyl, z.B. 4-Hydroxy-1,2,5-thiadiazol-3-yl, oder Aminotriazolyl, z.B. 5-Amino-1,2,4-triazol-3-yl bedeuten, Stereoisomere, Mischungen von diesen Stereoisomeren, Hydrate und pharmazeutisch verwendbare Salze von solchen Verbindungen.

Die vorliegende Erfindung betrifft hauptsächlich Verbindungen der Formel I, worin m Null, $R_1$ Wasserstoff, Niederalkyl, z.B. Methyl, Niederalkoxy, z.B. Methoxy, Halogen, z.B. Chlor, oder eine Gruppe $-CH_2-R_2$, worin $R_2$ Niederalkanoyloxy, z.B. Acetoxy, Carbamoyloxy, Triazolylthio, z.B. 1H-1,2,3-Triazol-5-ylthio, Tetrazolylthio, z.B. 1H-Tetrazol-5-ylthio, durch Niederalkyl, z.B. Methyl, Diniederalkylaminoniederalkyl, z.B. 2-Dimethylaminoäthyl, Sulfoniederalkyl, z.B. Sulfomethyl, Carboxyniederalkyl, z.B. Carboxymethyl, oder durch Carbamoyl substituiertes Tetrazolylthio, z.B. 1-Methyl-1H-tetrazol-5-ylthio, 1-Sulfomethyl-1H-tetrazol-5-ylthio, 1-Carboxymethyl-1H-tetrazol-5-ylthio oder 1-(2-Dimethylaminoäthyl)-1H-tetrazol-5-ylthio, Thiadiazolylthio, z.B. 1,3,4-Thiadiazol-5-ylthio, durch Niederalkyl,

z.B. Methyl, substituiertes Thiadiazolylthio, z.B. 2-Methyl-1,3,4-thiadiazol-5-ylthio, durch Niederalkyl, z.B. Methyl, substituiertes 5,6-Dioxotetrahydro-as-triazin-3-ylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, Pyridinio oder durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinio, z.B. 3- oder 4-Hydroxymethylpyridinio, 4-Carboxypyridinio, 3- oder 4-Carboxymethylpyridinio, 3- oder 4-Chlorpyridinio, 3- oder 4-Brompyridinio oder 3- oder 4-Carbamoylpyridinio bedeutet, $R_3$ Carboxyl, oder unter physiologischen Bedingungen spaltbares Carboxyl, z.B. Acyloxyniederalkoxycarbonyl, z.B. Niederalkanoyloxyniederalkoxycarbonyl, z.B. Niederalkanoyloxymethoxycarbonyl oder Niederalkanoyloxyäthoxycarbonyl, z.B. Pivaloyloxymethoxycarbonyl oder 2-Propionyloxyäthoxycarbonyl, oder Niederalkoxycarbonyloxyniederalkoxycarbonyl, z.B. 1-Aethoxycarbonyloxyäthoxycarbonyl oder tert.-Butoxycarbonyloxymethoxycarbonyl, $R_4$ Wasserstoff, $R_5$ Niederalkyl, z.B. Methyl oder Aethyl, Hydroxyniederalkyl, z.B. Hydroxymethyl, oder Hydroxyäthyl, Niederalkoxyniederalkyl, z.B. Methoxymethyl, 2-Methoxyäthyl oder 2-Aethoxyäthyl, Niederalkenyloxyniederalkyl, z.B. 2-Vinyloxyäthyl, Halogenniederalkyl, z.B. Chlormethyl oder 2-Chloräthyl, Niederalkylthioniederalkyl, z.B. 2-Methylthioäthyl oder 2-Aethylthioäthyl, Carboxyniederalkyl, z.B. Carboxymethyl oder 2-Carboxyäthyl, Carbamoylniederalkyl, z.B. Carbamoylmethyl, Cyanoniederalkyl, z.B. Cyanomethyl oder 1-Cyano- oder 2-Cyanoäthyl, oder eine Gruppe der Teilformel A, welche 2-Aminoäthyl, 2-Niederalkylaminoäthyl, z.B. 2-Methylaminoäthyl oder 2-n-Hexylaminoäthyl, 2-Diniederalkylaminoäthyl, z.B. 2-Dimethylaminoäthyl oder 2-Di-n-hexylaminoäthyl, 2-Sulfoaminoäthyl, Niederalkanoylaminoäthyl, z.B. 2-Formylaminoäthyl oder 2-Acetylaminoäthyl, 2-Niederalkoxyniederalkanoylaminoäthyl, z.B. 2-Methoxyacetylaminoäthyl, 2-Halogenniederalkanoylaminoäthyl, z.B. 2-Bromacetylaminoäthyl, 2-(α-Hydroxypropionylamino)-äthyl, 2-Glycylaminoäthyl, 2-(3-Amino-3-carboxypropionylamino)-äthyl,2-Acryloylaminoäthyl, 2-Propioloylaminoäthyl, 2-Cyclopropylcarbonylaminoäthyl, 2-Benzoylaminoäthyl, 2-(4-Aminobenzoylamino)-äthyl, 2-(4-Acetylaminobenzoylamino)-äthyl, 2-(4-Cyanobenzoylamino)-äthyl, 2-(4-Nitrobenzoylamino)-äthyl, 2-(3,4-Dinitrobenzoylamino)-äthyl, 2-Mandeloylaminoäthyl, 2-Phenylglycylaminoäthyl, 2-Nicotinoylaminoäthyl, 2-Isonicotinoylaminoäthyl, 2-(2-Furoylamino)-äthyl, 2-(2-Tienylcarbonylamino)-äthyl, 2-(2,6-Dihydroxy-1,3-pyrimid-4-ylcarbonylamino)-äthyl, 2-(4-Hydroxy-1,2,5-thiadiazol-3-ylcarbonylamino)-äthyl, 2-(2-Tetrazol-1-ylacetylamino)-äthyl, 2-[2-(2-Amino-1,3-thiazol-4-yl)-acetylamino]-äthyl, 2-Niederalkoxycarbonylaminoäthyl, z.B. 2-Methoxycarbonylaminoäthyl oder 2-Isopropoxycarbonylaminoäthyl, 2-(2-Amino-2-carboxyäthoxycarbonylamino)-äthyl, 2-Benzoyloxycarbonylaminoäthyl, 2-Niederalkylcarbamoylaminoäthyl, z.B. 2-Methylcarbamoylaminoäthyl, 2-Anilinocarbonylaminoäthyl, 2-Niederalkylthiocarbamoylaminoäthyl, z.B. 2-Methylthiocarbamoylaminoäthyl, 2.Niederalkansulfonylaminoäthyl, z.B. 2-Methansulfonylaminoäthyl, 2-Halogenmethansulfonylaminoäthyl, z.B. 2-Difluormethansulfonylaminoäthyl, 2-Cyanomethansulfonylaminoäthyl, 2-Benzolsulfonylaminoäthyl, 2-(4-Nitrobenzolsulfonylamino)-äthyl, 2-(2,4-Dinitrobenzolsulfonylaminoäthyl, 2-Benzoylcarbamoylaminoäthyl, 2-(2-Furoylcarbamoylamino)-äthyl, 2-(2-Oxo-1-imidazolidinocarbonylamino)-äthyl, 2-(4-Aethyl-2,3-dioxo-1-piperazinocarbonylamino)-äthyl und 2-(4-Methansulfonyl-1-piperazinocarbonylamino)-äthyl bedeutet, und $R_6$ Aminothiazolyl, z.B. 2-Amino-4-thiazolyl, Aminothiadiazolyl, z.B. 5-Amino-1,2,4-thiadiazolyl-3-yl, oder Aminotriazolyl, z.B. 5-Amino-1,2,4-triazol-3-yl, bedeutet, Stereoisomere, Mischungen von diesen Stereoisomeren, Hydrate und pharmazeutisch verwendbare Salze von solchen Verbindungen.

Die vorliegende Erfindung betrifft in erster Linie Verbindungen der Formel I, worin m Null, $R_1$ Wasserstoff, Niederalkoxy, z.B. Methoxy, Halogen, z.B. Chlor, oder eine Gruppe der Formel $-CH_2-R_2$, worin $R_2$

Niederalkanoyloxy, z.B. Acetoxy, Carbamoyloxy, Tetrazolylthio, z.B. 1H-Tetrazol-5-ylthio, durch Niederalkyl, z.B. Methyl, Diniederalkylaminoniederalkyl, z.B. 2-Dimethylaminoäthyl, Sulfoniederalkyl, z.B. Sulfomethyl, oder Carboxyniederalkyl, z.B. Carboxymethyl, substituiertes Tetrazolylthio, z.B. 1-Methyl-1H-tetrazol-5-ylthio, 1-(2-Dimethylaminoäthyl)-1H-tetrazol-5-ylthio, 1-Carboxymethyl-1H-tetrazol-5-ylthio, 1-Sulfomethyl-1H-tetrazol-5-ylthio oder 1-Carboxymethyl-1H-tetrazol-5-ylthio, durch Niederalkyl, z.B. Methyl, substituiertes 5,6-Dioxotetrahydro-as-triazin-3-ylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, Pyridinio oder durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinio, z.B. 3- oder 4-Hydroxymethylpyridinio, 4-Carboxypyridinio, 3- oder 4-Carboxymethylpyridinio, 3- oder 4-Chlorpyridinio, 3- oder 4-Brompyridinio oder 3- oder 4-Carbamoylpyridinio bedeutet, $R_3$ Carboxy, Niederalkanoyloxyniederalkoxycarbonyl, z.B. Niederalkanoyloxymethoxycarbonyl oder Niederalkanoyloxyäthoxycarbonyl, z.B. Pivaloyloxymethoxycarbonyl oder 2-Propionyloxyäthoxycarbonyl oder Niederalkoxycarbonyloxyniederalkoxycarbonyl, z.B. 1-Aethoxycarbonyloxyäthoxycarbonyl oder tert.-Butoxycarbonyloxymethoxycarbonyl, $R_4$ Wasserstoff, $R_5$ Niederalkyl, z.B. Methyl oder Aethyl, Niederalkoxyniederalkyl, z.B. Methoxymethyl, 2-Methoxyäthyl oder 2-Aethoxyäthyl, Niederalkenyloxyniederalkyl, z.B. 2-Vinyloxyäthyl, Halogenniederalkyl, z.B. Chlormethyl oder 2-Chloräthyl, Carboxyniederalkyl, z.B. Carboxymethyl oder 2-Carboxyäthyl, Cyanoniederalkyl, z.B. Cyanomethyl oder 1-Cyano- oder 2-Cyanoäthyl, oder eine Gruppe der Teilformel A, welche 2-Aminoäthyl, 2-Niederalkylaminoäthyl, z.B. 2-Methylaminoäthyl oder 2-Aethylaminoäthyl, 2-Diniederalkylaminoäthyl, z.B. 2-Dimethylaminoäthyl, 2-Sulfoaminoäthyl, Niederalkanoylaminoäthyl, z.B. 2-Formylaminoäthyl oder 2-Acetylaminoäthyl, Niederalkoxyniederalkanoylaminoäthyl, z.B. 2-Methoxyacetylaminoäthyl, Cyanoniederalkanoylaminoäthyl, z.B. 2-Cyanoacetylaminoäthyl, Niederalkanoylaminoäthyl, z.B. 2-Acryloylaminoäthyl, Niederalkinoylaminoäthyl, z.B. 2-Propionylaminoäthyl, Cycloalkanoylaminoäthyl, z.B. 2-Cyclopropanoylaminoäthyl, 2-(4-Hydroxy-1,2,5-thiadiazol-3-ylcarbonylamino)-äthyl, 2-(2-Tetrazol-5-ylacetylamino)-äthyl, 2-[2-(2-Amino-1,3-thiazol-4-yl)-acetylamino]-äthyl, 2-Niederalkoxycarbonylaminoäthyl, z.B. 2-Methoxycarbonylaminoäthyl, 2-Niederalkansulfonylaminoäthyl, z.B. 2-Methansulfonylaminoäthyl, 2-Benzolsulfonylaminoäthyl, 2-Benzolsulfonylaminoäthyl, worin Benzol durch Nitro oder Amino substituiert ist, z.B. 2-(4-Nitrobenzolsulfonylamino)-äthyl, 2-(2,4-Dinitrobenzolsulfonylamino)-äthyl, 2-(2-Oxo-1-imidazolidinocarbonylamino)-äthyl, 2-(4-Aethyl-2,3-dioxo-1-piperazinocarbonylamino)-äthyl, oder 2-(4-Methylsulfonyl-1-piperazino-carbonylamino)-äthyl, bedeutet, und $R_6$ Aminothiazolyl, z.B. 2-Amino-4-thiazolyl, bedeutet, Stereoisomere, Mischungen von diesen Stereoisomeren, Hydrate und pharmazeutisch verwendbare Salze von solchen Verbindungen.

Die Erfindung betrifft ganz besonders die in den Beispielen beschriebenen Verbindungen der Formel I, deren pharmazeutisch verwendbare Salze, sowie die dort beschriebenen Ausgangsstoffe und Zwischenprodukte.

Die Erfindung betrifft vor allem die im Versuchsbericht aufgezählten pharmazeutisch verwendbaren Salze von Verbindungen der Formel I bzw. deren Enantiomere.

Herstellungsverfahren:

Verbindungen der Formel I, worin die Carboxylgruppen in freier Form vorliegen oder in physiologisch spaltbarer Form verestert sind, Hydrate und Salze von solchen Verbindungen, die eine salzbildende Gruppe aufweisen, werden beispielsweise hergestellt, indem man

a) in einer Verbindung der Formel

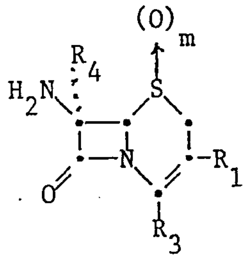

(II),

worin m, $R_1$, $R_3$ und $R_4$ die unter Formel I genannten Bedeutungen haben, und worin eine in $R_1$ vorhandene funktionelle Gruppe geschützt ist und die 7β-Aminogruppe gegebenenfalls durch eine die Acylierungsreaktion erlaubende Gruppe geschützt ist, die 7β-Aminogruppe durch Umsetzung mit einem den Acylrest einer Carbonsäure der Formel

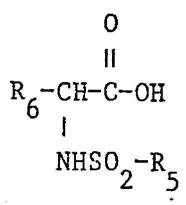

(III),

einführenden Acylierungsmittel, worin $R_5$ und $R_6$ die unter Formel I genannten Bedeutungen haben, und worin eine in $R_5$ und/oder $R_6$ vorhandene funktionelle Gruppe in geschützter Form vorliegt, acyliert oder

b) in einer Verbindung der Formel

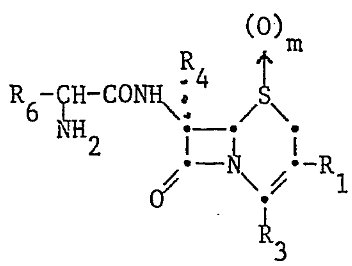

(IV),

worin m, $R_1$, $R_3$, $R_4$ und $R_6$ die unter Formel I genannten Bedeutungen haben, und worin eine in $R_1$ und/oder $R_6$ vorhandene funktionelle Gruppe geschützt ist und die 2-Aminogruppe gegebenenfalls durch eine die

Sulfonylierungsreaktion erlaubende Gruppe geschützt ist, die 2-Aminogruppe durch Umsetzung mit einem den $R_5$-Sulfonylrest einer Sulfonsäure der Formel

$$R_5 - SO_2 - OH \qquad (V),$$

einführenden Sulfonylierungsmittel, worin $R_5$ die unter Formel I genannte Bedeutung hat und eine in $R_5$ vorhandene funktionelle Gruppe in geschützter Form vorliegt, oder mit einem reaktionsfähigen, funktionellen Säurederivat oder einem Salz davon sulfonyliert oder

c) eine 2-Cephem-Verbindung der Formel

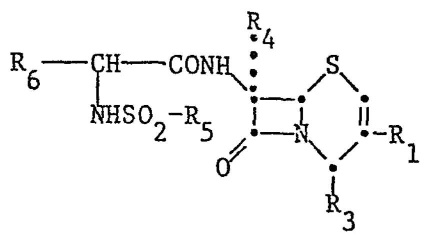

(VI),

worin $R_1$, $R_3$, $R_4$, $R_5$ und $R_6$ die unter Formel I genannten Bedeutungen haben und eine in $R_1$, $R_5$ und/oder $R_6$ vorhandene funktionelle Gruppe gegebenenfalls in geschützter Form vorliegt, zur entsprechenden 3-Cephem-Verbindung der Formel I isomerisiert und, wenn erwünscht, eine erfindungsgemäss erhältliche Verbindung der Formel I in eine andere definitionsgemässe Verbindung der Formel I umwandelt und/oder eine erfindungsgemäss erhältliche Verbindung der Formel I, worin m 0 bedeutet, in eine Verbindung der Formel I überführt, worin m 1 oder 2 bedeutet, und/oder eine Verbindung der Formel I, worin m 1 oder 2 bedeutet, in eine Verbindung der Formel I überführt, worin m 0 bedeutet, und/oder in einer Verbindung der Formel I in geschützter Form vorliegende funktionelle Gruppen in freie funktionelle Gruppen überführt, und/oder ein erhältliches Salz in die freie Verbindung oder in ein anderes Salz überführt, und/oder eine erhältliche freie Verbindung mit einer salzbildenden Gruppe in ein Salz überführt und/oder ein erhältliches Gemisch von isomeren Verbindungen der Formel I in die einzelnen Isomere auftrennt.

Verfahren a) (Acylierung):

In einem Ausgangsmaterial der Formel II ist eine in $R_1$ vorhandene funktionelle Gruppe, z.B. eine Carboxy-, Amino- oder Hydroxygruppe, durch eine weiter vorn genannte Schutzgruppe, z.B. eine Carboxy-, Amino- oder Hydroxyschutzgruppe, geschützt.

Die 7β-Aminogruppe in einem Ausgangsmaterial der Formel II ist gegebenenfalls durch eine die Acylierungsreaktion erlaubende Gruppe geschützt. Eine solche Gruppe ist beispielsweise eine organische Silylgruppe, ferner eine Ylidengruppe, die zusammen mit der Aminogruppe eine Schiff'sche Base bildet. Eine organische Silylgruppe ist z.B. eine solche Gruppe, die auch mit einer Carboxylgruppe $R_3$ eine geschützte Carboxylgruppe bilden kann. Es ist in erster Linie eine Triniederalkylsilylgruppe, insbesondere Trimethylsilyl. Bei der Silylierungsreaktion zum Schutz einer 4-Carboxylgruppe in einem Ausgangsmaterial der Formel II kann bei Verwendung eines Ueberschusses des Silylierungsmittels die Aminogruppe ebenfalls silyliert werden. Eine Ylidengruppe ist in erster Linie eine 1-Arylniederalkyliden-, insbesondere eine 1-Arylmethylengruppe, worin Aryl besonders für einen carbocyclischen, in erster Linie monocyclischen, Arylrest steht, z.B. für gegebenenfalls durch Niederalkyl, Hydroxy, Niederalkoxy und/oder Nitro substituiertes Phenyl.

Ein den Acylrest einer Carbonsäure der Formel III einführendes Acylierungsmittel ist die Carbonsäure der Formel III selbst oder ein reaktionsfähiges funktionelles Derivat oder ein Salz davon.
In einem Ausgangsmaterial der Formel III ist eine in $R_5$ und/oder $R_6$ vorhandene funktionelle Gruppe, z.B. eine Carboxylgruppe, welche an der Acylierungsreaktion nicht teilnehmen soll, eine Amino- oder Hydroxylgruppe durch eine weiter vorn genannte Schutzgruppe, z.B. eine Carboxyl-, Amino- oder Hydroxylschutzgruppe, geschützt.

In einem Ausgangsmaterial der Formel III kann eine vorhandene Aminogruppe auch in ionischer Form geschützt sein, z.B. in Form eines Säureadditionssalzes, welches beispielsweise mit einer starken anorganischen Säure, z.B. einer Halogenwasserstoffsäure, z.B. Salzsäure oder Schwefelsäure, oder mit einer organischen Säure, z.B. p-Toluolsulfonsäure, gebildet wird.

Falls eine freie Säure der Formel III zur Acylierung eingesetzt wird, wird die Reaktion üblicherweise in Gegenwart von geeigneten Kondensationsmitteln wie Carbodiimiden, beispielsweise N,N'-Diäthyl-, N,N'-Dipropyl-, N,N'-Dicyclohexyl- oder N-Aethyl-N'-3-dimethylaminopropylcarbodiimid, geeigneten Carbonylverbindungen, beispielsweise Carbonyldiimidazol, oder 1,2-Oxazoliumverbindungen, wie 2-Aethyl-5-phenyl-1,2-oxazolium-3'-sulfonat oder 2-tert.-Butyl-5-methyl-1,2-oxazoliumperchlorat, oder einer geeigneten Acylaminoverbindung, z.B. 2-Aethoxy-1-äthoxycarbonyl-1,2-dihydro-chinolin, durchgeführt.

Die Kondensationsreaktion wird vorzugsweise in einem wasserfreien Reaktionsmedium, vorzugsweise in Gegenwart eines Lösungsmittels, z.B. Methylenchlorid, Dimethylformamid, Acetonitril oder Tetrahydrofuran, gegebenenfalls unter Kühlen oder Erwärmen, z.B. in einem Temperaturbereich von etwa -40°C bis etwa +100°C, bevorzugt von etwa -20°C bis etwa +50°C, und gegebenenfalls unter Inertgas-, z.B. Stickstoffatmosphäre, durchgeführt.

Ein reaktionsfähiges, d.h. die Carboxamid-Funktion bildendes, funktionelles Derivat einer Carbonsäure der Formel III, ist in erster Linie ein Anhydrid der Carbonsäure der Formel III, vorzugsweise ein gemischtes Anhydrid. Ein gemischtes Anhydrid wird beispielsweise durch Kondensation mit einer anderen Säure, z.B. einer anorganischen Säure, z.B. einer Halogenwasserstoffsäure, gebildet und ist beispielsweise das entsprechende Carbonsäurehalogenid, z.B. das Carbonsäurechlorid oder

-bromid. Ein gemischtes Anhydrid wird ferner durch Kondensation mit Stickstoffwasserstoffsäure gebildet und ist beispielsweise das Carbonsäureazid. Weitere anorganische Säuren, die sich zur Bildung des gemischten Anhydrids eignen, sind Phosphor-haltige Säuren, z.B. Phosphorsäure, Diäthylphosphorsäure und phosphorige Säuren, Schwefel-haltige Säuren, z.B. Schwefelsäure oder Cyanwasserstoffsäure. Ein reaktionsfähiges, funktionelles Derivat einer Carbonsäure der Formel III wird ausserdem durch Kondensation mit einer organischen Carbonsäure gebildet, z.B. mit einer unsubstituierten oder durch Halogen, z.B. Fluor oder Chlor, substituierten Niederalkancarbonsäure, z.B. Pivalinsäure oder Trifluoressigsäure, mit einem Niederalkylhalbester der Kohlensäure, z.B. dem Aethyl- oder Isobutylhalbester der Kohlensäure, oder mit einer organischen, z.B. aliphatischen oder aromatischen, Sulfonsäure, z.B. Methansulfonsäure oder p-Toluolsulfonsäure.

Ein reaktionsfähiges funktionelles Derivat einer Carbonsäure der Formel III ist ebenfalls ein aktivierter Ester der Carbonsäure der Formel III, der beispielsweise durch Kondensation mit einem vinylogen Alkohol, d.h. mit einem Enol, z.B. einem vinylogen Niederalkenol, gebildet wird, ein Iminomethylesterhalogenid, z.B. Dimethyliminomethylesterchlorid, hergestellt aus der Carbonsäure der Formel III und z.B. Dimethyl-(1-chloräthyliden)-iminiumchlorid der Formel $[(CH_3)_2N^{\oplus}=C(Cl)CH_3]Cl^{\ominus}$, das man seinerseits z.B. aus N,N-Dimethylacetamid und Phosgen oder Oxalylchlorid erhalten kann, ein Arylester, z.B. ein durch Halogen, z.B. Chlor und/oder Nitro substituierter Phenylester, z.B. ein Pentachlorphenyl-, 4-Nitrophenyl- oder 2,3-Dinitrophenylester, ein N-heteroaromatischer Ester, z.B. N-Benztriazolester, oder ein N-Diacyliminoester, z.B. ein N-Succinylimino- oder N-Phthalyliminoester.

Die Acylierung mit einem reaktionsfähigen funktionellen Derivat der Carbonsäure der Formel III, z-B. mit einem entsprechenden Anhydrid, insbesondere einem Säurehalogenid, wird bevorzugt in Anwesenheit eines der genannten Kondensationsmittel, z.B. einem Carbodiimid, z.B.

Dicyclohexylcarbodiimid, oder einer geeigneten Base, durchgeführt. Eine geeignete Base ist beispielsweise ein Amin, z.B. ein tertiäres Amin, z.B. Triniederalkylamin, z.B. Trimethylamin, Triäthylamin oder Aethyl-di-isopropylamin, oder N,N-Diniederalkylanilin, z.B. N,N-Dimethylanilin, oder ein cyclisches tertiäres Amin, z.B. ein N-niederalkyliertes Morpholin, z.B. N-Methylmorpholin, oder ist beispielsweise eine Base vom Pyridin-Typ, z.B. Pyridin. Eine geeignete Base ist ferner eine anorganische Base, beispielsweise ein Alkalimetall- oder Erdalkalimetallhydroxid, -carbonat- oder -hydrogencarbonat, z.B. Natrium-, Kalium- oder Calciumhydroxid, -carbonat- oder-hydrogencarbonat, oder ist ein Oxiran, beispielsweise ein 1,2-Niederalkylenoxid, wie Aethylenoxid oder Propylenoxid.

Die Acylierung mit einem reaktionsfähigen, funktionellen Derivat der Carbonsäure der Formel III wird vorzugsweise in einem inerten, vorzugsweise wasserfreien, Lösungsmittel oder Lösungsmittelgemisch vorgenommen, beispielsweise in einem Carbonsäureamid, z.B. Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, cyclischen Aether, z.B. Tetrahydrofuran, einem Ester, z.B. Essigsäureäthylester, oder einem Nitril, z.B. Acetonitril, oder in Mischungen davon, gegebenenfalls bei erniedrigter oder erhöhter Temperatur, z.B. in einem Temperaturbereich von etwa -40°C bis etwa +100°C, bevorzugt von etwa -10°C bis etwa +50°C, und gegebenenfalls unter Inertgas-, z.B. Stickstoffatmosphäre.

Die Acylierung einer Verbindung der Formel II kann auch bei Verwendung eines geeigneten reaktionsfähigen, funktionellen Derivats der Säure der Formel III in Gegenwart einer geeigneten Acylase erfolgen. Solche Acylasen sind bekannt und können durch eine Anzahl von Mikroorganismen gebildet werden, z.B. durch Acetobacter, wie *Acetobacter aurantium*, Achromobacter, wie *Achromobacter aeris*, Aeromonas, wie *Aeromonas hydrophila*, oder Bacillus, wie *Bacillus megaterium 400*. In einer solchen enzymatischen Acylierung wird insbesondere ein Amid, Ester oder

Thioester, wie ein Niederalkyl-, z.B. Methyl- oder Aethylester, der Carbonsäure der Formel III als reaktionsfähiges, funktionelles Derivat eingesetzt. Ueblicherweise wird eine solche Acylierung in einem den entsprechenden Mikroorganismus enthaltenden Nährmedium, in einem Filtrat der Kulturbrühe oder, gegebenenfalls nach Isolierung der Acylase, inklusive nach Adsorption an einen Träger, in einem wässrigen, gegebenenfalls einen Puffer enthaltenden Medium, z.B. in einem Temperaturbereich von etwa +20°C bis etwa +40°C, bevorzugt bei etwa +37°C, durchgeführt.

Ein bei der Acylierungsreaktion eingesetztes, reaktionsfähiges funktionelles Derivat einer Säure der Formel III kann, wenn erwünscht, in situ gebildet werden. So kann man z.B. ein gemischtes Anhydrid in situ herstellen, indem man eine Säure der Formel III, worin funktionelle Gruppen gegebenenfalls geschützt sind, oder ein geeignetes Salz davon, z.B. ein Ammoniumsalz, welches z.B. mit einer organischen Base, wie Pyridin oder 4-Methylmorpholin gebildet wird, oder ein Metallsalz, z.B. ein Alkalimetallsalz, z.B. Natriumsalz, mit einem geeigneten Derivat einer anderen Säure, beispielsweise einem Säurehalogenid, einer unsubstituierten oder durch Halogen, z.B. Chlor, substituierten Niederalkancarbonsäure, z.B. Trichloracetylchlorid. einem Halbester eines Kohlensäurehalbhalogenids, z.B. Chlorameisensäureäthylester, oder -isobutylester, oder mit einem Halogenid einer Diniederalkylphosphorsäure, z.B. Diäthylphosphorbromidat, das man durch Umsetzen von Triäthylphosphit mit Brom bilden kann, umsetzt. Das so erhältliche gemischte Anhydrid lässt sich ohne Isolierung bei der Acylierungsreaktion verwenden.

Verfahren b) (Sulfonylierung):

In einem Ausgangsmaterial der Formel IV ist eine in $R_1$ und/oder $R_6$ vorhandene funktionelle Gruppe, z.B. eine Carboxyl-, Amino- oder Hydroxylgruppe durch eine weiter vorn genannte Schutzgruppe, z.B. eine Carboxyl-, Amino- oder Hydroxylschutzgruppe, geschützt.

Die 2-Aminogruppe in einem Ausgangsmaterial der Formel IV ist gegebenenfalls durch eine die Sulfonylierungsreaktion erlaubende Gruppe geschützt. Eine solche Gruppe ist beispielsweise eine organische Silylgruppe, z.B. eine Triniederalkylsilylgruppe, z.B. Trimethylsilyl oder eine Ylidengruppe, welche zusammen mit der Aminogruppe eine Schiff'sche Base bildet, und ist dieselbe Gruppe, durch welche die 7β-Aminogruppe in einem Ausgangsmaterial der Formel II gegebenenfalls substituiert ist, und welche die Acylierungsreaktion gemäss Verfahren a) erlaubt.

Ein den $R_5$-Sulfonylrest einer Sulfonsäure der Formel V einführendes Sulfonylierungsmittel ist die Sulfonsäure der Formel V selbst oder ein reaktionsfähiges, funktionelles Derivat davon.

In einem Ausgangsmaterial der Formel V ist eine in $R_5$ vorhandene funktionelle Gruppe, z.B. eine Carboxylgruppe, eine Amino- oder Hydroxylgruppe oder eine Sulfonylgruppe, welche an der Acylierungsreaktion nicht teilnehmen soll, durch eine weiter vorn genannte Schutzgruppe, z.B. eine Carboxyl-, Amino-, Hydroxy- oder eine Sulfonylschutzgruppe, geschützt.

In einem Ausgangsmaterial der Formel V kann eine vorhandene Aminogruppe wie eine in einem Ausgangsmaterial der Formel III vorhandene Aminogruppe in ionischer Form geschützt sein, z. B. in Form eines Säureadditionssalzes, z.B. als Hydrochlorid.

Falls eine freie Sulfonsäure der Formel V bei der Sulfonylierung eingesetzt wird, führt man die Sulfonylierung üblicherweise in Gegenwart desselben Kondensationsmittels durch, welches man bei der Acylierung der 7β-Aminogruppe in einer Verbindung der Formel II mit einer freien Carbonsäure der Formel III gemäss Verfahren a) verwendet, z.B. in Gegenwart eines Carbodiimids, z.B. N,N'-Dicyclohexylcarbodiimid.

Bei der Sulfonylierung mit einer freien Sulfonsäure der Formel V werden die gleichen Lösungsmittel verwendet und die gleichen Reaktionsbedingungen eingehalten wie bei der Acylierung mit einer freien Carbonsäure der Formel III gemäss Verfahren a).

Ein reaktionsfähiges, d.h. die Sulfonamid-Funktion bildendes, funktionelles Derivat einer Sulfonsäure der Formel V ist in erster Linie ein Anhydrid der Sulfonsäure der Formel V, vorzugsweise ein gemischtes Anhydrid. Ein gemischtes Anhydrid wird beispielsweise durch Kondensation mit einer anorganischen Säure,z.B. einer Halogenwasserstoffsäurem gebildet und ist beispielsweise das entsprechende Sulfonsäurehalogenid, z.B. das Sulfonsäurechlorid oder -bromid. Weitere anorganische Säuren, welche sich zur Bildung des gemischten Anhydrids eignen, sind Phosphorhaltige Säuren, z.B. Phosphorsäure, Diäthylphosphorsäure oder phosphorige Säure, oder Schwefel-haltige Säuren, z.B. Schwefelsäure. Ein reaktionsfähiges, funktionelles Derivat einer Sulfonsäure der Formel V wird ausserdem durch Kondensation mit einer organischen Carbonsäure gebildet, z.B. mit einer unsubstituierten oder durch Halogen, z.B. Fluor oder Chlor, substituierten Niederalkancarbonsäure, z.B. Pivalin- oder Trifluoressigsäure, mit einem Niederalkylhalbester der Kohlensäure, z.B. dem Aethyl- oder Isobutylhalbester der Kohlensäure, oder mit einer anderen Sulfonsäure, z.B. einer aliphatischen oder aromatischen Sulfonsäure, z.B. Methansulfonsäure oder p-Toluolsulfonsäure.

Ein reaktionsfähiges, funktionelles Derivat einer Sulfonsäure der Formel V ist ebenfalls ein aktivierter Ester der Sulfonsäure der Formel V. Ein aktivierter Ester wird beispielsweise durch Kondensation mit einem vinylogen Alkohol, d.h. mit einem Enol, z.B. einem vinylogen Niederalkenol, gebildet, oder ist beispielsweise ein durch Halogen, z.B. Chlor, und/oder Nitro substituierter Phenylester, z.B. ein Pentachlorphenyl-, ein 4-Nitrophenyl- oder ein 2,3-Dinitrophenylester, ein N-heteroaromatischer Ester, z.B. ein N-Bentriazolester, oder ist ein N-Diacyliminoester, z.B. ein N-Succinylimino- oder ein Phthalyliminoester.

Bei der Sulfonylierung mit einem reaktionsfähigen, funktionellen Derivat einer Sulfonsäure der Formel V werden die gleichen Lösungsmittel verwendet und die gleichen Reaktionsgedingungen eingehalten wie bei der Acylierung mit einem reaktionsfähigen, funktionellen Derivat einer Carbonsäure der Formel III gemäss Verfahren a).

Verfahren c) (Isomerisierung):

In einem 2-Cephem-Ausgangsmaterial der Formel VI weist die gegebenenfalls geschützte 4-Carboxylgruppe vorzugsweise die α-Konfiguration auf.

Man isomerisiert eine 2-Cephem-Verbindung der Formel VI, indem man diese mit einem basischen Mittel behandelt und die entsprechende 3-Cephem-Verbindung isoliert. Als basisches Mittel verwendet man eine organische, stickstoffhaltige Base, insbesondere eine tertiäre, heterocyclische Base aromatischen Charakters, in erster Linie eine Base des Pyridin-Typs, z.B. Pyridin, Picolin, Collidin oder Lutidin, ferner Chinolin, eine tertiäre aromatische Base, z.B. des Anilin-Typs, z.B. ein N,N-Diniederalkylanilin, z.B. N,N-Dimethylanilin oder N,N-Diäthylanilin, oder eine tertiäre aliphatische, azacycloaliphatische oder araliphatische Base, z.B. ein Triniederalkylamin, z.B. Trimethylamin oder N,N-Diisopropyl-N-äthylamin, ein N-Niederalkyl-azacycloalkan, z.B. N-Methyl-piperidin, oder ein N-Phenylniederalkyl-N,N-diniederalkylamin, z.B. N-Benzyl-N,N-dimethylamin, sowie ein Gemisch von solchen basischen Mitteln, z.B. das Gemisch einer Base vom Pyridintyp und eines Triniederalkylamins, z.B. Pyridin und Triäthylamin. Ferner kann man ein anorganisches oder organisches, basisches Salz, insbesondere ein basisches Salz einer mittelstarken bis starken Base mit einer schwachen Säure, z.B. ein Alkalimetall- oder Ammoniumsalz einer Niederalkancarbonsäure, z.B. Natriumacetat, Triäthylammoniumacetat oder N-Methylpiperidinacetat, sowie andere analoge Basen oder Gemische von solchen baischen Mitteln verwenden.

Bei der Isomerisierung einer 2-Cephem-Verbindung der Formel VI mit einem basischen Mittel arbeitet man vorzugsweise in einem wasserfreien

Medium, in An- oder Abwesenheit eines Lösungsmittels, wie eines gegebenenfalls halogenierten, z.B. chlorierten, aliphatischen, cycloaliphatischen oder aromatischen Kohlenwasserstoffs, oder eines Lösungsmittelgemisches, wobei die als Isomerisierungsmittel verwendete und unter den Reaktionsbedingungen flüssige Base gleichzeitig auch als Lösungsmittel dienen kann. Man arbeitet gegebenenfalls unter Kühlen oder unter Erwärmen, vorzugsweise in einem Temperaturbereich von etwa -30°C bis etwa +100°C und gegebenenfalls unter Inertgas-, z.B. Stickstoffatmosphäre.

Eine so erhältliche 3-Cephem-Verbindung der Formel I lässt sich in an sich bekannter Weise, z.B. durch Adsorptionschromatographie und/oder Kristallisation, von gegebenenfalls noch vorhandenem 2-Cephalosporin-Ausgangsstoff der Formel VI abtrennen.

Die Isomerisierung einer 2-Cephem-Verbindung der Formel VI zur entsprechenden 3-Cephem-Verbindung der Formel I erfolgt vorzugsweise, indem man die 2-Cephem-Verbindung der Formel VI in 1-Stellung mit einem geeigneten Oxydationsmittel oxydiert und, wenn erwünscht, ein gegebenenfalls erhältliches Isomerengemisch der 1-Oxide trennt und ein so erhältliches 1-Oxid der 3-Cephem-Verbindung der Formel I, worin m 1 bedeutet, zur 3-Cephem-Verbindung, worin m null bedeutet, reduziert.

Als Oxydationsmittel für die Oxydation des Schwefelatoms in 1-Stellung einer 2-Cephem-Verbindung der Formel VI eignen sich anorganische Persäuren, die ein Reduktionspotential von wenigstens +1,5 Volt aufweisen und aus nichtmetallischen Elementen bestehen, organische Persäuren oder Gemische aus Wasserstoffperoxid und Säuren, insbesondere organische Carbonsäuren mit einer Dissoziationskonstante von wenigstens $10^{-5}$, in Frage. Geeignete anorganische Persäuren sind beispielsweise Perjod- und Perschwefelsäure. Organische Persäuren sind beispielsweise Percarbon- und Persulfonsäuren, die als Persäure zugesetzt oder durch Verwendung von wenigstens einem Aequivalent Wasserstoffperoxid und einer Carbonsäure in situ gebildet werden können. Bei der in-situ Bildung der Persäure setzt man zweckmässig einen grossen Ueberschuss

der Carbonsäure, z.B. Essigsäure, als Lösungsmittel zu. Geeignete organische Persäuren sind vorzugsweise Perameisensäure, Peressigsäure, Trifluorperessigsäure, Permaleinsäure, Perbenzoesäure, 3-Chlorperbenzoesäure, Monoperphthalsäure oder p-Toluolpersulfonsäure.

Die Oxydation kann ebenfalls unter Verwendung von Wasserstoffperoxid mit katalytischen Mengen einer Säure mit einer Dissoziationskonstante von wenigstens $10^{-5}$ durchgeführt werden, wobei man niedrige Konzentrationen, z.B. 1-2% und weniger, aber auch grössere Mengen der betreffenden Säure einsetzen kann. Dabei hängt die oxydative Wirksamkeit des Gemisches in erster Linie von der Stärke der Säure ab. Geeignete Gemische sind z.B. Wasserstoffperoxid mit Essigsäure, Perchlorsäure oder Trifluoressigsäure.

Die obige Oxydation kann in Gegenwart von geeigneten sauren Katalysatoren durchgeführt werden. So kann z.B. die Oxydation mit einer Percarbonsäure in Gegenwart einer Säure mit einer Dissoziationskonstante von wenigstens $10^{-5}$ katalysiert werden, wobei ihre katalytische Wirksamkeit von ihrer Säurestärke abhängt. Als Katalysatoren geeignete Säuren sind z.B. Essigsäure, Perchlorsäure und Tirfluoressigsäure. Ueblicherweise verwendet man mindestens äquimolare Mengen des Oxydationsmittels, vorzugsweise einen geringen Ueberschuss von etwa 10% bis etwa 20%, wobei man auch grössere Ueberschüsse, d.h. bis zur 10fachen Menge des Oxidationsmittels oder darüber verwenden kann. Die Oxydation wird unter milden Bedingungen, z.B. bei Temperaturen von etwa -50°C bis etwa +100°C, vorzugsweise von etwa -10°C bis etwa 40°C durchgeführt.

Die Reduktion eines 1-Oxids der 3-Cephem-Verbindung, d.h. einer 3-Cephem-Verbindung der Formel I, worin m Null bedeutet, kann in an sich bekannter Weise durch Behandeln mit einem geeignetem Reduktionsmittel, wenn notwendig in Anwesenheit eines aktivierenden Mittels, durchgeführt werden. Geeignete Reduktionsmittel sind beispielsweise: Reduzierend wirkende Zinn-, Eisen-, Kupfer- oder Mangankationen, welche

in Form ihrer Salze, z.B. als Zinn-II-chlorid, -acetat oder -formiat, Eisen-II-chlorid, -sulfat, -oxalat, oder Ammoniumeisensulfat, Kupfer-I-chlorid oder -oxid, oder Mangan-II-chlorid, -sulfat, -acetat oder -oxid, oder als organische oder anorganische Komplexe, z.B. mit Aethylendiamintetraessigsäure oder Nitrolotriessigsäure, verwendet werden; reduzierend wirkende Dithionit-, Jod- oder Eisen-II-cyanid-anionen, welche in Form ihrer anorganischen oder organischen Salze, z.B. als Alkalimetall-, z.B. Natrium- oder Kaliumdithionit, Natrium- oder Kaliumjodid oder Natrium- oder Kaliumeisen-II-cyanid verwendet werden; Jodwasserstoffsäure, reduzierend wirkende trivalente, anorganische oder organische Phosphorverbindungen, wie Phosphine, ferner Ester, Amide und Halogenide der phosphonigen, phosphinigen oder phosphorigen Säure, sowie diesen Phosphor-Sauerstoffverbindungen entsprechende Phosphor-Schwefelverbindungen, worin in diesen Verbindungen organische Reste in erster Linie aliphatische, aromatische oder araliphatische Reste, z.B. gegebenenfalls substituiertes Niederalkyl, Phenyl oder Phenylniederalkyl, darstellen, z.B. Triphenylphosphin, Diphenylphosphonigsäuremethylester, Diphenylchlorphosphin, Phenyldichlorphosphin, Benzolphosphonigsäuredimethylester, Phosphorigsäuretriphenylester, Phosphorigsäuretrimethylester, Phosphortrichlorid, Phosphortribromid, ferner Phosphorigsäuretriphenylester-Halogenaddukte, z.B. Chlor- oder Bromaddukte, worin die Phenylreste gegebenenfalls durch Niederalkyl, z.B. Methyl, Niederalkoxy, z.B. Methoxy, oder durch Halogen, z.B. Chlor, substituiert sind, etc.; reduzierend wirkende Halogensilanverbindungen, die mindestens ein an das Siliciumatom gebundenes Wasserstoffatom aufweisen, und die ausser Halogen, wie Chlor, Brom oder Jod, auch organische Reste, wie aliphatische oder aromatische Gruppen, z.B. gegebenenfalls substituiertes Niederalkyl oder Phenyl, aufweisen können, beispielsweise Diphenylchlorsilan oder Dimethylchlorsilan, sowie auch Halogensilanverbindungen, worin alle Wasserstoffe durch organische Reste ersetzt sind, wie Triniederalkylhalogensilan, z.B. Trimethylchlorsilan oder Trimethyljodsilan, etc.; reduzierend wirkende quaternäre Chlormethylen-iminiumsalze, insbesondere -chloride oder -bromide,

worin die Iminiumgruppen durch einen bivalenten oder zwei monovalente organische Reste, wie gegebenenfalls substituiertes Niederalkylen bzw. Niederalkyl substituiert ist, wie N-Chlormethylen-N,N-dimethyliminiumchlorid oder N-Chlormethylen-pyrrolidiniumchlorid; oder komplexe Metallhydride, wie Natriumborhydrid, in Gegenwart von geeigneten Aktivierungsmitteln, wie Kobalt-II-chlorid, sowie Borandichlorid.

Aktivierende Mittel verwendet man zusammen mit solchen Reduktionsmitteln, welche keine oder nur schwache Lewissäure-Eigenschaften aufweisen. Diese werden in erster Linie zusammen mit Dithionit-, Jod- oder Eisen-II-cyanidsalzen und nicht halogenhaltigen trivalenten Phosphor-Reduktionsmitteln eingesetzt und sind insbesondere organische Carbon- und Sulfonsäurehalogenide, z.B. Phosgen, Oxalylchlorid, Essigsäurechlorid oder -bromid, oder Chloressigsäurechlorid.

Die Reduktion wird vorzugsweise in Gegenwart von Lösungsmitteln oder Gemischen davon durchgeführt, deren Auswahl in erster Linie durch die Löslichkeit der Ausgangsstoffe und die Wahl des Reduktionsmittels bestimmt wird, z.B. gegebenenfalls substituierte, z.B. halogenierte oder nitrierte, aliphatische, cycloaliphatische, aromatische oder araliphatische Kohlenwasserstoffe, z.B. Benzol, Methylenchlorid, Chloroform oder Nitromethan, geeignete Säurederivate, wie Niederalkancarbonsäureester oder -nitrile, z.B. Essigsäureäthylester oder Acetonitril, oder Amide von anorganischen oder organischen Säuren, z.B. Dimethylformamid oder Hexamethylphosphoramid, Aether, z.B. Diäthyläther, Tetrahydrofuran oder Dioxan, Ketone, z.B. Aceton, oder Sulfone, insbesondere aliphatische Sulfone, z.B. Dimethylsulfon oder Tetramethylensulfon, etc., zusammen mit den chemischen Reduktionsmitteln, wobei diese Lösungsmittel vorzugsweise kein Wasser enthalten.

Dabei arbeitet man gewöhnlicherweise bei Temperaturen von etwa -20°C bis etwa 100°C, wobei man bei Verwendung von sehr reaktionsfähigen Reduktionsmitteln bzw. Aktivierungsmitteln die Reaktion auch bei

tieferen Temperaturen durchführt und gegebenenfalls unter Inertgasatmosphäre, z.B. Stickstoffatmosphäre.

Nachoperationen:

In einer erhaltenen Verbindung der Formel I können auf übliche, an sich bekannte Weise noch nicht geschützte funktionelle Gruppen geschützt oder vorhandene Schutzgruppen gegen andere Schutzgruppen ausgetauscht werden, z.B. durch Abspalten der vorhandenen Schutzgruppe und Einführen der gewünschten anderen Schutzgruppe.

$R_1$-Umwandlungen:
In einer erhaltenen Verbindung der Formel I, worin funktionelle Gruppen gegebenenfalls geschützt sind, kann man in an sich bekannter Weise eine Gruppe $R_1$ durch einen anderen Rest $R_1$ ersetzen oder in einen anderen Rest $R_1$ umwandeln. So kann man beispielsweise in einer Verbindung der Formel I, worin $R_1$ eine Gruppe der Formel $-CH_2-R_2$ bedeutet und $R_2$ z.B. einen, durch nucleophile Substituenten ersetzbaren Rest darstellt, oder in einem Salz davon durch Behandeln mit einer Mercaptanverbindung, z.B. einer Heterocyclylmercaptan-Verbindung, oder mit einer Thiocarbonsäureverbindung einen solchen Rest $R_2$ durch eine verätherte Mercaptogruppe, z.B. Heterocyclylmercaptogruppe, bzw. eine veresterte Mercaptogruppe $R_2$ ersetzen.

Ein geeigneter, durch nucleophile Substituenten, z.B. eine verätherte Mercaptogruppe, ersetzbarer Rest ist beispielsweise eine durch eine niederaliphatische Carbonsäure veresterte Hydroxygruppe. Eine solche veresterte Hydroxygruppe ist insbesondere Acetyloxy und Acetoacetoxy.

Die Reaktion einer solchen Verbindung der Formel I mit einer geeigneten Mercaptanverbindung, z.B. Heterocyclylmercaptan-Verbindung, kann unter sauren, neutralen oder schwach basischen Bedingungen durchgeführt werden. Bei sauren Bedingungen arbeitet man in Gegenwart von

konzentrierter Schwefelsäure, welche gegebenenfalls durch ein anorganisches Lösungsmittel, z.B. Polyphosphorsäure, verdünnt wird. Bei neutralen oder schwach basischen Bedingungen führt man die Reaktion in Gegenwart von Wasser und gegebenenfalls einem mit Wasser mischbaren organischen Lösungsmittel durch.

Die basischen Bedingungen können beispielsweise durch Zugabe einer anorganischen Base, wie eines Alkalimetall- oder Erdalkalimetallhydroxids, -carbonats oder -hydrogencarbonats, z.B. durch Zugabe von Natrium-, Kalium- oder Calciumhydroxid, -carbonat oder -hydrogencarbonat, eingestellt werden. Als organische Lösungsmittel können z.B. mit Wasser mischbare Alkohole, z.B. Niederalkanole, wie Methanol oder Aethanol, Ketone, z.B. Niederalkanone, wie Aceton, Amide, z.B. Niederalkancarbonsäureamide, z.B. Dimethylformamid, oder Nitrile, z.B. Niederalkansäurenitrile, z.B. Acetonitril, verwendet werden.

In einer Verbindung der Formel I, worin $R_1$ eine Gruppe der Formel $-CH_2-R_2$ bedeutet, worin $R_2$ freies Hydroxy bedeutet, kann man die freie Hydroxygruppe durch den Acylrest einer gegebenenfalls N-substituierten Carbaminsäure verestern. Die Veresterung der freien Hydroxygruppe mit einer Isocyanat-Verbindung, z.B. Halogensulfonylisocyanat, z.B. Chlorsulfonylisocyanat, oder mit einem Carbaminsäurehalogenid, z.B. Carbaminsäurechlorid, führt zu N-unsubstituierten 3-Carbamoyloxymethyl-Cephalosporinen der Formel I. Die Veresterung der freien Hydroxygruppe mit einer N-substituierten Isocyanat-Verbindung oder mit einer N-mono- oder N,N-disubstituierten Carbaminsäure-Verbindung, z.B. einem entsprechend substituierten Carbaminsäurehalogenid, z.B. einem N-mono- oder N,N-disubstituierten Carbaminsäurechlorid, führt zu N-mono- oder N,N-disubstituierten 3-Carbamoyloxymethyl-Cephalosporinen der Formel I. Man arbeitet üblicherweise in Gegenwart eines Lösungs- oder Verdünnungsmittels und, wenn notwendig, unter Kühlen oder Erwärmen, in einem geschlossenen Gefäss und gegebenenfalls unter Inertgas-, z.B. Stickstoffatmosphäre. Die Verbindung der Formel I, worin $R_1$ eine

Gruppe der Formel $-CH_2-R_2$ bedeutet, worin $R_2$ freies Hydroxy bedeutet, kann man aus einer Verbindung der Formel I durch Abspaltung des Acetylrests aus einer Acetyloxygruppe $R_2$ herstellen, z.B. durch Hydrolyse in schwach-basischem Medium, z.B. in einer wässrigen Natriumhydroxydlösung bei pH 9-10, oder durch Behandeln mit einer geeigneten Esterase, wie einem entsprechenden Enzym aus Rhizobium tritolii, Rhizobium lupinii, Rhizobium japonicum oder Bacillus subtilis, oder einer geeigneten Citrus-Esterase, z.B. aus Orangenschalen.

Ferner kann man eine Verbindung der Formel I, worin $R_1$ eine Gruppe $-CH_2-R_2$ bedeutet, wobei $R_2$ z.B. den oben definierten, durch nucleophile Substituenten ersetzbaren Rest, z.B. Acetyloxy oder Acetacetoxy, darstellt, mit einer organischen Base, insbesondere einer tertiären, Stickstoff-haltigen Base, beispielsweise einem tertiären, aliphatischen Amin, oder vorzugsweise einer tertiären, heterocyclischen, aromatischen Stickstoffbase, z.B. Pyridin oder Pyrimidin mit den weiter vorn genannten Substituenten, unter neutralen oder schwach sauren Bedinngungen, bevorzugt bei einem pH-Wert von etwa 6,5, in Gegenwart von Wasser und gegebenenfalls in einem mit Wasser mischbaren organischen Lösungsmittel umsetzen. Man gelangt so zu Verbindungen der Formel I, worin $R_1$ den Rest der Formel-$CH_2-R_2$ und $R_2$ eine weiter vorn definierte Ammoniogruppe darstellt. Schwach saure Bedingungen können durch Zugabe einer geeigneten organischen oder anorganischen Säure, beispielsweise Essigsäure, Chlorwasserstoffsäure, Phosphorsäure oder Schwefelsäure, eingestellt werden. Als organische Lösungsmittel können beispielsweise die vorstehend genannten, mit Wasser mischbaren Lösungsmittel, verwendet werden. Zur Erhöhung der Ausbeute können der Reaktionsmischung Salze zugesetzt werden, beispielsweise Alkalimetall-, wie Natrium- und insbesondere Kaliumsalze, von anorganischen Säuren, wie Halogenwasserstoffsäure, z.B. Chlorwasserstoff- und insbesondere Jodwasserstoffsäure, sowie der Thiocyansäure, oder von organischen Säuren, wie Niederalkancarbonsäuren, z.B. Essigsäure. Geeignete Salze sind beispielsweise Natriumjodid, Kaliumjodid und Kaliumthiocyanat. Auch Salze von bestimmten Anionenaustauschern, z.B. flüssige Ionenaustauscher in Salzform, wie z.B. Amberlite LA-1 (flüssige sekundäre Amine mit einem Molekulargewicht von 351-393; Oel-löslich und wasserunlöslich; mAeq./g = 2,5-2,7, z.B. in Acetatform), mit Säuren, z.B. Essigsäure, können für diesen Zweck verwendet werden.

Acylierung der freien Aminogruppe:

In einer erhältlichen Verbindung der Formel

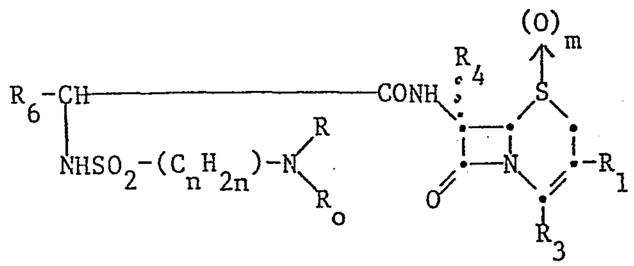

(I'),

worin m, $R_1$, $R_3$, $R_4$ und $R_6$ die unter Formel I genannten Bedeutungen haben, n eine ganze Zahl von 1 bis 4, $R_o$ Wasserstoff oder Niederalkyl und R Wasserstoff bedeuten, kann man die Aminogruppe in an sich bekannter Weise durch gegebenenfalls in Salzform vorliegendes Sulfo oder eine Acylgruppe substituieren.

Diese Substitution kann beispielsweise durch Umsetzung mit einem den Sulforest R oder einem den entsprechenden Acylrest R einführenden Acylierungsmittel erfolgen. Die Carboxylgruppe $R_3$, sowie in $R_1$ und $R_6$ vorhandene funktionelle Gruppen, z.B. Amino- oder Hydroxygruppen, sind durch die weiter vorn genannten Schutzgruppen geschützt.

Wird die Aminogruppe durch den Sulforest R substituiert, verwendet man als Acylierungsmittel beispielsweise einen Schwefeltrioxid-tert.-Amin-Komplex, z.B. den Schwefeltrioxid-Triäthylamin-Komplex.

Wird die Aminogruppe durch die Acylgruppe $R^a$-CO- substituiert, verwendet man als Acylierungsmittel beispielsweise die Carbonsäure $R^a$-CO-OH oder ein reaktionsfähiges, funktionelles Derivat davon. Ein reaktionsfähiges, funktionelles Derivat der Carbonsäure $R^a$-CO-OH ist

beispielsweise ein gemischtes Anhydrid oder ein aktivierter Ester, welcher in der unter Verfahren a) (Acylierung) geschilderten Weise durch Kondensation der Carbonsäure $R^a$-CO-OH mit einer anorganischen Säure, einer Carbonsäure, mit einem Halbester der Kohlensäure, einer Sulfonsäure oder durch Kondensation mit einem vinylogen Alkohol etc. erhalten werden kann.

Wird die Aminogruppe durch die Acylgruppe $R^a$-$SO_2$- substituiert, verwendet man als Acylierungsmittel beispielsweise die Sulfonsäure $R^a$-$SO_2$-OH oder ein reaktionsfähiges, funktionelles Derivat davon. Ein reaktionsfähiges, funktionelles Derivat der Sulfonsäure $R^a$-$SO_2$-OH ist beispielsweise ein gemischtes Anhydrid oder ein aktivierter Ester, welcher in der unter Verfahren b) (Sulfonylierung) geschilderten Weise durch Kondensation der Sulfonsäure $R^a$-$SO_2$-OH mit einer anorganischen Säure, einer Carbonsäure, mit einem Halbester der Kohlensäure, einer anderen Sulfonsäure oder durch Kondensation mit einem vinylogen Alkohol erhalten werden kann.

Bei der Acylierung der freien Aminogruppe mit einer freien Carbonsäure der Formel $R^a$-CO-OH und mit einer freien Sulfonsäure der Formel $R^a$-$SO_2$-OH werden die gleichen Kondensationsmittel, z.B. Carbodiimide, die gleichen Lösungsmittel verwendet und die gleichen Reaktionsbedingungen eingehalten wie bei der Acylierung gemäss Verfahren a).

Bei der Acylierung der freien Aminogruppe mit einem der weiter vorn beschriebenen reaktionsfähigen funktionellen Derivate werden die gleichen Lösungsmittel verwendet und die gleichen Reaktionsbedingungen eingehalten wie bei der Acylierung mit einem reaktionsfähigen, funktionellen Derivat einer Carbonsäure der Formel III gemäss Verfahren a).

Ein solches reaktionsfähiges, funktionelles Derivat ist beispielsweise ein Anhydrid, z.B. ein gemischtes Anhydrid, beispielsweise ein gemischtes Anhydrid mit einer organischen Säure, z.B. einer Halogenwasserstoffsäure, z.B. Chlorwasserstoff, beispielsweise das Acylchlorid, oder ist im Falle der Carbaminsäure oder Thiocarbaminsäure ein inneres Anhydrid, z.B. ein Cyanat oder Thiocyanat.

Wird die Aminogruppe durch eine Acylgruppe der Teilformeln $R^a$-O-CO-, $(R^a)R^bN$-CO-, $(R^a)R^bN$-CS-, $(R^a)R^bN$-$SO_2$-, $(R^a$-CO-$)R^bN$-CO-, $(R^a$-CO-$)R^bN$-CS- oder

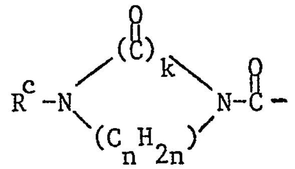

(B)

substituiert, verwendet man als Acylierungsmittel ein reaktionsfähiges funktionelles Derivat des entsprechenden Kohlensäurehalbesters, der entsprechenden Carbaminsäure, Thiocarbaminsäure, Amidosulfonsäure, Acylcarbaminsäure, Acylthiocarbaminsäure oder ein reaktionsfähiges, funktionelles Derivat der Carbonsäure der Formel:

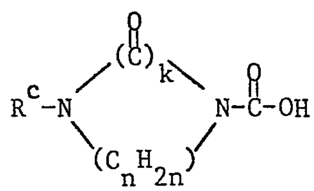

.

Alkylierung der Aminogruppe:

In einer erhältlichen Verbindung der Formel (I'), worin m, $R_1$, $R_3$, $R_4$ und $R_6$ die unter Formel I genannten Bedeutungen haben, n eine ganze Zahl von 1 bis 4, $R_o$ Wasserstoff und R Wasserstoff, gegebenenfalls in Salzform vorliegendes Sulfo oder eine Acylgruppe bedeuten, kann die Aminogruppe in an sich bekannte Weise durch ein den Niederalkylrest $R_o$ oder R einführendes, geeignetes Alkylierungsmittel, beispielsweise ein Alkylhalogenid, z.B. Methylbromid, alkyliert werden.

Umwandlung zum 1-Oxid, 1-Dioxid und 1-Sulfid:

Eine Verbindung der Formel I, worin der Index m 0 bedeutet, kann mit den unter Verfahren c) beschriebenen Oxydationsmitteln in das entsprechende 1-Oxid, worin der Index m den Wert 1 hat, umgewandelt werden.

1-Oxide der Formel I, welche in β-Konfiguration vorliegen, können in an sich bekannter Weise gemäss dem aus der Deutschen Offenlegungsschrift 30 13 996 bekannten Verfahren, und zwar durch Oxydation eines 1-Sulfids der Formeln I oder VI (m=0) mit einer Percarbonsäure, z.B. Peressigsäure oder m-Chlorperbenzoesäure hergestellt werden.

1-Oxide der Formel I, welche in α- oder β-Konfiguration vorliegen, können in an sich bekannter Weise gemäss dem aus der Deutschen Offenlegungsschrift 30 13 996 bekannten Verfahren und zwar durch Oxydation eines 1-Sulfids der Formel II, worin die 7β-Aminogruppe beispielsweise durch Ylidengruppen geschützt ist, die zusammen mit der Aminogruppe eine Schiff'sche Base bilden, mit einer Percarbonsäure, z.B. m-Chlorperbenzoesäure, chromatographischer Trennung der erhaltenen α- und β-1-Oxide der Formel II und anschliessender Acylierung mit einer Carbonsäure der Formel III hergestellt werden.

Eine Verbindung der Formel I, worin der Index m 0 oder 1 bedeutet, kann durch Umsetzung mit Sulfid- oder Sulfoxidgruppen in Sulfongruppen überführenden Oxydationsmitteln in das entsprechende 1-Dioxid, worin m den Wert 2 hat, überführt werden.

Solche Oxydationsmittel sind insbesondere Wasserstoffperoxid, organische Persäuren, insbesondere aliphatische Percarbonsäuren, z.B. Peressigsäure, Perbenzoesäure, Chlorperbenzoesäure, z.B. m-Chlorperbenzoesäure, oder Monoperphthalsäure, oxydierende anorganische Säuren oder

deren Salze, z.B. Salpetersäure, Chromsäure, Kaliumpermanganat, oder Alkalimetallhypochlorit, z.B. Natriumhypochlorit, sowie anodische Oxydation. Die Oxydation wird bevorzugt in einem geeigneten inerten Lösungsmittel, beispielsweise einem Halogenkohlenwasserstoff, z.B. Methylenchlorid, Chloroform oder Tetrachlorkohlenstoff, einem Alkohol, z.B. Methanol oder Aethanol, einem Keton, z.B. Aceton, einem Aether, z.B. Diäthyläther, Dioxan oder Tetrahydrofuran, einem Amid, z.B. Dimethylformamid, einem Sulfon, z.B. Dimethylsulfon, einer flüssigen organischen Carbonsäure, z.B. Essigsäure, oder in Wasser oder einem Gemisch dieser Lösungsmittel, insbesondere einem wasserhaltigen Gemisch, z.B. wässriger Essigsäure, bei Raumtemperatur oder unter Kühlen oder leichtem Erwärmen, d.h. bei etwa -20°C bis etwa +90°C, bevorzugt bei etwa -20°C bis etwa +30°C, durchgeführt. Die Oxydation kann auch stufenweise durchgeführt werden, indem zunächst bei niederer Temperatur, d.h. bei etwa -20°C bis etwa 0°C bis zur Sulfoxidstufe oxydiert wird, das gegebenenfalls isoliert wird, worauf in einem zweiten Schritt, bevorzugt bei höherer Temperatur, etwa bei Raumtemperatur, das Sulfoxid zum Sulfon, d.h. dem 1,1-Dioxid der Formel I, oxidiert wird.

Zur Aufarbeitung kann gegebenenfalls noch vorhandenes überschüssiges Oxydationsmittel durch Reduktion, insbesondere durch Behandlung mit einem Reduktionsmittel, wie einem Thiosulfat, z.B. Natriumthiosulfat, zerstört werden.

Ein 1-Oxid der Formel I, worin der Index m den Wert 1 hat, sowie ein 1-Dioxid, worin der Index m den Wert 2 hat, kann mit den unter Verfahren c) beschriebenen Reduktionsmitteln in das entsprechende 1-Sulfid, worin der Index m den Wert 0 hat, umgewandelt werden.

Abspaltung von Schutzgruppen:

In einer erhältlichen Verbindung der Formel I, worin eine oder mehrere funktionelle Gruppen geschützt sind, können diese, z.B. geschützte Carboxyl-, Amino-, Hydroxy- und/oder Sulfogruppen, in an sich bekannter Weise, mittels Solvolyse, insbesondere Hydrolyse, Alkoholyse oder

Acidolyse, oder mittels Reduktion, insbesondere Hydrogenolyse, gegebenenfalls stufenweise oder gleichzeitig, freigesetzt werden.

Eine geschützte Carboxylgruppe setzt man in an sich bekannter und je nach Art der Schutzgruppen in verschiedenartiger Weise, vorzugsweise mittels Solvolyse oder Reduktion, frei.

So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nukleophilen Verbindung, wie Phenol, Anisol oder Aethylenthioglykol, in freies Carboxyl überführen. Geeignet substituiertes Benzyloxycarbonyl, wie 4-Nitrobenzyloxycarbonyl, kann man durch chemische Reduktion in freies Carboxyl überführen, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoffionen-abgebenden Mittels, das zusammen mit dem Metall oder Metallsalz nascierenden Wasserstoff zu erzeugen vermag, wie einer Säure, in erster Linie einer geeigneten Carbonsäure, wie einer gegebenenfalls, z.B. durch Hydroxyl substituierten Niederalkancarbonsäure, z.B. Essigsäure, Ameisensäure, Glycolsäure, Diphenylglycolsäure, Milchsäure, Mandelsäure, 4-Chlor-mandelsäure oder Weinsäure, oder eines Alkohols oder Thiols, wobei man vorzugsweise Wasser zugibt. Durch Behandeln mit einem reduzierenden Metall oder Metallsalz kann man, wie oben beschrieben, auch 2-Halogenniederalkoxycarbonyl, gegebenenfalls nach Umwandlung einer 2-Bromniederalkoxycarbonylgruppe in eine entsprechende 2-Jodniederalkoxycarbonylgruppe, oder Aroylmethoxycarbonyl in freies Carboxyl umwandeln, wobei Aroylmethoxycarbonyl ebenfalls durch Behandeln mit einem nucleophilen, vorzugsweise salzbildenden Reagenz, wie Natriumthiophenolat oder Natriumjodid, gespalten werden kann. Substituiertes 2-Silyläthoxycarbonyl kann auch

durch Behandeln mit einem, das Fluoridanion lieferenden Salz der Fluorwasserstoffsäure, wie einem Alkalimetallfluorid, z.B. Natrium- oder Kaliumfluorid, in Anwesenheit eines macrocyclisches Polyäthers ("Kronenäther"), oder mit einem Fluorid einer organischen quaternären Base, wie Tetra-niederalkylammoniumfluorid oder Triniederalkylarylammoniumfluorid, z.B. Tetraäthylammoniumfluorid oder Tetrabutylammoniumfluorid, in Gegenwart eines aprotischen polaren Lösungsmittels, wie Dimethylsulfoxid oder N,N-Dimethylacetamid, in freies Carboxyl übergeführt werden. Mit einer organischen Silylgruppe, wie Triniederalkylsilyl, z.B. Trimethylsilyl, verestertes Carboxyl kann üblicherweise solvolytisch, z.B. durch Behandeln mit Wasser, einem Alkohol oder einer Säure, freigesetzt werden.

Eine geschützte Aminogruppe setzt man in an sich bekannter und je nach Art der Schutzgruppen in verschiedenartiger Weise, vorzugsweise mittels Solvolyse oder Reduktion, frei. 2-Halogenniederalkoxycarbonylamino, gegebenenfalls nach Umwandlung einer 2-Bromniederalkoxycarbonylaminogruppe in eine 2-Jodniederalkoxycarbonylaminogruppe, Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silyläthoxycarbonylamino kann durch Behandeln mit einer geeigneten Säure, z.B. Ameisen- oder Trifluoressigsäure, gegebenenfalls substituiertes Triarylmethylamino, Formylamino oder 2-Acetylniederalk-1-en-1-ylamino, z.B. durch Behandeln mit einer Säure, wie Mineralsäure, z.B. Chlorwasserstoffsäure, oder einer organischen Säure, z.B. Ameisen- oder Trifluoressigsäure, gegebenenfalls in Gegenwart von Wasser, und eine mit einer organischen Silylgruppe geschützte Aminogruppe, z.B. mittels

Hydrolyse oder Alkoholyse, gespalten werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsproduktes gespalten werden. Eine durch 2-substituiertes Silyläthoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxylgruppe angegeben, in die freie Aminogruppe übergeführt werden.

In Form einer Azidogruppe geschütztes Amino wird z.B. durch Reduktion in freies Amino übergeführt, durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20°C bis 25°C, oder auch unter Kühlen oder Erwärmen, durchgeführt.

Eine durch eine geeignete Acylgruppe, eine organische Silylgruppe oder durch gegebenenfalls substituiertes 1-Phenylniederalkyl geschützte Hydroxygruppe wird wie eine entsprechend geschützte Aminogruppe freigesetzt. Eine durch 2,2-Dichloracetyl geschützte Hydroxygruppe wird z.B. durch basische Hydrolyse, während eine durch tert.-Niederalkyl oder durch einen 2-oxa- oder 2-thiaaliphatischen oder -cycloaliphatischen Kohlenwasserstoffrest verätherte Hydroxygruppe durch Acidolyse,

z.B. durch Behandeln mit einer Mineralsäure oder einer starken Carbonsäure, z.B. Trifluoressigsäure, freigesetzt.

Eine geschützte, insbesondere veresterte, Sulfogruppe wird analog einer geschützten Carboxylgruppe freigesetzt.

Die beschriebenen Spaltungsreaktionen werden unter an sich bekannten Bedingungen durchgeführt, wenn notwendig unter Kühlen oder Erwärmen, und gegebenenfalls in einer Inertgas-, z.B. Stickstoffatomosphäre.

Bevorzugt werden bei Vorhandensein von mehreren geschützten funktionellen Gruppen die Schutzgruppen so gewählt, dass gleichzeitig mehr als eine solche Gruppe abgespalten werden können, beispielsweise acidolytisch, wie durch Behandeln mit Trifluoressigsäure oder Ameisensäure, oder reduktiv, wie durch Behandeln mit Zink und Essigsäure.

Veresterung einer freien Carboxygruppe: Die Umwandlung einer freien Carboxygruppe, z.B. der freien Carboxygruppe $R_3$, in eine veresterte Carboxygruppe, insbesondere in eine Carboxygruppe, die unter physiologischen Bedingungen spaltbar ist, erfolgt nach an sich bekannten Veresterungsmethoden. Beispielsweise setzt man eine Verbindung der Formel I, worin die zu veresternde Carboxylgruppe in freier Form und andere funktionelle Gruppen, z.B. Amino- oder Hydroxylgruppen, in geschützter Form vorliegen, oder eine Verbindung der Formel I, worin die zu veresternde Carboxygruppe in Form eines reaktionsfähigen, funktionellen Derivates vorliegt, oder ein Salz einer Verbindung der Formel I mit dem entsprechenden Alkohol oder einem reaktionsfähigen, funktionellen Derivat dieses Alkohols um.

Bei der Veresterung einer Verbindung der Formel I, worin die zu veresternde Carboxylgruppe in freier Form vorliegt, mit dem gewünschten Alkohol werden die gleichen Kondensationsmittel, z.B. Carbodiimide, die gleichen Lösungsmittel verwendet und die gleichen Reaktionsbedingungen eingehalten wie bei der Acylierung gemäss Verfahren a).

Eine Verbindung der Formel I, worin die zu veresternde Carboxygruppe in Form eines reaktionsfähigen, funktionellen Derivates vorliegt, ist beispielsweise ein gemischtes Anhydrid oder ein aktivierter Ester, welcher in der unter Verfahren a) (Acylierung) geschilderten Weise durch Kondensation der Carbonsäure der Formel I mit einer anorganischen Säure, einer Carbonsäure, mit einem Halbester der Kohlensäure, einer Sulfonsäure oder durch Kondensation mit einem vinylogen Alkohol erhalten werden kann.

Ein reaktionsfähiges, funktionelles Derivat des zu veresternden Alkohols ist in erster Linie der Ester, welcher durch Kondensation mit einer starken anorganischen oder organischen Säure gebildet wird, beispielsweise das entsprechende Halogenid, z.B. Chlorid, Bromid oder Jodid, oder die entsprechende Niederalkan-, z.B. die Methansulfonyloxy- oder 4-Methansulfonyloxyverbindung.

Bei der Veresterung einer Verbindung der Formel I, worin die zu veresternde Carboxylgruppe in Form eines reaktionsfähigen funktionellen Derivats vorliegt, mit dem entsprechenden Alkohol oder bei der Veresterung einer Verbindung der Formel I, worin die zu veresternde Carboxylgruppe in freier Form vorliegt, mit einem reaktionsfähigen, funktionellen Derivat des entsprechenden Alkohols werden die gleichen Lösungsmittel verwendet und die gleichen Reaktionsbedingungen eingehalten wie bei der Acylierung mit einem reaktionsfähigen, funktionellen Derivat einer Carbonsäure der Formel III gemäss Verfahren a).

Eine Verbindung der Formel I, worin die zu veresternde Carboxylgruppe in Form eines reaktionsfähigen, funktionellen Derivats vorliegt, kann man auch analog zu der im Verfahren a) (Acylierung) beschriebenen Methode in situ herstellen und ohne Isolierung mit dem entsprechenden Alkohol umsetzen.

Salzbildung:

Salze von Verbindungen der Formel I können in an sich bekannter Weise hergestellt werden. So kann man Salze von Verbindungen der Formel I z.B. durch Reaktion der sauren Gruppen mit Metallverbindungen, wie Alkalimetallsalzen von geeigneten Carbonsäuren, z.B. dem Natriumsalz der α-Aethylcapronsäure oder Natriumcarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauschreagenz. Innere Salze von Verbindungen der Formel I können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Bei sämtlichen weiter vorn genannten Umsetzungen, die unter basischen Bedingungen durchgeführt werden, können 3-Cephemverbindungen, gegebenenfalls teilweise, zu 2-Cephemverbindungen isomerisieren. Eine erhaltene 2-Cephemverbindung oder ein Gemisch aus einer 2- und einer 3-Cephemverbindung kann in an sich bekannter Weise zur gewünschten 3-Cephemverbindung isomerisiert werden.

Gemische von Isomeren können in an sich bekannter Weise, z.B. durch fraktionierte Kristallisation, Chromatographie, etc. in die einzelnen Isomeren aufgetrennt werden.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den vorstehend als besonders bevorzugt aufgeführten Verbindungen gelangt.

Pharmazeutische Präparate:

Die pharmakologisch verwendbaren Verbindungen der Formel I, deren Hydrate oder Salze können zur Herstellung von pharmazeutischen Präparaten verwendet werden.

Pharmazeutische Präparate enthalten eine wirksame Menge des reinen Wirkstoffs der Formel I selbst oder eine wirksame Menge des Wirkstoffs der Formel I im Gemisch mit anorganischen oder organischen, festen oder flüssigen, pharmazeutisch verwendbaren Trägerstoffen, die sich vorzugsweise zur parenteralen Verabreichung eignen.

Vorzugsweise verwendet man die Wirkstoffe der Formel I der vorliegenden Erfindung in Form von injizierbaren, z.B. intravenös, verabreichbaren Präparaten oder von Infusionslösungen. Solche Lösungen sind vorzugsweise isotonische wässrige Lösungen oder Suspensionen, welche z.B. aus lyophilisierten Präparaten, welche den reinen Wirkstoff oder den Wirkstoff zusammen mit einem Trägermaterial, z.B. Mannit, enthalten, vor Gebrauch hergestellt werden können. Die pharmazeutischen Präparate sind vorzugsweise sterilisiert und können Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe, z.B. andere

Wirkstoffe, enthalten können, enthalten etwa 0,1 % bis 100 %, insbesondere etwa 1 % bis zu 100 % des Wirkstoffes.

Die pharmazeutischen Präparate werden in an sich bekannter Weise, z.B. mittels konventioneller Lösungs- oder Lyophilisierungsverfahren, hergestellt.

Verwendung:
Verbindungen der Formel I, deren Hydrate oder pharmazeutisch verwendbare Salze können als antibiotisch wirksame Mittel in Form von pharmazeutischen Präparaten in einem Verfahren zur therapeutischen Behandlung des menschlichen oder tierischen Körpers angewendet werden, z.B. zur Behandlung von Infektionen, welche durch grampositive oder gramnegative Bakterien und Kokken, z.B. durch Enterobakterien, z.B. Escherichia coli, Klebsiella pneumoniae oder Proteus spp., verursacht werden.

Je nach Art der Infektionen und Zustand des infizierten Organismus verwendet man tägliche Dosen von etwa 0,5 g bis etwa 5 g s.c., i.v. oder i.m. zur Behandlung von Warmblütern (Menschen und Tiere) von etwa 70 kg Gewicht.
Ausgangsstoffe: Die im Verfahren zur Herstellung der Verbindungen der vorliegenden Erfindung verwendeten Ausgangsstoffe sind bekannt oder, können, falls sie neu sind, in an sich bekannter Weise hergestellt werden.

Ausgangsmaterialien der Formel II, sowie entsprechende Verbindungen mit geschützten funktionellen Gruppen, sind bekannt oder können auf an sich bekannte Weise hergestellt werden.

Verbindungen der Formel III, worin $R_5$ und $R_6$ die unter Formel I genannten Bedeutungen haben, und worin eine in $R_5$ und/oder $R_6$ vorhandene funktionelle Gruppe in freier oder in geschützter Form vorliegt, welche speziell für die Herstellung von Verbindungen der Formel I entwickelt worden sind, sind neu und ebenfalls Gegenstand der vorliegenden

Erfindung. Diese werden beispielsweise hergestellt, indem man in einer Verbindung der Formel:

$$R_6 - \underset{NH_2}{\underset{|}{CH}} - \overset{O}{\overset{\|}{C}} - OH \qquad (VII),$$

worin $R_6$ die unter Formel I genannten Bedeutungen hat und eine in $R_6$ vorhandene funktionelle Gruppe in geschützter Form vorliegt, die 2-Aminogruppe mit einer Sulfonsäure der Formel

$$R_5 - SO_2 - OH \qquad (V),$$

worin $R_5$ die unter Formel I genannte Bedeutung hat und eine in $R_5$ vorhandene funktionelle Gruppe in geschützter Form vorliegt, oder mit einem reaktionsfähigen, funktionellen Säurederivat oder einem Salz davon acyliert und, wenn erwünscht, in einer erhältlichen Verbindung vorhandene Schutzgruppen abspaltet und/oder eine erhältliche Verbindung der Formel III in eine andere Verbindung der Formel III umwandelt.

In einer Verbindung der Formel VII ist eine in $R_6$ vorhandene funktionelle Gruppe, z.B. eine Carboxy-, Amino- oder Hydroxygruppe durch eine weiter vorn genannte Schutzgruppe, z.B. durch eine Carboxyl-, Amino- oder Hydroxylschutzgruppe geschützt. Die 2-Aminogruppe in einer Verbindung der Formel VII ist gegebenenfalls durch eine die Sulfonylierungsreaktion erlaubende Gruppe geschützt. Eine solche Gruppe ist weiter vorn im Verfahren b) (Sulfonylierung) für Ausgangsmaterialien der Formel IV beschrieben.

In einer Verbindung der Formel V ist eine in $R_6$ vorhandene funktionelle Gruppe, z.B. eine Carboxyl-, Amino- oder Hydroxylgruppe durch eine weiter vorn genannte Schutzgruppe, z.B. durch eine Carboxyl-, Amino- oder Hydroxylschutzgruppe, geschützt.

Die Sulfonylierung einer Verbindung der Formel VII mit einer Sulfonsäure der Formel V oder mit einem reaktionsfähigen, funktionellen Derivat davon erfolgt in analoger Weise wie im Verfahren b) (Sulfonylierung) beschrieben.

In einer erhältlichen Verbindung der Formel III mit geschützten funktionellen Gruppen kann eine Schutzgruppe, gegebenenfalls selektiv, abgespalten oder eine, gegebenenfalls bei der Acylierungsreaktion frei gewordene, funktionelle Gruppe geschützt werden.

Verbindungen der Formeln IV und V, sowie entsprechende Verbindungen mit geschützten funktionellen Gruppen, sind bekannt oder können auf an sich bekannte Weise hergestellt werden.

2-Cephem-Verbindungen der Formel VI sind neu. Man kann sie analog dem Acylierungsverfahren a) oder analog dem Sulfonylierungsverfahren b) ausgehend von einer bekannten oder auf an sich bekannte Weise herstellbaren 2-Cephem-Verbindung der Formel

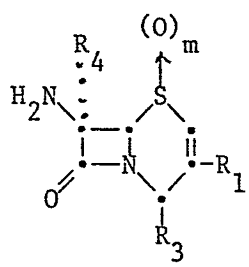

(VIII)

herstellen. Ausserdem können 2-Cephem-Verbindungen der Formel VI als Nebenprodukte bei den Verfahren a) und b) gebildet werden, insbesondere wenn unter basischen Bedingungen gearbeitet wird.

Die folgenden Beispiele dienen zur Illustration der Erfindung; Temperaturen werden in Celsiusgraden angegeben. Die Wellenlängen der UV-Spektren werden in Nanometern (nm) und die $\varepsilon$-Werte in Klammern angegeben. Für die IR-Spektren werden Wellenzahlen ($cm^{-1}$) angegeben.

In den Beispielen werden folgende Abkürzungen verwendet:

BOC: tert.-Butyloxycarbonyl

Cbz: Carbobenzyloxy

F: Schmelzpunkt

DC: Dünnschichtchromatogramm: auf Silicagel-Fertigplatten SL 254 der Fa. Antec, Birsfelden, Schweiz

Rf 96: Rf-Wert im Lösungsmittelsystem sec.-Butanol-Eisessig-Wasser 67:10:23.

Beispiel 1:

a) 3-Acetoxymethyl-7β-[(2R,S)-2-(2-aminothiazol-4-yl)-2-methansulfonylaminoacetamido]-3-cephem-4-carbonsäure-natriumsalz

2,6 g des gemäss Beispiel 1 b) erhältlichen 3-Acetoxymethyl-7β-[(2R,S)-2-(2-BOC-aminothiazol-4-yl)-2-methansulfonylaminoacetamido]-3-cephem-4-carbonsäurediphenylmethylesters werden in einer Mischung von 4 ml $CH_2Cl_2$ und 0,62 ml Anisol bei Zimmertemperatur gelöst. Dann gibt man 10 ml auf 0° vorgekühlte $CF_3COOH$ zu und rührt ohne Kühlung während 45 Minuten. Nach Zugabe von 750 ml Hexan-Aether-(2:1) wird 5 Minuten gerührt, der Niederschlag abgenutscht und mit 100 ml Hexan-Aether(1:1)-Mischung nachgewaschen. Darauf löst man den Filterrückstand in 20 ml Methanol, fügt 100 ml Wasser zu, stellt durch Zugabe von 1 N wässriger Natronlauge auf pH 7 und extrahiert mit Essigester. Die organische Phase wird dreimal mit Wasser gewaschen. Alle wässrigen Phasen werden vereinigt und im Vakuum auf ca. 10 ml eingeengt. Diese Lösung wird an 130 g silyliertem Kieselgel (Antec Opti U.P.C.-12) chromatographiert (Eluiermittel für Frakt. 1-25: Wasser, für die folgenden Fraktionen: Wasser-$CH_3CN$ 95:5; Fraktionsgrösse 25 ml). Die produkthaltigen Fraktionen werden vereinigt, auf ca. 10 ml Volumen eingeengt und in 400 ml Aethanol eingetragen. Das ausgefallene Produkt wird abfiltriert, je zweimal mit Aethanol und Diäthyläther gewaschen und getrocknet. Man erhält das Hydrat der Titelverbindung; F: oberhalb 180° (unter Zersetzung); $[\alpha]_D^{20°} = +101° \pm 1°$ (0,93 % in $H_2O$); IR: 3600-2400 (breit), 1170, 1748 (Schulter), 1730 (Schulter), 1692, 1610, 1530 (Nujol); UV: 253 (12100; $H_2O$).

b) 3-Acetoxymethyl-7β-[(2R,S)-2-(2-BOC-aminothiazol-4-yl)-2-methansulfonylaminoacetamido]-3-cephem-4-carbonsäurediphenylmethylester

3,2 g des gemäss Beispiel 1 c) erhältlichen 3-Acetoxymethyl-7β-[(2R,S)-2-(2-BOC-aminothiazol-4-yl)-2-aminoacetamido]-3-cephem-4-carbonsäurediphenylmethylesters werden in 30 ml absolutem Tetrahydrofuran und 0,37 ml Pyridin 3 Stunden mit 0,39 ml Methansulfochlorid bei Zimmertemperatur gerührt. Dann wird im Vakuum eingeengt,

in Essigester aufgenommen, mit 1 N Salzsäure und NaCl-Lösung gewaschen, mit 1 N $NaHCO_3$-Lösung neutralisiert, nochmals mit NaCl-Lösung gewaschen, über $Na_2SO_4$ getrocknet und im Vakuum eingedampft. Das resultierende Rohprodukt chromatographiert man an 200 g Kieselgel (Fraktionengrösse 50 ml; Eluiermittel: Aether). Durch Vereinigen der produkthaltigen Fraktionen erhält man die Titelverbindung; IR: 3400, 1780, 1715 (breit), 1630, 1525 ($CH_2Cl_2$); UV: 257 (13800; $C_2H_5OH$).

c) <u>3-Acetoxymethyl-7β-[(2R,S)-2-(2-BOC-aminothiazol-4-yl)-2-amino-acetamido]-3-cephem-4-carbonsäurediphenylmethylester</u>

Zu einer Lösung von 5,7 g des gemäss Beispiel 1 d) erhältlichen 3-Acetoxymethyl-7β-[(2R,S)-2-(2-BOC-aminothiazol-4-yl)-2-(2,2,2-trichloräthoxycarbonylamino)-acetamido]-3-cephem-4-carbonsäurediphenylmethylesters in 60 ml eines Essigsäure-Acetonitril-(1:1)-Gemisches gibt man unter Rühren bei 0° im Verlaufe von 10 Minuten portionenweise 5,7 g Zink-Staub zu und rührt anschliessend weitere 3 Stunden bei 0°. Dann wird die Reaktionslösung vom Zinkrückstand abgenutscht, mit Acetonitril nachgewaschen und im Rotationsverdampfer eingeengt. Der Rückstand wird mit Wasser versetzt, mit 2 N NaOH auf pH 8 gestellt, mit Essigester extrahiert und mit NaCl-Lösung neutral gewaschen. Das nach Trocknen über Natriumsulfat und Eindampfen erhaltene Rohprodukt chromatographiert man an 180 g Kieselgel, wobei Fraktionen à 150 ml genommen werden. Eluiermittel: Essigester und Essigester-Methanol(9:1). Durch Vereinigen und Eindampfen der Produkt-haltigen Fraktionen, sowie Umfällung des Eindampfrückstandes aus $CH_2Cl_2$-Hexan, erhält man die Titelverbindung; IR: 3370, 1780, 1740-1690 (breit), 1600 ($CH_2Cl_2$); UV: 257 (10500; $C_2H_5OH$).

d) <u>3-Acetoxymethyl-7β-[(2R,S)-2-(2-BOC-aminothiazol-4-yl)-2-(2,2,2-trichloräthoxycarbonylamino)-acetamido]-3-cephem-4-carbonsäure-diphenylmethylester</u>

4,3 g der gemäss Beispiel 1 e) erhältlichen (2R,S)-2-(2-BOC-Aminothiazol-4-yl)-2-(2,2,2-trichloräthoxycarbonylamino)-essigsäure und 4,2 g

3-Acetoxymethyl-7β-amino-3-cephem-4-carbonsäurediphenylmethylester werden in 50 ml absolutem Tetrahydrofuran zusammen mit 0,8 g Hydroxybenztriazol gelöst. Dann gibt man sofort, sowie nach 1 1/2 und nach 3 Stunden, je 0,71 g Dicyclohexylcarbodiimid in je 60 ml Tetrahydrofuran zu und rührt insgesamt 6 Stunden bei Zimmertemperatur. Das Reaktionsgemisch wird auf 1 Liter Hexan-Aether (9:1) gegossen, abgenutscht und mit Hexan gewaschen. Der Rückstand wird in 1 l Essigester gegeben und gerührt. Der in Essigester unlösliche Dicyclohexylharnstoff wird abfiltriert und die Essigesterlösung nacheinander mit gesättigten Natriumhydrogencarbonat- und Kochsalzlösungen gewaschen. Nach Trocknen über $Na_2SO_4$ und Eindampfen wird das Rohprodukt an 200 g Kieselgel chromatographiert (Fraktionengrösse 150 ml; Eluiermittel: Hexan-Aether 7:3). Dabei erhält man die Titelverbindung; IR 3390, 1780, 1725 (breit), 1690, 1635, 1528 ($CH_2Cl_2$); UV: 259 (13330; $C_2H_5OH$).

e) (2R,S)-2-(2-BOC-Aminothiazol-4-yl)-2-(2,2,2-trichloräthoxycarbonylamino)-essigsäure

5 g des gemäss Beispiel 1 f) erhältlichen (2R,S)-2-(2-BOC-Aminothiazol-4-yl)-2-(2,2,2-trichloräthoxycarbonylamino)-essigsäuremethylesters werden in 50 ml Methanol gelöst. Dann gibt man 25 ml 1 N wässrige NaOH zu und rührt 1/2 Stunde bei Zimmertemperatur. Dann extrahiert man mit Essigester und wäscht zweimal mit Wasser nach. Darauf werden die vereinigten Wasseranteile auf 0° abgekühlt, mit 4 N Salzsäure auf pH 3 gestellt, mit Essigester extrahiert, mit gesättigter wässriger NaCl-Lösung neutral gewaschen, über $Na_2SO_4$ getrocknet und eingedampft. Dabei resultiert die Titelverbindung, die ohne Charakterisierung weiterverarbeitet wird; IR: 3400, 3300-2750 (breit), 1725 (breit), 1540, 1500 ($CH_2Cl_2$).

f) (2R,S)-2-(2-BOC-Aminothiazol-4-yl)-2-(2,2,2-trichloräthoxycarbonylamino)-essigsäuremethylester

8,61 g (2R,S)-2-(2-BOC-Aminothiazol-4-yl)-glycinmethylester werden in einem Gemisch von 85 ml absolutem Tetrahydrofuran und 2,66 ml Pyridin

gelöst. Dann tropft man bei 5-10° eine Lösung von 4,4 ml Chlorameisensäure-2,2,2-trichloräthylester in 50 ml absolutem Tetrahydrofuran zu und rührt 1 Stunde im Eisbad nach. Darauf wird am Rotationsverdampfer eingeengt, in Essigester aufgenommen, mit gesättigter wässriger $NaHCO_3$-Lösung und mit gesättigter NaCl-Lösung bis zum Neutralpunkt gewaschen, über Natriumsulfat getrocknet und eingedampft. Das anfallende Rohprodukt wird an Kieselgel chromatographiert (Fraktionen à 200 ml; Eluiermittel: Hexan-Aether 1:1). Nach Kristallisation der produkthaltigen Fraktionen erhält man die Titelverbindung; IR: 3407, 1750 (Schulter), 1737, 1726 (Schulter), 1540, 1502 ($CH_2Cl_2$); UV: 258 (8806; $CH_3OH$).

Beispiel 2:

a) 7β-[(2R,S)-2-(2-Aminothiazol-4-yl)-2-methansulfonylaminoacetamido]-3-cephem-4-carbonsäurenatriumsalz

2,41 g des gemäss Beispiel 2 b) erhältlichen 7β-[(2R,S)-2-(2-BOC-Aminothiazol-4-yl)-2-methansulfonylaminoacetamido]-3-cephem-4-carbonsäurediphenylmethylesters werden in 6 ml $CH_2Cl_2$ und 2 ml Anisol mit 25 ml Trifluoressigsäure analog Beispiel 1 a) umgesetzt, aufgearbeitet, chromatographiert und umgefällt. Man erhält das Hydrat der Titelverbindung; F: oberhalb 200° (unter Zersetzung); $[\alpha]_D^{20°} = +117° \pm 1°$ (1,05 % in $H_2O$); IR: 3600-2500, 1760, 1680, 1600, 1520 (Nujol); UV 250 (10000; $C_2H_5OH$).

b) 7β-[(2R,S)-2-(2-BOC-Aminothiazol-4-yl)-2-methansulfonylaminoacetamido]-3-cephem-4-carbonsäurediphenylmethylester

1 g des gemäss Beispiel 2 c) erhältlichen 7β-[(2R,S)-2-(2-BOC-Aminothiazol-4-yl)-2-aminoacetamido]-3-cephem-4-carbonsäurediphenylmethylester-p-toluolsulfonsäuresalzes werden bei 0° in 10 ml absolutem Tetrahydrofuran und 0,219 ml Pyridin mit 0,119 ml Methansulfochlorid analog Beispiel 1 b) umgesetzt und aufgearbeitet. Das resultierende Rohprodukt chromatographiert man an 50 g Kieselgel (Eluiermittel: Toluol-Essigester (9:1) und (85:15); Fraktionengrösse 50 ml). Die produkt-

haltigen Fraktionen werden vereinigt und eingedampft. Der Eindampfrückstand wird aus Methylenchlorid-Hexan umgefällt. Man erhält die Titelverbindung; $[a]_D^{20°} = +11°\pm1°$ (1,07% in $CHCl_3$); IR: 3400, 3290, 1780, 1715, 1690 (Schulter), 1630, 1530 ($CH_2Cl_2$); UV: 259 (14300; $C_2H_5OH$).

Man stellt das Ausgangsmaterial folgendermassen her:

c) 7β-[(2R,S)-2-(2-BOC-Aminothiazol-4-yl)-2-aminoacetamido]-3-cephem-4-carbonsäurediphenylmethylester-p-toluolsulfonsäuresalz

7,21 g des gemäss Beispiel 2 d) erhältlichen 7β-[(2R,S)-2-(2-BOC-Aminothiazol-4-yl)-2-BOC-aminoacetamido]-3-cephem-4-carbonsäurediphenylmethylesters werden mit 3,8 g p-Toluolsulfonsäuremonohydrat in 100 ml Acetonitril 8 Stunden bei Zimmertemperatur gerührt. Nach Fällung mit 1000 ml Aether, Absaugen des Niederschlages, Waschen mit 500 ml Aether und Trocknen im Vakuum erhält man die Titelverbindung, welche ohne Charakterisierung weiterverarbeitet wird.

d) 7β-[(2R,S)-2-(2-BOC-Aminothiazol-4-yl)-2-BOC-aminoacetamido]-3-cephem-4-carbonsäurediphenylmethylester

2,21 g des gemäss Beispiel 2 e) erhältlichen (2R,S)-2-(2-BOC-Aminothiazol-4-yl)-2-BOC-aminoessigsäure werden in 50 ml absolutem Tetrahydrofuran gelöst, auf -20° abgekühlt und nacheinander mit 0,756 ml N-Methylmorpholin und 0,728 ml Chlorameisensäureisobutylester versetzt. Dann rührt man 3 Stunden bei -20°, senkt die Temperatur auf -40° ab, fügt 2,0 g 7β-Amino-3-cephem-4-carbonsäurediphenylmethylester in fester Form zu, rührt 10 Minuten bei -40°, sowie 2 1/2 Stunden bei 0° und arbeitet darauf wie folgt auf:
Die Reaktionsmischung wird in Essigester aufgenommen und nacheinander mit 1 N wässriger Salzsäure, gesättigter wässriger $NaHCO_3$-Lösung und NaCl-Lösung bis zum Neutralpunkt gewaschen. Man trocknet die organische Phase über Natriumsulfat, dampft sie im Vakuum ein und chromatographiert das Rohprodukt an 100 g Kieselgel (Eluiermittel: Toluol-Essigester (95:5) und -(4:1); Fraktionengrösse: 100 ml). Die produkt-

haltigen Fraktionen werden vereinigt, eingedampft und der Eindampfrückstand aus Methylenchlorid-Aether umgefällt. Man erhält die Titelverbindung; $[\alpha]_D^{20°} = +11°\pm1°$ (0,86% in $CHCl_2$); IR: 3390, 1778, 1715, 1692, 1635, 1528 ($CH_2Cl_2$); UV: 258 (14 500; $C_2H_5OH$).

e) (2R,S)-2-(2-BOC-Aminothiazol-4-yl)-2-BOC-aminoessigsäure

15 g (2R,S)-2-(2-BOC-Aminothiazol-4-yl)-glycinmethylester werden in einer Mischung von 52,5 ml Methanol und 34,5 ml Wasser mit 105 ml 1 N wässriger Natronlauge 1 Stunde bei Zimmertemperatur gerührt. Dann extrahiert man mit Essigester und wäscht zweimal mit je 50 ml Wasser nach. Die vereinigten wässrigen Phasen versetzt man mit 100 ml Dioxan und 14 g Di-t-butyl-pyrocarbonat und rührt anschliessend 3 Stunden bei Zimmertemperatur, wobei das pH durch Zugabe von 1 N wässriger Natronlauge bei 8 konstant gehalten wird(Titrator). Dann extrahiert man mit Essigester und wäscht dreimal mit Wasser. Die vereinigten Wasseranteile werden bei 0° mit 4 N Salzsäure auf pH 2 gestellt und mit Essigester extrahiert. Die Essigesterphase wäscht man mit Kochsalzlösung neutral, trocknet über Natriumsulfat und dampft im Vakuum ein. Das anfallende Rohprodukt wird aus einem Methanol-Methylenchlorid-Aether-Hexan-Gemisch umkristallisiert. Dabei erhält man die Titelverbindung; F: 168°; IR: 3410, 3300-2800 (breit), 1760 (Schulter), 1725, 1540, 1500 ($CH_2Cl_2$).

Beispiel 3:

a) 7β-[(2R,S)-2-(2-Aminothiazol-4-yl)-2-(4-aminobenzolsulfonylamino)-acetamido]-3-cephem-4-carbonsäure

0,4 g des gemäss Beispiel 3 b) erhältlichen 7β-[(2R,S)-2-(2-BOC-Aminothiazol-4-yl)-2-(4-aminobenzolsulfonylamino)-acetamido]-3-cephem-4-carbonsäurediphenylmethylesters werden in 0,98 ml $CH_2Cl_2$ und 0,3 ml Anisol mit 3,7 ml Trifluoressigsäure analog Beispiel 1 a) umgesetzt, aufgearbeitet, chromatographiert und umgefällt. Man erhält das Hydrat der Titelverbindung; F: oberhalb 205° (unter Zersetzung); $[\alpha]_D^{20°} = +102°\pm1°$ (0,78 % in $H_2O$); IR: 3600-2500 (breit), 1760, 1680, 1620, (Schulter), 1595, 1520 (Nujol); UV: 259

(21000; $H_2O$).

b) 7β-[(2R,S)-2-(2-BOC-Aminothiazol-4-yl)-2-(4-aminobenzolsulfonyl-amino)-acetamido]-3-cephem-4-carbonsäurediphenylmethylester

0,95 g des gemäss Beispiel 3 c) erhältlichen 7β-[(2R,S)-2-(2-BOC-Aminothiazol-4-yl)-2-(4-(2,2,2-trichloräthoxycarbonylamino)-benzol-sulfonylamino)-acetamido]-3-cephem-4-carbonsäurediphenylmethylesters werden in 10 ml Acetonitril-Essigsäure (1:1)-Gemisch mit 0,87 g Zinkstaub analog Beispiel 1c) umgesetzt, aufgearbeitet und chromatographiert. Dabei erhält man die Titelverbindung; $[\alpha]_D^{20°}$ = +15°$\pm$1° (0,77 % in $CHCl_3$); IR: 3390, 3280, 1770, 1835, 1690, 1620, 1590, 1530 ($CH_2Cl_2$); UV: 262 (29800; $C_2H_5OH$).

c) 7β-[(2R,S)-2-(2-BOC-Aminothiazol-4-yl)-2-(4-(2,2,2-trichloräthoxy-carbonylamino)-benzolsulfonylamino)-acetamido]-3-cephem-4-carbonsäurediphenylmethylester

3,27 g des gemäss Beispiel 4a) erhältlichen 7β-[(2R,S)-2-(2-BOC-Aminothiazol-4-yl)-2-aminoacetamido]-3-cephem-4-carbonsäurediphenylmethylester-p-toluolsulfonsäuresalzes werden bei Zimmertemperatur in 30 ml absolutem Tetrahydrofuran und 0,72 ml Pyridin mit 2,58 g 4-(2,2,2-Trichloräthoxycarbonylamino)-benzolsulfochlorid analog Beispiel 1 b) umgesetzt und aufgearbeitet. Das resultierende Rohprodukt chromatographiert man an 100 g Kieselgel (Eluiermittel: Toluol-Essigester (9:1) und (85:15); Fraktionengrösse 100 ml). Die produkthaltigen Fraktionen werden vereinigt, eingedampft und aus Methylenchlorid-Hexan umgefällt. Man erhält die Titelverbindung; $[\alpha]_D^{20°}$ = +7°$\pm$1° (0,94% in $CHCl_3$); IR: 3400, 3280, 1770, 1750, 1735, 1590, 1530 ($CH_2Cl_2$); UV: 254 (36400; $C_2H_5OH$).

d) 4-(2,2,2-Trichloräthoxycarbonylamino)-benzolsulfochlorid

17,3 g Sulfanilsäure werden bei 0° in 100 ml Pyridin aufgeschlämmt. Dann tropft man unter heftigem Rühren bei 0° im Verlaufe von 1 Stunde 15,1 ml Chlorameisensäure-2,2,2-trichloräthylester zu und rührt anschliessend 16 Stunden bei Zimmertemperatur. Dann dampft man im Vakuum bis zur Trockne ein, nimmt anschliessend mehrmals in Toluol auf und dampft jeweils wieder ein. Schliesslich trocknet man den Eindampfrückstand 60 Stunden im Vakuum bei 50°. Dann wird in 250 ml absolutem Chloroform aufgeschlämmt und auf 40° erwärmt. Darauf gibt man unter Rühren bei 40° im Verlaufe von 1 Stunde portionenweise total 33,75 g festes Phosphorpentachlorid zu und erhitzt anschliessend 4 Stunden am Rückfluss. Die abgekühlte Reaktionsmischung wird in 2,7 Liter Toluol aufgenommen, viermal mit Eiswasser gewaschen, über Natriumsulfat getrocknet und eingedampft. Nach Kristallisation des Rohprodukts aus $CH_2Cl_2$-Hexan erhält man die Titelverbindung. F: 90-91°; IR: 1757, 1591, 1525, 1407, 1375 ($CH_2Cl_2$).

Beispiel 4:

a) 7β-[(2R,S)-2-(2-BOC-Aminothiazol-4-yl)-2-aminoacetamido]-3-cephem-4-carbonsäurediphenylmethylester-p-toluolsulfonsäuresalz

2 g 7β-[(2R,S)-2-(2-BOC-Aminothiazol-4-yl)-2-Cbz-aminoacetamido]-3-cephem-4-carbonsäurediphenylmethylester werden in 50 ml absolutem Eisessig in Gegenwart von 2 g 10%igem Palladium/Kohle-Katalysator hydriert. Nach Aufnahme von 1 Aequivalent Wasserstoff (das bei der Hydrierung freigesetzte $CO_2$ wird in wässriger KOH absorbiert) wird die Reaktion abgebrochen, vom Katalysator abfiltriert und im Vakuum zur Trockne eingedampft. Der Eindampfrückstand wird in Essigester aufgenommen und nacheinander mit 1 N wässriger $NaHCO_3$-Lösung und mit NaCl-Lösung bis zum Neutralpunkt gewaschen. Darauf wird über Natriumsulfat getrocknet und eingedampft. Das rohe Amin löst man in 20 ml Acetonitril, gibt 1,0 g p-Toluolsulfonsäuremonohydrat zu und rührt 10 Minuten bei Zimmertemperatur. Nach Fällung mit 500 ml Aether, Absaugen des Niederschlages, Waschen mit 250 ml Aether und Trocknen im Vakuum erhält man die Titelverbindung, die mit dem im Beispiel 2 c) beschriebenen Toluolsulfonsäuresalz identisch ist.

Man stellt das Ausgangsmaterial folgendermassen her:

b) 7β-[(2R,S)-2-(2-BOC-Aminothiazol-4-yl)-2-Cbz-amino-acetamido]-3-cephem-4-carbonsäurediphenylmethylester

4,4 g der gemäss Beispiel 4 c) erhältlichen (2R,S)-2-(2-BOC-Aminothiazol-4-yl)-2-Cbz-aminoessigsäure und 3,63 g 7β-Amino-3-cephem-4-carbonsäurediphenylmethylesters werden analog Beispiel 2 d) umgesetzt (1,38 ml N-Methylmorpholin; 1,33 ml Chlorameisensäureisobutylester; 142 ml Tetrahydrofuran), aufgearbeitet, chromatographiert (Eluiermittel: Toluol-Essigester (9:1 und 4:1); Fraktionengrösse 300 ml) und umgefällt. Man erhält die Titelverbindung; $[\alpha]_D^{20°} = +12° \pm 1°$ (1,04% in $CHCl_3$); IR: 3400, 1790, 1727, 1698, 1638, 1543, 1498 ($CH_2Cl_2$); UV: 258 (15 000; $C_2H_5OH$).

c) (2R,S)-2-(2-BOC-Aminothiazol-4-yl)-2-Cbz-aminoessigsäure

7,4 g des gemäss Beispiel 4 d) erhältlichen (2R,S)-2-(2-BOC-Aminothiazol-4-yl)-2-Cbz-aminoessigsäuremethylesters werden in 270 ml Methanol gelöst. Dann gibt man 60 ml 1 N wässrige Natronlauge zu und rührt 3 Stunden bei Zimmertemperatur. Nach Aufarbeitung analog Beispiel 1 e) erhält man die Titelverbindung; IR: 3410, 3300-2800 (breit), 1760 (Schulter), 1725, 1542, 1503 ($CH_2Cl_2$); UV: 259 (8400; $C_2H_5OH$).

d) (2R,S)-2-(2-BOC-Aminothiazol-4-yl)-2-Cbz-aminoessigsäuremethylester

8,61 g (2R,S)-2-(2-BOC-Aminothiazol-4-yl)-glycinmethylester werden in einem Gemisch von 85 ml absolutem Tetrahydrofuran und 2,66 ml Pyridin gelöst. Dann tropft man unter Rühren bei 0° im Verlaufe von 15 Minuten eine Lösung von 4,74 ml Carbobenzoxychlorid in 50 ml absolutem Dioxan zu und rührt 3 Stunden bei 0° nach. Darauf wird in Essigester aufgenommen, mit 1 N wässriger HCl, mit 1 N wässriger $NaHCO_3$ und mit gesättigter wässriger NaCl-Lösung bis zum Neutralpunkt gewaschen, über Natriumsulfat getrocknet und eingedampft. Das anfallende Rohprodukt wird an 400 g Kieselgel chromatographiert (Fraktionen à 500 ml; Eluiermittel: Toluol-Essigester 95:5). Nach Vereinigung der produkthaltigen Fraktionen erhält man die Titelverbindung; IR: 3410, 1748 (Schulter),

1540, 1502; UV: 258 (8800; $C_2H_5OH$).

Beispiel 5:

a) 7β-[(2R,S)-2-(2-Aminothiazol-4-yl)-2-äthansulfonylaminoacetamido]-3-cephem-4-carbonsäurenatriumsalz

1,367 g des gemäss Beispiel 5 b) erhältlichen 7β-[(2R,S)-2-(2-BOC-Aminothiazol-4-yl)-äthansulfonylaminoacetamido]-3-cephem-4-carbonsäurediphenylmethylesters werden in 3,6 ml $CH_2Cl_2$ und 1,12 ml Anisol mit 13,9 ml Trifluoressigsäure analog Beispiel 1 a) umgesetzt, aufgearbeitet, chromatographiert und umgefällt. Man erhält das Hydrat der Titelverbindung; F: oberhalb 205° (unter Zersetzung); $[\alpha]_D^{20°} = +116°\pm1°$ (0,73 % in $H_2O$); IR: 3600, 2500 (breit), 1760, 1680, 1640 (Schulter), 1600, 1520 (Nujol); UV: 251 (9800; $H_2O$).

b) 7β-[(2R,S)-2-(2-BOC-Aminothiazol-4-yl)-2-äthansulfonylaminoacetamido]-3-cephem-4-carbonsäurediphenylmethylester

3,5 g 7β-[(2R,S)-2-(2-BOC-Aminothiazol-4-yl)-2-aminoacetamido]-3-cephem-4-carbonsäurediphenylmethylester-p-toluolsulfonsäuresalz werden in 40 ml absolutem Tetrahydrofuran in Gegenwart von 1 ml Pyridin mit 0,8 ml Aethansulfochlorid analog Beispiel 1 b) umgesetzt, aufgearbeitet, chromatographiert und umgefällt. Man erhält die Titelverbindung; $[\alpha]_D^{20°} = +18°\pm1°$ (0,97% in $CHCl_3$); IR: 3380, 3280, 1775, 1710, 1690 (Schulter), 1630, 1525 ($CH_2Cl_2$); UV: 258 (14300; $C_2H_5OH$).

Beispiel 6:

a) 7β-[(2R,S)-2-(2-Aminothiazol-4-yl)-2-(2-methylaminocarbonylamino-1,3,4-thiadiazol-5-ylsulfonylamino)-acetamido]-3-cephem-4-carbonsäurenatriumsalz

4,5 g des gemäss Beispiel 6 b) erhältlichen 7β-[(2R,S)-2-(2-BOC-Aminothiazol-4-yl)-2-(2-methylaminocarbonylamino-1,3,4-thiadiazol-5-ylsulfonylamino)-acetamido]-3-cephem-4-carbonsäurediphenylmethylesters wer-

den in 9 ml $CH_2Cl_2$ und 1,27 ml Anisol mit 20 ml Trifluoressigsäure analog Beispiel 1 a) umgesetzt, aufgearbeitet, chromatographiert und umgefällt. Man erhält das Hydrat der Titelverbindung. F: oberhalb 220° (unter Zersetzung); $[\alpha]_D^{20°} = +71° \pm 1°$ (0,93 % in $H_2O$); IR: 3600-2500 (breit), 1766, 1690, 1605, 1520 (Nujol); UV: 262 (14800; $H_2O$).

b) 7β-[(2R,S)-2-(2-BOC-Aminothiazol-4-yl)-2-(2-methylaminocarbonyl-amino-1,3,4-thiadiazol-5-ylsulfonylamino)-acetamido]-3-cephem-4-carbonsäurediphenylmethylester

4,5 g der gemäss Beispiel 6 c) erhältlichen (2R,S)-2-(2-BOC-Aminothiazol-4-yl)-2-(2-methylaminocarbonylamino-1,3,4-thiadiazol-5-ylsulfonylamino)-essigsäure und 3,3 g 7β-Amino-3-cephem-4-carbonsäurediphenylmethylester werden in 50 ml absolutem Tetrahydrofuran zusammen mit 0,8 g Hydroxybenztriazol gelöst. Dann gibt man sofort, sowie nach 1 1/2 und nach 3 Stunden, je 0,71 g Dicyclohexylcarbodiimid in je 60 ml Tetrahydrofuran zu und rührt insgesamt 6 Stunden bei Zimmertemperatur. Das Reaktionsgemisch wird auf 1 Liter Hexan-Aether (9:1)-Gemisch gegossen, abgenutscht und mit Hexan gewaschen. Der Rückstand wird in 1 Liter Essigester gegeben und gerührt. Der im Essigester unlösliche Dicyclohexylharnstoff wird abfiltriert und die Essigesterlösung nacheinander mit gesättigten Natriumhydrogencarbonat- und Kochsalzlösungen gewaschen. Das nach Trocknen und Eindampfen der Essigesterphase anfallende Rohprodukt chromatographiert man an 200 g Kieselgel (Eluiermittel: Aether-Essigester (1:1) und Essigester; Fraktionengrösse 100 ml). Nach Vereinigen und Umfällen der produkthaltigen Fraktionen aus $CH_2Cl_2$-Hexan erhält man die Titelverbindung; $[\alpha]_D^{20°} = +25° \pm 1°$ (1,01% in $CHCl_3$); IR: 3400-2806, 1778, 1700, 1530 ($CH_2Cl_2$); UV: 263 (21600; $C_2H_5OH$).

Das Ausgangsmaterial stellt man folgendermassen her:

c) (2R,S)-2-(2-BOC-Aminothiazol-4-yl)-2-(2-methylaminocarbonylamino-1,3,4-thiadiazol-5-ylsulfonylamino)-essigsäure

2,73 g (2R,S)-2-(2-BOC-Aminothiazol-4-yl)-glycin werden mit 8 ml N,O-

Bis-(trimethylsilyl)-acetamid in 24 ml $CH_2Cl_2$ 1 1/2 Stunden am Rückflusskühler erhitzt, wobei eine klare Lösung entsteht. Dann kühlt man auf 0° ab, gibt nacheinander 0,81 ml Pyridin und 3,8 g 2-Methylaminocarbonylamino-1,3,4-thiadiazol-5-ylsulfochlorid zu und rührt anschliessend 1 Stunde bei 0°, sowie 16 Stunden bei Zimmertemperatur. Dann nimmt man in Essigester auf, wäscht zweimal mit 1 N Salzsäure, sowie viermal mit gesättigter wässriger NaCl-Lösung, trocknet über Natriumsulfat und dampft im Vakuum zur Trockne ein. Die resultierende Titelverbindung wird ohne Charakterisierung weiterverarbeitet (Beispiel 6 b)).

Beispiel 7:

a) 7β-[(2R)-2-(Aminothiazol-4-yl)-2-methansulfonylaminoacetamido]-3-cephem-4-carbonsäurenatriumsalz

1,21 g des gemäss Beispiel 7 b) erhältlichen 7β-[(2R)-2-(2-BOC-Aminothiazol-4-yl)-2-methansulfonylaminoacetamido]-3-cephem-4-carbonsäurediphenylmethylesters werden in 3 ml $CH_2Cl_2$ und 1 ml Anisol mit 12 ml Trifluoressigsäure analog Beispiel 1 a) umgesetzt, aufgearbeitet, chromatographiert und umgefällt. Man erhält das Hydrat der Titelverbindung; F: oberhalb 206° (unter Zersetzung); IR: 3600-2500, 1766, 1680, 1600, 1520 (Nujol); UV: 250 (10500; $H_2O$).

b) 7β-[(2R)-2-(2-BOC-Aminothiazol-4-yl)-2-methansulfonylaminoacetamido]-3-cephem-4-carbonsäurediphenylmethylester

4,36 g des gemäss Beispiel 7 c) erhältlichen 7β-[(2R)-2-(2-BOC-Aminothiazol-4-yl)-2-aminoacetamido]-3-cephem-4-carbonsäurediphenylmethylesters werden mit 0,81 ml Methansulfochlorid in 50 ml absolutem Tetrahydrofuran und 0,56 ml Pyridin analog Beispiel 1 b) beschrieben umgesetzt (Reaktionsdauer 16 Stunden) und aufgearbeitet. Das resultierende Rohprodukt wird analog Beispiel 2 b) chromatographiert. Dabei erhält man die Titelverbindung; $[\alpha]_D^{20°} = -4° \pm 1°$ (0,94% in $CHCl_3$); IR: 3400, 3290, 1780, 1715, 1690 (Schulter), 1630, 1530 ($CH_2Cl_2$); UV: 259

Das Ausgangsmaterial kann man folgendermassen herstellen:

c) 7β-[(2R)-2-(2-BOC-Aminothiazol-4-yl)-2-aminoacetamido]-3-cephem-4-carbonsäurediphenylmethylester

4 g des gemäss Beispiel 7 d) erhältlichen 7β-[(2R)-2-(2-BOC-Aminothiazol-4-yl)-2-chloracetylaminoacetamido]-3-cephem-4-carbonsäurediphenylmethylesters werden mit 0,94 g Thioharnstoff in einem Gemisch von 70 ml Dioxan und 1,37 ml Essigsäure 5 Stunden bei Zimmertemperatur und anschliessend 10 Stunden bei 50° gerührt. Dann verdünnt man mit Essigester, wäscht einmal mit gesättigter wässriger $NaHCO_3$-Lösung, sowie mit gesättigter, wässriger NaCl-Lösung bis zum Neutralpunkt. Die nach Trocknen über $Na_2SO_4$ und Eindampfen erhaltene rohe Titelverbindung wird direkt weiterverarbeitet (Beispiel 7 b)).

d) 7β-[(2R)-2-(2-BOC-Aminothiazol-4-yl)-2-chloracetylaminoacetamido]-3-cephem-4-carbonsäurediphenylmethylester und 7β-[(2S)-2-(2-BOC-Aminothiazol-4-yl)-2-chloracetylaminoacetamido]-3-cephem-4-carbonsäurediphenylmethylester

9,6 g (2R,S)-2-(2-BOC-Aminothiazol-4-yl)-2-chloracetylaminoessigsäure werden mit 9,1 g 7β-Amino-3-cephem-4-carbonsäurediphenylmethylester in 80 ml Tetrahydrofuran analog Beispiel 6 b) umgesetzt (2,4 g Hydroxybenztriazol; dreimal 2,2 g Dicyclohexylcarbodiimid in je 20 ml Tetrahydrofuran) und aufgearbeitet. Das anfallende Rohprodukt wird in 4 gleichen Portionen an je 500 g Kieselgel chromatographiert (Eluiermittel: Toluol-Essigester 85:15-Gemisch; Fraktionen à 25 ml). Dabei eluiert man zuerst die (2R)-Titelverbindung: $[\alpha]_D^{20°} = -5°+1°$ (0,81% in $ChCl_3$); IR: 3380, 3250, 1780, 1715, 1700 (Schulter), 1670, 1510 (breit) ($CH_2Cl_2$); UV: 258 (13500; $C_2H_5OH$).

Die Konfigurationszuordnung am C-2-Kohlenstoffatom der Essigsäure in der Acylseitenkette erfolgt für die (2R)- und die (2S)-Verbindung aufgrund von Drehungsschiebungen und NMR-Vergleichen (CH-7) mit Ureidocephalosporinen; vgl. z.B. H. Breuer et al., J. Antibiot. 31, 546-560 sowie DOS 2 924 296.

Die anschliessend erluierten Fraktionen bestehen aus einem binären

Gemisch der obigen Verbindung mit dem entsprechenden (2S)-Isomeren, das durch nochmaliges Chromatographieren weiter aufgetrennt werden kann. Zuletzt wird reine (2S)-Titelverbindung eluiert; $[\alpha]_D^{20°} = +42°+1°$ (0,82% in $CHCl_3$); IR: 3390, 3270, 1780, 1715, 1700 (Schulter), 1670 (Schulter), 1520 (breit) ($CH_2Cl_2$); UV: 259 (14200; $C_2H_5OH$).

e) (2R,S)-2-(2-BOC-Aminothiazol-4-yl)-2-chloracetylaminoessigsäure

10 g (2R,S)-2-(2-BOC-Aminothiazol-4-yl)-glycinmethylester werden in 35 ml Methanol gelöst und nacheinander mit 23 ml $H_2O$ und 70 ml 1 N wässriger Natronlauge versetzt. Dann rührt man 1 Stunde bei Zimmertemperatur. Anschliessend wird auf 0° abgekühlt und im Verlaufe von 5 Minuten 2,9 ml Chloracetylchlorid zugetropft, wobei das pH durch Zugabe von 2 N Natronlauge (Titrator) bei 10 konstant zu halten ist. Dann wird bei pH 10 und 0° (2 N NaOH; Titrator) noch 2 Stunden gerührt. Zur Aufarbeitung stellt man mit 4N Salzsäure auf pH 2, extrahiert mit Essigester und wäscht 6 mal mit gesättigter, wässriger NaCl-Lösung. Das nach Trocknen über Natriumsulfat und Eindampfen erhaltene Rohprodukt wird aus einer Mischung $CH_3OH$-$CH_2Cl_2$-Hexan umgefällt. Dabei resultiert die Titelverbindung, die direkt weiterverarbeitet wird (Beispiel 7 d)).

Beispiel 8:

a) 7β-[(2S)-2-(2-Aminothiazol-4-yl)-2-methansulfonylaminoacetamido]-3-cephem-4-carbonsäurenatriumsalz

2,4 g des gemäss Beispiel 8 b) erhältlichen 7β-[(2S)-2-BOC-Aminothiazol-4-yl)-2-methansulfonylaminoacetamido]-3-cephem-4-carbonsäurediphenylmethylesters werden in 6 ml $CH_2Cl_2$ und 2 ml Anisol mit 25 ml Trifluoressigsäure analog Beispiel 1 a) umgesetzt, aufgearbeitet, chromatographiert und umgefällt. Man erhält das Hydrat der Titelverbindung; F: oberhalb 200° (unter Zersetzung); $[\alpha]_D^{20°} = +126° \pm 1°$ (0,94 % in $H_2O$); IR: 2600-2500, 1760, 1680, 1600, 1520 (Nujol); UV: 250 (9900; $H_2O$).

b) 7β-[(2S)-2-(2-BOC-Aminothiazol-4-yl)-2-methansulfonylacetamido]-3-cephem-4-carbonsäurediphenylmethylester

4,48 g des gemäss Beispiel 8 c) erhältlichen 7β-[(2S)-2-(2-BOC-Aminothiazol-4-yl)-2-aminoacetamido]-3-cephem-4-carbonsäurediphenylmethylesters werden mit 0,83 ml Methansulfonsäurechlorid in 5 ml Tetrahydrofuran und 0,58 ml Pyridin analog Beispiel 7 b) umgesetzt, aufgearbeitet und chromatographiert. Man erhält die Titelverbindung; $[\alpha]_D^{20°}$ = +36°±1° (0,89% $CHCl_3$); IR: 3400, 3290, 1780, 1715, 1690 (Schulter), 1630, 1530 ($CH_2Cl_2$); UV: 259 (14400: $C_2H_5OH$).

Das Ausgangsmaterial stellt man folgendermassen her:

c) 7β-[(2S)-2-(2-BOC-Aminothiazol-4-yl)-2-aminoacetamido]-3-cephem-4-carbonsäurediphenylmethylester

3,89 g des gemäss Beispiel 7 d) erhältlichen 7β-[(2S)-2-(2-BOC-Aminothiazol-4-yl)-2-chloracetylaminoacetamido]-3-cephem-4-carbonsäurediphenylmethylesters werden mit 0,91 g Thioharnstoff analog Beispiel 7 d) umgesetzt (70 ml Dioxan + 1,34 ml Essigsäure) und aufgearbeitet. Dabei resultiert die rohe Titelverbindung, die direkt weiterverarbeitet wird (Beispiel 8 b)).

Beispiel 9:

a) 7β-[(2R,S)-2-(2-Aminothiazol-4-yl)-2-(2-acetylamino-1,3,4-thiadiazol-5-ylsulfonylamino)-acetamido]-3-cephem-4-carbonsäurenatriumsalz

2 g des gemäss Beispiel 9 b) erhältlichen 7β-[(2R,S)-2-(2-BOC-Aminothiazol)-4-yl)-2-(2-acetylamino-1,3,4-thiadiazol-5-yl-sulfonylamino)-acetamido]-3-cephem-4-carbonsäurediphenylmethylesters werden in 5 ml $CH_2Cl_2$ und 0,57 ml Anisol mit 15 ml Trifluoressigsäure analog Beispiel 1 a) umgesetzt, aufgearbeitet, chromatographiert und umgefällt. Man erhält das Hydrat der Titelverbindung; F: oberhalb 220° (unter Zersetzung); $[\alpha]_D^{20°}$ = +89°±1° (0,92 % $H_2O$); IR: 3600-2500 (breit), 1760, 1690, 1600, 1520 (Nujol); UV: 263 (14800; $H_2O$).

b) 7β-[(2R,S)-2-(2-BOC-Aminothiazol-4-yl)-2-(2-acetylamino-1,3,4-thiadiazol-5-ylsulfonylamino)-acetamido]-3-cephem-4-carbonsäurediphenylmethylester

3 g (2R,S)-2-(2-BOC-Aminothiazol-4-yl)-2-(2-acetylamino-1,3,4-thiadiazol-5-ylsulfonylamino)-essigsäure und 1,83 g 7β-Amino-3-cephem-4-carbonsäurediphenylmethylester werden in 40 ml Tetrahydrofuran analog Beispiel 6 b) umgesetzt (0,8 g Hydroxybenztriazol 3 x 0,47 g Dicyclohexylcarbodiimid in je 40 ml Tetrahydrofuran), aufgearbeitet, chromatographiert und umgefällt. Man erhält die Titelverbindung; $[\alpha]_D^{20°}$ = +28°±1° (0,82% in $CHCl_3$); IR: 3400-2700 (breit), 1778, 1720-1690 (breit), 1525 ($CH_2Cl_2$); UV: 263 (20900; $C_2H_5OH$).

Beispiel 10:

a) 7β-[(2R,S)-2-(2-Aminothiazol-4-yl)-2-((1R,S)-1-cyanoäthansulfonylamino)-acetamido]-3-cephem-4-carbonsäurenatriumsalz

3,18 g des gemäss Beispiel 10 b) erhältlichen 7β-[(2R,S)-2-(2-BOC-Aminothiazol-4-yl)-2-((1R,S)-1-cyanoäthansulfonylamino)-acetamido]-3-cephem-4-carbonsäurediphenylmethylesters werden in 8 ml $CH_2Cl_2$ und 2,5 ml Anisol mit 30 ml Trifluoressigsäure analog Beispiel 1 a) umgesetzt, aufgearbeitet, chromatographiert und umgefällt. Man erhält das Hydrat der Titelverbindung; F: oberhalb 180° (unter Zersetzung); $[\alpha]_D^{20°}$ = +102°±1° (0,91 % in $H_2O$); IR: 3600-2400 (breit), 2255, 1760, 1680, 1640 (Schulter), 1605, 1520 (Nujol); UV: 250 (10000; $H_2O$).

b) 7β-[(2R,S)-2-(2-BOC-Aminothiazol-4-yl)-2-((1R,S)-1-cyanoäthansulfonylamino)-acetamido]-3-cephem-4-carbonsäurediphenylmethylester

3,92 g der gemäss Beispiel 10 c) erhältlichen (2R,S)-2-(2-BOC-Aminothiazol-4-yl)-2-((1R,S)-1-cyanoäthansulfonamido)-essigsäure und 3,3 g 7β-Amino-3-cephem-4-carbonsäurediphenylmethylester werden in 30 ml Tetrahydrofuran analog Beispiel 6 b) umgesetzt (0,915 g Hydroxybenztriazol; 3 x 0,8 g Dicyclohexylcarbodiimid in je 7 ml Tetrahydrofuran) und aufgearbeitet. Nach Chromatographieren des Rohprodukts (Eluiermittel: Toluol-Essigester 9:1, Fraktionengrösse 250 ml), Vereinigen

der produkthaltigen Fraktionen und Umfällung aus $CH_2Cl_2$-Hexan erhält man die Titelverbindung; $[\alpha]_D^{20°} = +8°\pm1°$ (1,02% in $CHCl_3$); IR: 3400, 3300, 2250 (schwach), 1780, 1720, 1700 (Schulter), 1635, 1530 ($CH_2Cl_2$); UV: 258 (14200; $C_2H_5OH$).

Man stellt das Ausgangsmaterial folgendermassen her:

c) (2R,S)-2-(2-BOC-Aminothiazol-4-yl)-2-((1R,S)-1-cyanoäthansulfonyl-amino)-essigsäure

2,73 g (2R,S)-2-(2-BOC-Aminothiazol-4-yl)-glycin werden in 24 ml $CH_2Cl_2$ mit 2,3 g 1-Cyanoäthansulfochlorid analog Beispiel 6 c) umgesetzt (8 ml N,O-Bis-(trimethylsilyl)-acetamid; 0,81 ml Pyridin) und aufgearbeitet. Man erhält die Titelverbindung, die ohne Charakterisierung weiterverarbeitet wird.

Beispiel 11:

a) 3-Acetoxymethyl-7β-[(2R,S)-2-(5-amino-1,2,4-thiadiazol-3-yl)-2-methansulfonylaminoacetamido]-3-cephem-4-carboxylat

Eine Lösung von 1,6 g der gemäss Beispiel 11 b) erhältlichen 3-Acetoxymethyl-7β-[(2R,S)-2-(5-BOC-amino-1,2,4-thiadiazol-3-yl)-2-methansulfonylaminoacetamido]-3-cephem-4-carbonsäure in 3 ml Methylenchlorid und 20 ml Trifluoressigsäure wird bei Raumtemperatur während 30 Minuten gerührt und anschliessend im Vakuum eingeengt. Der Rückstand wird mit Diäthyläther verrieben. Das Produkt wird in 20 ml Methanol gelöst und mit einer 50%igen Lösung von Natrium-2-Aethylhexanoat in Methanol auf pH 7,0 gestellt und mit Aethylacetat versetzt. Das ausgefallene Produkt wird abfiltriert und chromatographiert. Man erhält die Titelverbindung mit einem Rf: ca. 0.60 (Silicagel Opti UPC 12, Wasser-Methanol 95:5); IR (Nujol) 3310, 1765, 1610, 1155.

b) 3-Acetoxymethyl-7β-[2-(5-BOC-amino-1,2,4-thiadiazol-3-yl)-2-methansulfonylaminoacetamido]-3-cephem-4-carbonsäure

2,3 g (6,5 mMol) der gemäss Beispiel 11 c) erhältlichen (2R,S)-2-(5-BOC-Amino-1,2,4-thiadiazol-3-yl)-2-methansulfonylaminoessigsäure und 0,77 ml N-Methylmorpholin werden bei -5° zu einer gerührten Suspension eines Vilsmeyer-Reagenzes, hergestellt aus 0,63 ml Oxalylchlorid und 0,56 ml N,N-Dimethylformamid in 25 ml Methylenchlorid, gegeben. Das Gemisch wird während 30 Minuten bei 0° gerührt und anschliessend auf -10° abgekühlt. Zu diesem Gemisch tropft man eine frisch hergestellte Lösung von 1,69 g (6,2 mMol) 3-Acetoxymethyl-7β-amino-3-cephem-4-carbonsäure in 20 ml Methylenchlorid und 6,8 ml N,O-Bis-(trimethylsilyl)-acetamid und rührt bei 0° unter Stickstoff 2 Stunden. Die Reaktionslösung wird eingeengt, mit Aethylacetat verdünnt und mit verdünnter wässriger Natriumbicarbonatlösung extrahiert. Die wässrigen Extrakte werden mit 2 N HCl auf pH 2,0 gestellt und mit Aethylacetat extrahiert. Die organische Phase wird abgetrennt, mit verdünnter wässriger Natriumchloridlösung gewaschen, über Magnesiumsulfat getrocknet und im Vakuum eingeengt. Man erhält die Titelverbindung mit Rf 96: ca. 0,45 (Silicagel); IR: (Nujol): 1765, 1160.

Man stellt das Ausgangsmaterial folgendermassen her:

c) <u>(2R,S)-2-(5-BOC-Amino-1,2,4-thiadiazol-3-yl)-2-methansulfonylaminoessigsäure</u>

Eine Lösung von 5,0 g (13,6 mMol) des gemäss Beispiel 11 d) erhältlichen (2R,S)-2-(5-BOC-Amino-1,2,4-thiadiazol-3-yl)-2-methansulfonylaminoessigsäuremethylesters in 90 ml 95%igem Aethanol wird mit 4,4 g Kaliumhydroxid und 36,5 ml Wasser versetzt und während 1,5 Stunden bei Raumtemperatur gerührt. Das Reaktionsgemisch wird im Vakuum eingeengt, der Rückstand in Wasser gelöst, die Lösung mit verdünnter Salzsäure auf pH 8,5 gestellt und mit Aethylacetat extrahiert. Die wässrige Phase wird abgetrennt, mit 2 N HCl auf pH 2,0 gestellt und mit Aethylacetat extrahiert. Die organische Phase wird abgetrennt, mit wässriger Natriumchloridlösung gewaschen, über Magnesiumsulfat getrocknet und eingeengt. Man erhält die Titelverbindung vom Rf 96: ca. 0,50 (Silicagel); UV: 217 (5300), 245 (7700, Aethanol).

d) (2R,S)-2-(5-BOC-Amino-1,2,4-thiadiazol-3-yl)-2-methansulfonyl-aminoessigsäuremethylester

Eine Lösung von 8,6 g (29,6 mMol) des gemäss Beispiel 11 e) erhältlichen (2R,S)-2-(5-BOC-Amino-1,2,4-thiadiazol-3-yl)-2-aminoessigsäure-methylesters in 30 ml Methylenchlorid wird bei -5° mit 6 ml Triäthylamin und mit einer Lösung von 2,8 ml Mesylchlorid in 15 ml Methylenchlorid versetzt und während 1,5 Stunden bei 0° gerührt. Das Reaktionsgemisch wird im Vakuum eingeengt, der Rückstand in Aethylacetat aufgenommen und nacheinander mit verdünnter wässriger Natriumbicarbonatlösung, Wasser, 1 N Salzsäure und wässsriger Natriumchloridlösung gewaschen, über Magnesiumsulfat getrocknet und eingeengt. Nach Chromatographieren des Rückstands an Silicagel mit Toluol und steigendem Anteil Aethylacetat erhält man die Titelverbindung vom Rf: 0,35 (Silicagel, Toluol/Aethylacetat 1:1); NMR (60 MHz, $CDCl_3$): 1,56, 2,96, 3,70, 5,65 ppm.

e) (2R,S)-2-(5-BOC-Amino-1,2,4-thiadiazol-3-yl)-2-aminoessigsäuremethylester

Eine Suspension von 25,0 g (82,5 mMol) 2-(5-BOC-Amino-1,2,4-thiadiazol-3-yl)-2-hydroxyiminoessigsäuremethylester und 25 g 10%igem Palladium/Kohle-Katalysator in 250 ml Methanol wird 24 Stunden lang bei Raumtemperatur hydriert. Das Reaktionsgemisch wird über Hyflo-Supercel® filtriert und das Filtrat im Vakuum eingeengt. Man erhält die Titelverbindung mit einem Rf-Wert von 0,15 (Silicagel), Chloroform/Methanol, 95:5); NMR (60 MHz, $CDCl_3$): 1,56, 3,80, 7,07.

Beispiel 12:

a) 7β-[(2R,S)-2-(5-Amino-1,2,4-thiadiazol-3-yl)-2-methansulfonyl-aminoacetamido]-3-cephem-4-carbonsäure-natriumsalz

Eine Lösung von 2,3 g (3,30 mMol) des gemäss Beispiel 12 b) erhältlichen 7β-[(2R,S)-2-(5-BOC-Amino-1,2,4-thaidiazol-3-yl)-2-methansulfonylaminoacetamido]-3-cephem-4-carbonsäurediphenyl-

methylesters in 5 ml Methylenchlorid und 1,7 ml Anisol wird analog Beispiel 11 a) mit 23 ml Trifluoressigsäure versetzt, gerührt und eingeengt. Der Rückstand wird mit Diäthyläther verrieben. Das Produkt wird in 15 ml Methanol gelöst und mit einer 50%igen Lösung von Natrium-2-Aethylhexanoat in Methanol auf pH 7,0 gestellt und mit Aethylacetat versetzt. Das ausgefallene Produkt wird abfiltriert und chromatographiert (Silicagel Opti-UPC 12). Man erhält die Titelverbindung mit einem Rf-Wert von 0,70 (Silicagel Opti-UPC 12, Wasser-Methanol 95:5); IR (Nujol): 3310, 1765, 1600, 1155.

b) 7β-[(2R,S)-2-(5-BOC-Amino-1,2,4-thiadiazol-3-yl)-2-methansulfonylaminoacetamido]-3-cephem-4-carbonsäurediphenylmethylester

Eine Lösung von 1,80 g (5,0 mMol) 2-(5-BOC-Amino -1,2,4-thiadiazol-3-yl)-2-methansulfonylaminoessigsäure und 1,83 g (5 mMol) 7β-Amino-3-cephem-4-carbonsäurediphenylmethylester in 25 ml trockenem Tetrahydrofuran wird bei 0° mit 0,55 g 1-Hydroxybenztriazol und 1,25 g N,N'-Dicyclohexylcarbodiimid in 15 ml trockenem Tetrahydrofuran versetzt, 3,5 Stunden bei 0° gerührt und anschliessend auf Raumtemperatur erwärmt. Nach Filtrieren des Reaktionsgemisches wird das Filtrat mit Aethylacetat verdünnt und nacheinander mit wässriger verdünnter Natriumbicarbonat-salzsäure- und Natriumchloridlösung gewaschen,über Magnesiumsulfat getrocknet und im Vakuum eingeengt. Nach Verreiben des Rückstands mit Aether erhält man die Titelverbindung; Rf: 0,65 (Silicagel, Aethylacetat).

Beispiel 13:

a) 3-Acetoxymethyl-7β-[(2R,S)-2-(2-aminoäthansulfonylamino)-2-(2-aminothiazol-4-yl)-acetamido]-3-cephem-4-carbonsäure

Eine auf 0° gekühlte Lösung von 2,2 g (2,4 mMol) des gemäss Beispiel 13 b) erhältlichen 3-Acetoxymethyl-7β-[(2R,S)-2-(2-BOC-aminoäthansulfonylamino)-2-(2-BOC-aminothiazol-4-yl)-acetamido]-3-cephem-4-carbonsäurediphenylmethylesters und 2,2 ml Anisol in 11 ml absolutem

Methylenchlorid wird mit 11 ml kalter Trifluoressigsäure versetzt, während 90 Minuten bei 25° unter Stickstoffatmosphäre gerührt und anschliessend bei 0° mit 100 ml Diäthyläther versetzt. Der beige Niederschlag wird abfiltriert, mit wenig Diäthyläther gewaschen und nach dem Lösen in 10 ml Wasser mit Essigsäureäthylester (4 x 5 ml) extrahiert. Die auf 0° gekühlte saure wässrige Phase (pH ca. 1.8) wird durch tropfenweise Zugabe von 2N Natriumhydroxidlösung auf pH 5 gestellt und mit 40 ml Aethanol versetzt. Der gebildete Niederschlag wird abfiltriert, mit Aethanol-Wasser (3:1) gewaschen und zur vollständigen Entfernung des organischen Lösungsmittels in ca. 10 ml Wasser aufgeschlämmt und am Rotationsverdampfer eingeengt. Nach dem Trocknen (16 Stunden 25°, 0,05 Torr) wird die Titelverbindung in Form des Monohydrats erhalten; F: oberhalb 210° (unter Zersetzung);

DC (Silicagel, Entwickeln mit Ninhydrin): Rf 96: ca. 0,15, $[\alpha]_D^{20°}$ = +58°±1° (0,959 % in 0,1 N HCl); UV: 250 (15000, 0,1 N HCl).

b) 3-Acetoxymethyl-7β-[(2R,S)-2-(2-BOC-aminoäthansulfonylamino-)-2-(2-BOC-aminothiazol-4-yl)-acetamido]-3-cephem-4-carbonsäure-diphenylmethylester

Zu einer auf 0° abgekühlten Lösung von 14,4 g (30 mMol) der gemäss Beispiel 13 c) erhältlichen (2R,S)-2-(2-BOC-Aminoäthansulfonylamino)-2-(2-BOC-aminothiazol-4-yl)-essigsäure und 4,05 g (30 mMol) 1-Hydroxybenztriazol in 240 ml absolutem Tetrahydrofuran wird analog Beispiel 12 b) eine Lösung von 6,81 g (33 mMol) Dicyclohexylcarbodiimid in 60 ml absolutem Tetrahydrofuran innerhalb ca. 15 Minuten und anschliessend 11,83 g (27 mMol) 3-Acetoxymethyl-7β-amino-3-cephem-4-carbonsäure-diphenylmethylester gegeben. Nach einer Reaktionszeit von 4 Stunden bei Raumtemperatur wird der ausgefallene N,N'-Dicyclohexylharnstoff abfiltriert und das Filtrat am Rotationsverdampfer eingeengt. Man arbeitet analog Beispiel 12 b) auf und erhält die Titelverbindung; Rf: ca. 0,50 (Silicagel, Jod, Methylenchlorid, Essigester 1:1).

Das Ausgangsmaterial stellt man folgendermassen her:

c) (2R,S)-2-(2-BOC-Aminoäthansulfonylamino)-2-(2-BOC-aminothiazol-4-yl)-essigsäure

Zu einer gut gerührten Suspension von (2R,S)-2-(2-Aminoäthansulfonyl-amino-2-(2-BOC-aminothiazol-4-yl)-essigsäure und 11,3 g wasserfreiem Natriumcarbonat in 160 ml Dioxan und 80 ml Wasser werden bei Raumtemperatur 17,1 g Di-tert.-butyldicarbonat auf einmal zugefügt. Nach 2 Stunden wird das auf 0° gekühlte Reaktionsgemisch mit 4 N Salzsäure auf pH∿2 angesäuert und mit Essigester (2 x 300 ml) extrahiert. Die vereinigten organischen Phasen werden mit gesättigter Natriumchloridlösung gewaschen, über Natriumsulfat getrocknet und eingeengt. Der noch ölige Rückstand wird mit Petroläther verrieben, woraus nach Filtration und Trocknen die Titelverbindung als leicht beiges Pulver isoliert werden kann; F: oberhalb 83° (unter Zersetzung); Rf 96: ca. 0,65 (Silicagel, UV 366).

d) (2R,S)-2-(2-Aminoäthansulfonylamino)-2-(2-BOC-aminothiazol-4-yl)-essigsäure

Zu einer Lösung von 25,0 g (45 mMol) (2R,S)-2-(2-(2,2,2-Trichloräthoxycarbonylamino)-äthansulfonylamino)-2-(2-BOC-aminothiazol-4-yl)-essigsäure in 250 ml Essigsäure-Acetonitril 1:1 werden unter starkem Rühren bei 0° im Verlauf von 15 Minuten portionenweise 25 g Zink-Staub zugefügt. Nach einer Reaktionszeit von einer Stunde bei Raumtemperatur werden nochmals 12,5 g Zink-Staub zugegeben. Nach weiteren 3 Stunden wird die Reaktionsmischung vom Zink-Staub abfiltriert und am Rotationsverdampfer eingeengt. Zur Entfernung von überschüssiger Essigsäure wird der Rückstand noch zweimal in ca. 50 ml Toluol aufgeschlämmt, zur Trockne eingeengt und anschliessend mit 250 ml Diäthyläther verrieben. Die so erhaltene Titelverbindung wird ohne weitere Reinigung im nächsten Syntheseschritt (Beispiel 13 c) eingesetzt; Rf 96: ca. 3,38 (Silicagel, UV 366).

e) (2R,S)-2-(2-(2,2,2-Trichloräthoxycarbonylamino)-äthansulfonylamino)-2-(2-BOC-aminothiazol-4-yl)-essigsäure

Eine Suspension von 27,3 g (0,1 Mol) (2R,S)-2-Amino-2-(2-BOC-aminothiazol-4-yl)-essigsäure in 273 ml absolutem Methylenchlorid wird unter Feuchtigkeitsausschluss und Stickstoffatmosphäre unter Rühren mit 80,3 ml (0,33 Mol) und nach 30 Minuten nochmals mit 10 ml N,O-Bis-(trimethylsilyl)-acetamid versetzt. Nach einer Reaktionszeit von insgesamt 2 1/2 Stunden bei 25° wird das klare Reaktionsgemisch auf 0° gekühlt, mit 8,1 ml absolutem Pyridin und 31,9 g (0,1 Mol) 2-(2,2,2-Trichloräthoxycarbonylamino)-äthansulfochlorid, gelöst in 150 ml absolutem Methylenchlorid, versetzt. Nach 2,5 Stunden Reaktionszeit bei Raumtemperatur wird das Lösungsmittel abdestilliert und der in 1 l Essigester gelöste Rückstand mit 2 x 250 ml 1 N Salzsäure und 2 x 200 ml gesättigter Natriumchloridlösung gewaschen. Nach Trocknen der organischen Phase mit Natriumsulfat und Entfernen des Lösungsmittels am Rotationsverdampfer wird die Titelverbindung als blassgelbes Pulver erhalten; Rf 96: ca. 0,80 (Silicagel, UV: 366).

Beispiel 14:

a) 7β-[(2R,S)-2-(2-Aminoäthansulfonylamino)-2-(2-aminothiazol-4-yl)-acetamido]-3-cephem-4-carbonsäure

Analog Beispiel 1 a) oder 13 a) erhält man durch Umsetzen von 2,6 g (3,14 mMol) des gemäss Beispiel 14 b) erhältlichen 7β-[(2R,S)-2-(2-BOC-Aminoäthansulfonylamino)-2-(2-BOC-aminothiazol-4-yl)-acetamido]-3-cephem-4-carbonsäurediphenylmethylesters mit 13 ml Trifluoressigsäure und 2,6 ml Anisol in 13 ml absolutem Methylenchlorid die Titelverbindung in Form des Dihydrats. F: oberhalb 220° (unter Zersetzung); Rf 96: ca. 0,10 (Silicagel, Ninhydrin); Rf: ca. 0,40 und 0,50 (Silicagel Opti-UPC 12, UV 366, Wasser-Acetonitril 9:1); $[\alpha]_D^{20°} = 99° \pm 1°$ (0,524 % in 0,1 N HCl); UV: 250 (12900, 0,1 N HCl).

b) 7β-[(2R,S)-2-(2-BOC-Aminoäthansulfonylamino)-2-(2-BOC-aminothiazol-4-yl)-acetamido]-3-cephem-4-carbonsäurediphenylmethylester

Analog Beispiel 1 b) oder 13 b) erhält man durch Behandeln von 2,4 g (5 mMol) (2R,S)-2-(2-BOC-Aminoäthansulfonylamino)-2-(2-BOC-aminothiazol-4-yl)-essigsäure mit 1,64 g (4,5 mMol) 7β-Amino-3-cephem-4-carbonsäurediphenylmethylester in Gegenwart von 0,68 g 1-Hydroxybenztriazol und 1,13 g (5,5 mMol) N,N'-Dicyclohexylcarbodiimid in 50 ml Tetrahydrofuran die Titelverbindung; Rf: ca. 0,60 (Silicagel, UV 366, Methylenchlorid-Essigester 1:1).

Beispiel 15:

a) 3-(1-Methyl-1H-tetrazol-5-ylthiomethyl)-7β-[(2R,S)-2-(2-aminoäthansulfonylamino)-2-(2-aminothiazol-4-yl)-acetamido]-3-cephem-4-carbonsäure

Analog Beispiel 1 a) oder 13 a) erhält man durch Behandeln von 5,74 g (6 mMol) des gemäss Beispiel 15 b) erhältlichen 3-(1-Methyl-1H-tetrazol-5-ylthiomethyl)-7β-[(2R,S)-2-(2-BOC-aminoäthansulfonylamino)-2-(2-BOC-aminothiazol-4-yl)-acetamido]-3-cephem-4-carbonsäurediphenylmethylesters mit 30 ml Trifluoressigsäure und 5,7 ml Anisol in 30 ml absolutem Methylenchlorid die Titelverbindung in Form des Dihydrats; F: oberhalb 192° (unter Zersetzung); Rf 96: ca. 0,13 (Silicagel, Ninhydrin); $[\alpha]_D^{20°} = +45° \pm 1°$ (0,285 % in 0,1 N Salzsäure); UV: 252 (14000, in 0,1 N HCl).

b) 3-(1-Methyl-1H-tetrazol-5-ylthiomethyl)-7β-[(2R,S)-2-(2-BOC-äthansulfonylamino)-2-(2-BOC-aminothiazol-4-yl)-acetamido]-3-cephem-4-carbonsäurediphenylmethylester

Analog Beispiel 1 b) oder 13 b) erhält man durch Behandeln von 4,8 g (10 mMol) (2R,S)-2-(2-BOC-Aminoäthansulfonylamino)-(2-BOC-aminothiazol-4-yl)-essigsäure mit 4,33 g (9 mMol) 3-(1-Methyl-1H-tetrazol-5-ylthiomethyl)-7β-amino-3-cephem-4-carbonsäurediphenylmethylester in Gegenwart von 1,35 g (10 mMol) 1-Hydroxybenztriazol und 2,27 g (11 mMol)

0092830

N,N'-Dicyclohexylcarbodiimid in 80 ml Tetrahydrofuran als Lösungsmittel die Titelverbindung; Rf: ca. 0,44 und 0,54 (Silicagel UV 366, Aether-Essigester 1:1). Das 2R-, S-Diastereomerengemisch kann chromatographisch an Silicagel in die 2R- und 2S-Komponenten aufgetrennt werden.

Beispiel 16:

a) 3-Methoxy-7β-[(2R,S)-2-(2-aminoäthansulfonylamino)-2-(2-aminothiazol-4-yl)-acetamido]-3-cephem-4-carbonsäure

Analog Beispiel 1 a) oder 13 a) erhält man durch Behandeln von 6,15 g (8,3 mMol) des gemäss Beispiel 16 b) erhältlichen 3-Methoxy-7β-[(2R,S)-2-(2-BOC-aminoäthansulfonylamino)-2-(2-BOC-aminothiazol-4-yl)-acetamido]-3-cephem-4-carbonsäurediphenylmethylesters mit 32 ml Trifluoressigsäure und 6,2 ml Anisol in 32 ml absolutem Methylenchlorid die Titelverbindung in Form des Monohydrats. F: oberhalb 192° (unter Zersetzung); Rf 96: ca. 0,13 (Silicagel, Ninhydrin); $[\alpha]_D^{20°}$ = +110°±1° (0,33 %, in 0,1 N HCl); UV: 250 (13300, in 0,1 N HCl).

b) 3-Methoxy-7β-[(2R,S)-2-(2-BOC-aminoäthansulfonylamino)-2-(2-BOC-aminothiazol-4-yl)-acetamido]-3-cephem-4-carbonsäurediphenylmethylester

Analog Beispiel 1 b) oder 13 b) erhält man durch Behandeln von 4,8 g (10 mMol) (2R,S)-2-(2-Aminoäthansulfonylamino)-2-(2-BOC-aminothiazol-4-yl)-essigsäure mit 3,94 g (9 mMol) 3-Methoxy-7β-amino-3-cephem-4-carbonsäurediphenylmethylester in Gegenwart von 1,35 g 1-Hydroxybenztriazol und 2,27 g (11 mMol) N,N'-Dicyclohexylcarbodiimid in 80 ml Tetrahydrofuran als Lösungsmittel die Titelverbindung;
Rf: ca. 0,45 (Silicagel, UV 366, Methylenchlorid-Essigester 1:1).

Beispiel 17:

a) 3-Methoxy-7β-[(2R,S)-2-(2-aminoäthansulfonylamino)-2-(2-aminothiazol-4-yl)-acetamido]-3-cephem-4-carbonsäure

Zu einer Lösung von 1,01 g (1,2 mMol) der gemäss Beispiel 17 b) erhältlichen 3-Methoxy-7β-[(2R,S)-2-(2-(2,2,2-trichloräthoxycarbonylamino)-äthansulfonylamino)-2-(2-(2,2,2-trichloräthoxycarbonylamino)-thiazol-4-yl)-acetamido]-3-cephem-4-carbonsäure in 15 ml Essigsäure-Acetonitril 1:1 werden unter starkem Rühren bei Raumtemperatur 1,26 g Zink-Staub und nach einer Stunde nochmals 0,63 g Zink-Staub gegeben. Nach weiteren 2 Stunden wird im Rotationsverdampfer eingeengt. Der in 10 ml Wasser gelöste Rückstand wird mit Essigester (2 x 5 ml) extrahiert und die wässrige Phase mit 1 N Natriumhydroxidlösung auf pH 5 gestellt. Der gebildete Niederschlag wird abfiltriert und getrocknet. Die so erhaltene Titelverbindung ist mit dem gemäss Beispiel 16 a) erhaltenen Produkt identisch.

b) 3-Methoxy-7β-[(2R,S)-2-(2-(2,2,2-trichloräthoxycarbonylamino)-äthansulfonylamino)-2-(2-(2,2,2-trichloräthoxycarbonylamino)-thiazol-4-yl)-acetamido]-3-cephem-4-carbonsäure

Eine Lösung von 1,41 g (1,4 mMol) des gemäss Beispiel 17 c) erhältlichen 3-Methoxy-7β-[(2R,S)-2-(2-(2,2,2-trichloräthoxycarbonylamino)-äthansulfonylamino)-2-(2-(2,2,2-trichloräthoxycarbonylamino)-thiazol-4-yl)-acetamido]-3-cephem-4-carbonsäurediphenylmethylesters in 7 ml Methylenchlorid wird mit 7 ml Trifluoressigsäure versetzt und nach 2 Stunden am Rotationsverdampfer eingeengt. Der Rückstand wird zur Entfernung überschüssiger Trifluoressigsäure zweimal in Toluol (20 ml) aufgenommen und eingeengt. Die so erhaltene Titelverbindung kann ohne weitere Reinigung im nächsten Reaktionsschritt eingesetzt werden.
Rf 96: ca. 0,36 (Silicagel, UV 366).

Man stellt das Ausgangsmaterial folgendermassen her:

c) 3-Methoxy-7β-[(2R,S)-2-(2-(2,2,2-trichloräthoxycarbonylamino)-äthansulfonylamino)-2-(2-(2,2,2-trichloräthoxycarbonylamino)-thiazol-4-yl)-acetamido]-3-cephem-4-carbonsäurediphenylmethylester

Eine Lösung von 1,26 g (2,0 mMol) der gemäss Beispiel 17 d) erhältlichen (2R,S)-2-(2-(2,2,2-Trichloräthoxycarbonylamino)-äthansulfonylamino)-2-(2-(2,2,2-trichloräthoxycarbonylamino)-thiazol-4-yl)-essigsäure und 0,714 g (1,8 mMol) 3-Methoxy-7β-amino-3-cephem-4-carbonsäurediphenylmethylester in 20 ml absolutem Tetrahydrofuran in Gegenwart von 0,27 g 1-Hydroxybenztriazol und 0,45 g N,N'-Dicyclohexylcarbodiimid wird während 3,5 Stunden bei Raumtemperatur gerührt. Der gebildete N,N'-Dicyclohexylharnstoff wird abfiltriert und das Filtrat eingeengt. Darauf wird der in 60 ml Essigester gelöste Rückstand mit je 20 ml Eiswasser, 1 N Salzsäure, gesättigter Natriumhydrogencarbonat- und gesättigter Natriumchloridlösung gewaschen. Nach Trocknen der organischen Phase mit Natriumsulfat und Entfernen des Lösungsmittels am Rotationsverdampfer wird der Rückstand an Silicagel mit Methylenchlorid/Essigester 1:1 als Eluiermittel gereinigt, woraus die Titelverbindung als amorphes Pulver erhalten wird. Rf: ca. 0,46 (Silicagel, UV 366, Methylenchlorid-Essigester 1:1).

d) (2R,S)-2-(2-(2,2,2-Trichloräthoxycarbonylamino)-äthansulfonylamino)-2-(2-(2,2,2-trichloräthoxycarbonylamino)-thiazol-4-yl)-essigsäure

Eine Suspension von 1,74 g (5 mMol) (2R,S)-2-Amino-2-(2-(2,2,2-trichloräthoxycarbonylamino)-thiazol-4-yl)-essigsäure in 17 ml absolutem Methylenchlorid wird unter Rühren, Feuchtigkeitsausschluss und Stickstoffatmosphäre mit 2,45 ml N,O-Bis-(trimethylsilyl)-acetamid versetzt. Darauf wird das auf 0° gekühlte Reaktionsgemisch mit 0,4 ml absolutem Pyridin und 1,42 g (5 mMol) 2-(2,2,2-Trichloräthoxycarbonylamino)-äthansulfochlorid versetzt. Das Reaktionsgemisch wird noch 1 1/2 Stunde bei 0° und 4 Stunden bei Raumtemperatur gerührt, anschliessend mit Essigester (60 ml) verdünnt und mit 1 N Salzsäure und gesättigter Natriumchloridlösung gewaschen. Nach Trocknen der organischen Phase über Natriumsulfat und Entfernen des Lösungsmittels am Rotationsverdampfer wird das Rohprodukt an Silicagel mit Essigester als Eluiermittel gereinigt, woraus die Titelverbindung als beiges amorphes Pulver anfällt. Rf 96: ca. 0,75 (Silicagel, UV 366).

Die in den Beispielen 13 a), 13 b), 14 a), 14 b), 15 a) und 15 b) erhaltenen Verbindungen können auch nach dem im Beispiel 17 a) bis 17 d) beschriebenen Verfahren erhalten werden.

Beispiel 18:

3-(1-Carboxymethyl-1H-tetrazol-5-ylthiomethyl)-7β-[(2R,S)-2-(2-aminoäthansulfonylamino)-2-(2-aminothiazol-4-yl)-acetamido]-3-cephem-4-carbonsäure-natriumsalz

Eine Aufschlämmung von 1,07 g (2 mMol) 3-Acetoxymethyl-7β-[(2R,S)-2-(2-aminoäthansulfonylamino)-2-(2-aminothiazol-4-yl)-acetamido]-3-cephem-4-carbonsäure und 0,64 g (4 mMol) 1-Carboxymethyl-5-mercapto-1H-tetrazol in 6 ml Wasser wird durch Zugabe von 1 N Natronlauge auf pH 7 gestellt. Darauf wird die klare Lösung unter Stickstoff während 4 Stunden bei 65° gerührt, abgekühlt und in 300 ml Aethanol eingetragen. Der gebildete Niederschlag wird abfiltriert, in wenig Wasser gelöst und an 25 g silyliertem Silicagel (Antec Opti-$UPC_{12}$) mit Wasser als Eluiermittel gereinigt. Nach Vereinigen der dünnschichtchromatographisch einheitlichen Fraktionen wird das Lösungsmittel am Rotationsverdampfer abdestilliert. Der Rückstand wird im Hochvakuum getrocknet, woraus die Titelverbindung in Form des Dihydrats erhalten wird.

F: oberhalb 150° (unter Zersetzung); Rf: ca. 0,63 (Silicagel Opti-UPC 12, UV 366, Acetonitril-Wasser 1:9); UV: 251 (15000 in 0,1 N HCl).

Beispiel 19:

3-(1-Sulfomethyl-1H-tetrazol-5-ylthiomethyl)-7β-[(2R,S)-2-(2-amino-äthansulfonylamino)-2-(2-aminothiazol-4-yl)-acetamido]-3-cephem-4-carbonsäure-natriumsalz

Analog Beispiel 18 wird ausgehend von 1,07 g (2 mMol) 3-Acetoxymethyl-7β-[(2R,S)-2-(2-aminoäthansulfonylamino)-2-(2-aminothiazol-4-yl)-acetamido]-3-cephem-4-carbonsäure und 1,02 g (4 mMol) 1-Sulfomethyl-5-mercapto-1H-tetrazol-natriumsalz in 6 ml Wasser die Titelverbindung in Form des Dihydrats erhalten; F: oberhalb 180° (unter Zersetzung); Rf: ca. 0,20 (Silicagel Antec Opti-UPC 12, UV 366, Wasser); UV: 252 (14900, 0,1 N HCl).

Beispiel 20:

3-[1-(2-Dimethylaminoäthyl)-1H-tetrazol-5-ylthiomethyl]-7β-[(2R,S)-2-(2-aminoäthansulfonylamino)-2-(2-aminothiazol-4-yl)-acetamido]-3-cephem-4-carbonsäure

Analog Beispiel 18 wird ausgehend von 1,07 g (2 mMol) 3-Acetoxymethyl-7β-[(2R,S)-2-(2-aminoäthansulfonylamino)-2-(2-aminothiazol-4-yl)-acetamido]-3-cephem-4-carbonsäure und 0,69 g (4 mMol) 1-N,N-Dimethyl-aminoäthyl-5-mercapto-1H-tetrazol in 6 ml Wasser die Titelverbindung erhalten. Rf: ca. 0,18 (Silicagel Antec Opti-UPC 12, UV 366, Acetonitril-Wasser 2:8).

Beispiel 21:

a) 7β-[(2R,S)-2-(2-Methansulfonylaminoäthansulfonylamino)-2-(2-aminothiazol-4-yl)-acetamido]-3-cephem-4-carbonsäurenatriumsalz

Eine Lösung von 1,13 g (1,4 mMol) des gemäss Beispiel 21 b) erhältlichen 7β-[(2R,S)-2-(2-Methansulfonylaminoäthansulfonylamino)-2-(2-BOC-aminothiazol-4-yl)-acetamido]-3-cephem-4-carbonsäurediphenylmethylesters und 1,1 ml Anisol in 5,5 ml absolutem Methylenchlorid wird mit 5,5 ml Trifluoressigsäure versetzt, eine Stunde bei Raumtemperatur

unter Feuchtigkeitsausschluss gerührt und anschliessend bei 0° mit 60 ml kaltem Diäthyläther versetzt. Der beige Niederschlag wird abfiltriert, mit wenig Diäthyläther gewaschen, nach Lösen in 20 ml Wasser mit 1 N Natriumhydroxidlösung auf pH 7 gestellt und mit 3 x 10 ml Essigester extrahiert. Die wässrige Phase wird auf ca. 5 ml konzentriert und an silyliertem Silicagel (Antec Opti-UPC 12) mit Wasser als Eluiermittel gereinigt. Die dünnschichtchromatographisch einheitlichen Fraktionen werden vereinigt, am Rotationsverdampfer eingeengt und im Hochvakuum getrocknet, woraus die Titelverbindung in Form des Dihydrats erhalten wird. F: oberhalb 175° (unter Zersetzung); Rf 96: ca. 0,30 (Silicagel, UV 366) UV: 250 (10300, Wasser).

b) 7β-[(2R,S)-2-(2-Methansulfonylaminoäthansulfonylamino)-2-(2-BOC-aminothiazol-4-yl)-acetamido]-3-cephem-4-carbonsäurediphenylmethylester

Zu einer Lösung von 4,0 g (5,5 mMol) des gemäss Beispiel 21c) erhältlichen 7β-[(2R,S)-2-(2-Aminoäthansulfonylamino)-2-(2-BOC-aminothiazol-4-yl)-acetamido]-3-cephem-4-carbonsäurediphenylmethylesters in 60 ml absolutem Methylenchlorid werden unter Rühren und Feuchtigkeitsausschluss 0,48 ml absolutes Pyridin und anschliessend 0,5 ml Methansulfochlorid gegeben. Nach 4 Stunden wird das Reaktionsgemisch mit 300 ml Essigester verdünnt und mit 3 x 50 ml gesättigter Natriumchloridlösung gewaschen, über Natriumsulfat getrocknet und am Rotationsverdampfer eingeengt. Der Rückstand wird an Silicagel (4ofache Menge) mit Methylenchlorid/Essigester 1:1 als Eluiermittel gereinigt, woraus die Titelverbindung als farbloses, amorphes Pulver erhalten wird. Rf: ca. 0,28 (Silicagel, UV 366, Methylenchlorid-Essigester 1:1).

Das Ausgangsmaterial stellt man folgendermassen her:

c) 7β-[(2R,S)-2-(2-Aminoäthansulfonylamino)-2-(2-BOC-aminothiazol-4-yl)-acetamido]-3-cephem-4-carbonsäurediphenylmethylester

Zu einer auf 0° gekühlten Lösung von 5,25 g (5,8 mMol) des gemäss Beispiel 21 d) hergestellten 7β-[(2R,S)-2-(2-(2,2,2-Trichloräthoxycarbonylamino)-äthansulfonylamino)-2-(2-BOC-aminothiazol-4-yl)-acetamido]-3-cephem-4-carbonsäurediphenylmethylesters in 50 ml Essigsäure/Acetonitril (1:1) werden unter Rühren portionenweise 5,25 g Zink-Staub gegeben. Nach 1 Stunde Reaktionszeit bei Raumtemperatur werden nochmals 5 g Zink-Staub zugefügt. Nach insgesamt 5 Stunden Reaktionszeit wird das Reaktionsgemisch mit 200 ml Essigester verdünnt, vom Zink-Staub abfiltriert und mit kalter, gesättigter, wässriger Natriumhydrogencarbonat- und Natriumchloridlösung gewaschen. Nach Trocknen mit Natriumsulfat und Entfernen der Lösungsmittel am Rotationsverdampfer wird die Titelverbindung erhalten, die ohne weitere Reinigung im nächsten Reaktionsschritt eingesetzt werden kann. Rf: ca. 0,58 (Silicagel, UV 366).

d) 7β-[(2R,S)-2-(2-(2,2,2-Trichloräthoxycarbonylamino)-äthansulfonylamino)-2-(2-BOC-aminothiazol-4-yl)-acetamido]-3-cephem-4-carbonsäurediphenylmethylester

Eine Lösung von 11,12 g (20 mMol) der gemäss Beispiel 21 e) hergestellten (2R,S)-2-(2-(2,2,2-Trichloräthoxycarbonylamino)-äthansulfonylamino)-2-(2-BOC-aminothiazol-4-yl)-essigsäure und 7,3 g (20 mMol) 7β-Amino-3-cephem-4-carbonsäurediphenylmethylester in 200 ml absolutem Tetrahydrofuran in Gegenwart von 2,7 g 1-Hydroxybenztriazol und 4,54 g Dicyclohexylcarbodiimid wird während 3 Stunden bei Raumtemperatur gerührt. Darauf wird der gebildete Dicyclohexylharnstoff abfiltriert und das Filtrat eingeengt. Der in Essigester (300 ml) gelöste Rückstand wird mit 1 N Salzsäure, gesättigter Natriumhydrogencarbonat- und Natriumchloridlösung gewaschen. Nach Trocknen der organischen Phase mit Natriumsulfat wird das Lösungsmittel im Rotationsverdampfer entfernt und das anfallende Rohprodukt an Silicagel (40fache Menge) mit Methylenchlorid/Essigester (1:1) als Eluiermittel gereinigt, woraus die Titelverbindung als amorphes Pulver erhalten

wird. DC (Silicagel, Identifikation UV: Rf. ca. 0,65 (Methylenchlorid/Essigester 1:1).

e) 7β-[(2R,S)-2-(2-Aminoäthansulfonylamino)-2-(2-BOC-aminothiazol-4-yl)-acetamido]-3-cephem-4-carbonsäurediphenylmethylester

Eine Lösung von 0,828 g (1 mMol) 7β-[(2R,S)-2-(2-BOC-Aminoäthansulfonylamino)-2-(2-BOC-aminothiazol-4-yl)-acetamido]-3-cephem-4-carbonsäurediphenylmethylester in 10 ml absolutem Methylenchlorid wird unter Rühren bei Raumtemperatur mit 0,38 g p-Toluolsulfonsäure Monohydrat versetzt. Nach 5 Stunden wird das Reaktionsgemisch mit Diäthyläther (30 ml) verdünnt und mit gesättigter Natriumhydrogencarbonat- und Natriumchloridlösung gewaschen. Die organische Phase wird getrocknet (Natriumsulfat)und am Rotationsverdampfer eingeengt. Die so erhaltene Titelverbindung ist mit dem gemäss Beispiel 21 c) erhaltenen Produkt identisch.

Beispiel 22:

a) 3-(1-Methyl-1H-tetrazol-5-ylthiomethyl)-7β-[(2R,S)-2-(2-aminothiazol-4-yl)-2-methansulfonylaminoacetamido]-3-cephem-4-carbonsäurenatriumsalz

3,5 g des gemäss Beispiel 22 d) erhältlichen 3-(1-Methyl-1H-tetrazol-5-ylthiomethyl)-7β-[(2R,S)-2-(2-BOC-aminothiazol-4-yl)-2-methansulfonylaminoacetamido]-3-cephem-4-carbonsäurediphenylmethylesters werden in 7,5 ml $CH_2Cl_2$ und 2,45 ml Anisol mit 28 ml Trifluoressigsäure analog Beispiel 1 a) umgesetzt, aufgearbeitet, chromatographiert und umgefällt. Man erhält das Hydrat der Titelverbindung; F: oberhalb 165° (unter Zersetzung); $[\alpha]_D^{20°} = -9° \pm 1°$ (0,80 % in $H_2O$); IR: 3700-2500 (breit), 1762, 1685, 1603, 1521 (Nujol); UV: 260 (13600; $H_2O$).

b) 3-(1-Methyl-1H-tetrazol-5-ylthiomethyl)-7β-[(2R)-2-(2-aminothiazol-4-yl)-2-methansulfonylaminoacetamido]-3-cephem-4-carbonsäure-natriumsalz

2,18 g des gemäss Beispiel 22 e) erhältlichen 3-(1-Methyl-1H-tetrazol-5-ylthiomethyl)-7β-[(2R)-2-(2-BOC-aminothiazol-4-yl)-2-methansulfonylaminoacetamido]-3-cephem-4-carbonsäurediphenylmethylesters werden in 4,5 ml $CH_2Cl_2$ und 1,52 ml Anisol mit 17,4 ml Trifluoressigsäure analog Beispiel 1 a) umgesetzt, aufgearbeitet, chromatographiert und umgefällt. Man erhält das Hydrat der Titelverbindung; F: oberhalb 168° (unter Zersetzen); $[\alpha]_D^{20°}$ = -22°+1° (0,96% in $H_2O$); IR: 3700-2500 (breit), 1762, 1685, 1602, 1521 (Nujol); UV: 260 (3500; $H_2O$).

c) 3-(1-Methyl-1H-tetrazol-5-ylthiomethyl)-7β-[(2S)-2-(2-aminothiazol-4-yl)-2-methansulfonylaminoacetamido]-3-cephem-4-carbonsäure-natriumsalz

2,42 g des gemäss Beispiel 22 e) erhältlichen 3-(1-Methyl-1H-tetrazol-5-ylthiomethyl)-7β-[(2S)-2-(2-BOC-aminothiazol-4-yl)-2-methansulfonylaminoacetamido]-3-cephem-4-carbonsäurediphenylmethylesters werden in 5 ml $CH_2Cl_2$ und 1,69 ml Anisol mit 19,3 ml Trifluoressigsäure analog Beispiel 1 a) umgesetzt, aufgearbeitet, chromatographiert und umgefällt. Man erhält das Hydrat der Titelverbindung; F: oberhalb 168° (unter Zersetzung); $[\alpha]_D^{20°}$ = +8°±1+ (0,85% in $H_2O$); IR: 3700-2500 (breit), 1762, 1685, 1603, 1521 (Nujol); UV: 260 (13700, $H_2O$).

d) 3-(1-Methyl-1H-tetrazol-5-ylthiomethyl)-7β-[(2R,S)-2-(2-BOC-aminothiazol-4-yl)-2-methansulfonylaminoacetamido]-3-cephem-4-carbonsäurediphenylmethylester

3 g der gemäss Beispiel 22 f) erhältlichen (2R,S)-2-(2-BOC-Aminothiazol-4-yl)-2-methansulfonylaminoessigsäure werden mit 3,5 g 3-(1-Methyl-1H-tetrazol-5-ylthiomethyl)-7β-amino-3-cephem-4-carbonsäurediphenylmethylester in 40 ml Tetrahydrofuran analog Beispiel 6b) umgesetzt (0,76 g Hydroxybenztriazol; dreimal 0,7 g Dicyclohexylcarbodiimid in je 5 ml Tetrahydrofuran), aufgearbeitet, und chromatographiert. Man erhält die Titelverbindung; $[\alpha]_D^{20°} = -99°\pm1°$ (0,75% in $CHCH_3$); IR: 3400, 3300, 1780, 1720, 1695 (Schulter), 1600 (schwach), 1525 ($CH_2Cl_2$); UV: 260 (16000; EtOH).

e) 3-(1-Methyl-1H-tetrazol-5-ylthiomethyl)-7β-[(2R)-2-(2-BOC-aminothiazol-4-yl)-2-methansulfonylaminoacetamido]-3-cephem-4-carbonsäurediphenylmethylester
und
3-(1-Methyl-1H-tetrazol-5-ylthiomethyl)-7β-[(2S)-2-(2-BOC-aminothiazol-4-yl)-2-methansulfonylaminoacetamido]-3-cephem-4-carbonsäurediphenylmethylester

Das gemäss Beispiel 22 d) erhaltene binäre Gemisch der beiden Titelverbindungen chromatographiert man an 300 g Kieselgel (Stufensäule; Eluiermittel: Toluol-Essigester 4:1, 7:3 und 1:1 -Gemisch). Dabei eluiert man zuerst die Titelverbindung mit der 2R-Konfiguration (zur Konfigurationszuordnung vgl. Beispiel 7 d); $[\alpha]_D^{20°} = -126°\pm1°$ (0,75% in $CHCl_3$); IR: 3400, 3300, 1780, 1720, 1695 (Schulter), 1600 (schwach), 1525 ($CH_2Cl_2$); UV: 260 (16100; EtOH).

Die Folgefraktionen bestehen aus einem binären Gemisch der (2R)- und (2S)-Titelverbindungen. Zuletzt eluiert man die einheitliche (2S)-Titelverbindung; $[\alpha]_D^{20°} = -86°\pm1°$ (0,93% in $CHCl_3$); IR: 3400, 3300, 1780, 1720, 1695 (Schulter), 1600 (schwach), 1525 ($CH_2Cl_2$); UV: 260 (16200; EtOH).

Das Ausgangsmaterial stellt man folgendermassen her:

f) (2R,S)-2-(2-BOC-Aminothiazol-4-yl)-2-methansulfonylaminoessigsäure

5 g (2R,S)-2-(2-BOC-Aminothiazol-4-yl)-glycin werden in 45 ml $CH_2Cl_2$ mit 2,14 ml Mesylchlorid analog Beispiel 6 c) umgesetzt (15 ml N,O-Bis-(trimethylsilyl)-acetamid; 1,45 ml Pyridin) und aufgearbeitet. Man erhält die Titelverbindung, welche ohne Charakterisierung gemäss Beispiel 22 d) weiterverarbeitet wird.

Beispiel 23:

3-(4-Carbamoylpyridiniomethyl)-7β-[(2R,S)-2-(2-aminothiazol-4-yl)-2-methansulfonylaminoacetamido]-3-cephem-4-carbonsäure

2,81 g 3-Acetoxymethyl-7β-[(2R,S)-2-(2-aminothiazol-4-yl)-2-methansulfonylaminoacetamido]-3-cephem-4-carbonsäurenatriumsalz (Herstellung siehe Beispiel 1 a), 0,95 g Isonicotinamid, 8,6 g Natriumjodid und 0,94 g Trichloressigsäure werden in 5,75 ml Wasser 1 1/2 Stunde auf 70° erwärmt. Dann wird abgekühlt und in 1 Liter Aethanol eingetragen. Der dabei entstehende Niederschlag wird abfiltriert, mit Aether gewaschen und getrocknet. Anschliessend löst man in wenig Wasser und extrahiert die Wasserphase mit je 175 ml flüssigem Ionenaustauscher LA 1 (HOAC-Form),Hexan und Essigester (2 x). Darauf wird die Wasserphase mit 1 N NaOH auf pH 6,8 gestellt und im Vakuum eingeengt. Anschliessend stellt man 1 N Salzsäure auf pH 2,2 und chromatographiert an 100 g silyliertem Kieselgel (Antec Opti-UPC$_{12}$), Wasser- Acetonitril (95:5). Die produkthaltigen Fraktionen werden vereinigt, auf ca. 5 ml Volumen eingeengt und in 400 ml Aethanol eingetragen. Das ausgefallene Produkt wird abfiltriert, mit Aethanol und Diäthyläther gewaschen und getrocknet. Man erhält das Hydrat der Titelverbindung; F: oberhalb 160° (unter Zersetzung); $[\alpha]_D^{20°} = -3°\pm1°$ (0,78% in $H_2O$); IR: 2700-2500 (breit), 1779, 1688, 1610, 1568, 1522 (Nujol); UV: 260 (13600; $H_2O$).

Beispiel 24:

a) 7β-[(2R,S)-2-(2-Aminothiazol-4-yl)-2-(2-acetylaminoäthansulfonylamino)-acetamido]-3-cephem-4-carbonsäurenatriumsalz

1,7 g des gemäss Beispiel 24 b) herstellbaren 7β-[(2R,S)-2-(2-BOC-Aminothiazol-4-yl)-2-(2-acetylaminoäthansulfonylamino)-acetamido]-3-cephem-4-carbonsäurediphenylmethylesters werden in 4 ml $CH_2Cl_2$ und 1,28 ml Anisol mit 15 ml Trifluoressigsäure analog Beispiel 1 a) umgesetzt, aufgearbeitet und umgefällt. Man erhält die Titelverbindung; F: oberhalb 200° (unter Zersetzung); $[\alpha]_D^{20°} = +98°\pm1°$ (0,84 % in $H_2O$); IR: 3700-2600 (breit), 1760, 1650 (breit), 1600, 1521 (Nujol); UV: 251 (10200), 312 (1100; $H_2O$).

b) 7β-[(2R,S)-2-(2-BOC-Aminothiazol-4-yl)-2-(2-acetylaminoäthansulfonylamino)-acetamido]-3-cephem-4-carbonsäure-diphenylmethylester

2,21 g der gemäss Beispiel 24 c) herstellbaren (2R,S)-2-(2-BOC-Aminothiazol-4-yl)-2-(2-acetylaminoäthansulfonylamino)-essigsäure werden mit 1,9 g 7β-Amino-3-cephem-4-carbonsäurediphenylmethylester in 20 ml Tetrahydrofuran analog Beispiel 6 b) umgesetzt (0,53 g Hydroxybenztriazol; dreimal 0,46 g Dicyclohexylcarbodiimid in je 4 ml Tetrahydrofuran), aufgearbeitet und chromatographiert. Man erhält die Titelverbindung; $[\alpha]_D^{20°} = +23° \pm 1°$ (0,80% in $CHCl_3$); IR: 3400, 3300, 1781, 1720, 1675, 1529 ($CH_2Cl_2$); UV: 258 (13600; EtOH).

Herstellung des Ausgangsmaterials:

c) (2R,S)-2-(2-BOC-Aminothiazol-4-yl)-2-(2-acetylaminoäthansulfonylamino)-essigsäure

6,7 g (2R,S)-2-(2-BOC-Aminothiazol-4-yl)-2-(2-aminoäthansulfonylamino)-essigsäure werden in 24 ml $CH_2Cl_2$ mit 0,71 ml Acetylchlorid analog zu Beispiel 6 c) umgesetzt (10 ml N,O-Bis-(trimethylsilyl)-acetamid; 0,81 ml Pyridin) und aufgearbeitet. Man erhält die Titelverbindung, die ohne Charakterisierung gemäss Beispiel 24 b) weiterverarbeitet wird.

Beispiel 25:

a) 3-Acetoxymethyl-7β-[(2R,S)-2-(2-aminothiazol-4-yl)-2-(2-acetylaminoäthansulfonylamino)-acetamido]-3-cephem-4-carbonsäure-natriumsalz

1,32 g des gemäss Beispiel 25 b) erhältlichen 3-Acetoxymethyl-7β-[(2R,S)-2-(2-BOC-aminothiazol-4-yl)-2-(2-acetylaminoäthansulfonylamino)-acetamido]-3-cephem-4-carbonsäurediphenylmethylesters werden in 3,2 ml $CH_2Cl_2$ und 1 ml Anisol mit 12 ml Trifluoressigsäure analog Beispiel 1 a) umgesetzt, aufgearbeitet und umgefällt. Man erhält das Hydrat der Titelverbindung; F: oberhalb 160° (unter Zersetzung); $[\alpha]_D^{20°} = +61° \pm 1°$ (0,79 % in $H_2O$); IR: 3700-2500 (breit), 1762, 1680 (Schulter), 1605, 1624 (Nujol); UV: 258 (12700; EtOH).

b) 3-Acetoxymethyl-7β-[(2R,S)-2-(2-BOC-aminothiazol-4-yl)-2-(2-acetylaminoäthansulfonylamino)-acetamido]-3-cephem-4-carbonsäurediphenylmethylester

1,7 g (2R,S)-2-(2-BOC-Aminothiazol-4-yl)-2-(2-acetylaminoäthansulfonylamino)-essigsäure (Herstellung s. Beispiel 24 c)) werden mit 1,46 g 3-Acetoxymethyl-7β-amino-3-cephem-4-carbonsäurediphenylmethylester in 16 ml Tetrahydrofuran analog Beispiel 6 b) umgesetzt (0,4 g Hydroxybenztriazol; dreimal 0,35 g Dicyclohexylcarbodiimid in je 3 ml Tetrahydrofuran), aufgearbeitet und chromatographiert. Man erhält die Titelverbindung; $[\alpha]_D^{20°} = +6° \pm 1°$ (0,71% in $CHCl_3$); IR: 3400, 3300, 1787, 1725, 1695, 1677, 1540 $cm^{-1}$ ($CH_2Cl_2$); UV: 258 (14900; EtOH).

Beispiel 26:

a) 7β-[(2R,S)-2-(2-Aminothiazol-4-yl)-2-(2-benzoylaminoäthansulfonylamino)-acetamido]-3-cephem-4-carbonsäurenatriumsalz

1,46 g des gemäss 26 b) erhältlichen 7β-[(2R,S)-2-(2-BOC-Aminothiazol-4-yl)-2-(2-benzoylaminoäthansulfonylamino)-acetamido]-3-cephem-4-carbonsäurediphenylmethylesters werden in 3,2 ml $CH_2Cl_2$ und 1,0 ml Anisol mit 12 ml Trifluoressigsäure analog Beispiel 1 a) umgesetzt, aufgearbeitet und umgefällt. Man erhält das Hydrat der Titelverbindung; F: oberhalb 185° (unter Zersetzung); $[\alpha]_D^{20} = +79° \pm 1°$ (0,91 % in $H_2O$); IR: 3700-2500, 1760, 1680 (Schulter), 1640, 1600, 1577, 1525 (Nujol); UV: 230 (17300; $H_2O$).

b) 7β-[(2R,S)-2-(2-BOC-Aminothiazol-4-yl)-2-(2-benzoylaminoäthansulfonylamino)-acetamido]-3-cephem-4-carbonsäurediphenylmethylester

2,39 g der gemäss Beispiel 26 c) erhältlichen(2R,S)-2-(2-BOC-Aminothiazol-4-yl)-2-(2-benzoylaminoäthansulfonylamino)-essigsäure werden mit 2 g 7β-Amino-3-cephem-4-carbonsäurediphenylmethylester in 20 ml Tetrahydrofuran analog Beispiel 6 b) umgesetzt (0,56 g Hydroxybenztriazol; dreimal 0,49 g Dicyclohexylcarbodiimid in je 4 ml Tetrahydrofuran), aufgearbeitet und chromatographiert. Man erhält die Titel-

verbindung; $[\alpha]_D^{20°}$ = +20°±1° (1,01% in $CHCl_3$); IR: 3400, 3300, 1785, 1720, 1665, 1602, 1520 ($CH_2Cl_2$); UV: 255 (11500; EtOH).

Herstellung des Ausgangsmaterials:

c) (2R,S)-2-(2-BOC-Aminothiazol-4-yl)-2-(2-benzoylaminoäthansulfonylamino)-essigsäure

6,7 g (2R,S)-2-(2-BOC-Aminothiazol-4-yl)-2-(2-aminoäthansulfonylamino)-essigsäure werden in 24 ml Tetrahydrofuran mit 1,16 ml Benzoylchlorid analog Beispiel 6 c) umgesetzt(10 ml N,O-Bis-(trimethylsilyl)-acetamid; 0,81 ml Pyridin) und aufgearbeitet. Man erhält die Titelverbindung, die ohne Charakterisierung weiterverarbeitet wird.

Beispiel 27:

a) 7β-[(2R,S)-2-(2-Aminothiazol-4-yl)-2-(2-formylaminoäthansulfonylamino)-acetamido]-3-cephem-4-carbonsäure-natriumsalz

1,9 g des gemäss Beispiel 27 b) erhältlichen 7β-[(2R,S)-2-(2-BOC-Aminothiazol-4-yl)-2-(2-formylaminoäthansulfonylamino)-acetamido]-3-cephem-4-carbonsäurediphenylmethylesters werden in 10 ml $CH_2Cl_2$ und 0,57 ml Anisol mit 15 ml Trifluoressigsäure analog Beispiel 1 a) umgesetzt, aufgearbeitet, chromatographiert und umgefällt. Man erhält das Hydrat der Titelverbindung; F: oberhalb 215° (unter Zersetzung); $[\alpha]_D^{20°}$ = +101°±1° (1,22 % in $H_2O$); IR: 3700-2500 (breit), 1760, 1670, 1600, 1520 (Nujol); UV: 252 (9900), 318 (200; $H_2O$).

b) 7β-[(2R,S)-2-(2-BOC-Aminothiazol-4-yl)-2-(2-formylaminoäthansulfonylamino)-acetamido]-3-cephem-4-carbonsäurediphenylmethylester

2,3 g gemäss Beispiel 27 c) erhältliche (2R,S)-2-(2-BOC-Aminothiazol-4-yl)-2-(2-formylaminoäthansulfonylamino)-essigsäure werden mit 2,0 g 7β-Amino-3-cephem-4-carbonsäurediphenylmethylester in 30 ml Tetrahydrofuran analog Beispiel 6 b) umgesetzt (0,5 g Hydroxybenztriazol; dreimal 0,4 g Dicyclohexylcarbodiimid in je 6 ml Tetrahydrofuran), aufgearbeitet und chromatographiert. Man erhält die

Titelverbindung; $[\alpha]_D^{20°}$ = +18°±1° (0,99% in $CHCl_3$); IR: 3400, 3300, 1790, 1727, 1690, 1640, 1600, 1542 (Nujol); UV 260 (13400; EtOH).

Das Ausgangsmaterial stellt man folgendermassen her:

c) (2R,S)-2-(2-BOC-Aminothiazol-4-yl)-2-(2-formylaminoäthansulfonylamino)-essigsäure

6,7 g (2R,S)-2-(2-BOC-Aminothiazol-4-yl)-2-(2-aminoäthansulfonylamino)-essigsäure werden in 25 ml Tetrahydrofuran mit 1,35 ml des gemischten Anhydrids von Essig- und Ameisensäure [hergestellt aus Acetanhydrid und Ameisensäure; vgl. dazu G. Büchi et al., JACS 93, 2492 (1971)] analog Beispiel 6 c) umgesetzt (10 ml N,O-Bis-(trimethylsilyl)-acetamid; 0,81 ml Pyridin) und aufgearbeitet. Man erhält die Titelverbindung, die ohne Charakterisierung weiterverarbeitet wird.

Beispiel 28:

a) 3-(1-Methyl-1H-tetrazol-5-ylthiomethyl)-7β-[(2R)-2-(2-aminothiazol-4-yl)-2-(2-formylaminoäthansulfonylamino)-acetamido]-3-cephem-4-carbonsäurenatriumsalz

1,17 g des gemäss Beispiel 28 c) erhältlichen 3-(1-Methyl-1H-tetrazol-5-ylthiomethyl)-7β-[(2R)-2-(2-BOC-aminothiazol-4-yl)-2-(2-formylamino-äthansulfonylamino)-acetamido]-3-cephem-4-carbonsäurediphenylmethylesters werden in 5,5 ml $CH_2Cl_2$ und 0,29 ml Anisol mit 8,2 ml Trifluoressigsäure analog Beispiel 1 a) umgesetzt, aufgearbeitet und umgefällt. Man erhält das Hydrat der Titelverbindung; F: oberhalb 165° (unter Zersetzung); $[\alpha]_D^{20°}$ = -12°±1° (1,00 % in $H_2O$); IR: 3700-2600 (breit), 1760, 1675, 1605, 1520 (Nujol); UV: 259 (13200; $H_2O$).

b) 3-(1-Methyl-1H-tetrazol-5-ylthiomethyl)-7β-[(2S)-2-(2-aminothiazol-4-yl)-2-(2-formylaminoäthansulfonylamino)-acetamido]-3-cephem-4-carbonsäurenatriumsalz

1,1 g des gemäss Beispiel 28 c) erhältlichen 3-(1-Methyl-1H-tetrazol-5-ylthiomethyl)-7β-[(2S)-2-(2-BOC-aminothiazol-4-yl)-2-(2-formyl-

aminoäthansulfonylamino)-acetamido]-3-cephem-4-carbonsäurediphenylmethylesters werden in 5,5 ml $CH_2Cl_2$ und 0,29 ml Anisol mit 8,2 ml Trifluoressigsäure analog Beispiel 1 a) umgesetzt, aufgearbeitet und umgefällt. Man erhält das Hydrat der Titelverbindung; F: oberhalb 165° (unter Zersetzung); $[\alpha]_D^{20°} = +7°\pm1°$ (1,09 % in $H_2O$); IR: 2700-2600, 1762, 1672, 1605, 1524 (Nujol); UV: 259 (13500; $H_2O$).

c) <u>3-(1-Methyl-1H-tetrazol-5-ylthiomethyl)-7β-[(2R)-2-(2-BOC-aminothiazol-4-yl)-2-(2-formylaminoäthansulfonylamino)-acetamido]-3-cephem-4-carbonsäurediphenylmethylester</u>

und

<u>3-(1-Methyl-1H-tetrazol-5-ylthiomethyl)-7β-[(2S)-2-(2-BOC-aminothiazol-4-yl)-2-(2-formylaminoäthansulfonylamino)-acetamido]-3-cephem-4-carbonsäurediphenylmethylester</u>

3 g (2R,S)-2-(2-BOC-Aminothiazol-4-yl)-2-(2-formylaminoäthansulfonylamino)-essigsäure (Herstellung gemäss Beispiel 27 c) werden mit 2,77 g 3-(1-Methyl-1H-tetrazol-5-ylthiomethyl)-7β-amino-3-cephem-4-carbonsäurediphenylmethylester in 60 ml Tetrahydrofuran analog zu Beispiel 6 b) umgesetzt (0,65 g Hydroxybenztriazol; dreimal 0,52 g Dicyclohexylcarbodiimid in je 7 ml Tetrahydrofuran) und aufgearbeitet. Das anfallende Rohprodukt wird an 300 g Kieselgel (Stufensäule) chromatographiert (Eluiermittel: Toluol-Essigester 4:1-, 2:1- und 1:1-Gemisch und Essigester). Dabei eluiert man zuerst die Titelverbindnung mit der 2R-Konfiguration.
(Zur Konfiguration vgl. Beispiel 7 d); $[\alpha]_D^{20°} = -83°\pm1°$ (0,85% in $CHCl_3$); IR: 3400, 3300, 1788, 1722, 1691, 1605, 1542 ($CH_2Cl_2$); UV: 260 (16800; EtOH).
Die Folgefraktionen bestehen aus einem binären Gemisch der obigen (2R)-Verbindung mit dem (2S)-Isomeren.
Aus den letzten Fraktionen erhält man die Titelverbindung mit der 2S-Konfiguration; $[\alpha]_D^{20°} = -84°\pm1°$ (0,95% in $CHCl_3$); IR: 3400, 3300, 1789, 1722, 1691, 1605, 1542 ($CH_2Cl_2$); UV: 260 (17000; EtOH).

Beispiel 29:

a) 7β-[(2R,S)-2-(2-(2-Aminothiazol-4-ylacetamido)-äthansulfonylamino)-2-(2-aminothiazol-4-yl)-acetamido]-3-cephem-4-carbonsäure-natriumsalz

Eine Lösung von 3,9 g (4 mMol) 7β-[(2R,S)-2-(2-(2-BOC-Aminothiazol-4-ylacetamido)-äthansulfonylamino)-2-(2-BOC-aminothiazol-4-yl)-acetamido]-3-cephem-4-carbonsäurediphenylmethylester und 3,9 ml Anisol in 20 ml Methylenchlorid wird mit 20 ml Trifluoressigsäure versetzt, eine Stunde lang bei 25° unter Stickstoffatmosphäre gerührt und anschliessend bei 0° mit 300 ml Diäthyläther versetzt. Der beige Niederschlag wird abfiltriert, mit wenig Diäthyläther gewaschen und nach Auflösen in 30 ml Wasser mit 2N Natriumhydroxidlösung auf pH 7 gestellt. Nach Extraktion mit Essigsäureäthylester wird die wässrige Phase an silyliertem Silicagel (Opti-$UPC_{12}$) mit Wasser als Eluiermittel gereinigt. Man erhält die Titelverbindung in Form des Dihydrates. F: oberhalb 170° (Zersetzung); Rf 0,38 (Silicagel Opti-$UPC_{12}$, $H_2O$-$CH_3CN$, 8:2); $[\alpha]_D^{20°}$ = +89°±1° ($H_2O$; c = 0,152%); UV ($H_2O$): 251 (ε = 15 550).

b) 7β-[(2R,S)-2-(2-(2-BOC-Aminothiazol-4-ylacetamido)-äthansulfonylamino)-2-(2-BOC-aminothiazol-4-yl)-acetamido]-3-cephem-4-carbonsäurediphenylmethylester

Zu einer Lösung von 2,48 g (4 mMol) der gemäss Beispiel 29 c) erhältlichen (2R,S)-2-(2-(2-BOC-Aminothiazol-4-ylacetamido)-äthansulfonylamino)-2-(2-BOC-aminothiazol-4-yl)-essigsäure und 0,54 g (4 mMol) 1-Hydroxybenztriazol in 40 ml absolutem Tetrahydrofuran wird unter Rühren bei Raumtemperatur eine Lösung von 0,91 g (4,4 mMol) N,N'-Dicyclohexylcarbodiimid in 10 ml absolutem Tetrahydrofuran und nach ca. 10 Minuten 1,46 g (4 mMol) 7β-Amino-3-cephem-4-carbonsäurediphenylmethylester gegeben. Nach einer Reaktionszeit von 4 Stunden wird der ausgefallene N,N'-Dicyclohexylharnstoff abfiltriert und das Filtrat am Rotationsverdampfer eingeengt. Der in 400 ml Essigester gelöste Rückstand wird je zweimal mit 50 ml 1 N Salzsäure und gesättigter Natriumchloridlösung gewaschen. Das nach Trocknen über Natriumsulfat und Entfernen des Lösungsmittels am Rotationsverdanpfer erhaltene Rohprodukt wird an 160 g Silicagel mit Methylenchlorid-Essigester (1:1) als Eluiermittel gereinigt. Man erhält die Titelverbindung; Rf (Silicagel, Entwickeln mit Jod): 0,33 ($CHCl_3/CH_2OH/CH_3COOH$ 75:22:3).

Herstellung des Ausgangsmaterials:

c) (2R,S)-2-(2-(2-BOC-Aminothiazol-4-ylacetamido)-äthansulfonylamino)-2-(2-BOC-aminothiazol-4-yl)-essigsäure

Zu einer Lösung von 3,87 g (15 mMol) 2-BOC-Aminothiazol-4-ylessigsäure und 2,03 g (15 mMol) 1-Hydroxybenztriazol werden unter Rühren bei Raumtemperatur 3,4 g (16,5 mMol) N,N'-Dicyclohexylcarbodiimid gegeben. Nach 15 Minuten Reaktionszeit wird die entstandene Suspension mit 5,71 g (15 mMol) (2R,S)-2-(2-Aminoäthansulfonylamino)-2-(2-BOC-aminothiazol-4-yl)-essigsäure versetzt. Nach einer Reaktionszeit von 4 Stunden wird der entstandene N,N'-Dicyclohexylharnstoff abfiltriert und das Filtrat am Rotationsverdampfer eingeengt. Der in 500 ml Essigester gelöste Rückstand wird je dreimal mit 1 N Salzsäure und gesättigter Natriumchloridlösung gewaschen. Das nach Trocknen über Natriumsulfat und Entfernen des Lösungsmittels am Rotationsverdampfer erhaltene Rohprodukt wird an 400 g Silicagel mit Methylenchlorid-Essigester 1:2 als Eluiermittel gereinigt, woraus man die Titelverbindung als amorphes Pulver erhält.

Beispiel 30:

a) 7β-[(2R,S)-2-(2-Aminothiazol-4-yl)-2-(2-butyrylamino)-äthansulfonylamino)-acetamido]-3-cephem-4-carbonsäure

Analog Beispiel 29 a) erhält man durch Umsetzung von 3,1 g (3,9 mMol) 7β-[(2R,S)-2-(2-BOC-Aminothiazol-4-yl)-2-(2-butyrylamino)-äthansulfonylamino)-acetamido]-3-cephem-4-carbonsäurediphenylmethylester in Gegenwart von 3,1 ml Anisol mit 15 ml Trifluoressigsäure in 15 ml absolutem Methylenchlorid die Titelverbindung als 1,5-Hydrat;

F: oberhalb 170° (Zersetzung); Rf 96: ca. 0,43; $[\alpha]_D^{20°}$ = +86°±1° ($H_2O$, C = 0,872%); UV ($H_2O$): 250 ($\varepsilon$ = 10700).

b) 7β-[(2R,S)-2-(2-BOC-Aminothiazol-4-yl)-2-(2-butyrylamino)-äthansulfonylamino)-acetamido]-3-cephem-4-carbonsäurediphenylmethylester

Analog Beispiel 29 b) erhält man durch Umsetzung von 3,4 g (7,5 mMol) (2R,S)-2-(2-BOC-Aminothiazol-4-yl)-2-(2-butyrylamino)-äthansulfonylamino)-essigsäure mit 2,75 g (7,5 mMol) 7β-Amino-3-cephem-4-carbonsäurediphenylmethylester in Gegenwart von 1,01 g 1-Hydroxybenztriazol und 1,7 g N,N'-Dicyclohexylcarbodiimid in 80 ml Tetrahydrofuran die Titelverbindung; Rf (Silicagel): 0,45 (Essigester).

Herstellung des Ausgangsmaterials:

c) (2R,S)-2-(2-BOC-Aminothiazol-4-yl)-2-(2-butyrylamino)-äthansulfonylamino)-essigsäure

Eine Suspension von 3,80 g (10 mMol) (2R,S)-2-(2-Aminoäthansulfonylamino)-2-(2-BOC-aminothiazol-4-yl)-essigsäure in 90 ml absolutem Tetrahydrofuran wird unter Rühren, Feuchtigkeitsausschluss und Stickstoffatmosphäre mit 10 ml N,O-Bis-(trimethylsilyl)-acetamid versetzt. Nach einer Stunde Reaktionszeit bei 65° wird das Reaktionsgemisch auf 0° gekühlt, mit 0,88 ml Pyridin und 1,14 ml Buttersäurechlorid, gelöst in 10 ml Tetrahydrofuran, versetzt und weitere 15 Stunden bei Raumtemperatur gerührt. Nach Entfernen des Lösungsmittels wird der Rückstand in Essigester aufgenommen und je viermal mit 50 ml 0,5 N Salzsäure und gesättigter Natriumchloridlösung gewaschen. Nach Trocknen über Natriumsulfat wird das Lösungsmittel am Rotationsverdampfer entfernt, woraus die Titelverbindung erhalten wird, die ohne weitere Reinigung im nächsten Reaktionsschritt eingesetzt werden kann.

Beispiel 31:

a) 7β-[(2R,S)-2-(2-Aminothiazol-4-yl)-2-(2-acryloylaminoäthansulfonylamino)-acetamido]-3-cephem-4-carbonsäurenatriumsalz

1,84 g des gemäss Beispiel 31 b) erhältlichen 7β-[(2R,S)-2-(2-BOC-Aminothiazol-4-yl)-2-(2-acryloylaminoäthansulfonylamino)-acetamido]-3-cephem-4-carbonsäurediphenylmethylesters werden in 4,14 ml $CH_2Cl_2$ und 1,34 ml Anisol mit 15,5 ml Trifluoressigsäure analog Beispiel 1 a) umgesetzt, aufgearbeitet, chromatographiert und umgefällt. Man erhält das Hydrat der Titelverbindung; F: oberhalb 190° (unter Zersetzung); IR: 3700-2600 (breit), 1760, 1660, 1600, 1522 (Nujol); UV: 255 (10000; $H_2O$).

b) 7β-[(2R,S)-2-(2-BOC-Aminothiazol-4-yl)-2-(2-acryloylaminoäthansulfonylamino)-acetamido]-3-cephem-4-carbonsäurediphenylmethylester

2,88 g gemäss Beispiel 31 c) erhältliche (2R,S)-2-(2-BOC-Aminothiazol-4-yl)-2-(2-acryloylaminoäthansulfonylamino)-essigsäure werden mit 2,2 g 7β-Amino-3-cephem-4-carbonsäurediphenylmethylester in 20 ml Tetrahydrofuran analog Beispiel 6 b) umgesetzt (0,66 g Hydroxybenztriazol; dreimal 0,58 g Dicyclohexylcarbodiimid in je 5 ml Tetrahydrofuran), aufgearbeitet und chromatographiert. Man erhält die Titelverbindung; $[\alpha]_D^{20°} = +26° \pm 1°$ (1,01% in $CHCl_3$); IR: 3400, 2200, 1778, 1715, 1675, 1625, 1605 (Schulter), 1515 ($CH_2Cl_2$); UV: 257 (3400; EtOH).

Herstellung des Ausgangsmaterials:

c) (2R,S)-2-(2-BOC-Aminothiazol-4-yl)-2-(2-acryloylaminoäthansulfonylamino)-essigsäure

6,7 g (2R,S)-2-(2-BOC-Aminothiazol-4-yl)-2-(2-aminoäthansulfonylamino)-essigsäure werden in 24 ml Tetrahydrofuran mit 0,81 ml Acrylsäurechlorid analog zu Beispiel 6 c) umgesetzt (10 ml N,O-Bis-(trimethylsilyl)-acetamid; 0,81 ml Pyridin) und aufgearbeitet. Man erhält die Titelverbindung, die ohne Charakterisierung weiterverarbeitet wird.

Beispiel 32:

a) 7β-[(2S)-2-(2-Aminothiazol-4-yl)-2-(2-cyclopropylcarbonylaminoäthansulfonylamino)-acetamido]-3-cephem-4-carbonsäurenatriumsalz

1,4 g des gemäss Beispiel 32 c) erhältlichen 7β-[(2S)-2-(2-BOC-Aminothiazol-4-yl)-2-(2-cyclopropylcarbonylaminoäthansulfonylamino)-acetamido]-3-cephem-4-carbonsäurediphenylmethylesters werden in 7,4 ml $CH_2Cl_2$ und 0,4 ml Anisol mit 11,2 ml Trifluoressigsäure analog Beispiel 1 a) umgesetzt, aufgearbeitet und umgefällt. Man erhält das Hydrat der Titelverbindung; F: Zersetzung ab 200°; $[\alpha]_D^{20°}$ = +107°±1° (0,85% in $H_2O$); IR: 3700-2600, 1769, 1670 (Schulter), 1645, 1600, 1530 (Nujol); UV: 255 ( 11000; $H_2O$).

b) <u>7β-[(2R)-2-(2-Aminothiazol-4-yl)-2-(2-cyclopropylcarbonylaminoäthansulfonylamino)-acetamido]-3-cephem-4-carbonsäure-natriumsalz</u>

1 g des gemäss Beispiel 32 c) erhältlichen 7β-[(2R)-2-(2-BOC-Aminothiazol-4-yl)-2-(2-cyclopropylcarbonylaminoäthansulfonylamino)-acetamido]-3-cephem-4-carbonsäurediphenylmethylesters werden in 5,3 ml $CH_2Cl_2$ und 0,28 ml Anisol mit 8 ml Trifluoressigsäure analog Beispiel 1 a) umgesetzt, aufgearbeitet und umgefällt. Man erhält das Hydrat der Titelverbindung; F: Zersetzung ab 200°; $[\alpha[_D^{20°}$ = +77°±1° (0,56% in $H_2O$); IR: 3700-2500 (breit), 1760, 1670 (Schulter), 1645, 1600, 1525 (Nujol); UV: 255 (9600), 315 (600; $H_2O$).

c) <u>7β-[(2R)-2-(2-BOC-Aminothiazol-4-yl)-2-(2-cyclopropylcarbonylaminoäthansulfonylamino)-acetamido]-3-cephem-4-carbonsäurediphenylmethylester</u>

und

<u>7β-[(2S)-2-(2-BOC-Aminothiazol-4-yl)-2-(2-cyclopropylcarbonylaminoäthansulfonylamino)-acetamido]-3-cephem-4-carbonsäurediphenylmethylester</u>

2,4 g der gemäss Beispiel 32 d) erhältlichen (2R,S)-2-(2-BOC-Aminothiazol-4-yl)-2-(2-cyclopropylcarbonylaminoäthansulfonylamino)-essigsäure werden mit 1,93 g 7β-Amino-3-cephem-4-carbonsäurediphenylmethylester in 30 ml Tetrahydrofuran analog Beispiel 6 b) umgesetzt (0,54 g Hydroxybenztriazol; dreimal 0,46 g Dicyclohexylcarbodiimid in je 4 ml Tetrahydrofuran) und aufgearbeitet. Das anfallende Rohprodukt wird an

300 g Kieselgel (Stufensäule) chromatographiert (Eluiermittel: Toluol-Essigester 2:1). Dabei eluiert man zuerst die Titelverbindung mit der (2R)-Konfiguration; $[\alpha]_D^{20°} = +2°\pm1°$ (0,95% in $CH_2Cl_2$); IR: 3300, 1791, 1730, 1700, 1670, 1640, 1602, 1540 ($CH_2Cl_2$); UV: 258 (12500; EtOH).

Die Folgefraktionen bestehen aus einem binären Gemisch der (2R)- und (2S)-Titelverbindungen.

Zuletzt erhält man die (2S)-Titelverbindung; $[\alpha]_D^{20°} = +38°\pm1°$ (0,85% in $H_2O$); IR: 3400, 3300, 1792, 1739, 1704, 1670, 1640, 1602, 1540 ($CH_2Cl_2$); UV: 257 (11900; EtOH).

Herstellung des Ausgangsmaterials:

d) (2R,S)-2-(2-BOC-Aminothiazol-4-yl)-2-(2-cyclopropylcarbonylamino-äthansulfonylamino)-essigsäure

6,7 g (2R,S)-2-(2-BOC-Aminothiazol-4-yl)-2-(2-aminoäthansulfonylamino)-essigsäure werden in 24 ml Tetrahydrofuran mit 0,875 ml Cyclopropancarbonsäurechlorid analog Beispiel 6 c) umgesetzt (16 ml N,O-Bis-(trimethylsilyl)-acetamid; 0,81 ml Pyridin) und aufgearbeitet. Man erhält die Titelverbindung, die ohne Charakterisierung weiterverarbeitet wird.

Beispiel 33:

a) 7β-[(2R,S)-2-(2-Aminothiazol-4-yl)-2-(2-cyanacetylaminoäthansulfonylamino)-acetamido]-3-cephem-4-carbonsäurenatriumsalz

Analog Beispiel 29 a) erhält man durch Umsetzung von 2,23 g (2,8 mMol) des gemäss Beispiel 33 b) erhältlichen 7β-[(2R,S)-2-(2-BOC-Aminothiazol-4-yl)-2-(2-cyanacetylaminoäthansulfonylamino)-acetamido]-3-cephem-4-carbonsäurediphenylmethylesters in Gegenwart von 2,2 ml Anisol mit 11 ml Trifluoressigsäure die Titelverbindung in Form des Dihydrats; F: oberhalb 168° (unter Zersetzung); Rf (Silicagel Opti-$UPC_{12}$): ca. 0,35 ($H_2O$).

b) 7β-[(2R,S)-2-(2-BOC-Aminothiazol-4-yl)-2-(2-cyanacetylaminoäthansulfonylamino)-acetamido]-3-cephem-4-carbonsäurediphenylmethylester

Analog Beispiel 29 b) erhält man durch Umsetzung von 2,91 g (2R,S)-2-(2-BOC-Aminothiazol-4-yl)-2-(2-cyanacetylaminoäthansulfonylamino)-essigsäure mit 2,38 g 7β-Amino-3-cephem-4-carbonsäurediphenylmethylester in Gegenwart von 0,88 g 1-Hydroxybenztriazol und 1,48 g N,N'-Dicyclohexylcarbodiimid in 60 ml absolutem Tetrahydrofuran die die Titelverbindung als amorphes Pulver; Rf (Silicagel): ca. 0,18 (Methylenchlorid/Essigester 1:1).

Herstellung des Ausgangsmaterials:

c) (2R,S)-2-(2-BOC-Aminothiazol-4-yl)-2-(2-cyanacetylaminoäthansulfonylamino)-essigsäure

Eine Suspension von 3,8 g (10 mMol) (2R,S)-2-(2-Aminoäthansulfonylamino)-2-(2-BOC-aminothiazol-4-yl)-essigsäure in 100 ml absolutem Tetrahydrofuran wird unter Rühren, Feuchtigkeitsausschluss und Stickstoffatmosphäre mit 10 ml N,O-Bis(trimethylsilyl)-acetamid versetzt. Nach einer Stunde Reaktionszeit bei 65° wird das Reaktionsgemisch auf 0° gekühlt, mit 0,96 ml Pyridin und 1,0 ml Cyanessigsäurechlorid versetzt und anschliessend 3 Stunden bei Raumtemperatur gerührt. Nach Entfernen des Lösungsmittels wird der Rückstand in Essigester aufgenommen und je viermal mit 0,5 N Salzsäure und gesättigter Natriumchloridlösung gewaschen. Nach dem Trocknen über Natriumsulfat wird das Lösungsmittel am Rotationsverdampfer entfernt. Man erhält die Titelverbindung, welche ohne Reinigung im nächsten Reaktionsschritt eingesetzt wird.

Beispiel 34:

a) 7β-[(2R,S)-2-(2-Aminothiazol-4-yl)-2-(2-methoxyacetylaminoäthansulfonylamino)-acetamido]-3-cephem-4-carbonsäurenatriumsalz

3 g des gemäss Beispiel 34 b) herstellbaren 7β-[(2R,S)-2-(2-BOC-Aminothiazol-4-yl)-2-(2-methoxyacetylaminoäthansulfonylamino)-acetamido]-3-cephem-4-carbonsäurediphenylmethylesters werden in 8 ml $CH_2Cl_2$ und 0,9 ml Anisol mit 15 ml Trifluoressigsäure analog Beispiel 1 a) umgesetzt, aufgearbeitet, chromatographiert und umgefällt. Man erhält das Hydrat der Titelverbindung; F: Zersetzung oberhalb 150°; $[\alpha]_D^{20°} = +93° \pm 1°$ (1,22% in $H_2O$); IR: 3700-2600 (breit), 1760, 1665, 1600, 1525 (Nujol); UV: 252 (9600; $H_2O$).

b) 7β-[(2R,S)-2-(2-BOC-Aminothiazol-4-yl)-2-(2-methoxyacetylaminoäthansulfonylamino)-acetamido]-3-cephem-4-carbonsäurediphenylmethylester

3,6 g der gemäss Beispiel 36 c) erhältlichen (2R,S)-2-(2-BOC-Aminothiazol-4-yl)-2-(2-methoxyacetylaminoäthansulfonylamino)-essigsäure werden mit 2,9 g 7β-Amino-3-cephem-4-carbonsäurediphenylmethylester in 40 ml Tetrahydrofuran analog Beispiel 6 b) umgesetzt (1 g Hydroxybenztriazol;dreimal 0,53 g Dicyclohexylcarbodiimid in je 7 ml Tetrahydrofuran), aufgearbeitet und chromatographiert. Man erhält die Titelverbindung; $[\alpha]_D^{20°} = +19° \pm 1°$ (1,16% in $CHCl_3$); IR: 3400, 3300, 1790, 1728, 1692, 1640 (Schulter), 1600, 1530 ($CH_2Cl_2$); UV: 260 (14000; EtOH).

Das Ausgangsmaterial stellt man folgendermassen her:

c) (2R,S)-2-(2-BOC-Aminothiazol-4-yl)-2-(2-methoxyacetylaminoäthansulfonylamino)-essigsäure

6,7 g (2R,S)-2-(2-BOC-Aminothiazol-4-yl)-2-(2-aminoäthansulfonylamino)-essigsäure werden in 25 ml Tetrahydrofuran mit 0,92 ml Methoxyacetylchlorid analog Beispiel 6 c) umgesetzt (10 ml N,O-Bis-(trimethylsilyl)-acetamid; 0,81 ml Pyridin) und aufgearbeitet. Man erhält die Titelverbindung, die ohne Charakterisierung weiterverarbeitet wird.

Beispiel 35:

a) 7β-[(2R,S)-2-(2-Aminothiazol-4-yl)-2-(2-propioloylaminoäthansulfonylamino)-acetamido]-3-cephem-4-carbonsäurenatriumsalz

1,3 g 7β-[(2R,S)-2-(2-BOC-Aminothiazol-4-yl)-2-(2-propioloylaminoäthansulfonylamino)-acetamido]-3-cephem-4-carbonsäurediphenylmethylester werden in 7 ml $CH_2Cl_2$ und 0,37 ml Anisol mit 10,6 ml Trifluoressigsäure analog Beispiel 1 a) umgesetzt, aufgearbeitet und umgefällt. Man erhält das Hydrat der Titelverbindung; F: Zersetzung ab 220°; $[\alpha]_D^{20°}$ = +91°±1° (1,00% in $H_2O$); IR: 3700-2700 (breit), 2120, 1760, 1675 (Schulter), 1645, 1600, 1522 (Nujol); UV: 249 (9700), 314 (700; $H_2O$).

b) 7β-[(2R,S)-2-(2-BOC-Aminothiazol-4-yl)-2-(2-propioloylaminoäthansulfonylamino)-acetamido]-3-cephem-4-carbonsäurediphenylmethylester

3,1 g des gemäss Beispiel 35 c) erhältlichen 7β-[(2R,S)-2-(2-BOC-Aminothiazol-4-yl)-2-(2-aminoäthansulfonylamino)-acetamido]-3-cephem-4-carbonsäurediphenylmethylesters werden mit 0,258 ml Propiolsäure in 45 ml Tetrahydrofuran analog Beispiel 6 b) umgesetzt (0,56 g Hydroxybenztriazol; dreimal 0,29 g Dicyclohexylcarbodiimid in je 5 ml Tetrahydrofuran), aufgearbeitet und chromatographiert. Man erhält die Titelverbindung; IR: 3400, 3300, 2120, 1790, 1726, 1700 (Schulter), 1668, 1603, 1540 ($CH_2Cl_2$); UV: 258 (13 800; EtOH).

Herstellung des Ausgangsmaterials:

c) 7β-[(2R,S)-2-(2-BOC-Aminothiazol-4-yl)-2-(2-aminoäthansulfonylamino)-acetamido]-3-cephem-4-carbonsäurediphenylmethylester

12,9 g des gemäss Beispiel 35 d) erhältlichen 7β-[(2R,S)-2-(2-BOC-Aminothiazol-4-yl)-2-(2-(2,2,2-trichloräthoxycarbonylamino)-äthansulfonylamino)-acetamido]-3-cephem-4-carbonsäurediphenylmethylesters werden mit 14 g Zinkstaub in 150 ml Acetonitril-Essigsäure 1:1-Gemisch 3 Stunden bei 0° gerührt. Dann filtriert man den Zinkrückstand ab, wäscht mit Acetonitril und dampft im Vakuum zur Trockne ein. Den Eindampfrückstand nimmt man in 0,5 l Essigester-Wasser-1:1-Gemisch auf, stellt mit 1 N Natronlauge auf pH 8, verdünnt mit etwas Essigester und wäscht mit gesättigter wässriger NaCl-Lösung neutral. Dann trocknet man über $Na_2SO_4$, dampft im Vakuum ein und fällt das Rohprodukt aus $CH_2Cl_2$-Aether-Hexan einmal um. Man erhält die Titelverbindung; $[\alpha]_D^{20°} = +29°\pm1°$ (1,09% in $CHCl_3$); IR: 3450-2600 (breit), 1770, 1715, 1690 (Schulter), 1640, 1532 ($CH_2Cl_2$); UV: 258 (11 800; EtOH).

d) 7β-[(2R,S)-2-(2-BOC-Aminothiazol-4-yl)-2-(2-(2,2,2-trichloräthoxycarbonylamino)-äthansulfonylamino)-acetamido]-3-cephem-4-carbonsäurediphenylmethylester

11,8 g (2R,S)-2-(2-BOC-Aminothiazol-4-yl)-2-(2-(2,2,2-trichloräthoxycarbonylamino)-äthansulfonylamino)-essigsäure werden mit 7,05 g 7β-Amino-3-cephem-4-carbonsäurediphenylmethylester in 100 ml Tetrahydrofuran analog Beispiel 6 b) umgesetzt (1,95 g Hydroxybenztriazol; dreimal 1,7 g Dicyclohexylcarbodiimid in je 20 ml Tetrahydrofuran), aufgearbeitet und chromatographiert. Man erhält die Titelverbindung; $[\alpha]_D^{20°} = +14°\pm1°$ (0,90% in $CHCl_3$); IR: 3400, 3300, 1781, 1720, 1700 (Schulter), 1635, 1520 ($CH_2Cl_2$); UV: 258 (13600; EtOH).

Beispiel 36:

a) 7β-[(2R,S)-2-(2-((2R)-2-Amino-2-carboxyäthoxycarbonylamino)-äthansulfonylamino)-2-(2-aminothiazol-4-yl)-acetamido]-3-cephem-4-carbonsäurenatriumsalz

Analog Beispiel 29 a) erhält man durch Umsetzung von 1,5 g (1,35 mMol) 7β-[(2R,S)-2-(2-((2R)-2-BOC-Amino-2-diphenylmethoxycarbonyläthoxycarbonylamino)-äthansulfonylamino)-2-(2-BOC-aminothiazol-4-yl)-acetamido]-3-cephem-4-carbonsäurediphenylmethylester mit 1,5 ml Anisol und 7,5 ml Trifluoressigsäure in 7,5 ml Methylenchlorid die Titelverbindung in Form des Dihydrats; F: oberhalb 151°; Rf (Silicagel Opti-$UPC_{12}$): ca. 0,75 (Wasser/Acetonitril 9:1); $[\alpha]_D^{20°} = +68°\pm1°$ (0,659% in 0,1 N HCl); UV (in 0,1 N HCl): 252 ($\varepsilon$ = 12 900).

b) 7β-[(2R,S)-2-(2-((2R)-2-BOC-Amino-2-diphenylmethoxycarbonyläthoxycarbonylamino)-äthansulfonylamino)-2-(2-BOC-aminothiazol-4-yl)-acetamido]-3-cephem-4-carbonsäurediphenylmethylester

Analog Beispiel 29 b) erhält man durch Umsetzen von 1,56 g (2 mMol) (2R,S)-2-(2-((2R)-2-BOC-Amino-2-diphenylmethoxycarbonyläthoxycarbonylamino)-äthansulfonylamino)-2-(2-BOC-aminothiazol-4-yl)-essigsäure mit 0,73 g (2 mMol) 7β-Amino-3-cephem-4-carbonsäurediphenylmethylester in Gegenwart von 0,27 g 1-Hydroxybenztriazol und 0,45 g N,N'-Dicyclohexylcarbodiimid in 30 ml Tetrahydrofuran die Titelverbindung als gelbliches Pulver; Rf: ca. 0,63 (Methylenchlorid/Essigester 1:1).

Herstellung des Ausgangsmaterials:

c) (2R,S)-2-(2-((2R)-2-BOC-Amino-2-diphenylmethoxycarbonyläthoxycarbonylamino)-äthansulfonylamino)-2-(2-BOC-aminothiazol-4-yl)-essigsäure

Eine Suspension von 1,9 g (5 mMol) (2R,S)-2-(2-Aminoäthansulfonylamino)-2-(2-BOC-aminothiazol-4-yl)-essigsäure in 60 ml absolutem Tetrahydrofuran wird unter Rühren, Feuchtigkeitsausschluss und Stickstoffatmosphäre mit 5,0 ml N,O-Bis(trimethylsilyl)-acetamid versetzt. Nach einer Stunde Reaktionszeit bei 60° wird das Reaktionsgemisch auf Raumtemperatur abgekühlt, mit 0,4 ml Pyridin und 2,17 g (2R)-2-BOC-Amino-2-diphenylmethoxycarbonyläthoxycarbonylchlorid versetzt und anschliessend 2 Stunden gerührt. Nach Entfernen des Lösungsmittels wird der Rückstand in 250 ml Essigester aufgenommen und je dreimal mit 0,1 N Salzsäure und gesättigter Natriumchloridlösung gewaschen. Nach dem Trocknen über Natriumsulfat wird das Lösungsmittel im Rotationsverdampfer entfernt und der Rückstand an Silicagel mit Methylenchlorid-Essigester 1:1 als Eluiermittel gereinigt, woraus die Titelverbindung als amorphes Pulver erhalten wird; Rf (Silicagel): ca. 0,58 (Chlorform/Methanol/Eisessig 75:22:3).

Beispiel 37:

a) 7β-[(2R,S)-2-(2-Aminothiazol-4-yl)-2-(2-äthoxycarbonylaminoäthan-sulfonylamino)-acetamido]-3-cephem-4-carbonsäurenatriumsalz

Analog Beispiel 29 a) erhält man durch Umsetzen von 4,2 g (5,25 mMol) 7β-[(2R,S)-2-(2-BOC-Aminothiazol-4-yl)-2-(2-äthoxycarbonylamino-äthansulfonylamino)-acetamido]-3-cephem-4-carbonsäurediphenylmethylester mit 4,2 ml Anisol und 20 ml Trifluoressigsäure in 20 ml Methylenschlorid die Titelverbindung als 1,5 Hydrat; F: oberhalb 168° (unter Zersetzung); $[\alpha]_D^{20°} = +95° \pm 1°$ (1,027% in Wasser); UV: (Wasser): 251 ($\varepsilon$ = 10500).

b) 7β-[(2R,S)-2-(2-BOC-Aminothiazol-4-yl)-2-(2-äthoxycarbonylamino-äthansulfonylamino)-acetamido]-3-cephem-4-carbonsäurediphenylmethylester

Analog Beispiel 29 b) erhält man durch Behandeln mit 3,8 g (8,4 mMol) (2R,S)-2-(2-Aethoxycarbonylaminoäthansulfonylamino)-2-(2-BOC-aminothiazol-4-yl)-essigsäure mit 3,08 g (8,4 mMol) 7β-Amino-3-cephem-4-carbonsäurediphenylmethylester in Gegenwart von 1,13 g 1-Hydroxybenztriazol und 1,90 g N,N'-Dicyclohexylcarbodiimid die Titelverbindung als amorphes Pulver; Rf (Silicagel): ca. 0,58 (Essigester).

Herstellung des Ausgangsmaterials:

c) (2R,S)-2-(2-Aethoxycarbonylaminoäthansulfonylamino)-2-(2-BOC-aminothiazol-4-yl)-essigsäure

Eine Suspension von 3,8 g (10 mMol) (2R,S)-2-(2-Aminoäthansulfonyl-amino)-2-(2-BOC-aminothiazol-4-yl)-essigsäure in 90 ml absolutem Tetrahydrofuran wird unter Rühren, Feuchtigkeitsausschluss und Stickstoffatmosphäre mit 10 ml N,O-Bis(trimethylsilyl)-acetamid versetzt. Nach einer Stunde Reaktionszeit bei 65° wird das Reaktionsgemisch auf 0° gekühlt, mit 0,90 ml Pyridin und 1,05 ml Chlorameisensäureäthylester versetzt und anschliessend 4 Stunden bei Raumtemperatur gerührt. Nach Entfernen des Lösungsmittels wird der Rückstand in Essigester aufgenommen und je viermal mit 0,5 N Salzsäure und gesättigter Natriumchloridlösung gewaschen. Nach dem Trocknen über Natriumsulfat wird das Lösungsmittel im Rotationsverdampfer entfernt. Man erhält die Titelverbindung, die man ohne weitere Reinigung im nächsten Reaktionsschritt einsetzen kann.

Beispiel 38:

a) 7β-[(2R,S)-2-(2-(4-Nitrobenzolsulfonylamino)-äthansulfonylamino)-2-(2-aminothiazol-4-yl)-acetamido]-3-cephem-4-carbonsäurenatriumsalz

Analog Beispiel 29 a) erhält man durch Umsetzung von 2,0 g (2,19 mMol) 7β-[(2R,S)-2-(2-(4-Nitrobenzolsulfonylamino)-äthansulfonylamino)-2-(2-BOC-aminothiazol-4-yl)-acetamido]-3-cephem-4-carbonsäurediphenylmethylester in Gegenwart von 2,0 ml Anisol und 10 ml Trifluoressigsäure in 10 ml Methylenchlorid das 1,5 Hydrat der Titelverbindung als gelbliches Pulver; F: oberhalb 175° (Zersetzung); $[\alpha]_D^{20°} = +79° \pm 1°$ (0,066% in Wasser); UV (Wasser): 257 (ε = 19200 ; Rf 96 (Silicagel): ca. 0,50.

b) 7β-[(2R,S)-2-(2-(4-Nitrobenzolsulfonylamino)-äthansulfonylamino)-2-(2-BOC-aminothiazol-4-yl)-acetamido]-3-cephem-4-carbonsäurediphenylmethylester

Analog Beispiel 29 b) erhält man durch Behandeln von 3,4 g (6,0 mMol) (2R,S)-2-(2-(4-Nitrobenzolsulfonylamino)-äthansulfonylamino)-2-(2-aminothiazol-4-yl)-essigsäure mit 2,2 g (6,0 mMol) 7β-Amino-3-cephem-4-carbonsäurediphenylmethylester in Gegenwart von 0,81 g 1-Hydroxybenztriazol und 1,36 g N,N'-Dicyclohexylcarbodiimid in 60 ml Tetrahydrofuran die Titelverbindung als amorphes Pulver; Rf (Silicagel): ca 0,58 (Methylenchlorid/Essigester 1:1).

Herstellung des Ausgangsmaterials:

c) (2R,S)-2-(2-(4-Nitrobenzolsulfonylamino)-äthansulfonylamino)-2-(2-aminothiazol-4-yl)-essigsäure

Eine Suspension von 7,61 g (20 mMol) (2R,S)-2-(2-Aminoäthansulfonylamino)-2-(2-BOC-aminothiazol-4-yl)-essigsäure in 200 ml absolutem Tetrahydrofuran wird unter Rühren, Feuchtigkeitsausschluss und Stickstoffatmosphäre mit 20 ml N,O-Bis(trimethylsilyl)-acetamid versetzt. Nach einer Stunde bei 65° wird das Reaktionsgemisch auf 0° gekühlt, mit 2,4 ml Pyridin und 6,65 g 4-Nitrobenzolsulfochlorid versetzt und anschliessend 4 Stunden bei Raumtemperatur gerührt. Nach Entfernen des Lösungsmittels wird der Rückstand in 250 ml Essigester aufgenommen und je viermal mit 0,5 N Salzsäure und gesättigter Natriumchloridlösung gewaschen. Nach dem Trocknen über Natriumsulfat wird das Lösungsmittel am Rotationsverdampfer entfernt. Das Rohprodukt chromatographiert man an 350 g Silicagel mit Methylenchlorid-Essigester (4:1)-Gemisch als Eluiermittel. Man erhält die Titelverbindung als amorphes Pulver.

Beispiel 39:

a) 3-Acetoxymethyl-7β-[(2R,S)-2-(2-(4-nitrobenzolsulfonylamino)-äthansulfonylamino)-2-(2-aminothiazol-4-yl)-acetamido]-3-cephem-4-carbonsäurenatriumsalz

Analog Beispiel 29a) erhält man durch Umsetzung von 6,8 g (6,9 mMol) 3-Acetoxymethyl-7β-[(2R,S)-2-(2-(4-nitrobenzolsulfonylamino)-äthansulfonylamino)-2-(2-BOC-aminothiazol-4-yl)-acetamido]-3-cephem-4-carbonsäurediphenylmethylester in 6,8 ml Anisol mit 6,8 ml Trifluoressigsäure in 68 ml Methylenchlorid die Titelverbindung als blassgelbliches Pulver; F: oberhalb 155° (unter Zersetzung); Rf (Silicagel Opti $UPC_{12}$): ca. 0,23 (Wasser/Acetonitril 4:1); $[\alpha]_D^{20°} = +2° \pm 1°$ (0,858% in Wasser); UV (in Wasser): 258 (ε = 23300).

b) 3-Acetoxymethyl-7β-[(2R,S)-2-(2-(4-nitrobenzolsulfonylamino)-äthansulfonylamino)-2-(2-BOC-aminothiazol-4-yl)-acetamido]-3-cephem-4-carbonsäurediphenylmethylester

Analog Beispiel 29 b) erhält man durch Behandeln von 10,18 g (18 mMol) (2R,S)-2-(2-(4-Nitrobenzolsulfonylamino)-äthansulfonylamino) -2-(2-aminothiazol-4-yl)-essigsäure mit 7,9 g (18 mMol) 3-Acetoxymethyl-7β-amino-3-cephem-4-carbonsäurediphenylmethylester in Gegenwart von 2,43 g 1-Hydroxybenztriazol und 4,1 g N,N'-Dicyclohexylcarbodiimid in 150 ml Tetrahydrofuran die Titelverbindung als blassgelbes Pulver; Rf (Silicagel): ca. 0,53 (Methylenchlorid/Essigester 1:1).

Beispiel 40:

a) 7β-[(2R,S)-2-(2-(2,4-Dinitrobenzolsulfonylamino)-äthansulfonylamino)-2-(2-aminothiazol-4-yl)-acetamido]-3-cephem-4-carbonsäurenatriumsalz

Analog Beispiel 29 a) erhält man durch Umsetzung von 2,0 g (2,1 mMol) 7β-[(2R,S)-2-(2-(2,4-Dinitrobenzolsulfonylamino)-äthansulfonylamino)-2-(2-BOC-aminothiazol-4-yl)-acetamido]-3-cephem-4-carbonsäurediphenylmethylester in Gegenwart von 2,0 ml Anisol und 10 ml Trifluoressigsäure in 10 ml Methylenchlorid das Dihydrat der Titelverbindung als gelbliches Pulver; F: oberhalb 150°(Zersetzung); $[\alpha]_D^{20°} = +78° \pm 1°$ (0,59% in Wasser); Rf: ca. 0,30 (Silicagel Opti-UPC 12, Wasser-Acetonitril 4:1); UV: 245 (31500,in Wasser).

b) 7β-[(2R,S)-2-(2-(2,4-Dinitrobenzolsulfonylamino)-äthansulfonylamino)-2-(2-BOC-aminothiazol-4-yl)-acetamido]-3-cephem-4-carbonsäurediphenylmethylester

Analog Beispiel 29 b) erhält man durch Behandeln von 2,0 g (3,3 mMol) (2R,S)-2-(2-(2,4-Dinitrobenzolsulfonylamino)-äthansulfonylamino)-2-(2-BOC-aminothiazol-4-yl)-essigsäure mit 2,0 g 3-Acetoxymethyl-7β-amino-3-cephem-4-carbonsäurediphenylmethylester in Gegenwart von 0,44 g 1-Hydroxybenztriazol und 0,74 g N,N'-Dicyclohexylcarbodiimid in 40 ml Tetrahydrofuran die Titelverbindung als blassgelbes, amorphes Pulver; Rf (Silicagel): ca. 0,48 (Methylenchlorid/Essigester 1:1).

Herstellung des Ausgangsmaterials:

c) (2R,S)-2-(2-(2,4-Dinitrobenzolsulfonylamino)-äthansulfonylamino)-2-(2-BOC-aminothiazol-4-yl)-essigsäure

Eine Suspension von 3,80 g (10 mMol) (2R,S)-2-(2-Aminoäthansulfonylamino)-2-(2-BOC-aminothiazol-4-yl)-essigsäure in 100 ml absolutem Tetrahydrofuran wird unter Rühren, Feuchtigkeitsausschluss und Stickstoffatmosphäre mit 10 ml N,O-Bis(trimethylsilyl)-acetamid versetzt. Nach einer Stunde Reaktionszeit bei 65° wird das Reaktionsgemisch auf 0° gekühlt, mit 1,2 ml Pyridin und 4,0 g 2,4-Dinitrobenzolsulfochlorid versetzt und anschliessend 4 Stunden bei Raumtemperatur gerührt. Nach Entfernen des Lösungsmittels wird der Rückstand in 250 ml Essigester aufgenommen und je viermal mit 0,5 N Salzsäure und gesättigter Natriumchloridlösung gewaschen. Nach Trocknen über Natriumsulfat wird das Lösungsmittel am Rotationsverdampfer entfernt. Das Rohprodukt reinigt man an 250 g Silicagel mit Methylenchlorid-Essigester (4:1)-Gemisch als Eluiermittel. Man erhält die Titelverbindung als amorphes Pulver.

Beispiel 41:

a) 7β-[(2R,S)-2-(2-Aminothiazol-4-yl)-2-(2-cyanmethansulfonylaminoäthansulfonylamino)-acetamido]-3-cephem-4-carbonsäurenatriumsalz

0,5 g 7β-[(2R,S)-2-(2-BOC-Aminothiazol-4-yl)-2-(2-cyanmethansulfonylaminoäthansulfonylamino)-acetamido]-3-cephem-4-carbonsäurediphenylmethylester werden in 2 ml $CH_2Cl_2$ und 0,2 ml Anisol mit 5 ml Trifluoressigsäure analog Beispiel 1 a) umgesetzt, aufgearbeitet, chromatographiert und umgefällt. Man erhält das Hydrat der Titelverbindung; F: Zersetzung ab 220°; IR: u.a. 2374, 1755 (Nujol); UV: 251 (9700), 320 (650; $H_2O$).

b) 7β-[(2R,S)-2-(2-BOC-Aminothiazol-4-yl)-2-(2-cyanmethansulfonylaminoäthansulfonylamino)-acetamido]-3-cephem-4-carbonsäurediphenylmethylester

2,2 g (2R,S)-2-(2-BOC-Aminothiazol-4-yl)-2-(2-cyanmethansulfonylamino)-äthansulfonylamino)-essigsäure werden mit 0,6 g 7β-Amino-3-cephem-4-carbonsäurediphenylmethylester in 25 ml Tetrahydrofuran analog Beispiel 6 b) umgesetzt (0,6 g Hydroxybenztriazol; dreimal 0,3 g Dicyclohexylcarbodiimid in je 5 ml Tetrahydrofuran), aufgearbeitet und chromatographiert. Man erhält die Titelverbindung; IR: 3400, 3300, 2775, 1779, 1710, 1660, 1520 ($CH_2Cl_2$); UV: 259 (3200; EtOH).

Herstellung des Ausgangsmaterials:

c) (2R,S)-2-(2-BOC-Aminothiazol-4-yl)-2-(2-cyanmethansulfonylaminoäthansulfonylamino)-essigsäure

6,7 g (2R,S)-2-(2-BOC-Aminothiazol-4-yl)-2-(2-aminoäthansulfonylamino)-essigsäure werden in 25 ml Tetrahydrofuran mit 1,5 ml Cyanomethylsulfochlorid analog Beispiel 6 c) umgesetzt (10 ml N,O-Bis(trimethylsilyl)-acetamid; 0,81 ml Pyridin) und aufgearbeitet. Man erhält die Titelverbindung, die ohne Charakterisierung weiterverarbeitet wird.

Beispiel 42:

a) 7β-[(2R,S)-2-(2-Methylaminoäthansulfonylamino)-2-(2-aminothiazol-4-yl)-acetamido]-3-cephem-4-carbonsäure

3,4 g des gemäss Beispiel 42b) erhältlichen 7β-[(2R,S)-2-(2-Methylaminoäthansulfonylamino)-2-(2-BOC-aminothiazol-4-yl)-acetamido]-3-cephem-4-carbonsäurediphenylmethylesters werden in 10 ml Methylenchlorid gelöst, die Lösung sukzessive mit 1,3 ml Anisol und 62 ml Trifluoressigsäure versetzt und anschliessend unter Ausschluss von Luftfeuchtigkeit 1 Stunde bei Raumtemperatur gerührt. Aus der anfänglich klaren Lösung fällt ein voluminöser Niederschlag aus. Dann wird die Suspension auf ein eiskaltes Gemisch von Petroläther (600 ml) und Diäthyläther (300 ml) gegossen, das anfallende Trifluoracetat abgenutscht, mit Petroläther gewaschen und im Hochvakuum bei Raumtemperatur getrocknet. Darauf wird das rohe Trifluoracetat-Salz in 20 ml Aethanol-Wasser-(1:1)-Gemisch gelöst, die Lösung auf +5° abgekühlt, und unter Rühren und Kühlen durch Zutropfen von 2 N Natriumhydroxid-Lösung ein pH-Wert von 5.0 eingestellt. Dann wird die Lösung auf 600 ml Aethanol gegossen und am Rotationsverdampfer bei 50° auf ca. 100 ml Volumen eingeengt. Diese Operation wird zweimal mit je 300 ml Aethanol-Zusatz wiederholt, wobei das amorphe Produkt ausfällt. Es wird abgenutscht und sukzessive mit Aethanol, einem Gemisch von Aethanol-Diäthyläther und Diäthyläther gewaschen. Man erhält das Hydrat der Titelverbindung; F: Zersetzung ab 190°; $[\alpha]_D^{20°} = +91 \pm 1°$ (1,138 % in 0,1 N HCl). IR: 3320 (breit), 3195, 3120, 1775 (Schulter) 1765, 1690 (Schulter), 1680 (Schulter), 1670, 1640 (Schulter), 1615 (breit), 1520, 1380, 1350, 1150, 1120 (Schulter) (in Nujol).
Rf (Silicagel): 0.23 (n-Butanol-Pyridin-Eisessig-Wasser 42:24:4:30).

b) 7β-[(2R,S)-2-(2-Methylaminoäthansulfonylamino)-2-(2-BOC-aminothiazol-4-yl)-acetamido]-3-cephem-4-carbonsäurediphenylmethylester

Eine Lösung von 5,7 g 7β-[(2R,S)-2-(2-(N-Methyl-2,2,2-trichloräthoxycarbonylamino)-äthansulfonylamino)-2-(2-BOC-aminothiazol-4-yl)-

acetamido]-3-cephem-4-carbonsäurediphenylmethylester in 100 ml Acetonitril-Essigsäure-(1:1)-Gemisch wird unter Rühren mit 3,2 g Zink-Staub versetzt und 3 Stunden bei Raumtemperatur kräftig gerührt. Darauf wird die Suspension durch Celit® filtriert, das Nutschgut nochmals mit Acetonitril gewaschen und das Filtrat am Rotationsverdampfer bei 45° auf ca. 20 ml Volumen eingeengt. Die Lösung wird mit Aethylacetat verdünnt, sukzessive mit Wasser und 1 N Natriumhydrogencarbonat-Lösung (pH 8) mehrmals gewaschen, über Natriumsulfat getrocknet und am Rotationsverdampfer bei 45° eingedampft. Das Rohprodukt wird durch Chromatographieren an 25-facher Menge Kieselgel gereinigt. Eluiermittel: Methylenchlorid-Methanol-(97:3). Dabei erhält man die Titelverbindung als Schaum; Rf (Silicagel): ca. 0,45 (Methylenchlorid/Methanol 9:1); IR: 3280 (breit), 1785, 1720, 1690 (Schulter), 1635, 1545, 1380 (Schulter), 1370, 1330, 1185, 1145 (in Nujol).

c) <u>7β-[(2R,S)-2-(2-(N-Methyl-2,2,2-trichloräthoxycarbonylamino)-äthansulfonylamino)-2-(2-BOC-aminothiazol-4-yl)-acetamido]-3-cephem-4-carbonsäurediphenylmethylester</u>

Eine Lösung von 4,50 g (2R,S)-2-[2-(N-Methyl-2,2,2-trichloräthoxycarbonylamino)-äthansulfonylamino]-2-(2-BOC-aminothiazol-4-yl)-essigsäure, 2,58 g 7β-Amino-3-cephem-4-carbonsäurediphenylmethylester und 0,65 g 1-Hydroxy-benzotriazol in 52 ml Tetrahydrofuran wird auf +2° abgekühlt, eine Lösung von 0,82 g N,N'-Dicyclohexylcarbodiimid in 7 ml Tetrahydrofuran innerhalb 10 Minuten zugetropft und das Reaktionsgemisch im Eisbad gerührt. Nach 3 Stunden wird eine weitere Lösung von 0,82 g N,N'-Dicyclohexylcarbodiimid in 7 ml Tetrahydrofuran in das Reaktionsgemisch getropft. Nach insgesamt 6 Stunden Rühren bei 0° wird die Suspension abgenutscht, der Rückstand mit Aethylacetat gewaschen, das Filtrat mit Aethylacetat verdünnt, mit 1 N Natriumhydrogencarbonat-Lösung und Wasser mehrmals gewaschen, über Natriumsulfat getrocknet und am Rotationsverdampfer bei 45°

eingedampft. Das Rohprodukt wird durch Chromatographieren an 20-facher Menge Kieselgel gereinigt. Eluiermittel: Methylenchlorid mit 2 bis 5 % Methylacetat. Man erhält die Titelverbindung als Schaum. DC (Silicagel; Identification: UV 366): Rf = ca. 0.60 (Doppelflecken-Diastereomerengemisch-Toluol-Aethylacetat-1:1). IR: 3280 (breit), 1790, 1725, 1690 (Schulter), 1640, 1565 (Schulter), 1550, 1380, 1335, 1190, 1155 (Nujol).

Herstellung des Ausgangsmaterials:

d) (2R,S)-2-[2-(N-Methyl-2,2,2-trichloräthoxycarbonylamino)-äthansulfonylamino]-2-(2-BOC-aminothiazol-4-yl)-essigsäure

Eine Suspension von 5,0 g (2R,S)-2-Amino-2-(2-BOC-aminothiazol-4-yl)-essigsäure in 80 ml Acetonitril-Methylenchlorid-1:1-Gemisch wird mit 18,5 ml N,O-Bis-(trimethylsilyl)-acetamid versetzt und unter Feuchtigkeitsausschluss bei Raumtemperatur gerührt, wobei die Säure sich langsam löst. Nach 1 Stunde Rühren wird die klare Lösung auf 0° abgekühlt und mit 4,0 ml absolutem Pyridin versetzt. Man tropft eine Lösung von 9,0 g 2-(N-Methyl-2,2,2-trichloräthoxycarbonylamino)-äthansulfonylchlorid in 20 ml Methylenchlorid unter Rühren und Kühlen innerhalb 30 Minuten zu und rührt das Reaktionsgemisch 1,5 Stunden bei Raumtemperatur. Darauf wird die Suspension mit Aethylacetat verdünnt, etwas eingeengt,zweimal mit je 30 ml 1 N Salzsäure (pH ca. 2) und zweimal mit Sodalösung gewaschen, über Natriumsulfat getrocknet und am Rotationsverdampfer bei 50° eingedampft. Das Rohprodukt wird durch Chromatographieren an 20-facher Menge Kieselgel gereinigt. Eluiermittel: Methylenchlorid mit 10 bis 30 % Methylacetat. Man erhält die Titelverbindung als Schaum. DC (Silicagel; Identifikation: UV 366 nm). Rf = 0.48 (Methylenchlorid-Methanol-4:1). IR: 3200 (breit), 1715 (breit), 1680 (Schulter), 1540, 1370, 1325, 1185, 1145 (in Methylenchlorid).

e) 2-(N-Methyl-2,2,2-trichloräthoxycarbonylamino)-äthansulfochlorid

Eine Suspension von 50 g 2-Methylaminoäthansulfonsäure (hergestellt nach der Vorschrift von B. Josephsch, Biochemische Zeitschrift, Berlin, 265, 448 (1933) - CA 28: 7909 (1934) in 1400 ml Pyridin wird auf +10° abgekühlt. 50 ml Chlorameisensäure-2,2,2-trichloräthylester werden innerhalb 40 Minuten unter starkem Rühren und Kühlen zwischen +10 bis +15° hinzugetropft, und die Mischung 18 Stunden bei Raumtemperatur gerührt. Darauf wird die Suspension durch Celite® abgenutscht und der Rückstand mit Pyridin gewaschen. Das Filtrat wird am Rotationsverdampfer bei 55° eingedampft, der ölige Rückstand mehrmals mit Toluol versetzt, wobei das Pyridinium-Salz der 2-(N-Methyl-2,2,2-trichloräthoxycarbonylamino)-äthansulfonsäure kristallin ausfällt. Dieses wird mit Petroläther vermischt, abgenutscht und mit Petroläther gewaschen. F. 77-85° (unter Zersetzung).

Eine Lösung von 76 g 2-(N-Methyl-2,2,2-trichloräthoxycarbonylamino)-äthansulfonsäure-pyridiniumsalz in 380 ml Chloroform wird unter kräftigem Rühren innerhalb 30 Minuten bei Raumtemperatur portionsweise mit 38 g Phosphorpentachlorid versetzt, wobei unter Chlorwasserstoffentwicklung die Temperatur auf 35 - 40° ansteigt. Anschliessend wird das Reaktionsgemisch 3 Stunden am Rückfluss erhitzt, die Lösung auf +5° abgekühlt, mit 200 ml Benzol verdünnt, mit 150 ml eiskaltem Wasser gewaschen, über Natriumsulfat getrocknet und am Rotationsverdampfer bei 55° eingedampft. Die zurückbleibende, halbfeste Masse wird mit 300 ml Diäthyläther vermischt, die anfallenden Kristalle (Nebenprodukt) abgenutscht und der Rückstand mit etwas Diäthyläther gewaschen. Das Filtrat wird am Rotationsverdampfer bei 55° eingedampft. Es bleibt das ölige 2-(N-Methyl-2,2,2-trichloräthoxycarbonylamino)-äthansulfonylchlorid zurück. IR: 1715, 1620 (Schulter), 1370, 1180, 1160 (in Methylenchlorid).

Beispiel 43:

a) 7β-[(2R,S)-2-(2-Methoxyäthansulfonylamino)-2-(2-aminothiazol-4-yl)-acetamido]-3-cephem-4-carbonsäure

Analog Beispiel 42a) erhält man durch Behandeln von 1,8 g 7β-[(2R,S)-2-(2-Methoxyäthansulfonylamino)-2-(2-BOC-aminothiazol-4-yl)-acetamido]-3-cephem-4-carbonsäurediphenylmethylester (hergestellt nach Beispiel 42b) mit 50 ml Trifluoressigsäure und 0,8 ml Anisol in 5 ml Methylenchlorid das Hydrat der Titelverbindung. F: oberhalb 180° (Zersetzung); $[\alpha]_D^{20}$ = +96±1° (1,955 % in $H_2O$); IR: 3320 (breit), 3210, 1775 (Schulter), 1765 (breit), 1705 (Schulter), 1680, 1605, 1520, 1375, 1365, 1160, 1145 (in Nujol); DC (Silicagel; Identifikation: UV 366 nm): Rf = 0.39 (n-Butanol-Pyridin-Eisessig-Wasser-42:24:4:30).

b) 7β-[(2R,S)-2-(2-Methoxyäthansulfonylamino)-2-(2-BOC-aminothiazol-4-yl)-acetamido]-3-cephem-4-carbonsäurediphenylmethylester

Durch Umsetzung von 3,0 g (2R,S)-2-(2-Methoxyäthansulfonylamino)-2-(2-BOC-aminothiazol-4-yl)-essigsäure mit 2,6 g 7β-Amino-3-cephem-4-carbonsäurediphenylmethylester in Gegenwart von 0,55 g 1-Hydroxybenzotriazol und 1,54 g N,N'-Dicyclohexylcarbodiimid in 45 ml Tetrahydrofuran erhält man die Titelverbindung. Das Rohprodukt wird an 20-facher Menge Kieselgel gereinigt. Eluiermittel: Methylenchlorid-Methylacetat-(9:1). DC (Silicagel; Identifikation: UV 366 nm): Rf = 0,58 (Doppelflecken-Diastereomerengemisch-System: Toluol-Chloroform-Aethylacetat-Aethanol-32:32:32:5).

Herstellung des Ausgangsmaterials:

c) (2R,S)-2-(2-Methoxyäthansulfonylamino)-2-(2-BOC-aminothiazol-4-yl)-essigsäure

Eine Lösung von 5,4 g (2R,S)-2-(2-Methoxyäthansulfonylamino)-2-(2-BOC-aminothiazol-4-yl)-essigsäuremethylester in 45 ml Methanol wird mit 11 ml 2 N Natriumhydroxid-Lösung versetzt und bei Raumtemperatur

gerührt. Nach 2,5 Stunden Reaktionszeit werden weitere 6 ml 2 N Natriumhydroxid-Lösung zugesetzt. Nach insgesamt 5 Stunden Reaktionszeit wird der pH-Wert der klaren Lösung durch Zutropfen von 1 N Salzsäure auf 7,5 gestellt und der grösste Teil des Methanols am Rotationsverdampfer bei 50° abgedampft. Darauf wird die wässrige Lösung auf 0° abgekühlt, mit Aethylacetat überschichtet, unter Rühren durch Zusetzen von 20%iger, wässriger Zitronensäure angesäuert (pH 2,5-3,0) und zweimal mit Aethylacetat ausgezogen. Die organischen Extrakte werden vereinigt, zweimal mit etwas Sodalösung gewaschen, über Natriumsulfat getrocknet und am Rotationsverdampfer bei 50° eingedampft. Das Rohprodukt wird durch Chromatographieren an 20-facher Menge Kieselgel gereinigt. Eluiermittel: Methylenchlorid mit 30 bis 40% Methylacetat. Dabei erhält man die Titelverbindung als gelben Schaum. DC (Silicagel; Identifikation: UV 366 bm): Rf = 0,47 (n-Butanol-Eisessig-Wasser 67:10:23); IR: 3320 (breit), 3300 (Schulter), 2950, 1770 (Schulter), 1730, 1570 (Schulter), 1550, 1390, 1370, 1140, 1110 (in Methylenchlorid).

d) (2R,S)-2-(2-Methoxyäthansulfonylamino)-2-(2-BOC-aminothiazol-4-yl)-essigsäuremethylester

Eine Lösung von 5,0 g (2R,S)-2-(2-BOC-Aminothiazol-4-yl)-glycinmethylester in 40 ml Dioxan und 10 ml N-Methylmorpholin wird auf +2° abgekühlt. Man tropft eine Lösung von 30 ml 2-Methoxyäthansulfonylchlorid (hergestellt nach der Vorschrift von A.S. Matlack, J.org. Chem., 23, 729 (1958)) in 20 ml Dioxan innerhalb 20 Minuten unter Rühren und Kühlen und rührt das Reaktionsgemisch 2,5 Stunden bei Raumtemperatur. Die Suspension wird am Rotationsverdampfer bei 50° auf das halbe Volumen eingeengt, mit Aethylacetat verdünnt, mit Wasser, 20 %iger wässriger Zitronensäure (pH~3) und nochmals dreimal mit etwas Wasser gewaschen, und über Natriumsulfat getrocknet. Nach Abdampfen des Lösungsmittels am Rotationsverdampfer bei 50° bleibt die Titelverbindung als gelber Schaum zurück. DC (Silicagel; Identifikation: UV 366 nm): Rf = 0.54 (Toluol-Chloroform-Aethylacetat-Aethanol-32:32:32:5) IR: 3390, 3260, 1740 (Schulter),1720,

1535, 1370, 1330, 1185 (Schulter), 1125 (in Methylenchlorid).

Beispiel 44:

a) 7β-[(2R,S)-2-(2-Aminothiazol-4-yl)-2-cyanomethansulfonylamino-acetamido]-3-cephem-4-carbonsäurenatriumsalz

4,3 g 7β-[(2R,S)-2-(2-BOC-Aminothiazol-4-yl)-2-cyanomethansulfonyl-aminoacetamido]-3-cephem-4-carbonsäurediphenylmethylester werden in 20 ml $CH_2Cl_2$ und 1,4 ml Anisol mit 15 ml Trifluoressigsäure analog Beispiel 1a) umgesetzt, aufgearbeitet, chromatographiert und umgefällt. Man erhält das Hydrat der Titelverbindung. F: oberhalb 230° (Zersetzung); $[\alpha]_D^{20°}$ = +99°±1° (0,90 % in $H_2O$); IR: 3700 bis 2500, 2260, 1755, 1675, 1600, 1520 (Nujol); UV: 250 (9200), 320 (700; $H_2O$).

b) 7β-[(2R,S)-2-(2-BOC-Aminothiazol-4-yl)-2-cyanomethansulfonylamino-acetamido]-3-cephem-4-carbonsäurediphenylmethylester

3,9 g (2R,S)-2-(2-BOC-Aminothiazol-4-yl)-2-cyanomethansulfonylamino-essigsäure werden mit 3,8 g 7β-Amino-3-cephem-4-carbonsäurediphenylmethylester in 45 ml Tetrahydrofuran analog Beispiel 6b) umgesetzt (1,35 g Hydroxybenztriazol; dreimal 0,66 g Dicyclohexylcarbodiimid in je 8 ml Tetrahydrofuran), aufgearbeitet und chromatographiert. Man erhält die Titelverbindung. $[\alpha]_D^{20°}$ = +14°±1° (0,92 % in $CHCl_3$); IR: 3400, 3300, 2260 (schwach), 1780, 1720, 1700, 1635, 1530 ($CH_2Cl_2$); UV: 259 (14000; EtOH).

Herstellung des Ausgangsmaterials:

c) (2R,S)-2-(2-BOC-Aminothiazol-4-yl)-2-cyanomethansulfonylaminoes-sigsäure

3,4 g (2R,S)-2-(2-BOC-Aminothiazol-4-yl)-2-aminoessigsäure werden in 30 ml $CH_2Cl_2$ mit 2,6 g Cyanomethylsulfochlorid analog Beispiel 6c umgesetzt (10 ml N,O-Bis-(trimethylsilyl)-acetamid; 1,0 ml Pyridin) und aufgearbeitet. Man erhält die Titelverbindung, die ohne Charakterisierung weiterverarbeitet wird.

Beispiel 45:

a) 7β-[(2R,S)-2-(2-Aminothiazol-4-yl)-2-(2-((3R)-3-amino-3-carboxypropionylamino)-äthansulfonylamino)-acetamido]-3-cephem-4-carbonsäurenatriumsalz

3,1 g 7β-[(2R,S)-2-(2-BOC-Aminothiazol-4-yl)-2-(2-((3R)-3-BOC-amino-3-t.-butoxycarbonylpropionylamino)-äthansulfonylamino)-acetamido]-3-cephem-4-carbonsäurediphenylmethylester werden in 7,5 ml $CH_2Cl_2$ und 5 ml Anisol mit 60 ml Trifluoressigsäure analog Beispiel 1a) umgesetzt, aufgearbeitet und umgefällt. Man erhält das Hydrat der Titelverbindung. F: oberhalb 210° (Zersetzung); $[\alpha]_D^{20°}$ = +89°±1° (0,84 % in $H_2O$); IR: 3700 bis 2500 (breit), 1760, 1640, 1600, 1520 (Nujol); UV: 252 (9200), 316 (660; $H_2O$).

b) 7β-[(2R,S)-2-(2-BOC-Aminothiazol-4-yl)-2-(2-((3R)-3-BOC-amino-3-t.-butoxycarbonylpropionylamino)-äthansulfonylamino)-acetamido]-3-cephem-4-carbonsäurenatriumsalz

0,5 g 7β-[(2R,S)-2-(2-BOC-Aminothiazol-4-yl)-2-(2-aminoäthansulfonylamino)-acetamido]-3-cephem-4-carbonsäurediphenylmethylester (Herstellung siehe Beispiel 35c) werden mit 0,22 g (3R)-3-BOC-Amino-3-t.butoxycarbonylpropionsäure in 2,5 ml Tetrahydrofuran analog Beispiel 6b) umgesetzt (70 mg Hydroxybenztriazol; dreimal 60 mg Dicyclohexylcarbodiimid in je 0,5 ml Tetrahydrofuran), aufgearbeitet und chromatographiert. Man erhält die Titelverbindung; $[\alpha]_D^{20°}$ = +32°±1° (0,96 % in $CHCl_3$); IR: 3400, 3280, 1780, 1705, 1675, 1530 ($CH_2Cl_2$); UV: 257 (14 000; EtOH).

Beispiel 46:

a) 7β-[(2R,S)-2-(2-Aminothiazol-4-yl)-2-(2-pivaloylaminoäthansulfonylamino)-acetamido]-3-cephem-4-carbonsäurenatriumsalz

1,62 g 7β-[(2R,S)-2-(2-BOC-Aminothiazol-4-yl)-2-(2-pivaloylamino-

äthansulfonylamino)-acetamido]-3-cephem-4-carbonsäurediphenylmethylester werden in 3,6 ml $CH_2Cl_2$ und 1,13 ml Anisol mit 13,5 ml Trifluoressigsäure analog Beispiel 1a) umgesetzt, aufgearbeitet und umgefällt. Man erhält das Hydrat der Titelverbindung. F: oberhalb 144° (Zersetzung); $[\alpha]_D^{20°} = +92° \pm 1°$ (0,69 % in $H_2O$); IR: 3700 bis 2700 (breit), 1755, 1675 (Schulter) 1657, 1616, 1522 (Nujol); UV 250 (9700; $H_2O$).

b) 7β-[(2R,S)-2-(2-BOC-Aminothiazol-4-yl)-2-(2-pivaloylaminoäthansulfonylamino)-acetamido]-3-cephem-4-carbonsäurediphenylmethylester

2,06 g (2R,S)-2-(2-BOC-Aminothiazol-4-yl)-2-(2-pivaloylaminoäthansulfonylamino)-essigsäure werden mit 1,6 g 7β-Amino-3-cephem-4-carbonsäurediphenylmethylester in 17 ml Tetrahydrofuran analog Beispiel 6b) umgesetzt (0,45 g Hydroxybenztriazol; dreimal 0,39 g Dicyclohyexylcarbodiimid in jeweils 3,4 ml Tetrahydrofuran), aufgearbeitet und chromatographiert. Man erhält die Titelverbindung. $[\alpha]_D^{20°} = +20° \pm 1°$(0,69 % in $CHCl_3$); IR: 3450, 3400, 3280, 1780, 1715, 1695, 1650, 1520 $cm^{-1}$ ($CH_2Cl_2$); UV: 258 (14 000; EtOH).

Herstellung des Ausgangsmaterials:

c) (2R,S)-2-(2-BOC-Aminothiazol-4-yl)-2-(2-pivaloylaminoäthansulfonylamino)-essigsäure

8,5 g (2R,S)-2-(2-BOC-Aminothiazol-4-yl)-2-(2-aminoäthansulfonylamino)-essigsäure werden in 30 ml Tetrahydrofuran mit 1,56 ml Pivalinsäurechlorid analog Beispiel 6c) umgesetzt (12,5 ml N,O-Bis-(trimethylsilyl)-acetamid; 1,03 ml Pyridin) und aufgearbeitet. Man erhält die Titelverbindung, die ohne Charakterisierung weiterverarbeitet wird.

Beispiel 47:

a) 7β-[(2R,S)-2-(2-Aminothiazol-4-yl)-2-(2-((2R)-2-amino-2-phenylacetamido)-äthansulfonylamino)-acetamido]-3-cephem-4-carbonsäure

2,1 g 7β-[(2R,S)-2-(2-BOC-Aminothiazol-4-yl)-2-(2-((2R)-2-BOC-amino-2-phenylacetamido)-äthansulfonylamino)-acetamido]-3-cephem-4-carbonsäurediphenylmethylester werden in 10 ml $CH_2Cl_2$ und 0,57 ml Anisol mit 15 ml Trifluoressigsäure analog Beispiel 1a umgesetzt, aufgearbeitet, chromatographiert und umgefällt. Man erhält das Hydrat der Titelverbindung; F: oberhalb 240° (unter Zersetzung): $[\alpha]_D^{20°} = +57° \pm 1°$ (0,97 % in $H_2O$); IR: 3700 bis 2500 (breit), 1760, 1670, 1600, 1522 (Nujol); UV: 251 (9200), 320 (1000; $H_2O$).

b) 7β-[(2R,S)-2-(2-BOC-Aminothiazol-4-yl)-2-(2-((2R)-2-BOC-amino-2-2-phenylacetamido)-äthansulfonylamino)-acetamido]-3-cephem-4-carbonsäurediphenylmethylester

1 g 7β-[(2R,S)-2-(2-BOC-Aminothiazol-4-yl)-2-(2-aminoäthansulfonylamino)-acetamido]-3-cephem-4-carbonsäurediphenylmethylester (Herstellung siehe Beispiel 35c) werden mit 0,34 g N-BOC-(D)-Phenylglycin in 15 ml Tetrahydrofuran analog Beispiel 6b) umgesetzt (0,18 g Hydroxybenztriazol; dreimal 0,09 g Dicyclohexylcarbodiimid in je 2 ml Tetrahydrofuran), aufgearbeitet und chromatographiert. Man erhält die Titelverbindung. $[\alpha]_D^{20°} = -3° \pm 1°$ (0,99 % in $CHCl_3$); IR: 3400, 3300, 1790, 1725, 1680, 1639, 1600, 1541 $cm^{-1}$ ($CH_2Cl_2$); UV: 258 (13 100; EtOH).

Beispiel 48:

a) 7β-[(2R,S)-2-(2-Aminothiazol-4-yl)-2-(2-(2-aminoacetamido)-äthansulfonylamino)-acetamido]-3-cephem-4-carbonsäure

1,9 g 7β-[(2R,S)-2-(2-BOC-Aminothiazol-4-yl)-2-(2-(2-BOC-aminoacetamido)-äthansulfonylamino)-acetamido]-3-cephem-4-carbonsäurediphenylmethylester werden in 10 ml $CH_2Cl_2$ und 0,57 ml Anisol mit 15 ml Trifluoressigsäure analog Beispiel 1a) umgesetzt, aufgearbeitet, chromatographiert und umgefällt. Man erhält das Hydrat der Titelver-

bindung. F: oberhalb 230° (Zersetzung); $[\alpha]_D^{20°}$ = +78°±1° (0,92 % in HCOOH); IR: 3700 bis 2500, 1765, 1685, 1654, 1611, 1542, 1529 $cm^{-1}$ (Nujol); UV: 252 (9500), 318 (300; $H_2O$).

b) 7β-[(2R,S)-2-(2-BOC-Aminothiazol-4-yl)-2-(2-(2-BOC-aminoacetamido)-äthansulfonylamino)-acetamido]-3-cephem-4-carbonsäure-diphenylmethylester

3,1 g 7 β-[(2R,S)-2-(2-BOC-Aminothiazol-4-yl)-2-(2-aminoäthansulfonylamino)-acetamido]-3-cephem-4-carbonsäurediphenylmethylester (Herstellung siehe Beispiel 35c) werden mit 0,74 g N-BOC-Glycin in 45 ml Tetrahydrofuran analog Beispiel 6b) umgesetzt (0,56 g Hydroxybenztriazol; dreimal 0,29 g Dicyclohexylcarbodiimid in je 6 ml Tetrahydrofuran), aufgearbeitet und chromatographiert. Man erhält die Titelverbindung; $[\alpha]_D^{20°}$ = +24°±1° (1,00 % in $CHCl_3$); IR: 3400, 3300, 1790, 1725, 1690, 1640, 1542 $cm^{-1}$ ($CH_2Cl_2$); UV: 259 (14000; EtOH).

Beispiel 49:

a) 7β-[(2R,S)-2-(2-Aminothiazol-4-yl)-2-(2-methoxalylaminoäthansulfonylamino)-acetamido]-3-cephem-4-carbonsäurenatriumsalz

1,7 g 7β-[(2R,S)-2-(2-BOC-Aminothiazol-4-yl)-2-(2-methoxalylamino-äthansulfonylamino)-acetamido]-3-cephem-4-carbonsäurediphenylmethylester werden in 8 ml $CH_2Cl_2$ und 0,43 ml Anisol mit 12 ml Trilfuoressigsäure analog Beispiel 1a) umgesetzt, aufgearbeitet, chromatographiert und umgefällt. Man erhält das Hydrat der Titelverbindung; F: oberhalb 200° (unter Zersetzung); $[\alpha]_D^{20°}$ = +73°±1° (0,89 % in $H_2O$); IR: 3700 bis 2600 (breit), 1760, 1685, 1600, 1525 (Nujol).

b) 7β-[(2R,S)-2-(2-BOC-Aminothiazol-4-yl)-2-(2-methoxalylaminoäthansulfonylamino)-acetamido]-3-cephem-4-carbonsäurediphenylmethylester

3,2 g (2R,S)-2-(2-BOC-Aminothiazol-4-yl)-2-(2-methoxalylaminoäthansulfonylamino)-essigsäure werden mit 2,48 g 7β-Amino-3-cephem-4-carbonsäurediphenylmethylester in 26,2 ml Tetrahydrofuran analog Beispiel 6b) umgesetzt (0,69 g Hydroxybenztriazol; dreimal 0,6 g Dicyclohexylcarbodiimid in je 5,2 ml Tetrahydrofuran), aufgearbeitet und chromatographiert. Man erhält die Titelverbindung; $[\alpha]_D^{20°}$ = +17°±1° (0,84 % in $CHCl_3$); IR: 3400, 3300, 1788, 1725, 1705, 1637, 1602, 1540 ($CH_2Cl_2$); UV: 250 (9200; EtOH).

Herstellung des Ausgangsmaterials:

c) <u>(2R,S)-2-(2-BOC-Aminothiazol-4-yl)-2-(2-methoxalylaminoäthansulfonylamino)-essigsäure</u>

6,7 g (2R,S)-2-(2-BOC-Aminothiazol-4-yl)-2-(2-aminoäthansulfonylamino)-essigsäure werden in 24 ml Tetrahydrofuran mit 0,92 ml Oxalsäuremonomethylester-chlorid analog Beispiel 6c) umgesetzt (16 ml N,O-Bis-(trimethylsilyl)-acetamid; 0,81 ml Pyridin) und aufgearbeitet. Man erhält die Titelverbindung, die ohne Charakterisierung weiterverarbeitet wird.

Beispiel 50:

a) 7β-[(2R,S)-2-(2-Aminothiazol-4-yl)-2-(2-methoxymalonylaminoäthansulfonylamino)-acetamido]-3-cephem-4-carbonsäurenatriumsalz

2,817 g 7β-[(2R,S)-2-(2-BOC-Aminothiazol-4-yl)-2-(2-methoxymalonylaminoäthansulfonylamino)-acetamido]-3-cephem-4-carbonsäurediphenylmethylester werden in 6 ml $CH_2Cl_2$ und 1,92 ml Anisol mit 22 ml Trifluoressigsäure analog Beispiel 1a) umgesetzt, aufgearbeitet, chromatographiert und umgefällt. Man erhält das Hydrat der Titelverbindung. F: oberhalb 165° (unter Zersetzung); $[\alpha]_D^{20°}$ = +94°±1° (0,75 % in $H_2O$); IR: 3700 bis 2600 (breit), 1765, 1665, 1606, 1525 (Nujol); UV: 252 (9800; $H_2O$).

b) 7β-[(2R,S)-2-(2-BOC-Aminothiazol-4-yl)-2-(2-methoxymalonylaminoäthansulfonylamino)-acetamido]-3-cephem-4-carbonsäurediphenylmethylester

4,18 g (2R,S)-2-(2-BOC-Aminothiazol-4-yl)-2-(2-methoxymalonylaminoäthansulfonylamino)-essigsäure werden mit 2,9 g 7β-Amino-3-cephem-4-carbonsäurediphenylmethylester in 25 ml Tetrahydrofuran analog Beispiel 6b) umgesetzt (0,87 g Hydroxybenztriazol; dreimal 0,76 g Dicyclohexylcarbodiimid in je 5 ml Tetrahydrofuran), aufgearbeitet und chromatographiert. Man erhält die Titelverbindung; $[\alpha]_D^{20°} = +23° \pm 1°$ (0,82 % in $CHCl_3$); IR: 3400, 3300, 1778, 1710, 1696, 1630, 1520 ($CH_2Cl_2$); UV: 257 (14400; EtOH).

Herstellung des Ausgangsmaterials

c) (2R,S)-2-(2-BOC-Aminothiazol-4-yl)-2-(2-methoxymalonylaminoäthansulfonylamino)-essigsäure

6,7 g (2R,S)-2-(2-BOC-Aminothiazol-4-yl)-2-(2-aminoäthansulfonylamino)-essigsäure werden in 24 ml Tetrahydrofuran mit 1,07 ml Malonsäuremonomethylester-chlorid analog Beispiel 6c) umgesetzt (10 ml N,O-Bis-(trimethylsilyl)-acetamid; 0,81 ml Pyridin) und aufgearbeitet. Man erhält die Titelverbindung, die ohne Charakterisierung weiterverarbeitet wird.

Beispiel 51:

a) 7β-[(2R,S)-2-(2-Aminothiazol-4-yl)-2-(2-bromacetylaminoäthansulfonylamino)-acetamido]-3-cephem-4-carbonsäurenatriumsalz

1,5 g 7β-[(2R,S)-2-(2-BOC-Aminothiazol-4-yl)-2-(2-bromacetylaminoäthansulfonylamino)-acetamido]-3-cephem-4-carbonsäurediphenylmethylester werden in 8 ml $CH_2Cl_2$ und 0,4 ml Anisol mit 12 ml Trifluoressigsäure analog Beispiel 1a) umgesetzt, aufgearbeitet, chromatographiert und umgefällt. Man erhält das Hydrat der Titelverbindung.

F: oberhalb 220° (unter Zersetzung) IR: ua. 1770 (Nujol); UV: 253 (11100; $H_2O$).

b) 7β-[(2R,S)-2-(2-BOC-Aminothiazol-4-yl)-2-(2-bromacetylaminoäthansulfonylamino)-acetamido]-3-cephem-4-carbonsäurediphenylmethylester

3,3 g (2R,S)-2-(2-BOC-Aminothiazol-4-yl)-2-(2-bromacetylaminoäthansulfonylamino)-essigsäure werden mit 2,38 g 7β-Amino-3-cephem-4-carbonsäurediphenylmethylester in 25 ml Tetrahydrofuran analog Beispiel 6b) umgesetzt (0,66 g Hydroxybenztriazol; dreimal 0,76 g Dicyclohexylcarbodiimid in je 5 ml Tetrahydrofuran), aufgearbeitet und chromatographiert. Man erhält die Titelverbindung; IR: 3400, 3300, 1790, 1735, 1682, 1630, 1535 $cm^{-1}$ ($CH_2Cl_2$); UV: 256 (14500; EtOH).

Herstellung des Ausgangsmaterials:

c) (2R,S)-2-(2-BOC-Aminothiazol-4-yl)-2-(2-bromacetylaminoäthansulfonylamino)-essigsäure

6,7 g (2R,S)-2-(2-BOC-Aminothiazol-4-yl)-2-(2-aminoäthansulfonylamino)-essigsäure werden in 24 ml Tetrahydrofuran mit 0,82 ml Bromessigsäurebromid analog Beispiel 6 c) umgesetzt (16 ml N,O-Bis-(trimethylsilyl-acetamid; 0,81 ml Pyridin) und aufgearbeitet. Man erhält die Titelverbindung, die ohne Charakterisierung weiterverarbeitet wird.

Beispiel 52:

7β-[(2R,S)-2-(2-Aminothiazol-4-yl)-2-(2-(2-(1-methyl-1H-tetrazol-5-ylthio)-acetamido)-äthansulfonylamino)-acetamido]-3-cephem-4-carbonsäurenatriumsalz

1,5 g gemäss Beispiel 51a) herstellbares 7β-[(2R,S)-2-(2-Aminothiazol-4-yl)-2-(2-bromacetylaminoäthansulfonylamino)-acetamido]-3-cephem-4-carbonsäurenatriumsalz versetzt man in 30 ml Wasser mit

245 mg 1-Methyl-1H-mercaptotetrazol-natriumsalz und rührt 3 Stunden bei Zimmertemperatur, wobei durch Zugabe von 1 N Natronlauge der pH 7 konstant gehalten wird. Dann engt man im Vakuum ein, chromatographiert und fällt gemäss Beispiel 1a) um. Man erhält das Hydrat der Titelverbindung; F: oberhalb 172° (unter Zersetzung); IR: 3700 bis 2700 (breit), 1760, 1650, 1600, 1530 $cm^{-1}$ (Nujol); UV: 252 (8900; $H_2O$).

Beispiel 53:

a) 7β-[(2R,S)-2-(2-Aminothiazol-4-yl)-2-(2-methoxysuccinylaminoäthansulfonylamino)-acetamido]-3-cephem-4-carbonsäure-natriumsalz

1,5 g 7β-[(2R,S)-2-(2-BOC-Aminothiazol-4-yl)-2-(2-methoxysuccinylaminoäthansulfonylamino)-acetamido]-3-cephem-4-carbonsäurediphenylmethylester werden in 4 ml $CH_2Cl_2$ und 0,43 ml Anisol mit 10 ml Trifluoressigsäure analog Beispiel 1a) umgesetzt, aufgearbeitet, chromatographiert und umgefällt. Man erhält das Hydrat der Titelverbindung; F: oberhalb 210°(Zersetzung); $[\alpha]_D^{20°} = +89° \pm 1°$ (0,97 % in $CHCl_3$); IR: 3650 bis 2700 (breit), 1765, 1730, 1650, 1600, 1525 (Nujol); UV: 252 (9700), 310 (600; $H_2O$).

b) 7β-[(2R,S)-2-(2-BOC-Aminothiazol-4-yl)-2-(2-methoxysuccinylaminoäthansulfonylamino)-acetamido]-3-cephem-4-carbonsäurediphenylmethylester

4,2 g (2R,S)-2-(2-BOC-Aminothiazol-4-yl)-2-(2-methoxysuccinylaminoäthansulfonylamino)-essigsäure werden mit 3,1 g 7β-Amino-3-cephem-4-carbonsäurediphenylmethylester in 45 ml Tetrahydrofuran analog Beispiel 6b) umgesetzt (1,1 g Hydroxybenztriazol; dreimal 0,58 g Dicyclohexylcarbodiimid in je 6,6 ml Tetrahydrofuran) aufgearbeitet und chromatographiert. Man erhält die Titelverbindung. $[\alpha]_D^{20°} = +16° \pm 1°$ (1,00 % in $CHCl_3$); IR: 3400, 3300, 1790, 1730, 1680, 1630, 1543 ($CH_2Cl_2$); UV: 255 (12700; EtOH).

Herstellung des Ausgangsmaterials:

c) (2R,S)-2-(2-BOC-Aminothiazol-4-yl)-2-(2-methoxysuccinylaminoäthansulfonylamino)-essigsäure

6,7 g (2R,S)-2-(2-BOC-Aminothiazol-4-yl)-2-(2-aminoäthansulfonylamino)-essigsäure werden in 25 ml Tetrahydrofuran mit 1,22 ml Bernsteinsäuremonomethylester-chlorid analog Beispiel 6c) umgesetzt (10 ml N,O-Bis-(trimethylsilyl)-acetamid; 0,81 ml Pyridin) und aufgearbeitet. Man erhält die Titelverbindung, die ohne Charakterisierung weiterverarbeitet wird.

Beispiel 54:

a) 7β-[(2R,S)-2-(2-Aminothiazol-4-yl)-2-(2-hydroxymalonylaminoäthansulfonylamino)-acetamido]-3-cephem-4-carbonsäuredinatriumsalz

3,3 g 7β-[(2R,S)-2-(2-BOC-Aminothiazol-4-yl)-2-t.-butoxymalonylamino-äthansulfonylamino)-acetamido]-3-cephem-4-carbonsäurediphenylmethylester werden in 6,7 ml $CH_2Cl_2$ und 2,15 ml Anisol mit 25 ml Trifluoressigsäure analog Beispiel 1a) umgesetzt, aufgearbeitet, chromatographiert und umgefällt. Man erhält das Hydrat der Titelverbindung. F: oberhalb 200° (unter Zersetzung); $[\alpha]_D^{20°}$ = +93° +1° (0,76% in $H_2O$); IR: 3700 bis 2600 (breit), 1762, 1645, 1595, 1525 (Nujol); UV: 253 (1600), 314 (300; $H_2O$).

b) 7β-[(2R,S)-2-(2-BOC-Aminothiazol-4-yl)-2-(2-t.-butoxymalonylaminoäthansulfonylamino)-acetamido]-3-cephem-4-carbonsäurediphenylmethylester

6 g 7β-[(2R,S)-2-(2-BOC-Aminothiazol-4-yl)-2-(aminoäthansulfonylamino)-acetamido]-3-cephem-4-carbonsäurediphenylmethylester (Herstellung siehe Beispiel 35c) werden mit 1,5 g Malonsäuremono-t-butylester in 25 ml Tetrahydrofuran analog Beispiel 6b) umgesetzt (0,93 g Hydroxybenztriazol; dreimal 0,82 g Dicyclohexylcarbodiimid in je 5 ml Tetrahydrofuran), aufgearbeitet und chromatographiert. Man erhält die Titelverbindung; $[\alpha]_D^{20°}$ = +25°±1° (0,70 % in $CHCl_3$); IR: 3400, 1785, 1720, 1675, 1635, 1602, 1535 $cm^{-1}$ ($CH_2Cl_2$); UV: 258 (13700; EtOH).

Beispiel 55:

a) 7β-[(2R,S)-2-(2-Aminothiazol-4-yl)-2-(2-(4-nitrobenzoylamino)-äthansulfonylamino)-acetamido]-3-cephem-4-carbonsäurenatriumsalz

2,6 g 7β-[(2R,S)-2-(2-BOC-Aminothiazol-4-yl)-2-(2-(4-nitrobenzoylamino)-äthansulfonylamino)-acetamido]-3-cephem-4-carbonsäurediphenylmethylester werden in 6 ml $CH_2Cl_2$ und 0,76 ml Anisol mit 10 ml Trifluoressigsäure analog Beispiel 1a) umgesetzt, aufgearbeitet, chromatographiert und umgefällt. Man erhält das Hydrat der Titelverbindung. F: oberhalb 220° (unter Zersetzung). $[\alpha]_D^{20°}$ = +79°±1° (1,18 % in $H_2O$); IR: 3650 bis 2600 (breit), 1765, 1670, 1650, 1598, 1525 (Nujol); UV: 259 (18700; $H_2O$).

b) 7β-[(2R,S)-2-(2-BOC-Aminothiazol-4-yl)-2-(2-(4-nitrobenzoylamino)-äthansulfonylamino)-acetamido]-3-cephem-4-carbonsäurediphenylmethylester

7 g (2R,S)-2-(2-BOC-Aminothiazol-4-yl)-2-(2-(4-nitrobenzoylamino)-äthansulfonylamino)-essigsäure werden mit 3,6 g 7β-Amino-3-cephem-4-carbonsäurediphenylmethylester in 60 ml Tetrahydrofuran analog Beispiel 6b) umgesetzt (1,3 g Hydroxybenztriazol; dreimal 0,66 g Dicyclohexylcarbodiimid in je 6,6 ml Tetrahydrofuran), aufgearbeitet und chromatographiert. Man erhält die Titelverbindung. $[\alpha]_D^{20°}$ = +14°±1° (0,95 % in $CHCl_3$); IR: 3400, 3300, 1787, 1725, 1692, 1670, 1650, 1600, 1530 ($CH_2Cl_2$); UV: 259 (23200; EtOH).

Herstellung des Ausgangsmaterials:

c) (2R,S)-2-(2-BOC-Aminothiazol-4-yl)-2-(2-(4-nitrobenzoylamino)-äthansulfonylamino)-essigsäure

6,7 g (2R,S)-2-(2-BOC-Aminothiazol-4-yl)-2-(2-aminoäthansulfonylamino)-essigsäure werden in 25 ml Tetrahydrofuran mit 3,7 g p-Nitrobenzoylchlorid analog zu Beispiel 6c) umgesetzt (10 ml N,O-Bis-(trimethylsilyl)-acetamid; 0,81 ml Pyridin) und aufgearbeitet. Man erhält die Titelverbindung, die ohne Charakterisierung weiterverarbeitet wird.

Beispiel 56:

a) 7β-[(2R,S)-2-(2-Aminothiazol-4-yl)-2-(2-(4-acetamidobenzolsulfonylamino)-äthansulfonylamino)-acetamido]-3-cephem-4-carbonsäurenatriumsalz

Analog Beispiel 29a) erhält man durch Umsetzung von 1,4 g (1,5 mMol) 7β-[(2R,S)-2-(2-BOC-Aminothiazol-4-yl)-2-(2-(4-acetamidobenzolsulfonylamino)-äthansulfonylamino)-acetamido]-3-cephem-4-carbonsäurediphenylmethylester in Gegenwart von 1,4 ml Anisol und 7 ml Trifluoressigsäure in 7 ml Methylenchlorid die Titelverbindung als 2,5-Hydrat. F: oberhalb 185° (unter Zersetzung); Rf: ca. 0,45 (Silicagel Opti-UPC 12, Wasser-Acetonitril 4:1) UV: 256 (28700, Wasser).

b) 7β-[(2R,S)-2-(2-BOC-Aminothiazol-4-yl)-2-(2-(4-acetamidobenzolsulfonylamino)-äthansulfonylamino)-acetamido]-3-cephem-4-carbonsäurediphenylmethylester

Analog Beispiel 29b erhält man durch Behandeln von 1,44 g (2,5 mMol) (2R,S)-2-(2-BOC-Aminothiazol-4-yl)-2-(2-(4-acetamidobenzolsulfonylamino)-äthansulfonylamino)-essigsäure mit 0,92 g (2,5 mMol) 7β-Amino-3-cephem-4-carbonsäurediphenylmethylester in Gegenwart von 0,34 g 1-Hydroxybenztriazol und 0,57 g N,N'-Dicyclohexylcarbodiimid in 27 ml Tetrahydrofuran die Titelverbindung als blassgelbes, amorphes Pulver. Rf: ca. 0,10 (Silicagel, Methylenchlorid-Essigester 1:1).

Herstellung des Ausgangsmaterials:

c) (2R,S)-2-(2-BOC-Aminothiazol-4-yl)-2-(2-(4-acetamidobenzolsulfonylamino)-äthansulfonylamino)-essigsäure

Eine Suspension von 3,8 g (10 mMol) (2R,S)-2-(2-BOC-Aminothiazol-4-yl)-2-(2-aminoäthansulfonylamino)-essigsäure in 100 ml absolutem Tetrahydrofuran wird unter Rühren, Feuchtigkeitsausschluss und Stickstoffatmosphäre mit 10 ml N,O-Bis(trimethylsilyl)-acetamid versetzt. Nach einer Stunde Reaktionszeit bei 65° wird das Reaktionsgemisch

auf 0° abgekühlt, mit 0,88 ml Pyridin und 2,65 g 4-Acetamidobenzolsulfochlorid versetzt und anschliessend 20 Stunden bei Raumtemperatur gerührt. Nach Entfernung des Lösungsmittels wird der Rückstand in 300 ml Essigester aufgenommen und je dreimal mit 0,1 N Salzsäure und gesättigter, wässriger Natriumchloridlösung gewaschen. Nach Trocknen über Natriumsulfat wird das Lösungsmittel am Rotationsverdampfer entfernt, und der Rückstand an Silicagel mit Essigester-Methanol 4:1 als Eluiermittel gereinigt, woraus die Titelverbindung als amorphes Pulver erhalten wird. DC (Silicagel): Rf: ca. 0.23 (Chloroform-Methanol-Eisessig 75:22:3).

Beispiel 57:

a) 7β-[(2R,S)-2-(2-Aminothiazol-4-yl)-2-(2-isopropansulfonylaminoäthansulfonylamino)-acetamido]-3-cephem-4-carbonsäurenatriumsalz

0,4 g 7β-[(2R,S)-2-(2-BOC-Aminothiazol-4-yl)-2-(2-isopropansulfonylaminoäthansulfonylamino)-acetamido-3-cephem-4-carbonsäurediphenylmethylester werden in 0,84 ml $CH_2Cl_2$ und 0,27 ml Anisol mit 3,13 ml Trifluoressigsäure analog Beispiel 1a) umgesetzt, aufgearbeitet, chromatographiert und umgefällt. Man erhält das Hydrat der Titelverbindung. F: oberhalb 175° (unter Zersetzung); IR: 3700 bis 2700 (breit), 1762, 1680, 1602, 1520 (Nujol); UV: 250 (9800), 310 (1100; $H_2O$).

b) 7β-[(2R,S)-2-(2-BOC-Aminothiazol-4-yl)-2-(2-isopropansulfonylaminoäthansulfonylamino)-acetamido]-3-cephem-4-carbonsäurediphenylmethylester

3,2 g (2R,S)-2-(2-BOC-Aminothiazol-4-yl)-2-(2-isopropansulfonylaminoäthansulfonylamino)-essigsäure werden mit 2 g 7β-Amino-3-cephem-4-carbonsäurediphenylmethylester in 15 ml Tetrahydrofuran analog Beispiel 6b) umgesetzt (0,6 g Hydroxybenztriazol; dreimal 0,52 g Dicyclohexylcarbodiimid in je 4 ml Tetrahydrofuran) aufgearbeitet und chromatographiert. Man erhält die Titelverbindung. IR: 3405, 3290, 1781, 1720, 1678, 1520 ($CH_2Cl_2$); UV: 259 (13800; EtOH).

Herstellung des Ausgangsmaterials:

c) (2R,S)-2-(2-BOC-Aminothiazol-4-yl)-2-(2-isopropansulfonylamino-äthansulfonylamino)-essigsäure

8,3 g (2R,S)-2-(2-BOC-Aminothiazol-4-yl)-2-(2-aminoäthansulfonylamino)-essigsäure werden in 30 ml $CH_2Cl_2$ mit 2,66 g Isopropansulfochlorid analog Beispiel 6c) umgesetzt (10 ml N,O-Bis-(trimethylsilyl)-acetamid; 1,01 ml Pyridin) und aufgearbeitet. Man erhält die Titelverbindung, die ohne Charakterisierung weiterverarbeitet wird.

Beispiel 58:

a) 7β-[(2R,S)-2-(2-Aminothiazol-4-yl)-2-(2-(4-äthyl-2,3-dioxopiperazin-1-ylcarbonylamino)-äthansulfonylamino)-acetamido]-3-cephem-4-carbonsäurenatriumsalz

Analog Beispiel 29a) erhält man durch Umsetzen von 2,0 g (2,23 mMol) 7β-[(2R,S)-2-(2-BOC-Aminothiazol-4-yl)-2-(2-(4-äthyl-2,3-dioxo-piperazin-1-ylcarbonylamino)-äthansulfonylamino)-acetamido]-3-cephem-4-carbonsäurediphenylmethylester in Gegenwart von 2,0 ml Anisol mit 10 ml Trifluoressigsäure in 10 ml Methylenchlorid die Titelverbindung als Dihydrat. F: oberhalb 170° (unter Zersetzung); DC (Silicagel-Opti UPC-12): Rf: ca. 0,31 (Wasser-Acetonitril 8:2); $[\alpha]_D^{20°}$: +83°±1° (1,303 % in $H_2O$); UV: 240 (14600, $H_2O$).

b) 7β-[(2R,S)-2-(2-BOC-Aminothiazol-4-yl)-2-(2-(4-äthyl-2,3-dioxo-piperazin-1-ylcarbonylamino)-äthansulfonylamino)-acetamido]-3-cephem-4-carbonsäurediphenylmethylester

Analog Beispiel 29b) erhält man durch Behandeln von 2,20 g (4 mMol) (2R,S)-2-(2-BOC-Aminothiazol-4-yl)-2-(2-(4-äthyl-2,3-dioxopiperazin-1-ylcarbonylamino)-äthansulfonylamino)-essigsäure mit 1,46 g 7β-Amino-3-cephem-4-carbonsäurediphenylmethylester in Gegenwart von 0,54 g 1-Hydroxybenztriazol und 0,91 g N,N'-Dicyclohexylcarbodiimid in 40 ml Tetrahydrofuran die Titelverbindung als gelbliches Pulver her. DC (Silicagel): Rf: ca. 0.10 (Essigester).

Herstellung des Ausgangsmaterials:

c) (2R,S)-2-(2-BOC-Aminothiazol-4-yl)-2-(2-(4-äthyl-2,3-dioxopiperazin-1-ylcarbonylamino)-äthansulfonylamino)-essigsäure

Eine Suspension von 3,8 g (10 mMol) (2R,S)-2-(2-BOC-Aminothiazol-4-yl)-2-(2-aminoäthansulfonylamino)-essigsäure in 50 ml absolutem Tetrahydrofuran wird unter Rühren, Feuchtigkeitsausschluss, und Stickstoffatmosphäre mit 5 ml N,O-Bis(trimethylsilyl)-acetamid versetzt. Nach einer Stunde Reaktionszeit bei 65° wird das Reaktionsgemisch auf Raumtemperatur abgekühlt, mit 2 ml Pyridin und 5,12 g 4-Aethyl-2,3-dioxo-piperazin-1-ylcarbonylchlorid versetzt und anschliessend 5 Stunden gerührt. Nach Entfernung des Lösungsmittels wird der Rückstand in 250 ml Essigester aufgenommen und je dreimal mit 1 N Salzsäure und gesättigter wässriger Natriumchloridlösung gewaschen. Nach Trocknen über Natriumsulfat wird das Lösungsmittel am Rotationsverdampfer entfernt woraus die Titelverbindung als amorphes Pulver erhalten wird. DC (Silicagel): Rf: ca. 0.18 (Chloroform-Methanol-Eisessig 75:22:3).

Beispiel 59:

a) 7β-[(2R,S)-2-(2-Aminothiazol-4-yl)-2-isopropansulfonylaminoacetamido]-3-cephem-4-carbonsäurenatriumsalz

0,89 g 7β-[(2R,S)-2-(2-BOC-Aminothiazol-4-yl)-2-isopropansulfonylaminoacetamido]-3-cephem-4-carbonsäurediphenylmethylester werden in 2,15 ml $CH_2Cl_2$ und 0,69 ml Anisol mit 8 ml Trifluoressigsäure analog Beispiel 1a) umgesetzt, aufgearbeitet und umgefällt. Man erhält das Hydrat der Titelverbindung. F: oberhalb 220° (unter Zersetzung); $[\alpha]_D^{20°} = +95° \pm 1°$ (0,19 % in $H_2O$); IR: 3700 bis 2600 (breit), 1760, 1680, 1605, 1522 (Nujol); UV: 250 (10000), 310 (1500; $H_2O$).

b) 7β-[(2R,S)-2-(2-BOC-Aminothiazol-4-yl)-2-isopropansulfonylamino-acetamido]-3-cephem-4-carbonsäurediphenylmethylester

2,99 g (2R,S)-2-(2-BOC-Aminothiazol-4-yl)-2-isopropansulfonylamino-essigsäure werden mit 2,4 g 7β-Amino-3-cephem-4-carbonsäurediphenylmethylester in 18 ml Tetrahydrofuran analog Beispiel 6b) umgesetzt (0,72 g Hydroxybenztriazol; dreimal 0,62 g Dicyclohexylcarbodiimid in je 5 ml Tetrahydrofuran), aufgearbeitet und chromatographiert. Man erhält die Titelverbindung; $[\alpha]_D^{20°} = +16° \pm 1°$ (0,83 % in $CHCl_3$); IR: 3400, 3300, 1780, 1715, 1675, 1522 ($CH_2Cl_2$); UV: 258 (14200; EtOH).

Herstellung des Ausgangsmaterials

c) (2R,S)-2-(2-BOC-Aminothiazol-4-yl)-2-isopropansulfonylamino-essigsäure

3,4 g (2R,S)-2-(2-BOC-Aminothiazol-4-yl)-2-aminoessigsäure werden in 30 ml $CH_2Cl_2$ mit 2,66 g Isopropansulfochlorid analog Beispiel 6c) umgesetzt (10 ml N,O-Bis-(trimethylsilyl)-acetamid; 1,01 ml Pyridin) und aufgearbeitet. Man erhält die Titelverbindung, die ohne Charakterisierung weiterverarbeitet wird.

Beispiel 60:

a) 7β-[(2R,S)-2-(2-Aminothiazol-4-yl)-2-n.-octylsulfonylaminoacetamido]-3-cephem-4-carbonsäure-natriumsalz

Analog Beispiel 29a erhält man durch Umsetzung von 3.03 g (3,8 mMol) 7β-[(2R,S)-2-(2-BOC-Aminothiazol-4-yl)-2-n.octylsulfonylaminoacet-amido]-3-cephem-4-carbonsäurediphenylmethylester in Gegenwart von 3 ml Anisol und 15 ml Trifluoressigsäure in 15 ml Methylenchlorid die Titelverbindung in Form des Dihydrats. F: oberhalb 172° (unter Zersetzung); Rf 96: ca. 0.55; $[\alpha]_D^{20°} = +81 \pm 1°$ (1,042 % in Aethanol-Wasser 1:1); UV: 252 (9100, in Aethanol-Wasser 1:1).

b) 7β-[(2R,S)-2-(2-BOC-Aminothiazol-4-yl)-2-n.-octylsulfonylamino-acetamido]-3-cephem-4-carbonsäurediphenylmethylester

Analog Beispiel 29b) erhält man durch Behandeln von 2,25 g (5 mMol) (2R,S)-2-(2-BOC-Aminothiazol-4-yl)-2-n.octylsulfonylaminoessigsäure mit 1,83 g (5 mMol) 7β-Amino-3-cephem-4-carbonsäurediphenylmethylester in Gegenwart von 0,68 g 1-Hydroxybenztriazol und 1,14 g N,N'-Dicyclohexylcarbodiimid in 50 ml Tetrahydrofuran die Titelverbindung als gelbliches Pulver. Rf: ca. 0.61 (Silicagel, Methylenchlorid-Essigester 1:1).

Herstellung des Ausgangsmaterials:

c) (2R,S)-2-(2-BOC-Aminothiazol-4-yl)-2-n.-octylsulfonylaminoessigsäure

Eine Suspension von 2,73 g (10 mMol) (2R,S)-2-(2-BOC-Aminothiazol-4-yl)-2-aminoessigsäure in 30 ml absolutem Tetrahydrofuran wird unter Rühren, Feuchtigkeitsausschluss und Stickstoffatmosphäre mit 8 ml N,O-Bis(trimethylsilyl)-acetamid versetzt. Nach einer Stunde Reaktionszeit bei 60° wird das Reaktionsgemisch auf Raumtemperatur abgekühlt, mit 0,8 ml Pyridin und 1,96 ml 1-Octansulfochlorid versetzt und anschliessend 16 Stunden gerührt. Nach Entfernung des Lösungsmittels wird der Rückstand in 250 ml Essigester aufgenommen und je dreimal mit 0,5 N Salzsäure und gesättigter wässriger Natriumchloridlösung gewaschen. Nach Trocknen über Natriumsulfat wird das Lösungsmittel am Rotationsverdampfer entfernt, und der Rückstand an Silicagel mit Chloroform-Essigester 4:1 als Eluiermittel gereinigt, woraus die Titelverbindung als amorphes Pulver erhalten wird. Rf = ca. 0.58 (Silicagel, Chloroform-Methanol-Eisessig, 75:22:3).

Beispiel 61:

a) 7β-[(2R,S)-2-(2-Aminothiazol-4-yl)-2-p-toluolsulfonylaminoacetamido]-3-cephem-4-carbonsäure-natriumsalz

1,88 g 7β-[(2R,S)-2-(2-BOC-Aminothiazol-4-yl)-2-p-toluolsulfonylaminoacetamido]-3-cephem-4-carbonsäurediphenylmethylester werden in 4,4 ml $CH_2Cl_2$ und 1,4 ml Anisol mit 15 ml Trifluoressigsäure analog Beispiel 1a) umgesetzt, aufgearbeitet und umgefällt. Man erhält das Hydrat der Titelverbindung. F: oberhalb 213° (unter Zersetzung); $[\alpha]_D^{20°}$ = +96°±1° (0,91 % in $H_2O$); IR: 3650 bis 2700 (breit), 1755, 1657, 1609, 1518 (Nujol); UV: 230 (19000; $H_2O$).

b) 7β-[(2R,S)-2-(2-BOC-Aminothiazol-4-yl)-2-p-toluolsulfonylaminoacetamido]-3-cephem-4-carbonsäurediphenylmethylester

2,44 g (2R,S)-2-(2-BOC-Aminothiazol-4-yl)-2-p-toluolsulfonylaminoessigsäure werden mit 1,8 g 7β-Amino-3-cephem-4-carbonsäurediphenylmethylester in 20 ml Tetrahydrofuran analog Beispiel 6b) umgesetzt (0,52 g Hydroxybenztriazol; dreimal 0,46 g Dicyclohexylcarbodiimid in je 4 ml Tetrahydrofuran), aufgearbeitet und chromatographiert. Man erhält den 7β-[(2R,S)-2-(2-BOC-Aminothiazol-4-yl)-2-(p-toluolsulfonylamino)-acetamido]-3-cephem-4-carbonsäurediphenylmethylester; $[\alpha]_D^{20°}$ = +1°±1° (0,86 % in $CHCl_3$); IR: 3400, 3390, 1782, 1720, 1700, 1645, 1600, 1530 ($CH_2Cl_2$); UV: 259 (13000; EtOH)

Herstellung des Ausgangsmaterials:

c) (2R,S)-2-(2-BOC-Aminothiazol-4-yl)-2-p-toluolsulfonylaminoessigsäure

2,73 g (2R,S)-2-(2-BOC-Aminothiazol-4-yl)-2-aminoessigsäure werden in 24 ml Tetrahydrofuran mit 2,1 g p-Toluolsulfochlorid analog zu Beispiel 6c) umgesetzt (10 ml N,O-Bis-(trimethylsilyl)-acetamid; 0,81 ml Pyridin) und aufgearbeitet. Man erhält die Titelverbindung, die ohne Charakterisierung weiterverarbeitet wird.

Beispiel 62:

a) 7β-[(2R,S)-2-(2-Aminothiazol-4-yl)-2-p-nitrobenzolsulfonylamino-acetamido]-3-cephem-4-carbonsäurenatriumsalz

Analog Beispiel 29a) erhält man durch Umsetzung von 1,61 g (2,0 mMol) 7β-[(2R,S)-2-(2-BOC-Aminothiazol-4-yl)-2-p-nitrobenzolsulfonylamino-acetamido]-3-cephem-4-carbonsäurediphenylmethylester in Gegenwart von 3,5 ml Anisol mit 8 ml Trifluoressigsäure in 8 ml Methylenchlorid die Titelverbindung in Form des Monohydrats. F: oberhalb 188° (unter Zersetzung); Rf 96 : ca. 0.43; $[\alpha]_D^{20°} = \underline{+}11° \underline{+} 1°$ (0,268 % in Wasser); UV: 258 (18900, Wasser).

b) 7β-[(2R,S)-2-(2-BOC-Aminothiazol-4-yl)-2-p-nitrobenzolsulfonyl-aminoacetamido]-3-cephem-4-carbonsäurediphenylmethylester

Analog Beispiel 29b) erhält man durch Behandeln von 2,29 g (5 mMol) (2R,S)-2-(2-BOC-Aminothiazol-4-yl)-2-p-nitrobenzolsulfonylamino-essigsäure mit 1,83 g (5 mMol) 7β-Amino-3-cephem-4-carbonsäurediphenylmethylester in Gegenwart von 0,68 g 1-Hydroxybenztriazol und 1,15 g N,N'-Dicyclohexylcarbodiimid in 30 ml Tetrahydrofuran die Titelverbindung. Rf: ca. 0.83 (Methylenchlorid-Essigester 1:1).

Herstellung des Ausgangsmaterials:

c) (2R,S)-2-(2-BOC-Aminothiazol-4-yl)-2-p-nitrobenzolsulfonylamino-essigsäure

Eine Suspension von 2,73 g (10 mMol) (2R,S)-2-(2-BOC-Aminothiazol-4-yl)-2-aminoessigsäure in 27 ml absolutem Methylenchlorid wird unter Rühren, Feuchtigkeitsausschluss und Stickstoffatmosphäre mit 8 ml N,O-Bis(trimethylsilyl)-acetamid versetzt. Nach einer Stunde Reaktionszeit bei 60° wird das Reaktionsgemisch auf Raumtemperatur abgekühlt, mit 0,81 ml Pyridin und 2,21 g p-Nitrobenzolsulfochlorid versetzt und anschliessend 5 Stunden gerührt. Nach Entfernung des Lösungsmittels wird der Rückstand in 250 ml Essigester aufgenommen und je dreimal

mit 1,0 N Salzsäure und gesättigter, wässriger Natriumchloridlösung gewaschen. Nach Trocknen über Natriumsulfat wird das Lösungsmittel am Rotationsverdampfer entfernt, und der Rückstand an Silicagel mit Methylenchlorid-Essigester 1:1 als Eluiermittel gereinigt, woraus die Titelverbindung als amorphes Pulver erhalten wird. Rf 96 : ca. 0.65.

Beispiel 63:

a) 7β-[(2R,S)-2-(2-Aminothiazol-4-yl)-2-(4-acetamidobenzolsulfonylamino)-acetamido]-3-cephem-4-carbonsäurenatriumsalz

Analog Beispiel 29a) erhält man durch Umsetzung von 4,0 g (4.9 mMol) 7β-[(2R,S)-2-(2-BOC-Aminothiazol-4-yl)-2-(4-acetamidobenzolsulfonylamino)-acetamido]-3-cephem-4-carbonsäurediphenylmethylester in Gegenwart von 4 ml Anisol mit 20 ml Trifluoressigsäure in 20 ml Methylenchlorid die Titelverbindung in Form des Dihydrats. F: oberhalb 191° (unter Zersetzung); Rf: ca. 0.33 (Silicagel Opti-UPC 12, Wasser-Acetonitril 9:1); $[\alpha]_D^{20°} = +91° \pm 1°$ (0,982 % in Wasser); UV: 260 (27200, Wasser).

b) 7β-[(2R,S)-2-(2-BOC-Aminothiazol-4-yl)-2-(4-acetamidobenzolsulfonylamino)-acetamido]-3-cephem-4-carbonsäurediphenylmethylester

Analog Beispiel 29b) erhält man durch Behandeln von 2,35 g (5 mMol) (2R,S)-2-(2-BOC-Aminothiazol-4-yl)-2-(4-acetamidobenzolsulfonylamino)-essigsäure mit 1,83 g (5 mMol) 7β-Amino-3-cephem-4-carbonsäurediphenylmethylester in Gegenwart von 0,675 g 1-Hydroxybenztriazol und 1,15 g N,N'-Dicyclohexylcarbodiimid in 25 ml Tetrahydrofuran die Titelverbindung als beiges Pulver. Rf: ca. 0.40 (Silicagel, Essigester).

Herstellung des Ausgangsmaterials:

c) (2R,S)-2-(2-BOC-Aminothiazol-4-yl)-2-(4-acetamidobenzolsulfonylamino)-essigsäure

Eine Suspension von 2,73 g (10 mMol) (2R,S)-2-(2-BOC-Aminothiazol-4-yl)-2-aminoessigsäure in 30 ml absolutem Tetrahydrofuran wird unter Rühren, Feuchtigkeitsausschluss und Stickstoffatmosphäre mit 8 ml N,O-Bis(trimethylsilyl)-acetamid versetzt. Nach einer Stunde Reaktionszeit bei 60° wird das Reaktionsgemisch auf Raumtemperatur abgekühlt, mit 0,8 ml Pyridin und 2,34 g 4-Acetaminobenzolsulfochlorid versetzt und anschliessend 4 Stunden gerührt. Nach Entfernung des Lösungsmittels wird der Rückstand in 250 ml Essigester aufgenommen und je dreimal mit 1 N Salzsäure und gesättigter, wässriger Natriumchloridlösung gewaschen. Nach Trocknen über Natriumsulfat wird das Lösungsmittel am Rotationsverdampfer entfernt, und die Titelverbindung als amorphes Pulver erhalten. Rf 96 : ca. 0.68.

Beispiel 64:

a) 7β-[(2R,S)-2-(2-Aminothiazol-4-yl)-2-(2-aminonaphth-1-ylsulfonylamino)-acetamido]-3-cephem-4-carbonsäurenatriumsalz

1,57 g 7β-[(2R,S)-2-(2-BOC-Aminothiazol-4-yl)-2-(2-aminonaphth-1-ylsulfonylamino)-acetamido]-3-cephem-4-carbonsäurediphenylmethylester werden in 3,4 ml $CH_2Cl_2$ und 1,07 ml Anisol mit 12,5 ml Trifluoressigsäure analog zu Beispiel 1a) umgesetzt, aufgearbeitet und umgefällt. Man erhält das Hydrat der Titelverbindung. F: oberhalb 215° (unter Zersetzung); $[\alpha]_D^{20°} = +90° \pm 1°$ (0,078 % in $H_2O$); IR: 3650 bis 2700 (breit), 1762, 1680, 1628, 1605, 1555, 1520 (Nujol); UV: 243 (44800), 347 (3900; $H_2O$).

b) 7β-[(2R,S)-2-(2-BOC-Aminothiazol-4-yl)-2-(2-aminonaphth-1-ylsulfonylamino)-acetamido]-3-cephem-4-carbonsäurediphenylmethylester

2,71 g 7β-[(2R,S)-2-(2-BOC-Aminothiazol-4-yl)-2-(2-(2,2,2-trichlor-

äthoxycarbonylamino)-naphth-1-ylsulfonylamino)-acetamido]-3-cephem-4-carbonsäurediphenylmethylester werden mit 2,4 g Zinkstaub in 30 ml Acetonitril-Eisessig-1:1-Gemisch analog Beispiel 37c) umgesetzt und aufgearbeitet. Nach chromatographischer Reinigung des Rohproduktes an 100 g Kieselgel (Eluiermittel: Toluol-Essigester 1:1-Gemisch) erhält man die Titelverbindung. $[\alpha]_D^{20°} = -5° \pm 1°$ (0,70 % in $CHCl_3$); IR: 3500, 3395, 3300, 1785, 1720, 1700, 1628, 1600, 1535, 1508 ($CH_2Cl_2$); UV: 245 (57800), 350 (4300; EtOH).

c) 7β-[(2R,S)-2-(2-BOC-Aminothiazol-4-yl)-2-(2-(2,2,2-trichloräthoxycarbonylamino)-naphth-1-ylsulfonylamino)-acetamido]-3-cephem-4-carbonsäurediphenylmethylester

7,41 g 7β-[(2R,S)-2-(2-BOC-Aminothiazol-4-yl)-2-aminoacetamido]-3-cephem-4-carbonsäurediphenylmethylester und 7,41 g 2-(2,2,2-Trichloräthoxycarbonylamino)-naphthyl-1-sulfochlorid werden in 75 ml Tetrahydrofuran und 0,963 ml Pyridin 6 Stunden bei Zimmertemperatur gerührt. Dann nimmt man in Essigester auf, wäscht mit 1 N Salzsäure und gesättigter, wässriger NaCl-Lösung, trocknet über $Na_2SO_4$ und dampft ein. Nach chromatographischer Reinigung des Rohproduktes an 250 g Kieselgel (Eluiermittel: Toluol-Essigester 9:1-Gemisch) erhält man die Titelverbindung. $[\alpha]_D^{20°} = -5° \pm 1°$ (1,00 % in $CHCl_3$); IR: 3400, 3300, 1785, 1750, 1724, 1700, 1620, 1605 ($CH_2Cl_2$); UV: 249 (58800), 325 (4400; EtOH).

Herstellung des Ausgangsmaterials:

d) 7β-[(2R,S)-2-(2-BOC-Aminothiazol-4-yl)-2-aminoacetamido]-3-cephem-4-carbonsäurediphenylmethylester

7,52 g 7β-[(2R,S)-2-(2-BOC-Aminothiazol-4-yl)-2-chloracetylaminoacetamido]-3-cephem-4-carbonsäurediphenylmethylester (Herstellung siehe Beispiel 7d) werden mit 1,76 g Thioharnstoff analog Beispiel 7c) umgesetzt (125 ml Dioxan; 2,58 ml Essigsäure) und aufgearbeitet. Man erhält die Titelverbindung, die ohne Charakterisierung roh weiterverarbeitet wird.

Beispiel 65:

a) 7β-[(2R,S)-2-(2-Aminothiazol-4-yl)-2-(5-imidazolsulfonylamino)-acetamido]-3-cephem-4-carbonsäurenatriumsalz

1,6 g 7β-[(2R,S)-2-(2-Aminothiazol-4-yl)-2-(5-imidazolsulfonylamino)-acetamido]-3-cephem-4-carbonsäurediphenylmethylester werden in 10 ml $CH_2Cl_2$ und 0,56 ml Anisol mit 15 ml Trifluoressigsäure analog Beispiel 1a) umgesetzt, aufgearbeitet, chromatographiert und umgefällt. Man erhält das Hydrat der Titelverbindung; F: oberhalb 230° (unter Zersetzung) $[\alpha]_D^{20°}$ = +83°±1° (1,01 % in $H_2O$); IR: 3700 bis 2600 (breit), 1760 (breit), 1680, 1640, 1600, 1520 (Nujol); UV: 250 (9200; $H_2O$).

b) 7β-[(2R,S)-2-(2-Aminothiazol-4-yl)-2-(5-imidazolsulfonylamino)-acetamido]-3-cephem-4-carbonsäurediphenylmethylester

2,9 g 7β-[(2R,S)-2-(2-BOC-Aminothiazol-4-yl)-2-aminoacetamido]-3-cephem-4-carbonsäurediphenylmethylester (Herstellung siehe Beispiel 64d) und 0,8 g 5-Imidazolsulfochlorid werden in 30 ml Tetrahydrofuran und 0,55 ml N-Methylmorpholin analog Beispiel 64c) umgesetzt, aufgearbeitet und chromatographiert. Man erhält die Titelverbindung; $[\alpha]_D^{20°}$ = -6°±1° (0,077 % in $CHCl_3$); IR: 3450 bis 2700 (breit), 1785, 1725, 1700 (Schulter), 1640 (Schulter), 1602, 1545 ($CH_2Cl_2$). UV: 258 (14100; EtOH).

Beispiel 66:

a) 3-(1-Methyl-1H-tetrazol-5-ylthiomethyl)-7β-[(2R,S)-2-(2-aminothiazol-4-yl)-2-(2-(4-nitrobenzolsulfonylamino)-äthansulfonylamino)-acetamido]-3-cephem-4-carbonsäurenatriumsalz

Analog Beispiel 29a) erhält man durch Umsetzung von 3,7 g (3,6 mMol) 3-(1-Methyl-1H-tetrazol-5-ylthiomethyl)-7β-[(2R,S)-2-(2-BOC-aminothiazol-4-yl)-2-(2-(4-nitrobenzolsulfonylamino)-äthansulfonylamino)-acetamido]-3-cephem-4-carbonsäurediphenylmethylester in Gegenwart von 3,7 ml Anisol mit 18,5 ml Trifluoressigsäure in 18,5 ml Methylenchlorid das Dihydrat der Titelverbindung her. F: oberhalb 160° (unter Zersetzung);

Rf: ca. 0,10 (Silicagel Opti-UPC 12, Wasser-Acetonitril 4:1); $[\alpha]_D^{20°} = 22° \pm 1°$ (0,595 % in Wasser); UV: 259 (25000, in Wasser)

b) 3-(1-Methyl-1H-tetrazol-5-ylthiomethyl)-7β-[(2R,S)-2-(2-BOC-aminothiazol-4-yl)-2-(2-(4-nitrobenzolsulfonylamino)-äthansulfonylamino)-acetamido]-3-cephem-4-carbonsäurediphenylmethylester

Analog Beispiel 29b) erhält man durch Behandeln von 3,1 g (5,5 mMol) (2R,S)-2-(2-Aminothiazol-4-yl)-2-(2-(4-nitrobenzolsulfonylamino)-äthansulfonylamino)-essigsäure mit 2,72 g (5,5 mMol) 3-(1-Methyl-1H-tetrazol-5-ylthiomethyl)-7β-amino-3-cephem-4-carbonsäurediphenylmethylester in Gegenwart von 1-Hydroxybenztriazol und 1,25 g N,N'-Dicyclohexylcarbodiimid in 50 ml Tetrahydrofuran die Titelverbindung als gelbliches Pulver. Rf: ca. 0,43 (Silicagel, Methylenchlorid-Essigester 1:1).

Beispiel 67:

a) 3-Carbamoyloxymethyl-7β-[(2R,S)-2-(2-aminothiazol-4-yl)-2-(2-(4-nitrobenzolsulfonylamino)-äthansulfonylamino)-acetamido]-3-cephem-4-carbonsäurenatriumsalz

Analog Beispiel 29a) erhält man durch Umsetzung von 1,58 g (1,6 mMol) 3-Carbamoyloxymethyl-7β-[(2R,S)-2-(2-BOC-aminothiazol-4-yl)-2-(2-(4-nitrobenzolsulfonylamino)-äthansulfonylamino)-acetamido]-3-cephem-4-carbonsäurediphenylmethylester in Gegenwart von 1,7 ml Anisol mit 8 ml Trifluoressigsäure in 8 ml Methylenchlorid die Titelverbindung als blassgelbliches Pulver in Form des Dihydrats. F: oberhalb 112° (unter Zersetzung); Rf: ca. 0.18 Silicagel Opti-UPC 12, Wasser-Acetonitril 4:1); $[\alpha]_D^{20°} = +45 \pm 1°$ ( 0,766% in Wasser); UV: 256 (23700, in Wasser).

b) 3-Carbamoyloxymethyl-7β-[(2R,S)-2-(2-BOC-aminothiazol-4-yl)-2-(2-(4-nitrobenzolsulfonylamino)-äthansulfonylamino)-acetamido]-3-cephem-4-carbonsäurediphenylmethylester

Analog Beispiel 29b) erhält man durch Behandeln von 4,52 g (8,0 mMol) (2R,S)-2-(2-BOC-Aminothiazol-4-yl)-2-(2-(4-nitrobenzolsulfonylamino)-äthansulfonylamino)-essigsäure mit 3,51 g (8 mMol) 3-Carbamoyloxymethyl-7β-amino-3-cephem-4-carbonsäurediphenylmethylester in Gegenwart von 1,08 g 1-Hydroxybenztriazol und 1,81 g N,N'-Dicyclohexylcarbodiimid in 80 ml Tetrahydrofuran die Titelverbindung als blassgelbes, amorphes Pulver. Rf: ca. 0.33 (Silicagel, Methylenchlorid-Essigester 1:1).

Beispiel 68:

a) 7β-[(2R,S)-2-(2-Aminothiazol-4-yl)-2-(2-(4-aminobenzolsulfonylamino)-äthansulfonylamino)-acetamido]-3-cephem-4-carbonsäure-natriumsalz

Analog Beispiel 29a) erhält man durch Umsetzung von 1,77 g (2 mMol) 7β-[(2R,S)-2-(2-BOC-Aminothiazol-4-yl)-2-(2-(4-aminobenzolsulfonylamino)-äthansulfonylamino)-acetamido]-3-cephem-4-carbonsäurediphenylmethylester in Gegenwart von 1,8 ml Anisol mit 9 ml Trifluoressigsäure in 9 ml Methylenchlorid die Titelverbindung als gelbliches Pulver in Form des Trihydrats. F: oberhalb 170° (unter Zersetzung); $[\alpha]_D^{20°} = +72\pm1°$ (0,743 % in Wasser); UV: 257 (24200, in Wasser).

Herstellung des Ausgangsmaterials:

b) 7β-[(2R,S)-2-(2-BOC-Aminothiazol-4-yl)-2-(2-(4-aminobenzolsulfonylamino)-äthansulfonylamino)-acetamido]-3-cephem-4-carbonsäure-diphenylmethylester

Eine Lösung von 1,83 g (2 mMol) 7β-[(2R,S)-2-(2-BOC-Aminothiazol-4-yl)-2-(2-(4-nitrobenzolsulfonylamino)-äthansulfonylamino)-acetamido]-3-cephem-4-carbonsäurediphenylmethylester in 40 ml Essigsäureäthylester wird in Gegenwart

von 0,85 g 10%igem Palladium/Kohle-Katalysator bei Normaldruck und Raumtemperatur hydriert. Man filtriert ab, wäscht mit Essigester, engt das Filtrat ein und erhält die Titelverbindung als gelbliches Pulver. Rf: ca. 0.31 (Silicagel, Methylenchlorid-Essigester 1:1).

Beispiel 69:

a) 3-Carbamoyloxymethyl-7β-[(2R,S)-2-(2-aminothiazol-4-yl)-2-methansulfonylaminoacetamido]-3-cephem-4-carbonsäurenatriumsalz

3,9 g 3-Carbamoyloxymethyl-7β-[(2R,S)-2-(2-BOC-aminothiazol-4-yl)-2-methansulfonylaminoacetamido]-3-cephem-4-carbonsäurediphenylmethylester werden in 8,9 ml $CH_2Cl_2$ und 2,97 ml Anisol mit 33,4 ml Trifluoressigsäure analog Beispiel 1a) umgesetzt, aufgearbeitet, chromatographiert und umgefällt. Man erhält das Hydrat der Titelverbindung. F: oberhalb 160° (unter Zersetzung). $[\alpha]_D^{20°} = +62 \pm 1°$ (1,20, % in $H_2O$); IR: 3650 bis 2500 (breit), 1760, 1695, 1605, 1520 $cm^{-1}$; UV: 255 (12500, in $H_2O$).

b) 3-Carbamoyloxymethyl-7β-[(2R,S)-2-(2-BOC-aminothiazol-4-yl)-2-methansulfonylaminoacetamido]-3-cephem-4-carbonsäurediphenylmethylester

2,5 g (2R,S)-2-(2-BOC-Aminothiazol-4-yl)-2-methansulfonylaminoessigsäure (Herstellung siehe Beispiel 22c) werden mit 2,59 g 3-Carbamoyloxymethyl-7β-amino-3-cephem-4-carbonsäurediphenylmethylester in 33 ml Tetrahydrofuran analog Beispiel 6b) umgesetzt (0,63 g Hydroxybenztriazol; dreimal 0,58 g Dicyclohexylcarbodiimid in je 4 ml Tetrahydrofuran), aufgearbeitet und chromatographiert. Man erhält die Titelverbindung; $[\alpha]_D^{20°} = -9° \pm 1°$ (0,97 % in $CHCl_3$); IR 3500, 3400, 3270, 1770, 1700, 1680 (Schulter), 1560, 1515 $cm^{-1}$ ($CH_2Cl_2$); UV: 257 (16200; EtOH).

Beispiel 70:

a) 3-(1-Methyl-1H-tetrazol-5-ylthiomethyl)-7β-[(2R,S)-2-(2-aminothiazol-4-yl)-2-methansulfonylaminoacetamido]-3-cephem-4-carbonsäurepivaloyloxymethylesterhydrochlorid

1,65 g 3-(1-Methyl-1H-tetrazol-5-ylthiomethyl)-7β-[(2R,S)-2-(2-aminothiazol-4-yl)-2-methansulfonylaminoacetamido]-3-cephem-4-carbonsäure-natriumsalz (Herstellung siehe Beispiel 22a) und 0,735 ml Iodmethylpivalat werden in 16,5 ml Dimethylformamid 30 Minuten bei 0° gerührt. Dann fügt man 10 ml Phosphatpuffer von pH 8 zu und rührt nochmals 5 Minuten bei 0°. Anschliessend nimmt man in 25 ml Essigester auf, wäscht zweimal mit gesättigter wässriger NaCl-Lösung und trocknet über Natriumsulfat. Dann filtriert man ab und versetzt mit 4,5 ml 0,7 N HCl in $CH_2Cl_2$. Der dabei entstehende amorphe Niederschlag wird durch Dekantieren abgetrennt, dreimal mit Hexan gewaschen und bei Zimmertemperatur im Vakuum getrocknet. Anschliessend wird mit Aether digeriert, vom Aether abfiltriert und wiederum getrocknet. Man erhält die Titelverbindung. F: oberhalb 110° (unter Zersetzung); $[\alpha]_D^{20°}$ = $-21°\pm1°$ (1,07 % in DMSO); IR: 3600 bis 2400 (breit), 1782, 1750, 1695, 1628, 1545 $cm^{-1}$ (Nujol); UV: 258 (12000; $CH_3OH$).

Beispiel 71:

a) 3-(1-Methyl-1H-tetrazol-5-ylthiomethyl)-7β-[(2R,S)-2-(2-aminothiazol-4-yl)-2-cyanomethansulfonylaminoacetamido]-3-cephem-4-carbonsäurenatriumsalz

2,5 g 3-(1-Methyl-1H-tetrazol-5-ylthiomethyl)-7β-[(2R,S)-2-(2-BOC-aminothiazol-4-yl)-2-cyanomethansulfonylaminoacetamido]-3-cephem-4-carbonsäurediphenylmethylester werden in 5 ml $CH_2Cl_2$ und 0,7 ml Anisol mit 10 ml Trifluoressigsäure analog Beispiel 1a) umgesetzt, aufgearbeitet, chromatographiert und umgefällt. Man erhält das Hydrat der Titelverbindung. F: oberhalb 210° (unter Zersetzung); $[\alpha]_D^{20°}$ = $-9°\pm1°$ (0,91 % in $H_2O$); IR: 3650 bis 2500 (breit), 2260, 1760, 1685, 1605, 1520 (Nujol); UV: 257 (13600; in $H_2O$).

b) 3-(1-Methyl-1H-tetrazol-5-ylthiomethyl)-7β-[(2R,S)-2-(2-BOC-aminothiazol-4-yl)-2-cyanomethansulfonylaminoacetamido]-3-cephem-4-carbonsäurediphenylmethylester

2,6 g (2R,S)-2-(2-BOC-Aminothiazol-4-yl)-2-cyanomethansulfonylaminoessigsäure (Herstellung siehe Beispiel 44c) werden mit 3,3 g 3-(1-Methyl-1H-tetrazol-5-ylthiomethyl)-7β-amino-3-cephem-4-carbonsäurediphenylmethylester in 30 ml Tetrahydrofuran analog Beispiel 6b) umgesetzt (0,9 g Hydroxybenztriazol; dreimal 0,43 g Dicyclohexylcarbodiimid in je 6,6 ml Tetrahydrofuran), aufgearbeitet und chromatographiert. Man erhält die Titelverbindung. $[\alpha]_D^{20°}$ = -85°±1° (0,97 % in $CHCl_3$); IR: 3400, 3300, 2260, 1785, 1722, 1700 (Schulter), 1625, 1540 ($CH_2Cl_2$); UV: 258 (15800; EtOH).

Beispiel 72:

a) 3-Carbamoyloxymethyl-7β-[(2R,S)-2-(2-aminothiazol-4-yl)-2-(2-formylaminoäthansulfonylamino)-acetamido]-3-cephem-4-carbonsäurenatriumsalz

2,2 g 3-Carbamoyloxymethyl-7β-[(2R,S)-2-(2-BOC-aminothiazol-4-yl)-2-(2-formylaminoäthansulfonylamino)-acetamido]-3-cephem-4-carbonsäurediphenylmethylester werden in 4 ml $CH_2Cl_2$ und 0,636 ml Anisol mit 10 ml Trifluoressigsäure analog Beispiel 1a) umgesetzt, aufgearbeitet, chromatographiert und umgefällt. Man erhält das Hydrat der Titelverbindung. F: oberhalb 190° (unter Zersetzung); $[\alpha]_D^{20°}$ = +53°±1° (1,03 % in $H_2O$); IR: 3650 bis 2500 (breit), 1760, 1670, 1605, 1520 $cm^{-1}$ (Nujol); UV: 257 (13300; $H_2O$).

b) 3-Carbamoyloxymethyl-7β-[(2R,S)-2-(2-BOC-aminothiazol-4-yl)-2-(2-formylaminoäthansulfonylamino)-acetamido]-3-cephem-4-carbonsäurediphenylmethylester

2,3 g (2R,S)-2-(2-BOC-Aminothiazol-4-yl)-2-(2-formylaminoäthansulfonylamino)-essigsäure (Herstellung siehe Beispiel 27c) werden mit 2,4 g 3-Carbamoyloxymethyl-7β-amino-3-cephem-4-carbonsäurediphenylmethylester in 30 ml Tetrahydrofuran analog Beispiel 6b) umgesetzt (0,5 g

Hydroxybenztriazol; dreimal 1,2 g Dicyclohexylcarbodiimid in je 10 ml Tetrahydrofuran), aufgearbeitet und chromatographiert. Man erhält die Titelverbindung; $[\alpha]_D^{20°}$ = +1°$\pm$1° (1,01 % in $CHCl_3$); IR: u.a. 1785 $cm^{-1}$ ($CH_2Cl_2$); UV: 258 (14400; EtOH).

Beispiel 73:

a) 3-Acetoxymethyl-7β-[(2R,S)-2-(2-aminothiazol-4-yl)-2-(2-formylaminoäthansulfonylamino)-acetamido]-3-cephem-4-carbonsäurenatriumsalz.

2,7 g 3-Acetoxymethyl-7β-[(2R,S)-2-(2-BOC-aminothiazol-4-yl)-2-(2-formylaminoäthansulfonylamino)-acetamido]-3-cephem-4-carbonsäurediphenylmethylester werden in 14 ml $CH_2Cl_2$ und 0,73 ml Anisol mit 21 ml Trifluoressigsäure analog Beispiel 1a) umgesetzt, aufgearbeitet, chromatographiert und umgefällt. Man erhält das Hydrat der Titelverbindung. F: oberhalb 165° (unter Zersetzung); $[\alpha]_D^{20°}$ = +77°$\pm$1° (0,85 % in $H_2O$); IR: 3650 bis 2500 (breit), 1760, 1725 (Schulter), 1670, 1605, 1520 (Nujol); UV: 256 (12000; $H_2O$).

b) 3-Acetoxymethyl-7β-[(2R,S)-2-(2-BOC-aminothiazol-4-yl)-2-(2-formylaminoäthansulfonylamino)-acetamido]-3-cephem-4-carbonsäurediphenylmethylester

2,3 g (2R,S)-2-(2-BOC-Aminothiazol-4-yl)-2-(2-formylaminoäthansulfonylamino)-essigsäure (Herstellung siehe Beispiel 27c) werden mit 2,1 g 3-Acetoxymethyl-7β-amino-3-cephem-4-carbonsäurediphenylmethylester in 30 ml Tetrahydrofuran analog Beispiel 6b) umgesetzt (0,5 g Hydroxybenztriazol; dreimal 0,4 g Dicyclohexylcarbodiimid in je 6 ml Tetrahydrofuran), aufgearbeitet und chromatographiert. Man erhält die Titelverbindung; $[\alpha]_D^{20°}$ = +56°$\pm$1° (1,28 % in $CHCl_3$); IR: 3300, 1780, 1720, 1680, 1550 (Nujol); UV: 257 (15600, EtOH).

Beispiel 74:

7β-[(2R,S)-2-(2-Aminothiazol-4-yl)-2-(2-formylaminoäthansulfonylamino)-acetamido]-3-cephem-4-carbonsäurepivaloyloxymethylesterhydrochlorid

1 g 7β-[(2R,S)-2-(2-Aminothiazol-4-yl)-2-(2-formylaminoäthansulfonyl-amino)-acetamido]-3-cephem-4-carbonsäurenatriumsalz und 0,5 ml Iodmethylpivalat werden in 10 ml Dimethylformamid analog Beispiel 70) umgesetzt und aufgearbeitet. Man erhält die Titelverbindung; $[\alpha]_D^{20°}$ = +63°±1° (0,95 % in DMSO); IR: 3650 bis 2400 (breit), 1780, 1750, 1670 (breit), 1630, 1540 (Nujol); UV: 258 (11500, $CH_3OH$).

Beispiel 75:

a) 3-Carbamoyloxymethyl-7β-[(2S)-2-(2-aminothiazol-4-yl)-2-methan-sulfonylaminoacetamido]-3-cephem-4-carbonsäurenatriumsalz

1,65 g 3-Carbamoyloxymethyl-7β-[(2S)-2-(2-BOC-aminothiazol-4-yl)-2-methansulfonylaminoacetamido]-3-cephem-4-carbonsäurediphenylmethylester werden in 4,9 ml $CH_2Cl_2$ und 1,6 ml Anisol mit 18 ml Trifluor-essigsäure analog Beispiel 1a) umgesetzt, aufgearbeitet, chromato-graphiert und umgefällt. Man erhält das Hydrat der Titelverbindung. F: oberhalb 170° (unter Zersetzung); $[\alpha]_D^{20°}$ = +83°±1° (0,95 % in $H_2O$); IR: 3650 bis 2500 (breit), 1760, 1695, 1605, 1520 (Nujol); UV: 255 (12600; $H_2O$).

b) 3-Carbamoyloxymethyl-7β-[(2R)-2-(2-aminothiazol-4-yl)-2-methan-sulfonylaminoacetamido]-3-cephem-4-carbonsäurenatriumsalz

1,65 g 3-Carbamoyloxymethyl-7β-[(2R)-2-(2-BOC-aminothiazol-4-yl)-2-methansulfonylaminoacetamido]-3-cephem-4-carbonsäurediphenylmethylester werden in 4,9 ml $CH_2Cl_2$ und 1,6 ml Anisol mit 18 ml Tri-fluoressigsäure analog Beispiel 1a) umgesetzt, aufgearbeitet, chromatographiert und umgefällt. Man erhält das Hydrat der Titelverbindung. F. oberhalb 175° (unter Zersetzung); $[\alpha]_D^{20°}$ = +48°±1° (0,96 % in $H_2O$); IR: 3650 bis 2500 (breit), 1760, 1695, 1605, 1520 (Nujol); UV: 255 (12400; $H_2O$).

c) 3-Carbamoyloxymethyl-7β-[(2S)-2-(2-BOC-aminothiazol-4-yl)-2-methansulfonylaminoacetamido]-3-cephem-4-carbonsäurediphenylmethylester und

3-Carbamoyloxymethyl-7β-[(2R)-2-(2-BOC-aminothiazol-4-yl)-2-methansulfonylaminoacetamido]-3-cephem-4-carbonsäurediphenylmethylester

2,5 g (2R,S)-2-(2-BOC-Aminothiazol-4-yl)-2-methansulfonylaminoessigsäure (Herstellung siehe Beispiel 22c) werden mit 2,59 g 3-Carbamoyloxymethyl-7β-amino-3-cephem-4-carbonsäurediphenylmethylester in 33 ml Tetrahydrofuran analog Beispiel 6b) umgesetzt (0,63 g Hydroxybenztriazol; dreimal 0,58 g Dicyclohexylcarbodiimid in je 4 ml Tetrahydrofuran) und aufgearbeitet. Das anfallende Rohprodukt wird an 300 g Kieselgel (Stufensäule) chromatographiert [Eluiermittel: Toluol-Essigester 2:1- und 1:1-Gemisch]. Dabei eluiert man zuerst die Titelverbindung mit 2R-Konfiguration. (Zur Konfigurationszuordnung vgl. Beispiel 7 d); $[\alpha]_D^{20°} = -5° \pm 1°$ (1,08% in $CHCl_3$); IR: 3520, 3410, 3280, 1795, 1725, 1700 (Schulter), 1582, 1540 ($CH_2Cl_2$). UV: 259 (15200 in $C_2H_5OH$).

Höhere Fraktionen bestehen aus einem binären Gemisch der obigen (2R)-Verbindung mit dem (2S)-Isomeren.

Aus den letzten Fraktionen erhält man die Titelverbindung mit 2S-Konfiguration. $[\alpha]_D^{20°} = -7° \pm 1°$ (0,99% in $CHCl_3$); IR: 3520, 3410, 3290, 1780, 1725, 1695 (Schulter), 1582, 1540 ($CH_2Cl_2$). UV: 258 (16400; EtOH).

Beispiel 76:

a) 3-(1-Methyl-1H-tetrazol-5-ylthiomethyl)-7β-[(2R,S)-2-(2-aminothiazol-4-yl)-2-(2-methoxyäthansulfonylamino)-acetamido]-3-cephem-4-carbonsäurenatriumsalz

4 g 3-(1-Methyl-1H-tetrazol-5-ylthiomethyl)-7β-[(2R,S)-2-(2-BOC-

aminothiazol-4-yl)-2-(2-methoxyäthansulfonylamino)-acetamido]-3-cephem-4-carbonsäurediphenylmethylester werden in 7 ml Methylenchlorid gelöst. Die Lösung wird nacheinander mit 1,5 ml Anisol und 70 ml Trifluoressigsäure versetzt und unter Feuchtigkeitsausschluss 1 Stunde bei Raumtemperatur gerührt. Aus der anfänglich klaren Lösung fällt ein voluminöser Niederschlag aus. Danach wird die Suspension auf ein eiskaltes Gemisch von 600 ml Petroläther und 300 ml Diäthyläther gegossen, das anfallende Trifluoracetat abgenutscht, mit Petroläther gewaschen und im Hochvakuum bei Raumtemperatur getrocknet. Darauf wird das rohe Trifluoracetatsalz in 30 ml Acetonitril-Wasser Gemisch gelöst, die Lösung auf +5° abgekühlt und unter Rühren und Kühlen durch Zutropfen von 1 N Natronlauge ein pH-Wert von 5,8 eingestellt. Dann wird die Lösung auf 600 ml Aethanol gegossen und am Rotationsverdampfer bei 50° auf ca. 100 ml Volumen eingeengt. Diese Massnahme wird zweimal mit je 300 ml Aethanol Zusatz wiederholt, wobei das amorphe Produkt ausfällt. Es wird abgenutscht, und sukzessive mit Aethanol, einem Gemisch von Aethanol-Diäthyläther und Diäthyläther gewaschen. Man erhält das Hydrat der Titelverbindung. Schmelzpunkt ab 146° unter Zersetzung; $[\alpha]_D^{20}$ = $-7\pm1°$ (2,039 % in 0,1 N $NaHCO_3$). IR: 3310 (breit), 3200, 1785 (Schulter), 1765 (breit), 1685, 1610 (breit), 1525, 1375, 1350, 1175, 1145 (in Nujol). Rf: ca. 0,44 (Silicagel, UV 366, n-Butanol-Pyridin-Eisessig-Wasser-42:24:4:30).

b) <u>3-(1-Methyl-1H-tetrazol-5-ylthiomethyl)-7β-[(2R,S)-2-(2-BOC-aminothiazol-4-yl)-2-(2-methoxyäthansulfonylamino)-acetamido]-3-cephem-4-carbonsäurediphenylmethylester</u>

Analog Beispiel 42c) erhält man durch Umsetzung von 3,0 g (2R,S)-2-(2-BOC-Aminothiazol-4-yl)-essigsäure (Herstellung siehe Beispiel 43d) mit 3-(1-Methyl-1H-tetrazol-5-ylthiomethyl)-3-cephem-4-carbonsäurediphenylmethylester in Gegenwart von 0,50 g 1-Hydroxybenzotriazol und 1,60 g (2 x 0,80 g) N,N'-Dicyclohexylcarbodiimid in 50 ml Gemisch von Dioxan-Tetrahydrofuran-(1:1) die Titelverbin-

dung. Das Rohprodukt wird an 20-facher Menge Kieselgel gereinigt. Eluiermittel: Methylenchlorid mit 10 bis 20 % Methylacetat. Rf: ca. 0,52 (Silicagel, UV 336, Doppelflecken-Diastereomerengemisch, Chloroform-Aethylacetat-Aethanol 42,5:42,5:5).

Beispiel 77:

a) 7β-[(2R,S)-2-(2-Aminothiazol-4-yl)-2-(2-methylcarbamoylaminoäthansulfonylamino)-acetamido]-3-cephem-4-carbonsäurenatriumsalz

Analog Beispiel 76a) erhält man durch Umsetzung von 3,4 g 7β-[(2R,S)-2-(2-BOC-Aminothiazol-4-yl)-2-(2-methylcarbamoylaminoäthansulfonylamino)-acetamido]-3-cephem-4-carbonsäurediphenylmethylester mit 60 ml Trifluoressigsäure in 5 ml Methylenchlorid und 1,2 ml Anisol und anschliessender Behandlung des Trifluoracetatsalzes mit 1 N Natronlauge die Titelverbindung. Das amorphe Produkt schmilzt oberhalb 178° unter Zersetzung. $[\alpha]_D^{20°} = +83 \pm 1°$ (2,039 % in 0,1 N $NaHCO_3$). IR: 3320 (breit), 3190 (breit), 1760 (breit), 1645, 1600, 1565, 1520, 1375, 1365, 1165 (Schulter), 1140 (in Nujol). Rf: 0,33 (Silicagel, UV 366, n-Butanol-Pyridin-Eisessig-Wasser 42:24:4:30).

b) 7β-[(2R,S)-2-(2-BOC-Aminothiazol-4-yl)-2-(2-methylcarbamoylaminoäthansulfonylamino)-acetamido]-3-cephem-4-carbonsäurediphenylmethylester

Analog Beispiel 42c) erhält man die Titelverbindung durch Umsetzung von 3,0 g (2R,S)-2-(2-BOC-Aminothiazol-4-yl)-2-(2-methylcarbamoylaminoäthansulfonylamino)-essigsäure mit 2,50 g 7β-Amino-3-cephem-4-carbonsäure-diphenylmethylester in Gegenwart von 1,60 g (2 x 0,80 g) N,N'-Dicyclohexylcarbodiimid in 50 ml Tetrahydrofuran. Das Rohprodukt wird an 25-facher Menge Kieselgel gereinigt. Eluiermittel: Methylenchlorid-Methylacetat-(1:1). F: oberhalb 140° (unter Zersetzung); Rf: 0,15 (Silicagel, UV 366, Doppelflecken-Diastereomerengemisch, Chloroform-Aethylacetat-Aethanol 42,5:42,5:5).

Herstellung des Ausgangsmaterials:

c) (2R,S)-2-(2-BOC-Aminothiazol-4-yl)-2-(2-methylcarbamoylaminoäthansulfonylamino)-essigsäure

Eine Lösung von 3,8 g (2R,S)-2-(2-BOC-Aminothiazol-4-yl)-2-(2-methylcarbamoylaminoäthansulfonylamino)-essigsäuremethylester in 15 ml Methanol und 10 ml Wasser wird mit 18 ml 1 N Natriumhydroxid versetzt und das Reaktionsgemisch 4 Stunden bei 30° gerührt. Anschliessend wird die entstehende Säure analog Beispiel 43d) isoliert. Nach Abdampfen des Lösungsmittels bleibt die Titelverbindung als Schaum zurück. F: 93-96° (unter Zersetzung).

d) (2R,S)-2-(2-BOC-Aminothiazol-4-yl)-2-(2-methylcarbamoylaminoäthansulfonylamino)-essigsäuremethylester

Zu einer Lösung von 4,70 g (2R,S)-2-(2-BOC-Aminothiazol-4-yl)-2-(2-aminoäthansulfonylamino)-essigsäuremethylester in 40 ml Tetrahydrofuran wird bei 2° unter Rühren 30 Minuten lang eine Lösung von 0,80 g Methylisocyanat in 8 ml Tetrahydrofuran getropft. Anschliessend rührt man das Reaktionsgemisch 4 Stunden bei +2° und 1 Stunde bei Raumtemperatur. Dann wird die Lösung am Rotationsverdampfer bei 50° zur Trockne eingedampft und das Rohprodukt an 20-facher Menge Kieselgel gereinigt. Eluiermittel: Methylenchlorid mit 55 bis 70 % Methylacetat. Man erhält die Titelverbindung als Schaum. F: oberhalb 70° (unter Zersetzung). Rf: ca. 0,07 (Silicagel, UV 336, Toluol, Chloroform, Aethylacetat 1:1:1).

Beispiel 78:

a) 7β-[(2R,S)-2-(2-Aminothiazol-4-yl)-2-(2-anilinoformamidoäthansulfonylamino)-acetamido]-3-cephem-4-carbonsäurenatriumsalz

Analog Beispiel 76a) erhält man durch Umsetzung von 2,70 g 7β-[(2R,S)-2-(2-BOC-Aminothiazol-4-yl)-2-(2-anilinoformamidoäthan-

sulfonylamino)-acetamido]-3-cephem-4-carbonsäurediphenylmethylester mit 50 ml Trifluoressigsäure in 1,0 ml Anisol und 5 ml Methylenchlorid, und anschliessender Behandlung des Trifluoracetatsalzes mit 1 N Natronlauge die Titelverbindung. Das amorphe Produkt schmilzt oberhalb 210° unter Zersetzung. IR: 3360, 3305, 3270, 3180 (breit), 1785 (Schulter), 1760, 1650, 1640, 1590, 1560, 1535, 1510, 1375, 1365, 1145, 113 (in Nujol). Rf 96: ca. 0,29.

b) 7β-[(2R,S)-2-(2-BOC-Aminothiazol-4-yl)-2-(2-anilinoformamidoäthansulfonylamino)-acetamido]-3-cephem-4-carbonsäurediphenylmethylester

Analog Beispiel 42c) erhält man die Titelverbindung durch Umsetzung von 2,8 g (2R,S)-2-(2-BOC-Aminothiazol-4-yl)-2-(2-anilinoformamidoäthansulfonylamino)-essigsäure mit 2,10 g 7β-Amino-3-cephem-4-carbonsäurediphenylmethylester in Gegenwart von 1,20 g (2 x 0,60 g) N,N'-Dicyclohexylcarbodiimid in 50 ml Tetrahydrofuran. Das Rohprodukt wird an 20-facher Menge Kieselgel gereinigt. Eluiermittel: Methylenchlorid mit 15 bis 25 % Methylacetat. F: oberhalb 128-131° unter Zersetzung. Rf: ca. 0,43 (Silicagel, UV 366, Doppelflecken, Diastereomerengemisch, Toluol-Chloroform-Athylacetat 1:1:1 + 5 % Aethanol).

Herstellung des Ausgangsmaterials:

c) (2R,S)-2-(2-BOC-Aminothiazol-4-yl)-2-(2-anilinoformamidoäthansulfonylamino)-essigsäure

Analog Beispiel 43d) erhält man durch Umsetzung von 2,7 g (2R,S)-2-(2-BOC-Aminothiazol-4-yl)-2-(2-anilinoformamidoäthansulfonylamino)-essigsäuremethylester in 25 ml Methanol mit 6 ml 2 N Natronlauge und 4 Stunden Rühren bei 40° die Titelverbindung. Rf 96: ca. 0,69.

d) (2R,S)-2-(2-BOC-Aminothiazol-4-yl)-2-(2-anilinoformamidoäthansulfonylamino)-essigsäuremethylester

Eine Lösung von 4,54 g (2R,S)-2-(2-BOC-Aminothiazol-4-yl)-2-(2-aminoäthansulfonylamino)-essigsäuremethylester in 30 ml Tetrahydrofuran wird mit 1,60 ml Phenylisocyanat in 20 ml Tetrahydrofuran analog Beispiel 77d) umgesetzt, das Reaktionsgemisch am Rotationsverdampfer bei 50° zur Trockne eingedampft, und das Rohprodukt an 15-facher Menge Kieselgel gereinigt. Eluiermittel: Methylenchlorid mit 15 bis 25 % Methylacetat. Man erhält die Titelverbindung als Schaum.
Rf: 0,24 (Silicagel, UV 366, Toluol-Chloroform-Aethylacetat-1:1:1 + 3 % Aethanol).

Beispiel 79:

a) 3-(1-Methyl-1H-tetrazol-5-ylthiomethyl)-7β-[(2R,S)-2-(2-aminothiazol-4-yl)-2-(2-methylcarbamoylaminoäthansulfonylamino)-acetamido]-3-cephem-4-carbonsäurenatriumsalz

Analog Beispiel 43a) erhält man durch Umsetzung von 3,6 g 3-(1-Methyl-1H-tetrazol-5-ylthiomethyl)-7β-[(2R,S)-2-(2-BOC-aminothiazol-4-yl)-2-(2-methylcarbamoylaminoäthansulfonylamino)-acetamido]-3-cephem-4-carbonsäurediphenylmethylester mit 45 ml Trifluoressigsäure in 2,10 ml Anisol und 5 ml Methylenchlorid und anschliessender Behandlung des Trifluoracetatsalzes mit 1 N Natronlauge die Titelverbindung. F: oberhalb 160° unter Zersetzung; $[\alpha]_D^{20} = -34\pm1°$ (2,207 % in Dimethylsulfoxid); Rf: 0,35 (Silicagel, UV 366, n-Butanol-Pyridin-Eisessig-Wasser 42:24:4:30.

b) 3-(1-Methyl-1H-tetrazol-5-ylthiomethyl)-7β-[(2R,S)-2-(2-BOC-aminothiazol-4-yl)-2-(2-methylcarbamoylaminoäthansulfonylamino)-acetamido]-3-cephem-4-carbonsäurediphenylmethylester

Analog Beispiel 42c) erhält man durch Umsetzung von 2,20 g (2R,S)-2-(2-BOC-Aminothiazol-4-yl)-2-(2-BOC-Methylaminoäthansulfonylamino)-essigsäure mit 1,8 g 3-(1-Methyl-1H-tetrazol-5-ylthiomethyl)-7β-amino-3-cephem-4-carbonsäurediphenylmethylester in Gegenwart von

0,27 g 1-Hydroxybenzotriazol und 0,90 g (2 x 0,45 g ) N,N'-Dicyclohexylcarbodiimid in 45 ml Tetrahydrofuran die Titelverbindung. Das Rohprodukt wird an 40-facher Menge Kieselgel gereinigt. Eluiermittel: Methylenchlorid-Methylacetat (85:15). Rf: ca. 0,48 (Silicagel, UV 366, Doppelflecken, Diastereomerengemisch, Toluol-Aethylacetat 1:1).

Herstellung des Ausgangsmaterials:

c) (2R,S)-2-(2-BOC-Aminothiazol-4-yl)-2-(2-BOC-Methylaminoäthansulfonylamino)-essigsäure

9,5 g (2R,S)-2-[2-(N-Methyl-2,2,2-trichloräthoxycarbonylamino)-äthansulfonylamino]-2-(2-BOC-aminothiazol-4-yl)-essigsäure (Herstellung Beispiel 42d) werden in 100 ml Acetonitril-Eisessig 1:1 mit 12,0 g (9,5 g + 2,5 g) Zinkstaub behandelt. Aufarbeitung analog Beispiel 13d). Man gibt eine Lösung des Rohprodukts auf n-Hexan, wobei die amorphe (2R,S)-2-(2-N-Methylaminoäthansulfonylamino)-2-(2-BOC-aminothiazol-4-yl)-essigsäure ausfällt. Rf: ca. 0,13 (Silicagel, UV 366, Chloroform-Methanol-Eisessig-Wasser 45:12:1:2). 45 g dieser Säure werden in 60 ml Dioxan und 30 ml Wasser mit 6,4 ml Di-tert.-butyl-dicarbonat in Gegenwart von 4,3 g Natriumcarbonat analog Beispiel 13c) umgesetzt. Nach Vermischen des Rohproduktes mit Petroläther erhält man die amorphe Titelverbindung. Rf:ca. 0,58 (Silicagel, UV 366, Chloroform-Methanol-Eisessig-Wasser 45:12:1:2).

Beispiel 80:

a) 7β-[(2R,S)-2-(2-Aminothiazol-4-yl)-2-(2-n-butylaminoäthansulfonylamino)-acetamido]-3-cephem-4-carbonsäure

Analog Beispiel 76a) wird durch Umsetzung von 7β-[2R,S)-2-(2-BOC-Aminothiazol-4-yl)-2-(2-n-butylamino-äthansulfonylamino)-acetamido]-3-cephem-4-carbonsäurediphenylmethylester mit 50 ml Trifluoressigsäure in 1,0 ml Anisol und 5 ml Methylenchlorid und anschliessender Behandlung des Trifluorecatetsalzes mit 1 N Natronlauge die Titelverbindung erhalten. Das amorphe Produkt schmilzt bei 166-173° unter Zersetzung. IR: 3310 (breit), 3190, 1785 (Schulter), 1765 (breit), 1680, 1600 (breit), 1520, 1355 (breit), 1175 (Schulter), 1150 (in Nujol). Rf: ca. 0.38 (Silicagel, UV 366, n-Butanol-Pyridin-Eisessig-Wasser 42:24:4:30).

b) 7β-[(2R,S)-2-(2-BOC-Aminothiazol-4-yl)-2-(2-BOC-n-butylamino-äthansulfonylamino)-acetamido]-3-cephem-4-carbonsäurediphenylmethylester

Analog Beispiel 43c) erhält man durch Umsetzung von 2,3 g (2R,S)-2-(2-BOC-Aminothiazol-4-yl)-2-(2-BOC-n.-butylaminoäthansulfonylamino)-essigsäure mit 1,5 g 7β-Amino-3-cephem-4-carbonsäurediphenylmethylester in Gegenwart von 0,98 g (2 x 0,49 g) Dicyclohexylcarbodiimid in 50 ml Tetrahydrofuran die Titelverbindung. Das Rohprodukt wird an 25-facher Menge Kieselgel gereinigt. Eluiermittel: Methylenchlorid mit 7 bis 10 % Methylacetat. Rf: ca. 0,45 (Silicagel, UV 366, Toluol-Aethylacetat 2:1).

Herstellung des Ausgangsmaterials

c) (2R,S)-2-(2-BOC-Aminothiazol-4-yl)-2-(2-BOC-n.-butylaminoäthansulfonylamino)-essigsäure

7,1 g (2R,S)-2-(2-BOC-Aminothiazol-4-yl)-2-(N-butyl-2,2,2-trichloräthoxycarbonylamino)-äthansulfonylamino)-essigsäure werden in 80 ml Eisessig-Acetonitril (1:1) mit 14,0 g (10,0 g und 4,0 g) Zinkstaub behandelt. Das Reaktionsgemisch wird analog Beispiel 13d) aufgearbeitet. Beim Vermischen des Rohproduktes mit Diäthyläther erhält man die amorphe (2R,S)-2-(2-BOC-Aminothiazol-4-yl)-2-n.butylaminoäthansulfonylaminoessigsäure. Rf 96: ca. 0,42 (Silicagel, UV 366). 8,9 g dieser Säure werden in 80 ml Dioxan gelöst und in 20 ml Wasser mit 4,7 ml Di-tert.-butyl-dicarbonat in Gegenwart von 2,5 g Natriumcarbonat analog Beispiel 13c) umgesetzt. Das Rohprodukt wird an 20-facher Menge Kieselgel gereinigt. Eluiermittel: Methylenchlorid mit 10 bis 30 % Methylacetat. Man erhält die Titelverbindung. Rf 96: 0,45 (Silicagel, UV 366).

d) (2R,S)-2-(2-BOC-Aminothiazol-4-yl)-2-(N-butyl-2,2,2-trichloräthoxycarbonylamino)-äthansulfonylamino)-essigsäure

Die Umsetzung des Trimethylsilylesters von 10 g (2R,S)-2-Amino-2-(2-BOC-aminothiazol-4-yl)-essigsäure mit 20 g öligem 2-(N-n-Butyl-2,2,2-trichloräthoxycarbonylamino)-äthansulfonylchlorid (hergestellt nach Beispiel 42e) erfolgt analog Beispiel 42d). Das Rohprodukt wird an 20-facher Menge Kieselgel gereinigt. Eluiermittel: Methylenchlorid mit 10 bis 30 % Methylacetat. Man erhält die Titelverbindung. Rf 96: 0,64 (Silicagel, UV 366).

Beispiel 81:

a) 3-(1-Methyl-1H-tetrazol-5-ylthiomethyl)-7β-[(2R,S)-2-(2-aminothiazol-4-yl)-2-(2-methoxyacetylaminoäthansulfonylamino)-acetamido]-3-cephem-4-carbonsäurenatriumsalz

3,5 g gemäss Beispiel 81 b) herstellbarer 3-(1-Methyl-1H-tetrazol-5-ylthiomethyl)-7β-[(2R,S)-2-(2-BOC-aminothiazol-4-yl)-2-(2-methoxyacetylaminoäthansulfonylamino)-acetamido]-3-cephem-4-carbonsäurediphenylmethylester werden in 6 ml $CH_2Cl_2$ und 0,9 ml Anisol mit 10 ml Trifluoressigsäure analog Beispiel 1 a) umgesetzt, aufgearbeitet, chromatographiert und umgefällt. Man erhält das Hydrat der Titelverbindung; F: Zersetzung oberhalb 205°; $[\alpha]_D^{20°} = -2°\pm1°$ (0,93% in $H_2O$); IR: 3650-2500 (breit), 1763, 1665, 1600, 1520 (Nujol); UV: 258 (12600; $H_2O$).

b) 3-(1-Methyl-1H-tetrazol-5-ylthiomethyl)-7β-[(2R,S)-2-(2-BOC-aminothiazol-4-yl)-2-(2-methoxyacetylaminoäthansulfonylamino)-acetamido]-3-cephem-4-carbonsäurediphenylmethylester

5,5 g gemäss Beispiel 36 c) erhältliche (2R,S)-2-(2-BOC-Aminothiazol-4-yl)-2-(2-methoxyacetylaminoäthansulfonylamino)-essigsäure werden mit

6,0 g 3-(1-Methyl-1H-tetrazol-5-ylthiomethyl)-7β-amino-3-cephem-4-carbonsäurediphenylmethylester in 55 ml Tetrahydrofuran analog Beispiel 6 b) umgesetzt (1,6 g Hydroxybenztriazol; dreimal 0,83 g Dicyclohexylcarbodiimid in je 5 ml Tetrahydrofuran), aufgearbeitet und chromatographiert. Man erhält die Titelverbindung; $[\alpha]_D^{20°} = -73° \pm 1°$ (0,88% in $CHCl_3$); IR: 3400, 3180 (breit), 1787, 1720, 1683, 1538 ($CH_2Cl_2$); UV: 260 (16800; EtOH).

Beispiel 82:

3-(1-Methyl-1H-tetrazol-5-ylthiomethyl)-7β-[(2R,S)-2-(2-aminothiazol-4-yl)-2-(2-methoxyacetylaminoäthansulfonylamino)-acetamido]-3-cephem-4-carbonsäurepivaloyloxymethylesterhydrochlorid

0,5 g 3-(1-Methyl-1H-tetrazol-5-ylthiomethyl)-7β-[(2R,S)-2-(2-aminothiazol-4-yl)-2-(2-methoxyacetylaminoäthansulfonylamino)-acetamido]-3-cephem-4-carbonsäurenatriumsalz (Herstellung siehe Beispiel 81 a) und 0,19 ml Jodmethylpivalat werden in 5 ml Dimethylformamid analog Beispiel 70 a) umgesetzt, aufgearbeitet und in das Hydrochlorid überführt. Man erhält die Titelverbindung; F: oberhalb 150° (unter Zersetzung); $[\alpha]_D^{20°} = -18° \pm 1°$ (1,03% in DMSO); IR: 3660-2300 (breit), 1780, 1748, 1690 (Schulter), 1660-1620 (breit), 1540 (Nujol); UV: 260 (12000; $CH_3OH$).

Beispiel 83:

a) 3-(1-Methyl-1H-tetrazol-5-ylthiomethyl)-7β-[(2R,S)-2-(2-aminothiazol-4-yl)-2-(2-benzoylaminoäthansulfonylamino)-acetamido]-3-cephem-4-carbonsäurenatriumsalz

6 g des gemäss Beispiel 83 b) herstellbaren 3-(1-Methyl-1H-tetrazol-5-ylthiomethyl)-7β-[(2R,S)-2-(2-BOC-aminothiazol-4-yl)-2-(2-benzoylaminoäthansulfonylamino)-acetamido]-3-cephem-4-carbonsäurediphenylmethylesters werden in 10 ml $CH_2Cl_2$ und 1,5 ml Anisol mit 15 ml Trifluoressigsäure analog Beispiel 1 a) umgesetzt, aufgearbeitet, chromatographiert und umgefällt. Man erhält das Hydrat der Titelverbindung; F: oberhalb 205° (unter Zersetzung); $[\alpha]_D^{20°} = +25 \pm 1°$

(1,02% in $H_2O$); IR: 3660-2500 (breit), 1760, 1680 (Schulter), 1630 (Schulter), 1600, 1578, 1522 (Nujol); UV: 230 (20000), 260 (Schulter; $H_2O$).

b) <u>3-(1-Methyl-1H-tetrazol-5-ylthiomethyl)-7β-[(2R,S)-2-(2-BOC-aminothiazol-4-yl)-2-(2-benzoylaminoäthansulfonylamino)-acetamido]-3-cephem-4-carbonsäurediphenylmethylester</u>

8 g gemäss Beispiel 26 c) erhältliche (2R,S)-2-(2-BOC-Aminothiazol-4-yl)-2-(2-benzoylaminoäthansulfonylamino)-essigsäure werden mit 7,4 g 3-(1-Methyl-1H-tetrazol-5-ylthiomethyl)-7β-amino-3-cephem-4-carbonsäurediphenylmethylester in 80 ml Tetrahydrofuran analog Beispiel 6 b) umgesetzt (2,0 g Hydroxybenztriazol; dreimal 1 g Dicyclohexylcarbodiimid in je 8,3 ml Tetrahydrofuran, aufgearbeitet und chromatographiert. Man erhält die Titelverbindung; $[\alpha]_D^{20°} = -67°\pm1°$ (1,00% in $CHCl_3$); IR: 3400, 3300 (breit), 1788, 1722, 1698 (Schulter), 1663, 1601, 1579, 1538 ($CH_2Cl_2$); UV: 259 (15800; $CH_2Cl_3$).

<u>Beispiel 84:</u>

<u>3-(1-Methyl-1H-tetrazol-5-ylthiomethyl)-7β-[(2R,S)-2-(2-aminothiazol-4-yl)-2-(2-benzoylaminoäthansulfonylamino)-acetamido]-3-cephem-4-carbonsäurepivaloyloxymethylesterhydrochlorid</u>

0,8 g 3-(1-Methyl-1H-tetrazol-5-ylthiomethyl)-7β-[(2R,S)-2-(2-aminothiazol-4-yl)-2-(2-benzoylaminoäthansulfonylamino)-acetamido]-3-cephem-4-carbonsäurenatriumsalz (Herstellung siehe Beispiel 83 a) und 0,3 ml Jodmethylpivalat werden in 8 ml Dimethylformamid analog Beispiel 70 a) umgesetzt, aufgearbeitet und in das Hydrochlorid überführt. Man erhält die Titelverbindung; F: oberhalb 195° (unter Zersetzung); $[\alpha]_D^{20°} = -12°\pm1°$ (0,69% in DMSO); IR: 3660-2500 (breit), 1782, 1750, 1692, 1630, 1600 (Schulter), 1577, 1535 (Nujol); UV: 220 (21000), 258 (13700; $CH_3OH$).

Beispiel 85:

a) 3-(1-Methyl-1H-tetrazol-5-ylthiomethyl)-7β-[(2R,S)-2-(2-aminothiazol-4-yl)-2-(2-acryloylaminoäthansulfonylamino)-acetamido]-3-cephem-4-carbonsäurenatriumsalz

2,13 g gemäss Beispiel 85 b) erhältlicher 3-(1-Methyl-1H-tetrazol-5-ylthiomethyl)-7β-[(2R,S)-2-(2-BOC-aminothiazol-4-yl)-2-(2-acryloylaminoäthansulfonylamino)-acetamido]-3-cephem-4-carbonsäurediphenylmethylesters werden in 4,1 ml $CH_2Cl_2$ und 1,35 ml Anisol mit 15,5 ml Trifluoressigsäure analog Beispiel 1 a) umgesetzt, aufgearbeitet, chromatographiert und umgefällt. Man erhält das Hydrat der Titelverbindung; F: oberhalb 157° (unter Zersetzung); $[\alpha]_D^{20°}$ = 0° (0,75% in $H_2O$); IR: 3660-2500, 1763, 1690 (Schulter), 1660, 1625 (Schulter), 1600, 1550 (Schulter), 1525 (Nujol); UV: 255 (13500; $H_2O$).

b) 3-(1-Methyl-1H-tetrazol-5-ylthiomethyl)-7β-[(2R,S)-2-(2-BOC-aminothiazol-4-yl)-2-(2-acryloylaminoäthansulfonylamino)-acetamido]-3-cephem-4-carbonsäurediphenylmethylester

2,88 g gemäss Beispiel 31 c) erhältliche (2R,S)-2-(2-BOC-aminothiazol-4-yl)-2-(2-acryloylaminoäthansulfonylamino)-essigsäure werden mit 2,9 g 3-(1-Methyl-1H-tetrazol-5-ylthiomethyl)-7β-amino-3-cephem-4-carbonsäurediphenylmethylester in 30 ml Tetrahydrofuran analog Beispiel 6 b) umgesetzt (0,66 g Hydroxybenztriazol; dreimal 0,58 g Dicyclohexylcarbodiimid in je 4 ml Tetrahydrofuran), aufgearbeitet und chromatographiert. Man erhält die Titelverbindung; $[\alpha]_D^{20°}$ = -66°±1° (0,98% in $CHCl_3$); IR: 3390, 3290, 1775, 1710, 1690 (Schulter), 1670 (Schulter), 1620, 1600 (Schulter), 1515 ($CH_2Cl_2$); UV: 260 (16000; EtOH).

Beispiel 86:

3-Carbamoyloxymethyl-7β-[(2R,S)-2-(2-aminothiazol-4-yl)-2-methansulfonylaminoacetamido]-3-cephem-4-carbonsäurepivaloyloxymethylesterhydrochlorid

1,815 g 3-Carbamoyloxymethyl-7β-[(2R,S)-2-(2-aminothiazol-4-yl)-2-

methansulfonylaminoacetamido]-3-cephem-4-carbonsäurenatriumsalz (Herstellung siehe Beispiel 69a) und 0,9 ml Jodmethylpivalat werden in 18,15 ml Dimethylformamid analog Beispiel 70 a) umgesetzt, aufgearbeitet und in das Hydrochlorid überführt. Man erhält die Titelverbindung; F: oberhalb 123° (unter Zersetzung); $[\alpha]_D^{20°} = +30 \pm 1°$ (0,81% in DMSO); IR: 3660-2300 (breit), 1780, 1745, 1700, 1630, 1545 (Nujol); UV: 260 (11800; $CH_3OH$).

Beispiel 87:

a) 3-Carbamoyloxymethyl-7β-[(2R)-2-(2-aminothiazol-4-yl)-2-(2-acryloylaminoäthansulfonylamino)-acetamido]-3-cephem-4-carbonsäurenatriumsalz

2,62 g gemäss Beispiel 87 c) erhältlicher 3-Carbamoyloxymethyl-7β-[(2R)-2-(2-BOC-aminothiazol-4-yl)-2-(2-acryloylaminoäthansulfonylamino)-acetamido]-3-cephem-4-carbonsäurediphenylmethylester werden in 5,4 ml $CH_2Cl_2$ und 1,76 ml Anisol mit 20 ml Trifluoressigsäure analog Beispiel 1 a) umgesetzt, aufgearbeitet, chromatographiert und umgefällt. Man erhält das Hydrat der Titelverbindung; F: oberhalb 155° (unter Zersetzung); $[\alpha]_D^{20°} = +40° \pm 1°$ (0,85% in $H_2O$); IR: 3660-2500 (breit), 1760, 1700, 1658, 1605, 1522 (Nujol); UV: 253 (12800; $H_2O$).

b) 3-Carbamoyloxymethyl-7β-[(2S)-2-(2-aminothiazol-4-yl)-2-(2-acryloylaminoäthansulfonylamino)-acetamido]-3-cephem-4-carbonsäurenatriumsalz

3,45 g des gemäss Beispiel 87 c) erhältlichen 3-Carbamoyloxymethyl-7β-[(2S)-2-(2-BOC-aminothiazol-4-yl)-2-(2-acryloylaminoäthansulfonylamino)-acetamido]-3-cephem-4-carbonsäurediphenylmethylesters werden in 7 ml $CH_2Cl_2$ und 2,3 ml Anisol mit 26 ml Trifluoressigsäure analog Beispiel 1 a) umgesetzt, aufgearbeitet, chromatomatographiert und umgefällt. Man erhält das Hydrat der Titelverbindung; F: oberhalb 160° (unter Zersetzung); $[\alpha]_D^{20°} = +69° \pm 1°$ (0,92% in $H_2O$); IR 3660-2500 (breit, 1762, 1700 (Schulter), 1600, 1605, 1522 (Nujol); UV: 253 (13800; $H_2O$).

c) 3-Carbamoyloxymethyl-7β-[(2R)-2-(2-BOC-aminothiazol-4-yl)-2-(2-acryloylaminoäthansulfonylamino)-acetamido]-3-cephem-4-carbonsäurediphenylmethylester

und

3-Carbamoyloxymethyl-7β-[(2S)-2-(2-BOC-aminothiazol-4-yl-2-(2-acryloylaminoäthansulfonylamino)-acetamido]-3-cephem-4-carbonsäurediphenylmethylester

9,93 g gemäss Beispiel 31 c) erhältliche (2R,S)-2-(2-BOC-Aminothiazol-4-yl)-2-(2-acryloylaminoäthansulfonylamino)-essigsäure werden mit 8,95 g 3-Carbamoyloxymethyl-7β-amino-3-cephem-4-carbonsäurediphenylmethylester in 70 ml Tetrahydrofuran analog Beispiel 6 b) umgesetzt (2,28 g Hydroxybenztriazol; dreimal 2,0 g Dicyclohexylcarbodiimid in je 10 ml Tetrahydrofuran) und aufgearbeitet. Das erhaltene Rohprodukt chromatographiert man an 700 g Kieselgel [Eluiermittel: Toluol-Essigester 7:3, 3:2, 1:1 und 1:2-Gemisch. Dabei eluiert man zuerst die Titelverbindung mit der 2R-Konfiguration (zur Konfigurationszuordnung vgl. Beispiel 7 d); $[\alpha]_D^{20°} = -5°\pm1°$ (0,75% in $CHCl_3$); IR: 3520, 3420, 3300, 3200 (Schulter), 1775, 1712, 1670 (Schulter), 1620, 1580, 1520 ($CH_2Cl_2$); UV: 259 (16000; EtOH).

Die Folgefraktionen bestehen aus einem binären Gemisch der obigen (2R)-Verbindung mit dem (2S)-Isomeren.

Aus den letzten Fraktionen erhält man die Titelverbindung mit der 2S-Konfiguration; $[\alpha]_D^{20°} = +4°\pm1°$ (0.91% in $CHCl_3$); IR 3550, 3429, 330, 3200 (Schulter), 1778, 1715, 1700 (Schulter), 1670 (Schulter), 1620 (Schulter), 1600, 1580, 1522 ($CH_2Cl_2$); UV: 259 (16200; EtOH).

Beispiel 88:

3-Carbamoyloxymethyl-7β-[(2R)-2-(2-aminothiazol-4-yl)-2-(2-acryloylaminoäthansulfonylamino)-acetamido]-3-cephem-4-carbonsäurepivaloyloxymethylesterhydrochlorid

0,54 g 3-Carbamoyloxymethyl-7β-[(2R)-2-(2-aminothiazol-4-yl)-2-(2-acryloylaminoäthansulfonylamino)-acetamido]-3-cephem-4-carbonsäurenatriumsalz (Herstellung siehe Beispiel 87 a) und 0,23 ml Jodmethylpivalat werden in 5,4 ml Dimethylformamid analog Beispiel 70 a) umgesetzt, aufgearbeitet und in das Hydrochlorid überführt. Man erhält die Titelverbindung; F: oberhalb 130° (unter Zersetzung); IR: 3660-2400 (breit), 1780, 1748, 1700, 1660, 1627, 1545 (Nujol); UV 259 (11000; $CH_3OH$).

Beispiel 89:

3-Carbamoyloxymethyl-7β-[(2S)-2-(2-aminothiazol-4-yl)-2-(2-acryloylaminoäthansulfonylamino)-acetamido]-3-cephem-4-carbonsäurepivaloyloxymethylesterhydrochlorid

0,67 g 3-Carbamoyloxymethyl-7β-[(2S)-2-(2-aminothiazol-4-yl)-2-(2-acryloylaminoäthansulfonyloxyamino)-acetamido]-3-cephem-4-carbonsäurenatriumsalz (Herstellung siehe Beispiel 87 b) und 0,29 ml Jodmethylpivalat werden in 6,7 ml Dimethylformamid analog Beispiel 70 a) umgesetzt, aufgearbeitet und in das Hydrochlorid überführt. Man erhält die Titelverbindung; F: oberhalb 125° (unter Zersetzung); IR: 3660-2400 (breit), 1780, 1748, 1700, 1660, 1627, 1545 (Nujol); UV: 259 (11000; $CH_3OH$).

Beispiel 90:

a) 3-(1-Methyl-1H-tetrazol-5-ylthiomethyl)-7β-[(2R,S)-2-(2-aminothiazol-4-yl)-2-(2-acetylaminoäthansulfonylamino)-acetamido]-3-cephem-4-carbonsäurenatriumsalz

3,5 g gemäss Beispiel 90 b) herstellbarer 3-(1-Methyl-1H-tetrazol-5-ylthiomethyl)-7β-[(2R,S)-2-(2-BOC-aminothiazol-4-yl)-2-(2-acetylaminoäthansulfonylamino)-acetamido]-3-cephem-4-carbonsäure-diphenylmethylester werden in 6 ml $CH_2Cl_2$ und 0,9 ml Anisol mit 10 ml Trifluoressigsäure analog Beispiel 1 a) umgesetzt, aufgearbeitet, chromatographiert und umgefällt. Man erhält das Hydrat der Titelverbindung; F: oberhalb 180° (unter Zersetzung); $[\alpha]_D^{20°} = -2°\pm1°$ (0,81% in $H_2O$); IR: 3660-2500 (breit), 1765, 1690-1600 (breit), 1522 (Nujol); UV: 260 (13200; $H_2O$).

b) <u>3-(1-Methyl-1H-tetrazol-5-ylthiomethyl)-7β-[(2R,S)-2-(2-BOC-aminothiazol-4-yl)-2-(2-acetylaminoäthansulfonylamino)-acetamido]-3-cephem-4-carbonsäurediphenylmethylester</u>

4,7 g gemäss Beispiel 24 c) herstellbare (2R,S)-2-(2-BOC-Aminothiazol-4-yl)-2-(2-acetylaminoäthansulfonylamino)-essigsäure werden mit 4,5 g 3-(1-Methyl-1H-tetrazol-5-ylthiomethyl)-7β-amino-3-cephem-4-carbonsäurediphenylmethylester in 50 ml Tetrahydrofuran analog Beispiel 6 b) umgesetzt (1,2 g Hydroxybenztriazol; dreimal 0,6 g Dicyclohexylcarbodiimid in je 5 ml Tetrahydrofuran), aufgearbeitet und chromatographiert. Man erhält die Titelverbindung; $[\alpha]_D^{20°} = -76°\pm1°$ (1,47% in $CHCl_3$); IR: 3420 (Schulter), 3398, 3300 (breit), 1789, 1722, 1676, 1543 ($CH_2Cl_2$); UV: 260 (16600; EtOH).

<u>Beispiel 91:</u>
<u>3-(1-Methyl-1H-tetrazol-5-ylthiomethyl)-7β-[(2R,S)-2-(2-aminothiazol-4-yl)-2-(2-acetylaminoäthansulfonylamino)-acetamido]-3-cephem-4-carbonsäurepivaloyloxymethylesterhydrochlorid</u>

0,5 g 3-(1-Methyl-1H-tetrazol-5-ylthiomethyl)-7β-[(2R,S)-2-(2-aminothiazol-4-yl)-2-(2-acetylaminoäthansulfonylamino)-acetamido]-3-cephem-4-carbonsäurenatriumsalz (Herstellung siehe Beispiel 90 a) und 0,2 ml Jodmethylpivalat werden in 5 ml Dimethylformamid analog Beispiel 70 a) umgesetzt, aufgearbeitet und in das Hydrochlorid überführt. Man erhält die Titelverbindung; F: oberhalb 180° (unter Zersetzung); $[\alpha]_D^{20°} = -20°\pm1°$ (1,08% in DMSO); IR: 3660-2500 (breit), 1785, 1750, 1694, 1630, 1545 (Nujol); UV: 260 (12800; $CH_3OH$).

Beispiel 92:

a) 3-Carbamoyloxymethyl-7β-[(2R)-2-(2-aminothiazol-4-yl)-2-(2-methoxyacetylaminoäthansulfonylamino)-acetamido]-3-cephem-4-carbonsäure-natriumsalz

1,1 g gemäss Beispiel 92 c) herstellbarer 3-Carbamoyloxymethyl-7β-[(2R)-2-(2-BOC-aminothiazol-4-yl)-2-(2-methoxyacetylaminoäthansulfonylamino)-acetamido]-3-cephem-4-carbonsäurediphenylmethylester werden in 5 ml $CH_2Cl_2$ und 0,3 ml Anisol mit 10 ml Trifluoressigsäure analog Beispiel 1 a) umgesetzt, aufgearbeitet, chromatographiert und umgefällt. Man erhält das Hydrat der Titelverbindung; F: oberhalb 190° (unter Zersetzung); $[\alpha]_D^{20°} = +49° \pm 1°$ (0,84% in $H_2O$); IR: 3660-2500 (breit), 1762, 1670, 1610, 1525 (Nujol); UV: 255 (12200; $H_2O$).

b) 3-Carbamoyloxymethyl-7β-[(2S)-2-(2-aminothiazol-4-yl)-2-(2-methoxyacetylaminoäthansulfonylamino)-acetamido]-3-cephem-4-carbonsäure-natriumsalz

1,7 g gemäss Beispiel 92 c) herstellbarer 3-Carbamoyloxymethyl-7β-[(2S)-2-(2-BOC-aminothiazol-4-yl)-2-(2-methoxyacetylaminoäthansulfonylamino)-acetamido]-3-cephem-4-carbonsäurediphenylmethylester werden in 5 ml $CH_2Cl_2$ und 0,47 ml Anisol mit 10 ml Trifluoressigsäure analog Beispiel 1 a) umgesetzt, aufgearbeitet, chromatographiert und umgefällt. Man erhält das Hydrat der Titelverbindung; F: oberhalb 190° (unter Zersetzung); $[\alpha]_D^{20°} = +6° \pm 1°$ (0,99% in $H_2O$); IR: 3660-2500 (breit), 1762, 1705 (Schulter), 1670, 1605, 1525 (Nujol); UV 255 (12800; $H_2O$).

c) 3-Carbamoyloxymethyl-7β-[(2R)-2-(2-BOC-aminothiazol-4-yl)-2-(2-methoxyacetylaminoäthansulfonylamino)-acetamido]-3-cephem-4-carbonsäurediphenylmethylester

und

3-Carbamoyloxymethyl-7β-[(2S)-2-(2-BOC-aminothiazol-4-yl)-2-(2-methoxyacetylaminoäthansulfonylamino)-acetamido]-3-cephem-4-carbonsäure-diphenylmethylester

6,7 g gemäss Beispiel 34 c) erhältliche (2R,S)-2-(2-BOC-Aminothiazol-4-yl)-2-(2-methoxyacetylaminoäthansulfonylamino)-essigsäure werden mit 6 g 3-Carbamoyloxymethyl-7β-amino-3-cephem-4-carbonsäurediphenylmethylester in 70 ml Tetrahydrofuran analog Beispiel 6 b) umgesetzt (1,8 g Hydroxybenztriazol; dreimal 0,93 g Dicyclohexylcarbodiimid in je 6,6 ml Tetrahydrofuran) und aufgearbeitet. Das erhaltene Rohprodukt chromatographiert man an 400 g Kieselgel [Eluiermittel: Toluol-Essigester 4:1, 1:1 und 1:2-Gemisch und Essigester. Dabei eluiert man zuerst die Titelverbindung mit der 2R-Konfiguration (zur Konfigurationszuordnung vgl. Beispiel 7 d); $[\alpha]_D^{20°} = +20°\pm1°$ (0,25% in $CHCl_3$); IR: 3530, 3415, 3280 (breit), 1787, 1728, 1690, 1583, 1542 ($CH_2Cl_2$; UV: 259 (16600; EtOH).

Die Folgefraktionen bestehen aus einem binären Gemisch der obigen (2R)-Verbindung mit dem (2S)-Isomeren.

Aus den letzten Fraktionen erhält man die Titelverbindung der 2S-Konfiguration; $[\alpha]_D^{20°} = +7°\pm1°$ (0,92% in $CHCl_3$); IR: 3525, 3418, 3290 (breit), 1785, 1728, 1685, 1584, 1542 ($CH_2Cl_2$); UV: 259 (16000; EtOH).

Beispiel 93:

a) 3-Acetoxymethyl-7β-[(2R,S)-2-(2-aminothiazol-4-yl)-2-(2-propioloylaminoäthansulfonylamino)-acetamido]-3-cephem-4-carbonsäure-natriumsalz

10,0 g des gemäss Beispiel 93 b) erhältlichen 3-Acetoxymethyl-7β-[(2R,S)-2-(2-BOC-aminothiazol-4-yl)-2-(2-propioloylaminoäthansulfonylamino)-acetamido]-3-cephem-4-carbonsäurediphenylmethylesters werden in 15 ml $CH_2Cl_2$ und 2,82 ml Anisol mit 20 ml Trifluoressigsäure

analog Beispiel 1 a) umgesetzt, aufgearbeitet, chromatographiert und umgefällt. Man erhält das Hydrat der Titelverbindung; F: oberhalb 180° (unter Zersetzung); $[\alpha]_D^{20°} = +155° \pm 1°$ (1,08% in $H_2O$); IR: 3660-2500 (breit), 2110, 1758, 1680 (Schulter), 1650-1610 (breit), 1520 (Nujol); UV: 250 (16000; $H_2O$).

b) 3-Acetoxymethyl-7β-[(2R,S)-2-(2-BOC-aminothiazol-4-yl)-2-(2-propioloylaminoäthansulfonylamino)-acetamido]-3-cephem-4-carbonsäurediphenylmethylester

15 g des gemäss Beispiel 83 c) erhältlichen 3-Acetoxymethyl-7β-[(2R,S)-2-(2-BOC-aminothiazol-4-yl)-2-(2-aminoäthansulfonylamino)-acetamido]-3-cephem-4-carbonsäurediphenylmethylesters werden mit 1,15 ml Propiolsäure in 200 ml Tetrahydrofuran analog Beispiel 6 b) umgesetzt (2,5 g Hydroxybenztriazol; dreimal 1,3 g Dicyclohexylcarbodiimid in je 20 ml Tetrahydrofuran), aufgearbeitet und chromatographiert. Man erhält die Titelverbindung; IR: 3400, 3300, 2120, 1790, 1726, 1700 (Schulter), 1668, 1603, 1540 ($CH_2CH_2$); UV: 258 (13500; EtOH).

Herstellung des Ausgangsmaterials:

c) 3-Acetoxymethyl-7β-[(2R,S)-2-(2-BOC-aminothiazol-4-yl)-2-(2-aminoäthansulfonylamino)-acetamido]-3-cephem-4-carbonsäurediphenylmethylester

5,5 g des gemäss Beispiel 83 d) erhältlichen 3-Acetoxymethyl-7β-[(2R,S)-2-(2-BOC-aminothiazol-4-yl)-2-(2-(2,2,2-trichloräthoxycarbonylamino)-äthansulfonylamino)-acetamido]-3-cephem-4-carbonsäurediphenylmethylesters werden mit 5,5 g Zinkstaub in 60 ml Acetonitril-Essigsäure 1:1-Gemisch analog Beispiel 35 c) umgesetzt und aufgearbeitet. Man erhält die Titelverbindung; $[\alpha]_D^{20°} = +67° \pm 1°$ (0,78% in $CHCl_3$); IR: 3320 (Schulter), 3270, 1785, 1740 (Schulter), 1725, 1680 (Schulter), 1626, 1550 (Nujol); UV: 257 (12400; EtOH).

d) 3-Acetoxymethyl-7β-[(2R,S)-2-(2-BOC-aminothiazol-4-yl)-2-(2-(2,2,2-trichloräthoxycarbonylamino)-äthansulfonylamino)-acetamido]-3-cephem-4-carbonsäurediphenylmethylester

4,6 g gemäss Beispiel 13 e) erhältliche (2R,S)-2-(2-BOC-aminothiazol-4-yl)-2-(2,2,2,2-Trichloräthoxycarbonylamino)-äthansulfonylamino)-essigsäure werden mit 3,29 g 3-Acetoxymethyl-7β-amino-3-cephem-4-carbonsäurediphenylmethylester in 40 ml Tetrahydrofuran analog Beispiel 6 b) umgesetzt (o,76 g Hydroxybenztriazol; dreimal 0,67 g Dicyclohexylcarbodiimid in je 4 ml Tetrahydrofuran), aufgearbeitet und chromatographiert. Man erhält die Titelverbindung; $[\alpha]_D^{20°} = 0°$ (0,79% in $CHCl_3$); IR: 3420 (Schulter), 3400, 3290, 1787, 1740 (Schulter), 1730, 1700 (Schulter), 1605 (schwach), 1541, 1525 (Schulter), 1498 (Nujol); UV: 257 (15300; EtOH).

Beispiel 94:

7β-[(2R,S)-2-(2-Aminothiazol-4-yl)-2-methansulfonylaminoacetamido]-3-cephem-4-carbonsäurepivaloyloxymethylesterhydrochlorid

1,78 g 7β-[(2,R,S)-2-(2-Aminothiazol-4-yl)2-methansulfonylacetamido]-3-cephem-4-carbonsäurenatriumsalz (Herstellung siehe Beispiel 2 a) und 1,027 ml Jodmethylpivalat werden in 17,8 ml Dimethylformamid analog Beispiel 70 a) umgesetzt, aufgearbeitet und in das Hydrochlorid überführt. Man erhält die Titelverbindung; F: oberhalb 140° (unter Zersetzung); $[\alpha]_D^{20°} = +70° \pm 1°$ (0,88% in DMSO); IR: 3660-2300 (breit), 1780, 1750, 1695, 1630, 1540 (Nujol); UV: 256 (11000;$CH_3OH$).

Beispiel 95:

3-(1-Sulfomethyl-1H-tetrazol-5-ylthiomethyl)-7β-[(2R,S)-2-(2-aminothiazol-4-yl)-2-methansulfonylaminoacetamido]-3-cephem-4-carbonsäuredinatriumsalz

Analog Beispiel 18 wird ausgehend von 1,26 g 3-Acetoxymethyl-7β-[(2R,S)-2-(2-aminothiazol-4-yl)-2-methansulfonylaminoacetamido]-3-cephem-4-carbonsäurenatriumsalz (Herstellung siehe Beispiel 1 a) und

1,33 g 1-Sulfomethyl-5-mercapto-1H-tetrazolnatriumsalz in 4,2 ml Wasser das Hydrat der Titelverbindung erhalten; F: oberhalb 180° (unter Zersetzung); $[\alpha]_D^{20°}$=+9°±1° (0,77% in $H_2O$); IR: 3660-2500 (breit), 1760, 1682, 1605, 1550 (Schulter), 1522 (Nujol); UV: 260 (14800; $H_2O$).

Beispiel 96:

3-(4-Carbamoylpyridiniomethyl)-7β-[(2R,S)-2-(2-aminothiazol-4-yl)-2-(2-formylaminoäthansulfonylamino)-acetamido]-3-cephem-4-carbonsäure

3,3 g 3-Acetoxymethyl-7β-[(2R,S)-2-(2-aminothiazol-4-yl)-2-(2-formylaminoäthansulfonylamino)-acetamido]-3-cephem-4-carbonsäurenatriumsalz (Herstellung siehe Beispiel 73 a), 1,0 g Isonicotinamid, 9,1 g Natriumjodid und 1,0 g Trichloressigsäure werden in 6,1 ml Wasser analog Beispiel 23 umgesetzt, aufgearbeitet, chromatographiert und umgefällt. Man erhält die Titelverbindung; F: oberhalb 175° (unter Zersetzung); IR: 2700-2500 (breit), 1778, 1720 (Schulter), 1688, 1610, 1570, 1520 (Nujol); UV: 260 (13000; $H_2O$).

Beispiel 97:

a) 3-Carbamoyloxymethyl-7β-[(2R)-2-(2-aminothiazol-4-yl)-2-äthansulfonylaminoacetamido]-3-cephem-4-carbonsäurenatriumsalz

1,8 g gemäss Beispiel 97 c) erhältlicher 3-Carbamoyloxymethyl-7β-[(2R)-2-(2-BOC-aminothiazol-4-yl)-2-äthansulfonylaminoacetamido]-3-cephem-4-carbonsäurediphenylmethylester werden in 3,75 ml $CH_2Cl_2$ und 1,32 ml Anisol mit 15 ml Trifluoressigsäure analog Beispiel 1a) umgesetzt, aufgearbeitet, chromatographiert und umgefällt. Man erhält die Titelverbindung; F: oberhalb 170° (unter Zersetzung); $[\alpha]_D^{20°}$ = +44°±1° (0,98% in $H_2O$); IR: 3660-2500 (breit), 1761, 1697, 1605, 1521 (Nujol); UV 258 (12400; $H_2O$).

b) 3-Carbamoyloxymethyl-7β-[(2S)-2-(aminothiazol-4-yl)-2-äthansulfonylaminoacetamido]-3-cephem-4-carbonsäurenatriumsalz

2,07 g gemäss Beispiel 97 c) erhältlicher 3-Carbamoyloxymethyl-7β-

[(2S)-2-(2-BOC-aminothiazol-4-yl)-2-äthansulfonylaminoacetamido-3-cephem-4-carbonsäurediphenylmethylester werden in 4,3 ml $CH_2Cl_2$ und 1,52 ml Anisol mit 17,25 ml Trifluoressigsäure analog Beispiel 1 a) umgesetzt, aufgearbeitet, chromatographiert und umgefällt. Man erhält die Titelverbindung; F: oberhalb 170° (unter Zersetzung); $[\alpha]_D^{20°}$ = +78°$\pm$1° (1,06% in $H_2O$); IR: 3660-2500 (breit), 1760, 1700, 1605, 1520 (Nujol); UV: 255 (12700; $H_2O$).

c) 3-Carbamoyloxymethyl-7β-[(2R)-2-(2-BOC-aminothiazol-4-yl)-2-äthansulfonylaminoacetamido]-3-cephem-4-carbonsäurediphenylmethylester

und

3-Carbamoyloxymethyl-7β-[(2S)-2-(2-BOC-aminothiazol-4-yl)-2-äthansulfonylaminoacetamido]-3-cephem-4-carbonsäurediphenylmethylester

3 g der gemäss Beispiel 97 d) erhältlichen (2R,S)-2-(2-BOC-Aminothiazol-4-yl)-2-äthansulfonylaminoessigsäure werden mit 3 g 3-Carbamoyloxymethyl-7β-amino-3-cephem-4-caronsäurediphenylmethylester in 40 ml Tetrahydrofuran analog Beispiel 6 b) umgesetzt (0,73 g Hydroxybenztriazol; dreimal 0,67 g Dicyclohexylcarbodiimid in je 4 ml Tetrahydrofuran) und aufgearbeitet. Das anfallende Rohprodukt chromatographiert man an 450 g Kieselgel [Stufensäule; Eluiermittel: Toluol-Essigester 3:2, 1:1 und 1:2-Gemisch. Dabei eluiert man zuerst die Titelverbindung mit der 2R-Konfiguration (zur Konfigurationszuordnung vgl. Beispiel 7 d); $[\alpha[_D^{20°}$ = -26°$\pm$1° (0,79% in $CHCl_3$); IR: 3530, 3420, 3300, 1778, 1715, 1698 (Schulter), 1582, 1530 ($CH_2Cl_2$);UV:260 (15800; EtOH).

Die Folgefraktionen bestehen aus einem Gemisch der (2R)- und (2S)-Titelverbindungen. Zuletzt eluiert man die einheitliche 2S Titelverbindung; $[\alpha]_D^{20°}$ = -12°$\pm$1° (0,83% in $CHCl_3$); IR: 3530, 3420, 3300, 1775, 1715, 1695 (Schulter), 1582, 1530 ($CH_2Cl_2$); UV: 260 (16000; EtOH).

Das Ausgangsmaterial stellt man folgendermassen her:

d) (2R,S)-2-(2-BOC-Aminothiazol-4-yl)-2-äthansulfonylaminoessigsäure

3 g (2R,S)-2-(2-BOC-Aminothiazol-4-yl)-2-aminoessigsäure werden in

30 ml $CH_2Cl_2$ mit 1,82 ml Aethansulfochlorid analog Beispiel 6 c) umgesetzt (10,5 ml N,O-Bis-(trimethylsilyl)-acetamid; 1,043 ml Pyridin) und aufgearbeitet. Man erhält die Titelverbindung, die ohne Charakterisierung weiterverarbeitet wird.

Beispiel 98:

a) 7β-[(2R,S)-2-(2-Aminothiazol-4-yl)-2-(2-methansulfonylaminoäthansulfonylamino)-acetamido]-3-cephem-4-carbonsäurenatriumsalz

Analog Beispiel 21 a) wird ausgehend von 8,07 g (10 mMol) 7β-[2R,S)-2-(2-BOC-Aminothiazol-4-yl)-2-(2-methansulfonylaminoäthansulfonylamino)-acetamido]-3-cephem-4-carbonsäurediphenylmethylester, gelöst in 40 ml absolutem Methylenchlorid, mit 8 ml Anisol und 40 ml Trifluoressigsäure die Titelverbindung erhalten, die mit derjenigen des Beispiel 21 identisch ist. F: oberhalb 175° (unter Zersetzung); UV: 253 (10500), Wasser); $[\alpha]_D^{20°} = +95° \pm 1°$ (0,880% in Wasser).

b) 7β-[(2R,S)-2-(2-BOC-Aminothiazol-4-yl)-2-(2-methansulfonylaminoäthansulfonylamino)-acetamido]-3-cephem-4-carbonsäurediphenylmethylester

Eine Lösung von 9,17 g (20 mMol) der gemäss Beispiel 98 c) hergestellten (2R,S)-2-(2-BOC-aminothiazol-4-yl)-2-(2-methansulfonylaminoäthansulfonylamino-essigsäure und 7,3 g (20 mMol) 7β-Amino-3-cephem-4-carbonsäurediphenylmethylester in 200 ml absolutem Tetrahydrofuran in Gegenwart von 2,7 g 1-Hydroxybenztriazol und 4,54 g Dicyclohexylcarbodiimid wird während 16 Stunden bei Raumtemperetaur gerührt. Darauf wird der gebildete Dicyclohexylharnstoff abfiltriert und das Filtrat eingeengt. Der in 600 ml Essigester gelöste Rückstand wird mit 0,5 N Salzsäure und Natriumchloridlösung gewaschen. Nach Trocknen der organischen Phase mit Natriumsulfat wird das Lösungsmittel im Rotationsverdampfer entfernt und das anfallende Rohprodukt an Silicagel (30fache Menge) mit Methylenchlorid/Essigester (1:1) als Eluiermittel gereinigt, woraus die Titelverbindung als amorphes Pulver erhalten wird. DC (Silicagel, Identifikation UV 366); Rf:

ca. 0,25 (Methylenchlorid/Essigester 1:1).

c) (2R,S)-2-(2-BOC-Aminothiazol-4-yl)-2-(2-methansulfonylaminoäthansulfonylamino)-essigsäure

Zu einer Suspension von 22,82 g (60 mMol) des gemäss Beispiel 13 d) erhältlichen (2R,S)-2-(2-Aminoäthansulfonylamino)-2-(2-BOC-aminothiazol-4-yl)-essigsäure in 480 ml absolutem Tetrahydrofuran werden unter Rühren und Feuchtigkeitsausschluss bei Raumtemperatur 60 ml N,O-Bis-(trimethylsilyl)-acetamid gegeben. Das Reaktionsgemisch wird eine Stunde bei 65° gerührt, auf 0° gekühlt und mit 7,1 ml absolutem Pyridin und 7 ml Methansulfochlorid versetzt. Nach 2 Stunden Reaktionszeit wird das auf Raumtemperatur erwärmte Reaktionsgemisch nochmals mit 2,4 ml Pyridin und 2,3 ml Methansulfochlorid versetzt. Nach weiteren 16 Stunden Reaktionszeit wird das Lösungsmittel am Rotationsverdampfer entfernt, der Rückstand in Essigester gelöst und je dreimal mit kalter 1N Salzsäure und gesättigter Natriumchloridlösung gewaschen. Die über Natriumsulfat getrocknete organische Phase wird am Rotationsverdampfer vom Lösungsmittel befreit, woraus die Titelverbindung als beiges amorphes Pulver erhalten wird, das ohne weitere Reinigung im nächsten Syntheseschritt eingesetzt werden kann. Rf 96: 0,48 (Silicagel, UV 366).

Beispiel 99:

7β-[(2R,2)-2-(2-Aminothiazol-4-yl)-2-(2-methansulfonylaminoäthansulfonylamino)-acetamido]-3-cephem-4-carbonsäurepivaloyloxymethylester-hydrochlorid

600 mg 7β-[(2R,S)-2-(2-Aminothiazol-4-yl)-2-(2-methansulfonylaminoäthansulfonylamino)-acetamido]-3-cephem-4-carbonsäurepivalat werden in 6 ml Dimethylformamid 30 Minuten bei 0° gerührt. Dann fügt man 10 ml Phosphatpuffer pH 8 zu und rührt nochmals 5 Minuten bei 0°. Anschliessend nimmt man in 25 ml Essigester auf, wäscht zweimal mit gesättigter wässriger Natriumchloridlösung und trocknet über Natriumsulfat. Dann filtriert man ab und versetzt mit 1,6 ml 0,7 N HCl in

Methylenchlorid. Der dabei entstehende amorphe Niederschlag wird durch Dekantieren abgetrennt, dreimal mit Hexan gewaschen und bei Zimmertemperatur im Vakuum getrocknet. Anschliessend wird mit Aether digeriert, vom Aether abfiltriert und wiederum getrocknet. Man erhält die Titelverbindung, die noch 0,5 g Dimethylformamid enthält); F: oberhalb 100° (unter Zersetzung); $[\alpha]_D^{20°} = +60° \pm 1°$ (0,810% in DMSO); UV: 255 (10300; $CH_3OH$).

Beispiel 100:

a) 3-Acetoxymethyl-7β-[(2R,S)-2-(2-aminothiazol-4-yl)-2-(2-methansulfonylaminoäthansulfonylamino)-acetamido]-3-cephem-4-carbonsäure-natriumsalz

Analog Beispiel 21 a) wird ausgehend von 7,03 g (8 mMol) gemäss Beispiel 100 b) hergestelltem 3-Acetoxymethyl-7β-[2R,S)-2-(2-BOC-aminothiazol-4-yl)-2-(2-methansulfonylaminoäthansulfonylamino)-acetamido]-3-cephem-4-carbonsäurediphenylmethylester, gelöst in 35 ml Methylenchlorid, in Gegenwart von 7 ml Anisol mit 35 ml Trifluoressigsäure die Titelverbindung in Form des 1,5-Hydrats erhalten. F: oberhalb 115° (unter Zersetzung); Rf: 0,28 (Silicagel, UV 366); $[\alpha]_D^{20°} = +48° \pm 1°$ (0,826% in Wasser); UV: 255 (11900, Wasser).

b) 3-Acetoxymethyl-7β-[(2R,S)-2-(2-BOC-aminothiazol-4-yl)-2-(2-methansulfonylaminoäthansulfonylamino)acetamido]-3-cephem-4-carbonsäurediphenylmethylester

Analog Beispiel 98 b) wird ausgehend von 9,17 g (20 mMol) gemäss Beispiel 98 c) hergestellte (2R,S)-2-(2-BOC-Aminothiazol-4-yl)-2-(2-methansulfonylaminoäthansulfonylamino)-essigsäure, gelöst in 200 ml absolutem Tetrahydrofuran, in Gegenwart von 2,7 g 1-Hydroxybenztriazol und 4,54 g Dicyclohexylcarbodiimid mit 8,77 g (20 mMol) 3-Acetoxymethyl-7β-amino-3-cephem-4-carbonsäurediphenylmethylester die Titelverbindung als amorphes Pulver erhalten. Rf: 0,58 (Silicagel, UV: 366; Essigester).

Beispiel 101:

3-Acetoxymethyl-7β-[(2R,S)-2-(2-aminothiazol-4-yl)-2-(2-methansulfonylaminoäthansulfonylamino)-acetamido]-3-cephem-4-carbonsäurepivaloyloxymethylesterhydrochlorid

1,9 g (3 mMol) 3-Acetoxymethyl-7β-[(2R,S)-2-(2-aminothiazol-4-yl)-2-(2-methansulfonylaminoäthansulfonylamino)-acetamido]-3-cephem-4-carbonsäurenatriumsalz und 0,78 ml Jodmethylpivalat werden in 19 ml Dimethylformamid analog Beispiel 99 umgesetzt und aufgearbeitet, woraus die Titelverbindung erhalten wird, die 0,5 Aequivalente Dimethylformamid enthält. F: oberhalb 95° (unter Zersetzung); $[\alpha]_D^{20°} = +24 \pm 1°$ (1,07% in DMSO); UV: 260 (12100 in Methanol).

Beispiel 102:

a) 3-(1-Methyl-1H-tetrazol-5-ylthiomethyl)-7β-[(2R,S)-2-(2-aminothiazol-4-yl)-2-(2-methansulfonylaminoäthansulfonylamino)-acetamido]-3-cephem-4-carbonsäurenatriumsalz

Analog Beispiel 21 a) wird ausgehend von 7,15 g (7,5 mMol) des gemäss Beispiel 102 b) hergestellten 3-(1-Methyl-1H-tetrazol-5-ylthiomethyl)-7β-[(2R,S)-2-(2-BOC-aminothiazol-4-yl)-2-(2-methansulfonylaminoäthansulfonylamino)-acetamido]-3-cephem-4-carbonsäurediphenylmethylester, gelöst in 37 ml Methylenchlorid, in Gegenwart von 7,2 ml Anisol mit 37 ml Trifluoressigsäure der Titelverbindung in Form des Dihydrates erhalten. F: oberhalb 123° (unter Zersetzung); Rf: 0,25 (Silicagel, UV 266); $[\alpha]_D^{20°} = -4° \pm 1°$ (0,802% in Wasser); UV: 260 (13450; Wasser).

b) 3-(1-Methyl-1H-tetrazol-5-ylthiomethyl)-7β-[(2R,S)-2-(2-BOC-aminothiazol-4-yl)-2-(2-methansulfonylaminoäthansulfonylamino)-acetamido]-3-cephem-4-carbonsäurediphenylmethylester

Analog Beispiel 98 b) wird ausgehend von 9,17 g (20 mMol) der gemäss Beispiel 98 c) hergestellten (2R,S)-2-(2-BOC-aminothiazol-4-yl)-2-(2-methansulfonylamincäthansulfonylamino)-essigsäure, gelöst in 200 ml absolutem Tetrahydrofuran, in Gegenwart von 2,7 g 1-Hydroxybenztriazol

und 4,54 g Dicyclohexylcarbodiimid mit 19,1 g (20 mMol) 3-(1-Methyl-1H-tetrazol-5-ylthiomethyl)-7β-amino-3-cephem-4-carbonsäurediphenylmethylester die Titelverbindung als amorphes Pulver erhalten. Rf: 0,50 (Silicagel; UV 366, Essigester).

Beispiel 103:

a) 3-Carbamoyloxymethyl-7β-[(2R,S)-2-(2-aminothiazol-4-yl)-2-(2-methansulfonylaminoäthansulfonylamino)-acetamido]-3-cephem-4-carbonsäurenatriumsalz

Analog Beispiel 21 a) wird ausgehend von 1,05 g (1,2 mMol) des gemäss Beispiel 103 d) hergestellten 3-Carbamoyloxymethyl-7β-[(2R,S)-2-(2-BOC-aminothiazol-4-yl)-2-(2-methansulfonylaminoäthansulfonylamino)-acetamido]-3-cephem-4-carbonsäurediphenylmethylesters, gelöst in 6 ml Methylenchlorid, in Gegenwart von 1 ml Anisol mit 6 ml Trifluoressigsäure die Titelverbindung in Form des Dihydrates erhalten. F: oberhalb 108° (unter Zersetzung); Rf:96: 0,22 (Silicagel, UV 366); $[\alpha]_D^{20°}$ = +44°±1° (1,105% in Wasser); UV: 255 (12600, Wasser).

b) 3-Carbamoyloxymethyl-7β-[(2R)-2-(2-aminothiazol-4-yl)-2-(2-methansulfonylaminoäthansulfonylamino)-acetamido]-3-cephem-4-carbonsäurenatriumsalz

Analog Beispiel 21 a) wird ausgehend von 2,51 g (2,85 mMol) des gemäss Beispiel 103 d) hergestellten 3-Carbamoyloxymethyl-7β-[(2R)-2-(2-BOC-aminothiazol-4-yl)-2-(2-methansulfonylaminoäthansulfonylamino)-acetamido]-3-cephem-4-carbonsäurediphenylmethylesters,gelöst in 14 ml Methylenchlorid,in Gegenwart von 2,5 ml Anisol mit 14 ml Trifluoressigsäure die Titelverbindung in Form des 1,5-Hydrates erhalten. F: oberhalb 105° (unter Zersetzung); Rf 96: 0,22 (Silicagel, UV 366); $[\alpha]_D^{20°}$ = +35°±1° (0,884% in Wasser); UV: 255 (2000, Wasser).

c) 3-Carbamoyloxymethyl-7β-[(2R,S)-2-(aminothiazol-4-yl)-2-(2-methansulfonylaminoäthansulfonylamino)—acetamido]-4-carbonsäurenatriumsalz

Analog Beispiel 21 a) wird ausgehend von 3,96 g (4,5 mMol) des gemäss Beispiel 103 d) hergestellten 3-Carbamoyloxymethyl-7β-[(2S)-2-(2-BOC-aminothiazol-4-yl)-2-(2-methansulfonylaminoäthansulfonylamino)-acetamido]-3-cephem-4-carbonsäurediphenylmethylesters gelöst in 22,5 ml Methylenchlorid, in Gegenwart von 4 ml Anisol mit 22,5 ml Trifluoressigsäure die Titelverbindung in Form des Dihydrates erhalten. F: oberhalb 122° (unter Zersetzung); Rf 96: 0,22 (Silicagel, UV 366); $[\alpha]_D^{20°} = +59°\pm1°$ (0,958% in Wasser); UV: 255 (13400, Wasser).

d) 3-Carbamoyloxymethyl-7β-[(2R,S)-2-(2-BOC-aminothiazol-4-yl)-2-(2-methansulfonylaminoäthansulfonylamino)-acetamido]-3-cephem-4-carbonsäurediphenylmethylester,

3-Carbamoyloxymethyl-7β-[(2R)-2-(2-BOC-aminothiazol-4-yl)-2-(2-methansulfonylaminoäthansulfonylamino)-acetamido]-3-cephem-4-carbonsäurediphenylmethylester

und

3-Carbamoyloxymethyl-7β-[(2S)-2-(2-BOC-aminothiazol-4-yl)-2-(2-methansulfonylaminoäthansulfonylamino)-acetamido]-3-cephem-4-carbonsäurediphenylmethylester

Analog Beispiel 98 b) wird ausgehend von 9,17 g (20 mMol) der gemäss Beispiel 98 c) hergestellten (2R,S)-2-(2-BOC-Aminothiazol-4-yl)-2-(2-methansulfonylamino)-äthansulfonylaminoessigsäure, gelöst in 200 ml absolutem Tetrahydrofuran, in Gegenwart von 2,7 g 1-Hydroxybenztriazol und 4,53 g Dicyclohexylcarbodiimid mit 8,8 g (20 mMol) 3-Carbamoyloxymethyl-7β-amino-3-cephem-4-carbonsäurediphenylmethylester die Titelverbindung als 2R,S-Diastereomerengemisch erhalten. Das Rohprodukt wird an Silicagel (1000 g) mit Methylenchlorid-Essigester 1:1 und Essigester als Eluiermittel chromatographiert, woraus die Titelverbindungen mit 2R-Konfiguration (Rf: 0,48, Silicagel, Essigester), 2R,S-Konfiguration und 2S-Konfiguration (Rf: 0,43 Silicagel, Essigester) als amorphe Pulver isoliert werden (zur Konfigutationszuordnung vgl. Beispiel 7 d).

Beispiel 104:

a) 3-Carbamoyloxymethyl-7β-[(2S)-2-(2-aminothiazol-4-yl)-2-(2-(4-nitrobenzolsulfonylamino)-äthansulfonylamino)-acetamido]-3-cephem-4-carbonsäurenatriumsalz

In Ergänzung zu Beispiel 67 und in Analogie zu Beispiel 29 a) erhält man durch Umsetzung von 0,85 g (0,86 mMol) 3-Carbamoyloxymethyl-7β-[(2S)-2-(2-BOC-aminothiazol-4-yl)-2-(2-(4-nitrobenzolsulfonylamino)-äthansulfonylamino)-acetamido]-3-cephem-4-carbonsäurediphenylmethylester in Gegenwart von 0,85 ml Anisol mit 5 ml Trifluoressigsäure in 5 ml Methylenchlorid die Titelverbindung als gelbliches Pulver in Form des Dihydrates. F: oberhalb 155° (unter Zersetzung); Rf: ca. 0,45 (Silicagel Opti-UPC 12, Wasser-Acetonitril 4:1); $[\alpha]_D^{20°}$ = +5°±1° (0,673% in Wasser); UV: 258 (23200, in Wasser).

b) 3-Carbamoyloxymethvl-7β-[(2R)-2-(2-aminothiazol-4-yl)-2-(2-(4-nitrobenzolsulfonylamino)-äthansulfonylamino)-acetamido]-3-cephem-4-carbonsäurenatriumsalz

In Ergänzung zu Beispiel 67 und in Analogie zu Beispiel 29a) erhält man durch Umsetzung von 0,8 g (0,81 mMol) 3-Carbamoyloxymethyl-7β-[(2R)-2-(2-BOC-aminothiazol-4-yl)-2-(2-(4-nitrobenzolsulfonylamino)-äthansulfonylamino)-acetamido]-3-cephem-4-carbonsäurediphenylmethylester in Gegenwart von 0,8 ml Anisol mit 4 ml Trifluoressigsäure in 4 ml Methylenchlorid die Titelverbindung als blassgelbliches Pulver in Form des Dihydrates. F: oberhalb 112° (unter Zersetzung); Rf: ca. 0,45 (Silicagel Opti-UPC 12, Wasser-Acetonitril 4:1); $[\alpha]_D^{20°}$ = +36°+1° (0,515% in Wasser); UV: 256 (21900, in Wasser).

c) 3-Carbamoyloxymethyl-7β-[(2R)-2-(2-BOC-aminothiazol-4-yl)-2-(2-(4-nitrobenzolsulfonylamino)-äthansulfonylamino)-acetamido]-3-cephem-4-carbonsäurediphenylmethylester

und

3-Carbamoyloxymethyl-7β-[(2S)-2-(2-BOC-aminothiazol-4-yl)-2-(2-(4-nitrobenzolsulfonylamino)-äthansulfonylamino)-acetamido]-3-cephem-4-carbonsäurediphenylmethylester

Analog Beispiel 29 b) erhält man durch Behandlung von 4,52 g (8,0 mMol) (2R,S)-2-(2-BOC-Aminothiazol-4-yl)-2-(2-(4-nitrobenzolsulfonylamino)-äthansulfonylamino)-essigsäure mit 3,51 g (8 mMol) 3-Carbamoyloxymethyl-7β-amino-3-cephem-4-carbonsäurediphenylmethylester in Gegenwart von 1,08 g 1-Hydroxybenztriazol und 1,81 g Dicyclohexylcarbodiimid in 80 ml Tetrahydrofuran das Rohprodukt als 2R,S-Diastereomerengemisch. Das Rohprodukt wird an Silicagel (300 g) mit Methylenchlorid-Essigester 7:3 als Eluiermittel chromatographiert, und aus den ersten Fraktionen die Titelverbindung mit 2R-Konfiguration (Rf: 0,38) und aus den anschliessenden Fraktionen die Titelverbindung mit 2S-Konfiguration (Rf: 0,33, Silicagel, Methylenchlorid/Essigester 1:1) eluiert.

Beispiel 105:

a) 3-Carbamoyloxymethyl-7β-[(2S)-2-(2-aminothiazol-4-yl)-2-(2-(4-aminobenzolsulfonylamino)-äthansulfonylamino)-acetamido]-3-cephem-4-carbonsäurenatriumsalz

Analog Beispiel 29 a) erhält man durch Umsetzung von 3,06 g (3,2 mMol) des gemäss Beispiel 105 c) erhaltenen 3-Carbamoyloxymethyl-7β-[(2S)-2-(2-BOC-Aminothiazol-4-yl)-2-(2-(4-aminobenzolsulfonylamino)-äthansulfonylamino)-acetamido]-3-cephem-4-carbonsäurediphenylmethylesters in Gegenwart von 15 ml Anisol mit 3 ml Trifluoressigsäure in 15 ml Methylenchlorid die Titelverbindung als gelbliches Pulver in Form des Dihydrats. F: oberhalb 150° (unter Zersetzung); $[\alpha]_D^{20°} = +52 \pm 1°$ (1,037% in Wasser); UV: 258 (28500, in Wasser).

b) 3-Carbamoyloxymethyl-7β-[(2R)-2-(2-Aminothiazol-4-yl)-2-(2-(4-aminobenzolsulfonylamino)-äthansulfonylamino)-acetamido]-3-cephem-4-carbonsäurenatriumsalz

Analog Beispiel 29 a) erhält man durch Umsetzung von 2,6 g (2,7 mMol) des gemäss Beispiel 105 d) erhaltenen 3-Carbamoyloxymethyl-7β-[(2R)-2-(2-BOC-aminothiazol-4-yl)-2-(2-(4-aminobenzolsulfonylamino)-äthan-

sulfonylamino)-acetamido]-3-cephem-4-carbonsäurediphenylmethylesters in Gegenwart von 2,6 ml Anisol mit 13 ml Trifluoressigsäure in 13 ml Methylenchlorid die Titelverbindung als gelbliches Pulver in Form des Dihydrats. F: oberhalb 150° (unter Zersetzung); $[\alpha]_D^{20°} = +38° \pm 1°$ (1,03% in Wasser); UV: 258 (28900, in Wasser).

c) 3-Carbamoyloxymethyl-7β-[(2S)-2-(2-BOC-aminothiazol-4-yl)-2-(2-(4-aminobenzolsulfonylamino)-äthansulfonylamino)-acetamido-3-cephem-4-carbonsäurediphenylmethylester

Eine Lösung von 3,5 g (3,55 mMol) des gemäss Beispiel 104 c) erhaltenen 3-Carbamoyloxymethyl-7β-[(2S)-2-(2-BOC-aminothiazol-4-yl)-2-(2-(4-nitrobenzolsulfonylamino)-äthansulfonylamino)-acetamido]-3-cephem-4-carbonsäurediphenylmethylesters in 70 ml Essigester wird in Gegenwart von 1,7 g 10%igem Palladium/Kohle-Katalysator bei Normaldruck und Raumtemperatur hydriert. Man filtriert ab und wäscht mit Essigester, engt das Filtrat ein und erhält die Titelverbindung als gelbes Pulver.
Rf: 0,44 (Silicagel, UV 266; Essigester).

d) 3-Carbamoyloxymethyl-7β-[(2R)-2-(2-BOC-aminothiazol-4-yl)-2-(2-(4-aminobenzolsulfonylamino)-äthansulfonylamino)-acetamido]-3-cephem-4-carbonsäurediphenylmethylester

Eine Lösung von 2,96 g (3 mMol) des gemäss Beispiel 104 c) erhaltenen 3-Carbamoyloxymethyl-7β-[(2R)-2-(2-BOC-aminothiazol-4-yl)-2-(2-(4-nitrobenzolsulfonylamino)-äthansulfonylamino)-acetamido]-3-cephem-4-carbonsäurediphenylmethylesters in 600 ml Essigester wird in Gegenwart von 1,5 g 10%igem Palladium/Kohle-Katalysator bei Normaldruck und Raumtemperatur hydriert. Nach Aufnahme von 3 Aequivalenten Wasserstoffgas wird der Katalysator abfiltriert und mit Essigester gewaschen. Das Filtrat wird eingeengt, woraus die Titelverbindung erhalten wird.
Rf: 0,46 (Silicagel, UV 366; Essigester).

Beispiel 106:

a) 3-Acetoxymethyl-7β-[(2R,S)-2-(2-aminothiazol-4-yl)-2-(2-(4-amino-benzolsulfonylamino)-äthansulfonylamino)-acetamido]-3-cephem-4-carbonsäurenatriumsalz

Analog Beispiel 29 a) erhält man durch Umsetzung von 3,75 g (2,9 mMol) 3-Acetoxymethyl-7β-[(2R,S)-2-(2-BOC-aminothiazol-4-yl)-2-(2-(4-amino-benzolsulfonylamino)-äthansulfonylamino)-acetamido]-3-cephem-4-carbonsäurediphenylmethylester in Gegenwart von 3,8 ml Anisol mit 19 ml Trifluoressigsäure in 19 ml Methylenchlorid die Titelverbindung als gelbliches Pulver in Form des 1,5 Hydrats. F: oberhalb 152° (unter Zersetzung); $[\alpha]_D^{20°} = +48° \pm 1°$ (0,869% in Wasser); UV: 258 (28500, in Wasser).

b) 3-Acetoxymethyl-7β-[(2R,S)-2-(2-BOC-aminothiazol-4-yl)-2-(2-(4-aminobenzolsulfonylamino)-äthansulfonylamino)-acetamido]-3-cephem-4-carbonsäurediphenylmethylester

Eine Lösung von 7,5 g (7,6 mMol) des gemäss Beispiel 39 b) erhaltenen 3-Acetoxymethyl-7β-[(2R,S)-2-(2-BOC-aminothiazol-4-yl)-2-(2-(4-nitro-benzolsulfonylamino)-äthansulfonylamino-acetamido]-3-cephem-4-carbonsäurediphenylmethylesters in 40 ml Essigsäureäthylester wird in Gegenwart von 3,5 g 10%igem Palladium-Kohle-Katalysator bei Normaldruck und Raumtemperatur hydriert. Nach Aufnahme von 3 Aequivalenten Wasserstoffgas wird der Katalysator abfiltriert und mit Essigester gewaschen. Das Lösungsmittel wird am Rotationsverdampfer entfernt, woraus die Titelverbindung erhalten wird. Rf: 0,58 (Silicagel, UV 366, Essigester.

Beispiel 107:

a) 3-(1-Methyl-1H-tetrazol-5-ylthiomethyl)-7β-[(2R,S)-2-(2-(2,4-dinitro-benzolsulfonylamino)-äthansulfonylamino)-2-(2-aminothiazol-4-yl)-acetamido]-3-cephem-4-carbonsäurenatriumsalz

Analog Beispiel 29 a) erhält man durch Umsetzung von 5,6 g (5,15 mMol) 3-(1-Methyl-1H-tetrazol-5-ylthiomethyl)-7β-[(2R,S)-2-(2-(2,4-dinitro-

benzolsulfonylamino)-äthansulfonylamino)-2-(2-BOC-aminothiazol-4-yl)-acetamidoy-3-cephem-4-carbonsäurediphenylmethylester in Gegenwart von 5,6 ml Anisol und 20 ml Trifluoressigsäure in 28 ml Methylenchlorid das 1,5 Hydrat der Titelverbindung als gelbliches Pulver.

b) 3-(1-Methyl-1H-tetrazol-5-ylthiomethyl-7β-[(2R,S)-2-(2-(2,4-dinitrobenzolsulfonylamino)-äthansulfonylamino)-2-(2-BOC-aminothiazol-4-yl)-acetamido]-3-cephem-4-carbonsäurediphenylmethylester

Analog Beispiel 29 b) erhält man durch Behandeln von 6,1 g (10 mMol) der gemäss Beispiel 40 c) erhaltenen (2R,S)-2-(2-(2,4-Dinitrobenzolsulfonylamino)-äthansulfonylamino)-2-(2-BOC-aminothiazol-4-yl)-essigsäure mit 4,94 g 3-(1-Methyl-1H-tetrazol-5-ylthiomethyl)-7β-amino-3-cephem-4-carbonsäurediphenylmethylester in Gegenwart von 1,35 g 1-Hydroxybenztriazol und 2,26 g Dicyclohexylcarbodiimid in 120 ml Tetrahydrofuran (Reaktionszeit: 16 Stunden bei Raumtemperatur) die Titelverbindung als blassgelbes, amorphes Pulver; Rf: 0,70 (Silicagel, UV 366; Essigester).

Beispiel 108:

a) 3-Carbamoyloxymethyl-7β-[(2R,S)-2-(2-(2,4-dinitrobenzolsulfonylamino)-äthansulfonylamino)-2-(2-aminothiazol-4-yl)-acetamido]-3-cephem-4-carbonsäurenatriumsalz

Analog Beispiel 29 a) erhält man durch Umsetzung von 7,0 g (6,78 mMol) 3-Carbamoyloxymethyl)-7β-[(2R,S)-2-(2-(2,4-dinitrobenzolsulfonylamino)-äthansulfonylamino)-2-(2-BOC-aminothiazol-4-yl)-acetamido]-3-cephem-4-carbonsäurediphenylmethylester in Gegenwart von 7,0 ml Anisol und 35 ml Trifluoressigsäure in 35 ml Methylenchlorid das Dihydrat der Titelverbindung als gelbliches Pulver;
$[\alpha]_D^{20°} = +40° \pm 1°$ (0,977% in Wasser); Rf: 0,10 (Silicagel Opti-UPC 12, Wasser); UV: 250 (23000 in Wasser).

b) 3-Carbamoyloxymethyl-7β-[(2R,S)-2-(2-(2,4-dinitrobenzolsulfonylamino)-äthansulfonylamino)-2-(2-BOC-aminothiazol-4-yl)-acetamido]-3-cephem-4-carbonsäurediphenylmethylester

Analog Beispiel 29 b) erhält man durch Behandeln von 9,17 g (15 mMol) der gemäss Beispiel 40 c) erhaltenen (2R,S)-2-(2-(2,4-Dinitrobenzolsulfonylamino)-äthansulfonylamino)-2-(2-BOC-aminothiazol-4-yl)-essigsäure mit 6,6 g Carbamoyloxymethyl-7β-amino-3-cephem-4-carbonsäurediphenylmethylester in Gegenwart von 2,03 g 1-Hydroxybenztriazol und 3,4 g Dicyclohexylcarbodiimid in 200 ml Tetrahydrofuran (Reaktionszeit 16 Stunden bei Raumtemperatur) die Titelverbindung als blassgelbes, amorphes Pulver; DC (Silicagel, Identifikation UV 266): Rf 0,25 (Methylenchlorid/Essigester 1:1).

Beispiel 109:

a) 7β-[(2R,S)-2-(2-Aminothiazol-4-yl)-2-(2,4-dinitrobenzolsulfonylamino)-acetamido]-3-cephem-4-carbonsäurenatriumsalz

Analog Beispiel 29 a) erhält man durch Umsetzung von 5,02 g (5,9 mMol) 7β-[2R,S)-2-(2-BOC-Aminothiazol-4-yl)-2-(2,4-dinitrobenzolsulfonylamino)-acetamido]-3-cephem-4-carbonsäurediphenylmethylester in Gegenwart von 5 ml Anisol mit 25 ml Trifluoressigsäure in 25 ml Methylenchlorid die Titelverbindung in Form des Dihydrats. F: oberhalb 150° (unter Zersetzung); $[\alpha]_D^{20°} = +24° \pm 1°$ (0,362% in Wasser); UV: 246 (19800, Wasser); Rf.: 0,33 (Silicagel OPTI-UPC 12, Wasser/Acetonitril 4:1).

b) 7β-[(2R,S)-2-(2-BOC-Aminothiazol-4-yl)-2-(2,4-dinitrobenzolsulfonyl)-aminoacetamido]-3-cephem-4-carbonsäurediphenylmethylester

Analog Beispiel 29 b) erhält man durch Behandeln von 10,06 g (20 mMol) (2R,S)-2-(2-BOC-Aminothiazol-4-yl)-2-(2,4-dinitrobenzolsulfonylamino)-essigsäure mit 7,33 g (20 mMol) 7β-Amino-3-cephem-4-carbonsäurediphenylmethylester in Gegenwart von 2,7 g 1-Hydroxybenztriazol und 4,54 g N,N'-Dicyclohexylcarbodiimid in 200 ml Tetrahydrofuran (Reaktionszeit

16 Stunden bei Raumtemperatur) die Titelverbindung. Rf: 0,70 (Methylenchlorid/Essigsäureäthylester 1:1).

Herstellung des Ausgangsmaterials:

c) (2R,S)-2-(2-BOC-Aminothiazol-4-yl)-2-(2,4-dinitrobenzolsulfonyl-aminoessigsäure

Eine Suspension von 40,95 g (0,15 Mol) (2R,S)-2-(2-BOC-Aminothiazol-4-yl)-2-aminoessigsäure in 500 ml absolutem Tetrahydrofuran wird unter Rühren, Feuchtigkeitsausschluss und Stickstoffatmosphäre mit 109 ml N,O-Bis(trimethylsilyl)-acetamid versetzt. Nach einer Reaktionszeit von einer Stunde bei 60° wird das Reaktionsgemisch auf 0° abgekühlt, mit 13 ml Pyridin und 48 g (0,18 Mol) 2,4-Dinitrobenzolsulfochlorid versetzt und anschliessend 16 Stunden bei Raumtemperatur gerührt. Nach Entfernung des Lösungsmittels wird der Rückstand in 1500 ml Essigester aufgenommen und je dreimal mit 1,0 N Salzsäure und gesättigter, wässriger Natriumchloridlösung gewaschen. Nach Trocknen über Natriumsulfat wird das Lösungsmittel am Rotationsverdampfer entfernt. Der Rückstand wird in Aether aufgeschlämmt und filtriert. woraus die Titelverbindung als amorphes Pulver erhalten wird. Rf: 0,35 (Silicagel, UV 366; Chloroform/Methanol/Essigsäure 75:22:13).

Beispiel 110:

a) 3-Carbamoyloxymethyl-7β-[(2S)-2-(2-aminothiazol-4-yl)-2-(2,4-dinitrobenzolsulfonylamino)-acetamido]-3-cephem-4-carbonsäure-natriumsalz

Analog Beispiel 29 a) erhält man durch Umsetzung von 2,78 g (3 mMol) 3-Carbamoyloxymethyl-7β-[(2S)-2-(2-BOC-aminothiazol-4-yl)-2-(2,4-dinitrobenzolsulfonylamino-acetamido]-3-cephem-4-carbonsäurediphenylmethylester in Gegenwart von 2,8 ml Anisol mit 14 ml Trifluoressigsäure in 14 ml Methylenchlorid die Titelverbindung in Form des Dihydrates. F: oberhalb 131° (unter Zersetzung); Fr 96: 0,38 (Silicagel, UV 366); $[\alpha]_D^{20°} = +88° \pm 1$ (0,497% in Wasser); UV: 250 (22300, Wasser).

b) 3-Carbamoyloxymethyl-7β-[(2R)-2-(2-aminothiazol-4-yl)-2-(2,4-dinitrobenzolsulfonylamino)-acetamido]-3-cephem-4-carbonsäure-natriumsalz

Analog Beispiel 29 a) erhält man durch Umsetzung von 3,51 g (2,8 mMol) 3-Carbamoyloxymethyl-7β-[(2R)-2-(2-BOC-aminothiazol-4-yl)-2-(2,4-dinitrobenzolsulfonylamino)-acetamido]-3-cephem-4-carbonsäurediphenylmethylester in Gegenwart von 3,5 ml Anisol mit 17,5 ml Trifluoressigsäure in 17,5 ml Methylenchlorid die Titelverbindung in Form des Dihydrats. F: oberhalb 130° (unter Zersetzung); Rf 96: ca. 0,30 (Silicagel, UV 266); $[\alpha]_D^{20°} = -36° \pm 1°$ (0,556% in Wasser); UV: 250 (21800, Wasser).

c) 3-Carbamoyloxymethyl-7β-[(2R)-2-(2-BOC-aminothiazol-4-yl)-2-(2-(2,4-dinitrobenzolsulfonylamino)-äthansulfonylamino)-acetamido]-3-cephem-4-carbonsäurediphenylmethylester
und
3-Carbamoyloxymethyl-7β-[(2S)-2-(2-BOC-aminothiazol-4-yl)-2-(2-(2,4-dinitrobenzolsulfonylamino)-äthansulfonylamino)-acetamido]-3-cephem-4-carbonsäurediphenylmethylester

Analog Beispiel 29 b) erhält man durch Behandeln von 10,06 g (20 mMol) die gemäss Beispiel 109 c) erhaltene (2R,S)-2-(2-BOC-Aminothiazol-4-yl)-2-(2-(2,4-dinitrobenzolsulfonylamino)-äthansulfonylamino)-essigsäure mit 8,79 g (20 mMol) 3-Carbamoyloxymethyl-7β-amino-3-cephem-4-carbonsäurediphenylmethylester in Gegenwart von 2,7 g 1-Hydroxybenztriazol und 4,54 g Dicyclohexylcarbodiimid in 80 ml Tetrahydrofuran (Reaktionszeit 16 Stunden bei Raumtemperatur) das Rohprodukt als 2R,S-Diastereomerengemisch. Das Rohprodukt wird an Silicagel (1000 g) mit Toluol/Essigester 4:1 als Eluiermittel chromatographiert und in den ersten Fraktionen die Titelverbindung mit der 2R-Konfiguration (Rf: 0,63) und den letzten Fraktionen die Titelverbindung mit der 2S-Konfiguration (Rf: 0,15, Silicagel, Methylenchlori/Essigester 1:1) eluiert.

Beispiel 111:

a) 7β-[(2R,S)-2-(2-Aminothiazol-4-yl)-2-(2-difluormethansulfonylaminoäthansulfonylamino)-acetamido]-3-cephem-4-carbonsäurenatriumsalz

Analog Beispiel 21 a) wird ausgehend von 1,85 g (2,2 mMol) 7β-[(2R,S)-2-(2-BOC-Aminothiazol-4-yl)-2-(2-difluormethansulfonylaminoäthansulfonylamino)-acetamido]-3-cephem-4-carbonsäurediphenylmethylester, gelöst in 10 ml Methylenchlorid, in Gegenwart von 1,85 ml Anisol mit 10 ml Trifluoressigsäure die Titelverbindung in Form des 1,5- Hydrats erhalten. F: oberhalb 170° (unter Zersetzung); Rf 96: 0,38 (Silicagel, UV 366); $[\alpha]_D^{20°}$ = +98°±1° (0,890% in Wasser); UV 250 (10600, Wasser).

b) 7β-[(2R,S)-2-(2-BOC-Aminothiazol-4-yl)-2-(2-difluormethansulfonylaminoäthansulfonylamino)-acetamido]-3-cephem-4-carbonsäurediphenylmethylester

Analog Beispiel 98 b) wird ausgehend von 2,47 g (5 mMol) (2R,S)-2-(2-BOC-Aminothiazol-4-yl)-2-(2-difluormethansulfonylaminoäthansulfonylamino)-essigsäure, gelöst in 60 ml absolutem Tetrahydrofuran, in Gegenwart von 0,68 g 1-Hydroxybenztriazol und 1,13 g Dicyclohexylcarbodiimid mit 1,83 g (5 mMol) 7β-Amino-3-cephem-4-carbonsäurediphenylmethylester die Titelverbindung als amorphes Pulver erhalten. Rf: 0,73 (Silicagel, UV 366; Essigester).

c) (2R,S)-2-(2-BOC-Aminothiazol-4-yl)-2-(2-difluormethansulfonylaminoäthansulfonylamino)-essigsäure

Analog Beispiel 98 c) wird nach Veresterung von 11,41 g (30 mMol) (2R,S)-2-(2-Aminoäthansulfonylamino)-2-(2-BOC-aminothiazol-4-yl)-essigsäure mit 30 ml N,O-Bis-(trimethylslyl)-acetamid in 150 ml Tetrahydrofuran nach Umsetzung mit 5,87 g Difluormethansulfochlorid in Gegenwart von 3,1 ml Pyridin die Titelverbindung erhalten, die noch an Silicagel (300 g) mit Methylenchlorid-Essigester als Eluiermittel gereinigt werden kann. Rf 96: 0,71 (Silicagel, UV 366).

Beispiel 112:

a) 3-(1-Methyl-1H-tetrazol-5-ylthiomethyl)-7β-[(2R,S)-2-(2-aminothiazol-4-yl)-2-(2-difluormethansulfonylaminoäthansulfonylamino)-acetamido]-3-cephem-4-carbonsäurenatriumsalz

Analog Beispiel 21 a) wird ausgehend von 2,6 g (2,68 mMol) 3-(1-Methyl-1H-5-ylthiomethyl)-7β-[(2R,S)-2-(2-BOC-aminothiazol-4-yl)-2-(2-difluormethansulfonylaminoäthansulfonylamino)-acetamido]-3-cephem-4-carbonsäurediphenylmethylester,gelöst in 13 ml Methylenchlorid,in Gegenwart von 2,6 ml Anisol und 13 ml Trifluoressigsäure die Titelverbindung in Form des Dihydrats erhalten. F: oberhalb 150° (unter Zersetzung); Rf 96: 0,38 (Silicagel, UV 366); $[\alpha]_D^{20°} = -4° \pm 1°$ (0,984% in Wasser); UV 258 (14400, Wasser).

b) 3-(1-Methyl-1H-tetrazol-5-ylthiomethyl)-7β-[(2R,S)-2-(2-BOC-aminothiazol-4-yl)-2-(2-difluormethansulfonylaminoäthansulfonylamino)-acetamido]-3-cephem-4-carbonsäurediphenylmethylester

Analog Beispiel 98 b) wird ausgehend von 2,47 g (5 mMol) (2R,S)-2-(2-BOC-Aminothiazol-4-yl)-2-(2-difluormethansulfonylaminoäthansulfonylamino)-essigsäure, gelöst in 60 ml absolutem Tetrahydrofuran, in Gegenwart von 0,68 g 1-Hydroxybenztriazol und 1,13 g Dicyclohexylcarbodiimid mit 2,47 g (5 mMol) 3-(1-Methyl-1H-tetrazol-5-ylthiomethyl)-7β-amino-3-cephem-4-carbonsäurediphenylmethylester die Titelverbindung als amorphes Pulver erhalten. Rf: 0,65 (Silicagel, UV 366, Essigester).

Beispiel 113:

a) 3-Carbamoyloxymethyl-7β-[(2R,S)-2-(2-aminothiazol-4-yl)-2-(2-difluormethansulfonylaminoäthansulfonylamino)-acetamido]-3-cephem-4-carbonsäurenatriumsalz

Analog Beispiel 21 a) wird ausgehend von 1,5 g (1,64 mMol) 3-Carbamoyloxymethyl)-7β-[(2R,S)-2-(2-BOC-aminothiazol-4-yl)-2-(2-difluormethansulfonylaminoäthansulfonylamino)-acetamido]-3-cephem-4-carbonsäurediphenylmethylester, gelöst in 7,5 ml Methylenchlorid, in Gegenwart

von 1,5 ml Anisol mit 7,5 ml Trifluoressigsäure die Titelverbindung in Form des 1,8 Hydrats erhalten. F: oberhalb 145° (unter Zersetzung); Rf 96: 0,34 (Silicagel, UV 366); $[\alpha]_D^{20°}$ = +50°±1° (0,921% in Wasser); UV 256 (13000, Wasser).

b) 3-Carbamoyloxymethyl)-7β-[(2R,S)-2-(2-BOC-aminothiazol-4-yl)-2-(2-difluormethansulfonylaminoäthansulfonylamino)-acetamido]-3-cephem-4-carbonsäurediphenylmethylester

Analog Beispiel 98 b) wird ausgehend von 1,7 g (3,44 mMol) (2R,S)-2-(2-BOC-Aminothiazol-4-yl)-2-(2-difluormethansulfonylaminoäthansulfonylamino)-essigsäure, gelöst in 40 ml absolutem Tetrahydrofuran, in Gegenwart von 0,47 g 1-Hydroxybenztriazol und 0,78 g Dicyclohexylcarbodiimid mit 1,51 g (3,44 mMol) 3-Carbamoyloxymethyl-7β-amino-3-cephem-4-carbonsäurediphenylmethylester die Titelverbindung als amorphes Pulver erhalten. Rf: 0,51 (Silicagel, UV 366, Essigester).

Beispiel 114:

a) 7β-[(2R,S)-2-(2-Aminothiazol-4-yl)-2-difluormethansulfonylaminoacetamido]-3-cephem-4-carbonsäurenatriumsalz

Analog Beispiel 21 a) wird ausgehend von 3,5 g (4,76 mMol) 7β-[(2R,S)-2-(2-BOC-Aminothiazol-4-yl)-2-difluormethansulfonylaminoacetamido]-3-cephem-4-carbonsäurediphenylmethylester, gelöst in 17,5 ml Methylenchlorid, in Gegenwart von 3,5 ml Anisol mit 17,5 ml Trifluoressigsäure die Titelverbindung in Form des 2,5 Hydrats erhalten. F: oberhalb 161° (unter Zersetzung); Rf 96: 0,30 (Silicagel, UV 366); $[\alpha]_D^{20°}$ = +106°±1° (0,746% in Wasser); UV 252 (10500, Wasser).

b) 7β-[(2R,S)-2-(2-BOC-Aminothiazol-4-yl)-2-difluormethansulfonylaminoacetamido]-3-cephem-4-carbonsäurediphenylmethylester

Analog Beispiel 98 b) wird ausgehend von 1,94 g (5 mMol) (2R,S)-2-(2-BOC-Aminothiazol-4-yl)-2-difluormethansulfonylaminoessigsäure, gelöst in 40 ml absolutem Tetrahydrofuran, in Gegenwart von 0,68 g 1-Hydroxybenztriazol und 1,13 g Dicyclohexylcarbodiimid mit 1,89 g

7β-Amino-3-cephem-4-carbonsäurediphenylmethylester die Titelverbindung als amorphes Pulver erhalten. Rf: 0,58 (Silicagel, UV 366, Essigester).

c) (2R,S)-2-(2-BOC-Aminothiazol-4-yl)-2-difluormethansulfonylamino-essigsäure

Analog Beispiel 98 c) wird nach Veresterung von 13,65 g (50 mMol) (2R,S)-2-Amino-2-(2-BOC-aminothiazol-4-yl)-essigsäure mit 40 ml N,O-Bis(trimethyl)-acetamid in 200 ml Tetrahydrofuran und nach Umsetzung mit 9,03 g Difluormethansulfochlorid in Gegenwart von 4,8 ml Pyridin die Titelverbindung erhalten, die noch an Silicagel (420 g) mit Methylenchlorid/Essigester als Eluiermittel gereinigt werden kann. Rf 96: 0,61 (Silicagel, UV 366).

Beispiel 115:

a) 3-Carbamoyloxymethyl-7β-[(2R,S)-2-(2-aminothiazol-4-yl)-2-difluormethansulfonylaminoacetamido]-3-cephem-4-carbonsäurenatriumsalz

Analog Beispiel 21 a) wird ausgehend von 6,47 g (8,0 mMol) 3-Carbamoyloxymethyl-7β-[(2R,S)-2-(2-BOC-aminothiazol-4-yl)-2-difluormethansulfonylaminoacetamido]-3-cephem-4-carbonsäurediphenylmethylester, gelöst in 32,5 ml Methylenchlorid, in Gegenwart von 6,5 ml Anisol mit 32,5 ml Trifluoressigsäure die Titelverbindung in Form des Dihydrats erhalten. F: oberhalb 142° (unter Zersetzung); Rf 96: 0,28 (Silicagel, UV 366); $[\alpha]_D^{20°} = +57° \pm 1°$ (1,152% in Wasser); UV 257 (13000, Wasser).

b) 3-Carbamoyloxymethyl-7β-[(2R,S)-2-(2-BOC-aminothiazol-4-yl)-2-difluormethansulfonylaminoacetamido]-3-cephem-4-carbonsäurediphenylmethylester

Analog Beispiel 98 b) wird ausgehend von 4,65 g (12 mMol) der gemäss Beispiel 114 c) erhaltenen (2R,S)-2-(2-BOC-Aminothiazol-4-yl)-2-difluormethansulfonylaminoessigsäure, gelöst in 95 ml absolutem Tetrahydrofuran , in Gegenwart von 1,62 g 1-Hydroxybenztriazol und

2,72 g Dicyclohexylcarbodiimid mit 5,3 g 3-Carbamoyloxymethyl-7β-amino-3-cephem-4-carbonsäurediphenylmethylester die Titelverbindung als amorphes Pulver erhalten. Rf: 0,58 (Silicagel, UV 366; Essigester).

Beispiel 116:

a) 7β-[(2R,S)-2-(2-Aminothiazolyl-4-yl)-2-(2-pyrid-3-ylsulfonyl-aminoäthansulfonylamino)-acetamido]-3-cephem-4-carbonsäure-natriumsalz

Analog Beispiel 21 a) wird ausgehend von 4,0 g (4,5 mMol) 7β-[(2R,S)-2-(2-BOC-Aminothiazol-4-yl)-2-(2-pyrid-3-ylsulfonylaminoäthan-sulfonylamino)-acetamido]-3-cephem-4-carbonsäurediphenylmethylester, gelöst in 20 ml Methylenchlorid in Gegenwart von 3,3 ml Anisol mit 20 ml Trifluoressigsäure die Titelverbindung in der Form des 1,5-Hydrats erhalten. F: oberhalb 164° (unter Zersetzung); Rf 96: 0,30 (Silicagel, UV 366); $[\alpha]_D^{20°} = +80° \pm 1°$ (0,779% in Wasser); UV 254 (12300, Wasser).

b) 7β-[(2R,S)-2-(2-BOC-Aminothiazol-4-yl)-2-(2-pyrid-3-ylsulfonyl-aminoäthansulfonylamino)-acetamido]-3-cephem-4-carbonsäure-diphenylmethylester

Analog Beispiel 98 b) wird ausgehend von 4,7 g (9 mMol) (2R,S)-2-(2-BOC-Aminothiazol-4-yl)-2-(2-pyrid-3-ylsulfonylaminoäthansulfonyl-amino -essigsäure,gelöst in 90 ml absolutem Tetrahydrofuran, in Gegenwart von 1,22 g 1-Hydroxybenztriazol und 2,10 g Dicyclohexylcarbodiimid mit 3,5 g (9 mMol) 7β-Amino-3-cephem-4-carbonsäurediphenylmethylester (Reaktionszeit 5 Stunden) die Titelverbindung als amorphes Pulver erhalten. Rf: 0,45 (Silcagel, UV 366; Methylenchlorid/Essigester 1:1).

c) (2R,S)-2-(2-BOC-Aminothiazol-4-yl)-2-(2- pyrid-3-ylsulfonylamino-äthansulfonylamino)-essigsäure

Analog Beispiel 98 c) wird nach Veresterung von 7,61 g (20 mMol) (2R,S)-2-(2-Aminoäthansulfonylamino)-2-(2-BOC-aminothiazol-4-yl)-essigsäure mit 16 ml N,O-Bis-(trimethylsilyl)-acetamid in 60 ml Tetrahydrofuran und nach Umsetzung mit 5,14 g 3-Pyridinsulfochloridhydrochlorid in Gegenwart von 4,9 ml N-Methylmorpholin die Titelverbindung erhalten. Rf 96: 0,40 (Silicagel, UV 366).

Beispiel 117:

a) 7β-[(2R,S)-2-(2-Aminothiazol-4-yl)-2-pyrid-3-ylsulfonylamino-acetamido]-3-cephem-4-carbonsäurenatriumsalz

Analog Beispiel 21 a) wird ausgehend von 3,43 g (4,5 mMol) 7β-[(2R,S)-2-(2-BOC-Aminothiazol-4-yl)-2-pyrid-3-ylsulfonylaminoacetamido]-3-cephem-4-carbonsäurediphenylmethylester, gelöst in 20 ml Methylenchlorid, in Gegenwart von 3,3 ml Anisol und 20 ml Trifluoressigsäure die Titelverbindung in Form des Dihydrats erhalten. F: oberhalb 179° (unter Zersetzung); Rf 96: 0,35 (Silicagel, UV 366); $[\alpha]_D^{20°} = +94° \pm 1°$ (0,929% in Wasser); UV 254 (11800, Wasser).

b) 7β-[(2R,S)-2-(2-BOC-Aminothiazol-4-yl)-2-pyrid-3-ylsulfonylamino-acetamido]-3-cephem-4-carbonsäurediphenylmethylester

Analog Beispiel 98 b) wird ausgehend von 2,9 g (7 mMol) (2R,S)-2-(2-BOC-Aminothiazol-4-yl)-2-pyrid-3-ylsulfonylaminoessigsäure, gelöst in 70 ml absolutem Tetrahydrofuran, in Gegenwart von 0,95 g 1-Hydroxybenztriazol und 1,6 g Dicyclohexylcarbodiimid mit 2,6 g 7β-Amino-3-cephem-4-carbonsäurediphenylmethylester die Titelverbindung als amorphes Pulver erhalten. Rf: 0,38 (Silicagel, UV 366; Methylenchlorid/Essigester 1:1).

c) (2R,S)-2-(2-BOC-Aminothiazol-4-yl)-2-pyrid-3-ylsulfonylamino-essigsäure

Analog Beispiel 98 c) wird nach Veresterung von 2,73 g (10 mMol) (2R,S)-2-Amino-2-(2-BOC-aminothiazol-4-yl)-essigsäure mit 30 ml N,O-Bis(trimethylsilyl)-acetamid in 30 ml Tetrahydrofuran und nach Umsatz mit 2,57 g 3-Pyridinsulfochloridhydrochlorid in Gegenwart von 1,6 ml N-Methylmorpholin die Titelverbindung erhalten. Rf 96: 0,49 (Silicagel, UV 266).

Beispiel 118:

a) 3-Acetoxymethyl-7β-[(2R,S)-2-(2-(2-aminothiazol-4-ylacetamido)-äthansulfonylamino)-2-(2-aminothiazol-4-yl)-acetamido]-3-cephem-4-carbonsäurenatriumsalz

Analog Beispiel 29 a) wird ausgehend von 1,04 g (1mMol) 3-Acetoxymethyl-7β-[(2R,S)-2-(2-(2-BOC-aminothiazol-4-ylacetamido)-äthansulfonylamino)-2-(2-BOC-aminothiazol-4-yl)-acetamido]-3-cephem-4-carbonsäurediphenylmethylester, gelöst in 5 ml Methylenchlorid, in Gegenwart von 1 ml Anisol mit 5 ml Trifluoressigsäure die Titelverbindung in Form des Dihydrats erhalten. F: oberhalb 150° (unter Zersetzung); Rf: 0,25 (Silicagel Opti UPC 12, UV 366, Wasser/Acetonitril 4:1); $[\alpha]_D^{20°}$ = +57°±1° (1,183% in Wasser); UV 254 (18500, Wasser).

b) 3-Acetoxymethyl-7β-[(2R)-2-(2-(2-Aminothiazol-4-ylacetamido)- äthansulfonylamino)-2-(2-aminothiazol-4-yl)-acetamido]-3-cephem-4-carbonsäurenatriumsalz

Analog Beispiel 29 a) wird ausgehend von 1,60 g (1,54 mMol) 3-Acetoxymethyl-7β-[(2R)-2-(2-(2-BOC-aminothiazol-4-ylacetamido)-äthansulfonylamino)-2-(2-BOC-aminothiazol-4-yl)-acetamido]-3-cephem-4-carbonsäurediphenylmethylester, gelöst in 8 ml Methylenchlorid, in Gegenwart von 1,6 ml Anisol mit 8 ml Trifluoressigsäure die Titelverbindung in Form des Dihydrats erhalten. F: oberhalb 151° (unter Zersetzung); Rf: 0,25 (Silicagel Opti UPC 12, UV 366, Wasser/Acetonitril 4:1); $[\alpha]_D^{20°}$ = +39° ±1° (1,111% in Wasser); UV 254 (17700, Wasser).

c) 3-Acetoxymethyl-7β-[(2S)-2-(2-(2-Aminothiazol-4-ylacetamido]-äthansulfonylamino)-2-(2-aminothiazol-4-yl)-acetamido]-3-cephem-4-carbonsäurenatriumsalz

Analog Beispiel 29 a) wird ausgehend von 1,90 g (1,83 mMol) 3-Acetoxymethyl-7β-[(2S)-2-(2-(2-BOC-aminothiazol-4-ylacetamido)-äthansulfonylamino)-2-(2-BOC-aminothiazol-4-yl)-acetamido]-3-cephem-4-carbonsäurediphenylmethylester, gelöst in 9,5 ml Methylenchlorid, in Gegenwart

von 1,9 ml Anisol mit 9,5 ml Trifluoressigsäure die Titelverbindung in Form des Dihydrats erhalten. F: oberhalb 150° (unter Zersetzung); Rf: 0,25 (Silicagel Opti UPC 12, UV 366, Wasser/Acetonitril 4:1); $[\alpha]_D^{20°}$ +61°±1° (1,473% in Wasser); UV 254 (18800, Wasser).

d) 3-Acetoxymethyl-7β-[(2R,S)-2-(2-(2-BOC-aminothiazol-4-ylacetamido)-äthansulfonylamino)-2-(2-BOC-aminothiazol-4-yl)-acetamido]-3-cephem-4-carbonsäurediphenylmethylester,

3-Acetoxymethyl-7β-[(2R)-2-(2-(2-BOC-aminothiazol-4-ylacetamido)-äthansulfonylamino)-2-(2-BOC-aminothiazol-4-yl)-acetamido]-3-cephem-4-carbonsäurediphenylmethylester und

3-Acetoxymethyl-7β-[(2S)-2-(2-(2-BOC-aminothiazol-4-ylacetamido)-äthansulfonylamino)-2-(2-BOC-aminothiazol-4-yl)-acetamido]-3-cephem-4-carbonsäurediphenylmethylester

Analog Beispiel 29 b) wird ausgehend von 3,5 g (5,7 mMol) der gemäss Beispiel 29 c) erhaltenen (2R,S)-2-(2-(2-BOC-Aminothiazol-4-ylacetamido)-äthansulfonylamino)-2-(2-BOC-aminothiazol-4-yl)-essigsäure, gelöst in 60 ml Tetrahydrofuran, in Gegenwart von 0,77 g 1-Hydroxybenztriazol und 1,29 g Dicyclohexylcarbodiimid mit 2,5 g (5,7 mMol) 3-Acetoxymethyl-7β-amino-3-cephem-4-carbonsäurediphenylmethylester (Reaktionszeit 16 Stunden bei Raumtemperatur) die Titelverbindung als 2R,S-Diastereomerengemisch erhalten. Das Rohprodukt wird an Silicagel (280 g) mit Methylenchlorid/Essigester 1:1 als Eluiermittel chromatographiert, und in den ersten Fraktionen die Titelverbindung mit 2R-Konfiguration (Rf: 0,58, Silicagel, Essigester), in den Folgefraktionen die Titelverbindung mit 2R,S-Konfiguration und in den letzten Fraktionen die Titelverbindung mit 2S-Konfiguration (Rf: 0,48, Silicagel, Essigester) eluiert.

Beispiel 119:

a) 3-Carbamoyloxymethyl-7β-[(2R)-2-(2-(2-aminothiazol-4-ylacetamido)-äthansulfonylamino)-2-(2-aminothiazol-4-yl)-acetamido]-3-cephem-4-carbonsäurenatriumsalz

Analog Beispiel 29 a) wird ausgehend von 4,17 g (4 mMol) 3-Carbamoyl-oxymethyl-7β-[(2R)-2-(2-(2-BOC-aminothiazol-4-ylacetamido)-äthansulfonylamino)-2-(2-BOC-aminothiazol-4-yl)-acetamido]-3-cephem-4-carbonsäurediphenylmethylester, gelöst in 20 ml Methylenchlorid, in Gegenwart von 4 ml Anisol mit 20 ml Trifluoressigsäure die Titelverbindung in Form des Dihydrats erhalten. F: oberhalb 155° (unter Zersetzung); Rf: 0,20 (Silicagel Opti UPC 12, UV 366, Wasser/Acetonitril 4:1), $[\alpha]_D^{20°} = +34° \pm 1°$ (1,341% in Wasser); UV 256 (18500, Wasser).

b) 3-Carbamoyloxymethyl-7β-[(2S)-2-(2-(2-aminothiazol-4-ylacetamido)-äthansulfonylamino)-2-(2-aminothiazol-4-yl)acetamido]-3-cephem-4-carbonsäurenatriumsalz

Analog Beispiel 29 a) wird ausgehend von 4,17 g (4 mMol) 3-Carbamoyl-oxymethyl-7β-[(2S)-2-(2-(2-BOC-aminothiazol-4-ylacetamido)-äthansulfonylamino)-2-(2-BOC-aminothiazol-4-yl)-acetamido]-3-cephem-4-carbonsäurediphenylmethylester, gelöst in 20 ml Methylenchlorid, in Gegenwart von 4 ml Anisol mit 20 ml Trifluoressigsäure die Titelverbindung in Form des 2,5-Hydrats erhalten. F: oberhalb 160° (unter Zersetzung); Rf: 0,18 (Silicagel Opti UPC 12, UV 366, Wasser/Acetonitril 4:1); $[\alpha]_D^{20°} = +57° \pm 1°$ (0,884% in Wasser); UV 257 (18500, Wasser).

c) 3-Carbamoyloxymethyl-7β-[(2R)-2-(2-(2-BOC-aminothiazol-4-ylacetamido)-äthansulfonylamino)-2-(2-BOC-aminothiazol-4-yl)-acetamido]-3-cephem-4-carbonsäurediphenylmethylester

und

3-Carbamoyloxymethyl-7β-[(2S)-2-(2-(2-BOC-aminothiazol-4-ylacetamido)-äthansulfonylamino)-2-(2-BOC-aminothiazol-4-yl)-acetamido]-3-cephem-4-carbonsäurediphenylmethylester

Analog Beispiel 29 b) wird ausgehend von 7,44 g (12 mMol) der gemäss Beispiel 29 c) erhaltenen (2R,S)-2-(2-(2-BOC-Aminothiazol-4-ylacetamido)-äthansulfonylamino)-2-(2-BOC-aminothiazol-4-yl)-essigsäure, gelöst in 120 ml Tetrahydrofuran, in Gegenwart von 1,62 g 1-Hydroxybenztriazol und 2,72 g Dicyclohexylcarbodiimid mit 5,27 g (12 mMol) mit 3-Carbamoyloxymethyl-7β-amino-3-cephem-4-carbonsäurediphenylmethylester (Reaktionszeit 16 Stunden bei Raumtemperatur) die Titelverbindung als 2R,S-Diastereomerengemisch erhalten. Das Rohprodukt wird an Silicagel (700 g) mit Methylenchlorid/Essigester 1:1 als Eluiermittel chromatographiert, woraus die Titelverbindungen mit 2R-Konfiguration (Rf: 0,33, Silicagel, Essigester) resp. 2S-Konfiguration (Rf: 0,25, Silicagel, Essigester) als amorphe Pulver isoliert werden.

Beispiel 120:

a) 3-Carbamoyloxymethyl-7β-[(2R)-2-(2-aminoäthansulfonylamino)-2-(2-aminothiazol-4-yl)-acetamido]-3-cephem-4-carbonsäure

Analog Beispiel 13 a) wird ausgehend von 3,6 g (4 mMol) 3-Carbamoyloxymethyl-7β-[(2R)-2-(2-BOC-aminoäthansulfonylamino)-2-(2-BOC-aminothiazol-4-yl-acetamido]-3-cephem-4-carbonsäurediphenylmethylester, gelöst in 16 ml absolutem Methylenchlorid, in Gegenwart von 3,2 ml Anisol und 16 ml Trifluoressigsäure die Titelverbindung in Form des 1,5-Hydrats erhalten. F: oberhalb 140° (unter Zersetzung); Rf: 0,75 (Silicagel Opti UPC 12, Wasser/Acetonitril 4:1); UV: 254 (13800, 0,1 N HCl).

b) 3-Carbamoyloxymethyl-7β-[(2S)-2-(2-aminoäthansulfonylamino)-2-(2-aminothiazol-4-yl)-acetamido]-3-cephem-4-carbonsäure

Analog Beispiel 13 a) wird ausgehend von 4,96 g (5,5 mMol) 3-Carbamoyloxymethyl-7β-[(2S)-2-(2-BOC-aminoäthansulfonylamino)-2-(2-BOC-aminothiazol-4-yl)-acetamido]-3-cephem-4-carbonsäurediphenylmethylester, gelöst in 22 ml absolutem Methylenchlorid, in Gegenwart von 4,4 ml Anisol und 22 ml Trifluoressigsäure die Titelverbindung in Form

des 1,5-Hydrats erhalten. F: oberhalb 142° (unter Zersetzung); Rf: 0,75 (Silicagel Opti UPC 12, Wasser/Acetonitril 4:1); UV 254 (13600, 0,1 N HCl).

c) 3-Carbamoyloxymethyl-7β-[(2R)-2-(2-BOC-aminoäthansulfonylamino)-2-(2-BOC-aminothiazol-4-yl)-acetamido]-3-cephem-4-carbonsäurediphenylmethylester
und
3-Carbamoyloxymethyl-7β-[(2S)-2-(2-BOC-aminoäthansulfonylamino)-2-(2-BOC-aminothiazol-4-yl)-acetamido]-3-cephem-4-carbonsäurediphenylmethylester

Analog Beispiel 13 b) erhält man durch Behandeln von 9,6 g (20 mMol) (2R,S)-2-(2-BOC-Aminoäthansulfonylamino)-2-(2-BOC-aminothiazol-4-yl)-essigsäure mit 8,79 g (20 mMol) 3-Carbamoyloxymethyl-7β-amino-3-cephem-4-carbonsäurediphenylmethylester in Gegenwart von 2,7 g 1-Hydroxybenztriazol und 4,54 g Dicyclohexylcarbodiimid in 200 ml Tetrahydrofuran das Rohprodukt als 2R,S-Diastereomerengemisch. Das Rohprodukt wird an Silicagel (1000 g) mit Methylenchlorid/Essigester 7:3 resp. 1:1 als Eluiermittel chromatographiert, woraus die Titelverbindungen mit 2R-Konfiguration (Rf: 0,65) und 2S-Konfiguration (Rf: 0,55, Silicagel, Diäthyläther/Essigester 1:1) erhalten werden.

Beispiel 121:

a) 7β-[(2R,S)-2-(2-Aminothiazol-4-yl)-2-(2-phenylaminoäthansulfonylamino)-acetamido]-3-cephem-4-carbonsäurenatriumsalz

Analog Beispiel 76 a) wird durch Umsetzung von 1,9 g 7β-[(2R,S)-2-(2-BOC-Aminothiazol-4-yl)-2-(2-phenylaminoäthansulfonylamino)-acetamido]-3-cephem-4-carbonsäurediphenylmethylester mit 40 ml Trifluoressigsäure in 1,0 ml Anisol und 4 ml Methylenchlorid und anschliessende Behandlung des Trifluoracetatsalzes mit 1N Natriumhydroxid-Lösung die Titelverbindung erhalten. F: ab 176° (unter Zersetzung). IR: 3320 (breit), 3200, 1775 (Schulter), 1760, 1745 (Schulter), 1680,

1645, 1600, 1375, 1365, 1325 (Schulter), 1145 (in Nujol); Rf: ca. 0,74 (Silicagel, UV 366, n-Butanol-Pyridin-Eisessig-Wasser 42:24:4:30).

b) 7β-[(2R,2)-2-(2-BOC-Aminothiazol-4-]1)-2-(2-phenylaminoäthansulfonylamino)-acetamido]-3-cephem-4-carbonsäurediphenylmethylester

6,0 g 7β-[(2R,S)-2-(2-BOC-Aminothiazol-4-yl)-2-(2-phenyl-2-trichloräthoxycarbonylaminoäthansulfonylamino)-acetamido]-3-cephem-4-carbonsäurediphenylmethylester, hergestellt analog Beispiel 42 c)- e), werden in 60 ml Acetonitril-Eisessig (1:1) mit 20,0 g (2 x 10,0 g) Zinkstaub behandelt, analog Beispiel 13 d) aufgearbeitet, wobei die Titelverbindung als Schaum erhalten wird. Rf: ca. 0,43 (Silicagel, UV 366, Doppelflecken, Diastereomerengemisch, Toluol-Chloroform-Essigester 1:1:1 + 1 % Aethanol).

Beispiel 122:

a) 7β-[(2R,S)-2-(2-Aminothiazol-4-yl)-2-(3-(2-furoyl)-ureido)-äthansulfonylamino)-acetamido]-3-cephem-4-carbonsäurematriumsalz

Analog Beispiel 76 a) wird durch Umsetzung von 1,9 g 7β-[(2R,S)-2-(2-BOC-Aminothiazol-4-yl)-2-(3-(2-furoyl)-ureido-äthansulfonylamino)-acetamido]-3-cephem-4-carbonsäurediphenylmethylester mit 50 ml Trifluoressigsäure in 0,40 ml Anisol und 5 ml Methylenchlorid und anschliessende Behandlung des Trifluoracetatsalzes mit 1 N Natronlauge die Titelverbindung erhalten. F: ab 196° unter Zersetzung).
IR: 3310 (breit), 3200, 1765 (breit), 1690 (breit), 1600 (breit), 1375, 1365, 1340 (Schulter), 1175, 1150 (in Nujol); Rf: ca. 0,38 (Silicagel, UV 366, n-Butanol-Pyridin-Eisessig-Wasser 42:24:4:30).

b) 7β-[(2R,S)-2-(2-BOC-Aminothiazol-4-yl)-2-(3-(2-furoyl)-ureido)-äthansulfonylamino)-acetamido]-3-cephem-4-carbonsäurediphenylmethylester

Eine Lösung von 3,50 g 7β-[(2R,S)-2-(2-BOC-Aminothiazol-4-yl)-2-(2-aminoäthansulfonyamino)-acetamido]-3-cephem-4-carbonsäurediphenylmethylester in 100 ml Aethylacetat wird vorgelegt, auf +3° abgekühlt,

unter Rühren und Kühlen innerhalb einer Stunde eine Lösung von 0,85 g 2-Furoylisocyanat in 50 ml Aethylacetat hinzugetropft und anschliessend das Reaktionsgemisch 5 Stunden im Eisbad gerührt. Darauf wird die Aethylacetatlösung mit Wasser gewaschen, über Natriumsulfat getrocknet, am Rotationsverdampfer bei 50° eingedampft und das Rohprodukt durch Chromatographieren an 60-facher Menge Kieselgel gereinigt. Eluiermittel: Methylenchlorid mit 25 bis 40% Methylacetat. Man erhält die Titelverbindung als Schaum. Rf: ca. 0,27 (Silicagel, UV 366, Doppelflecken, Diastereomerengemisch, Toluol-Chloroform-Aethylacetat 1:1:1 + 5% Aethanol).

Beispiel 123:

a) 7β-[(2R,S)-2-(2-Aminothiazol-4-yl)-2-(2-(3-äthoxycarbonylureido)-äthansulfonylamino)-acetamido]-3-cephem-4-carbonsäurenatriumsalz

Analog Beispiel 76 a) wird durch Umsetzung von 1,29 g 7β-[(2R,S)-2-(2-BOC-Aminothiazol-4-yl)-2-(2-(3-äthoxycarbonylureido)-äthansulfonylamino)-acetamido]-3-cephem-4-carbonsäurediphenylmethylester mit 50 ml Trifluoressigsäure in 0,40 ml Anisol und 5 ml Methylenchlorid und anschliessende Behandlung des Trifluoracetatsalzes mit 1N Natronlauge die Titelverbindung erhalten. F: ab 232° unter Zersetzung; IR: 3320 (breit), 3200, 1775 (Schulter), 1760 (breit), 1730, 1690 (breit), 1640, 1605, 1375, 1365, 1175 (Schulter), 1145 (in Nujol); Rf: ca. 0,42 (Silicagel, UV 366, n-Butanol-Pyridin-Eisessig-Wasser 42:24:4:30).

b) 7β-[(2R,S)-2-(2-BOC-Aminothiazol-4-yl)-2-(2-(3-äthoxycarbonylureido)-äthansulfonylamino)-acetamido]-3-cephem-4-carbonsäurediphenylmethylester

Analog Beispiel 122 b) wird durch Umsetzung von 3,40 g 7β-[(2R,S)-2-(2-BOC-Aminothiazol-4-yl)-2-(2-aminoäthansulfonylamino)-acetamido]-3-cephem-4-carbonsäure-diphenylmethylester mit 2,5 ml Aethoxycarbonylisocyanat in 150 ml (100 ml und 50 ml) Aethylacetat die Titelverbindung erhalten. Das Rohprodukt wird durch Chromatographieren an 20-facher

Menge Kieselgel gereinigt. Eluiermittel: Methylenchlorid mit 15 bis 25% Methylacetat. Rf: ca. 0,16 (Silicagel, UV 366, Doppelflecken, Diastereomerengemisch, Toluol-Chloroform-Aethylacetat 1:1:1 + 3% Aethanol).

Beispiel 124:

a) 7β-[(2R,S)-2-(2-Aminothiazol-4-yl)-2-(2-(3-benzolsulfonylureido)-äthansulfonylamino)-acetamido]-3-cephem-4-carbonsäurenatriumsalz

Analog Beispiel 76 a) wird durch Umsetzung von 1,5 g 7β-[(2R,S)-2-(2-BOC-Aminothiazol-4-yl)-2-(2-(3-benzolsulfonylureido)-äthansulfonylamino)-acetamido]-3-cephem-4-carbonsäurediphenylmethylester mit 50 ml Trifluoressigsäure in 0,40 ml Anisol und 5 ml Methylenchlorid und anschliessende Behandlung des Trifluoracetatsalzes mit 1N Natronlauge die Titelverbindung erhalten. F: ab 200° unter Zersetzung. IR: 3320 (breit), 3200, 1785, 1760, 1685, 1640 (Schulter), 1600 (breit), 1375, 1365, 1350 (Schulter), 1165 (Schulter), 1135 (in Nujol); Rf: ca. 0,37 (Silicagel, UV 366, n-Butanol-Pyridin-Eisessig-Wasser 42:24:4:30).

b) 7β-[(2R,S)-2-(2-BOC-Aminothiazol-4-yl)-2-(2-(3-benzolsulfonylureido)-äthansulfonylamino)-acetamido-3-cephem-4-carbonsäurediphenylmethylester

Analog Beispiel 122 b) wird durch Umsetzung von 3,50 g 7β-[(2R,S)-2-(2-BOC-Aminothiazol-4-yl)-2-(2-aminoäthansulfonylamino)-acetamido]-3-cephem-4-carbonsäurediphenylmethylester mit 1,10 g Benzolsulfonylisocyanat in 120 ml Aethylacetat (100 ml und 20 ml) die Titelverbindung erhalten. Das Rohprodukt wird durch Chromatographieren an 20-facher Menge Kieselgel gereinigt. Eluiermittel: Methylenchlorid mit 20 bis 30% Methylacetat. Rf: ca 0,14 (Silicagel, UV 366, Doppelflecken, Diastereomerengemisch, Toluol-Chloroform-Aethylacetat 1:1:1 + 3% Aethanol).

Beispiel 125:

a) 3-Carbamoyloxymethyl-7β-[(2R,S)-2-(2-aminothiazol-4-yl)-2-(2-methoxyäthansulfonylamino)-acetamido]-3-cephem-4-carbonsäurenatriumsalz

Analog Beispiel 76 a) wird durch Umsetzung von 6 g 3-Carbamoyloxymethyl-7β-[(2R,S)-2-(2-BOC-aminothiazol-4-yl)-2-(2-methoxyäthansulfonylamino)-acetamido]-3-cephem-4-carbonsäurediphenylmethylester mit 100 ml Trifluoressigsäure in 2,50 ml Anisol und 10 ml Methylenchlorid und anschliessende Behandlung des Trifluoracetatsalzes mit 1N Natronlauge die Titelverbindung erhalten. F: ab 148° unter Zersetzung). IR: 3320 (breit), 3200, 1785 (Schulter), 1760, 1700 (breit), 1605 (breit), 1380, 1330, 1145, 1115 $cm^{-1}$ (in Nujol). Rf: 0,37 (Silicagel, UV 366, n-Butanol-Pyridin-Eisessig-Wasser 42:24:4:30).

b) 3-Carbamoyloxymethyl-7β-[(2R,S)-2-(2-BOC-aminothiazol-4-yl)-2-(2-methoxyäthansulfonylamino)-acetamido]-3-cephem-4-carbonsäurediphenylmethylester

Analog Beispiel 42 c) erhält man durch Umsetzung von 4,30 g (2R,S)-2-(2-BOC-Aminothiazol-4-yl)-2-(2-methoxyäthansulfonylamino)-essigsäure mit 4,0 g 3-Carbamoyloxymethyl-7β-amino-3-cephem-4-carbonsäurediphenylmethylester in Gegenwart von 2,40 g (2 x 1,20 g) N,N'-Dicyclohexylcarbodiimid in insgesamt 110 ml Tetrahydrofuran die Titelverbindung. Das Rohprodukt wird chromatographisch an 25-facher Menge Kieselgel gereinigt. Eluiermittel: Methylenchlorid mit 25 bis 45% Methylacetat. Rf: ca. 0,33 (Silicagel, UV 366, Doppelflecken, Diastereomerengemisch, Toluol-Aethanol 9:1).

Beispiel 126:

7β-[(2R,S)-2-(2-Aminothiazol-4-yl)-2-(2-methoxyäthansulfonylamino)-acetamido]-3-carbamoyloxymethyl-3-cephem-4-carbonsäurepivaloyloxymethylesterhydrochlorid

1,90 g 3-Carbamoyloxymethyl-7β-[(2R,S)-2-(2-aminothiazol-4-yl)-2-(2-methoxyäthansulfonylamino)-acetamido]-3-cephem-4-carbonsäurenatriumsalz

und 0,90 g Jodmethylpivalat werden in 12 ml N,N-Dimethylformamid analog Beispiel 70 a) umgesetzt und aufgearbeitet. F: ab 112° unter Zersetzung. Rf: ca. 0,36 (Silicagel Opti-UPC 12, UV 366, Acetonitril-Wasser-1:1).

Beispiel 127:

a) 3-Carbamoyloxymethyl-7β-[(2R,S)-2-(2-aminothiazol-4-yl)-2-(2-methylaminoäthansulfonylamino)-acetamido]-3-cephem-4-carbonsäurenatriumsalz

Analog Beispiel 76 a) wird durch Umsetzung von 3,1 g 3-Carbamoyloxymethyl-7β-[(2R,S)-2-(2-BOC-aminothiazol-4-yl)-2-(2-BOC-methylaminoäthansulfonylamino)-acetamido]-3-cephem-4-carbonsäurediphenylmethylester mit 46 ml Trifluoressigsäure in 1,10 ml Anisol und 7,5 ml Methylenchlorid und anschliessende Behandlung des Trifluoracetatsalzes mit 1N Natronlauge die Titelverbindung erhalten. F: ab 180 unter Zersetzung. IR: 3330 (breit), 3190 (breit), 1785 (Schulter), 1765, 1695 (breit), 1610 (breit), 1375, 1365, 1325, 1155, 1130 (Schulter) (in Nujol). Rf: ca. 0,24 (Silicagel, UV 366, n-Butanol-Pyridin-Eisessig-Wasser 42:24:4:30).

b) 3-Carbamoyloxymethyl-7β-[2R,S)-2-(2-BOC-aminothiazol-4-yl)-2-(2-BOC-methylaminoäthansulfonylamino)-acetamido]-3-cephem-4-carbonsäurediphenylmethylester

Analog Beispiel 42 c) erhält man durch Umsetzung von 2,0 g (2R,S)-2-(2-BOC-Aminothiazol-4-yl)-2-(2-BOC-methylaminoäthansulfonylamino)-essigsäure (Herstellung siehe Beispiel 79 c)) und 2,0 g 3-Carbamoyloxymethyl-7β-amino-3-cephem-4-carbonsäurediphenylmethylester in Gegenwart von 0,37 g 1-Hydroxy-benztriazol und 1,30 g (0,70 und 0,60 g) N,N'-Dicyclohexylcarbodiimid in 90 ml Tetrahydrofuran die Titelverbindung. Das Rohprodukt wird durch Chromatographieren an 40-facher Menge Kieselgel gereinigt. Eluiermittel: Methylenchlorid-Methylacetat-(7:3). Rf: ca. 0,32 (Silicagel, UV 366, Doppelflecken, Diastereomerengemisch, Toluol-Aethylacetat 1:1).

Beispiel 128:

a) 7β-[(2R,S)-2-(2-Aminothiazol-4-yl)-2-(2-sulfoaminoäthansulfonylamino)-acetamido]-3-cephem-4-carbonsäuredinatriumsalz

Analog Beispiel 76 a) wird durch Umsetzung von 3,1 g 7β-[(2R,S)-2-(2-BOC-Aminothiazol-4-yl)-2-(2-sulfoaminoäthansulfonylamino)-acetamido]-3-cephem-4-carbonsäurediphenylmethylester mit 40 ml Trifluoressigsäure in 1,25 ml Anisol und 6 ml Methylenchlorid und anschliessende Behandlung des Trifluoracetatsalzes mit 1N Natronlauge die Titelverbindung erhalten. F: ab 205° unter Zersetzung. IR: 3420 (breit), 3340 (Schulter), 3200 (breit), 1780 (Schulter), 1755, 1680, 1635 (Schulter), 1600, 1360, 1325 (Schulter), 1180, 1145 (in Nujol). Rf: ca. 1,18 (Silicagel, UV 366, n-Butanol-Pyridin-Eisessig-Wasser 42:24:4:30).

b) 7β-[(2R,S)-2-(2-BOC-Aminothiazol-4-yl)-2-(2-sulfoaminoäthansulfonylamino)-acetamido]-3-cephem-4-carbonsäurediphenylmethylester

Eine Lösung von 5,0 g 7β-[(2R,S)-2-(2-BOC-Aminothiazol-4-yl)-2-(2-aminoäthansulfonylamino)-acetamido]-3-cephem-4-carbonsäurediphenylmethylester in 40 ml Methylenchlorid wird auf +2° abgekühlt, mit 2,7 ml N-Methylmorpholin versetzt, unter Rühren und Kühlen innert 5 Minuten 3,6 ml Chlorsulfonsäuretrimethylsilylester hinzugetropft und das Reaktionsgemisch 4 Stunden bei Raumtemperatur gerührt. Anschliessend wird die Suspension mit Aethylacetat verdünnt, das Methylenchlorid am Rotationsverdampfer bei 50° abgezogen, die Aethylester-Lösung sukzessive mit 20%iger Zitronensäure-Lösung und Wasser (2 x) gewaschen. Die organische Phase wird über Natriumsulfat getrocknet, filtriert und am Rotationsverdampfer bei 50° eingedampft. Das Rohprodukt wird durch Chromatographieren an 30-facher Menge Kieselgel gereinigt. Eluiermittel: Chloroform-Methanol-32%ige wässrige Essigsäure 15:4:1. Man erhält die Titelverbindung als Schaum. Rf: ca. 0,36 (Silicagel, UV 366, Chloroform-Methanol-32%ige wässrige Essigsäure 15:4:1).

Beispiel 129:

a) 7β-[(2R,S)-2-(2-Aminothiazol-4-yl)-2-(vinylsulfonylamino)-acetamido]-3-cephem-4-carbonsäurenatriumsalz

Analog Beispiel 76 a) wird durch Umsetzung von 1,9 g 7β-[(2R,S)-2-(2-BOC-Aminothiazol-4-yl)-2-(vinylsulfonylamino)-acetamido]-3-cephem-4-carbonsäurediphenylmethylester mit 60 ml Trifluoressigsäure in 0,70 ml Anisol und 5 ml Methylenchlorid und anschliessende Behandlung des Trifluoracetatsalzes mit 1N Natronlauge die Titelverbindung erhalten. F: ab 210° unter Zersetzung. IR: 3300 (breit), 3190 (breit), 1775 (Schulter), 1755 (breit), 1680, 1640 (Schulter), 1600 (breit), 1375, 1365, 1150, 1120 (Schulter) (in Nujol). Rf: ca. 0,27 (Silicagel Opti-UPC 12, UV 366, Acetonitril mit 5% Wasser).

b) 7β-[(2R,S)-2-(2-BOC-Aminothiazol-4-yl)-2-(vinylsulfonylamino)-acetamido]-3-cephem-4-carbonsäurediphenylmethylester

Analog Beispiel 42 c) erhält man durch Umsetzung von 1,50 g (2R,S)-2-(2-BOC-Aminothiazol-4-yl)-2-(vinylsulfonylamino)-essigsäure und 1,50 g 7β-Amino-3-cephem-4-carbonsäure in Gegenwart von 1,0 g ( 2 x 0,50 g) N,N'-Dicyclohexylcarbodiimid in einem Gemisch von 20 ml Tetrahydrofuran und 10 ml N,N-Dimethylformamid die Titelverbindung. Das Rohprodukt wird durch Chromatographieren an 20-facher Menge Kieselgel gereinigt, Eluiermittel: Methylenchlorid mit 5% Methylacetat, und erhält die Titelverbindung als Schaum. Rf: ca. 0,70 (Silicagel, UV 366, Doppelflecken,Diastereomerengemisch, Toluol-Chloroform-Aethylacetat 1:1:1 + 3% Aethanol).

c) (2R,S)-2-(2-BOC-Aminothiazol-4-yl)-2-(vinylsulfonylamino)-essigsäure

40 g (2R,S)-2-(2-BOC-Aminothiazol-4-yl)-2-(vinylsulfonylamino)-essigsäureäthylester werden in 350 ml Aethanol gelöst, unter Rühren und leichtem Kühlen innerhalb 20 min 350 ml 2N Natronlauge hineingetropft und das Reaktionsgemisch bei Raumtemperatur gerührt. Nach 2 Stunden Reaktionszeit werden weitere 100 ml 2N Natronlauge in die klare,

dunkelorange gefärbte Lösung getropft. Nach insgesamt 5,5 Stunden Reaktionszeit bei Raumtemperatur wird der pH-Wert des Gemisches mit 10 %iger Salzsäure auf 7,5 gestellt, der grösste Teil des Aethanols am Rotationsverdampfer bei 50° abdestilliert und die wässrige Lösung mit Diäthyläther ausgezogen. Dann wird die wässrige Phase abgetrennt, mit Aethylacetat überschichtet, im Eisbad abgekühlt, unter Rühren und Kühlen durch Zutropfen von 20 %iger Phosphorsäure sauer gestellt (pH 2,0) und zweimal mit Aethylacetat ausgezogen. Die organischen Phasen werden vereinigt, mit Wasser 2 mal gewaschen, über Natriumsulfat getrocknet, filtriert und am Rotationsverdampfer bei 50° eingedampft. Der ölige Rückstand erstarrt beim Vermischen mit einem Gemisch von Methylenchlorid-Petroläther (1:1). F: 119-122°. Rf: ca. 0,64 (Silicagel, UV 366, n-Butanol-Eisessig-Wasser 67:10:23).

d) <u>(2R,S)-2-(2-BOC-Aminothiazol-4-yl)-2-(vinylsulfonylamino)-essigsäureäthylester</u>

Eine Lösung von 50 g (2R,S)-2-(2-BOC-Aminothiazol-4-yl)-glycinäthylester in 400 ml Dioxan und 45 ml N-Methylmorpholin wird auf +10° abgekühlt und unter starkem Rühren und Kühlen innerhalb 30 Minuten eine Lösung von 40 g 2-Bromäthansulfonylchlorid, hergestellt nach der Vorschrift von C.S. Marcel et al., J.Am.Chem.Soc., 49, 1833 (1927), in 100 ml Dioxan hinzugetropft. Das Reaktionsgemisch wird 5 Stunden bei Raumtemperatur gerührt. Darauf wird der grösste Teil des Lösungsmittels am Rotationsverdampfer bei 50° abgedampft und die verbleibende Suspension mit Aethylacetat verdünnt, sukzessive mit 20 %iger Zitronensäurelösung, Wasser, 1 N Natriumhydrogencarbonat-Lösung und Wasser (3 x) gewaschen. Die organische Phase wird über Natriumsulfat getrocknet, filtriert und am Rotationsverdampfer bei 50° eingedampft. Das Rohprodukt wird durch Chromatographieren an 10facher Menge Kieselgel gereinigt und die Titelverbindung als Schaum erhalten. Rf: ca. 0,46 (Silicagel, UV 366, Toluol-Aethylacetat 1:1).

Beispiel 130:

a) 3-Carbamoyloxymethyl-7β-[(2R,S)-2-(2-aminothiazol-4-yl)-2-(vinylsulfonylamino)-acetamido]-3-cephem-4-carbonsäurenatriumsalz

Analog Beispiel 76 a) wird durch Umsetzung von 1,8 g 3-Carbamoyloxymethyl-7β-[(2R,S)-2-(2-BOC-aminothiazol-4-yl)-2-(vinylsulfonylamino)-acetamido]-3-cephem-4-carbonsäurediphenylmethylester mit 40 ml Trifluoressigsäure in 1,0 ml Anisol und 4 ml Methylenchlorid und anschliessende Behandlung des Trifluoracetatsalzes mit 1N Natronlauge die Titelverbindung erhalten. F: ab 168° unter Zersetzung. IR: 3330 (breit), 3190, 1785 (Schulter), 1760, 1700 (breit), 1610 (breit), 1620, 1375, 1365 (schwach), 1325, 1150, 1120 (in Nujol). Rf: ca. 0,24 (Silicagel, UV 366, n-Butanol-Pyridin-Eisessig-Wasser 42:24:4:30).

b) 3-Carbamoyloxymethyl-7β-[(2R,S)-2-(2-BOC-aminothiazol-4-yl)-2-(vinylsulfonylamino)-acetamido]-3-cephem-4-carbonsäurediphenylmethylester

Analog Beispiel 42 c) erhält man durch Umsetzung von 1,30 g (2R,S)-2-(2-BOC-Aminothiazol-4-yl)-2-(vinylsulfonylamino)-essigsäure (Herstellung siehe Beispiel 129 c)) und 1,70 g 3-Carbamoyloxymethyl-7β-amino-3-cephem-4-carbonsäurediphenylmethylester in Gegenwart von 0,90 g (0,50 g und 0,40 g) N,N'-Dicyclohexylcarbodiimid in insgesamt 40 ml Tetrahydrofuran die Titelverbindung. Das Rohprodukt wird durch Chromatographieren an 25-facher Menge Kieselgel gereinigt, Eluiermittel Methylenchlorid mit 15 bis 20% Methylacetat, und erhält die Titelverbindung als Schaum. Rf: ca. 0,18 (Silicagel, UV 366, Doppelflecken, Diastereomerengemisch, Toluol-Chloroform-Aethylacetat 1:1:1 + 3% Aethanol).

Beispiel 131:

a) 3-(1-Methyl-1H-tetrazol-5-ylthiomethyl)-7β-[(2R,S)-2-(2-aminothiazol-4-yl)-2-(2-methylaminoäthansulfonylamino)-acetamido]-3-cephem-4-carbonsäurepivaloyloxymethylesterhydrochlorid

Eine Suspension von 2,20 g 3-(1-Methyl-1H-tetrazol-5-ylthiomethyl-7β-[(2R,S)-2-(2-BOC-aminothiazol-4-yl)-2-(2-BOC-methylaminoäthansulfonyl-amino-acetamido]-3-cephem-4-carbonsäurepivaloyloxymethylester in 0,90 ml Anisol und 5 ml Methylenchlorid wird mit 40 ml Trifluoressigsäure versetzt, das Reaktionsgemisch unter Ausschluss der Luftfeuchtigkeit 45 min bei Raumtemperatur gerührt, und anschliessend auf ein eiskaltes Gemisch von 800 ml Petroläther und 200 ml Diäthyläther gegossen, wobei das Trifluoracetatsalz ausfällt. Es wird abgenutscht, mit einem Gemisch von Petroläther-Diäthyläther gewaschen und bei Raumtemperatur am Hochvakuum getrocknet. Das Trifluoracetatsalz wird in ca. 300 ml Aethylacetat gelöst und die Lösung sukzessive mit 1N Natriumhydrogencarbonat-Lösung (2 x 60 ml) und Sodalösung gewaschen. Die organische Phase wird über Natriumsulfat getrocknet, filtriert, mit überschüssigem Chlorwasserstoff in Methylenchlorid versetzt und am Rotationsverdampfer bei 20-30° auf ca. 50 ml eingeengt. Die anfallende Titelverbindung wird abgenutscht, mit Aethylacetat und Diäthyläther gewaschen, und bei Raumtemperatur am Hochvakuum getrocknet. F: ab 110° unter Zersetzung. IR: 3180 (breit), 1785 (Schulter), 1775, 1750, 1690, 1625, 1375, 1365 (Schulter), 1335, 1150, 1115 (in Nujol). Rf: ca. 0,28 (Silicagel Opti UPC 12, UV 366, Acetonitril-Wasser 4:1).

b) <u>3-(1-Methyl-1H-tetrazol-5-ylthiomethyl)-7β-[(2R,S)-2-(2-BOC-aminothiazol-4-yl)-2-(2-BOC-methylaminoäthansulfonylamino)-acetamido]-3-cephem-4-carbonsäurepivaloyloxymethylester</u>

Analog Beispiel 42 c) erhält man durch Umsetzung von 2,60 g (2R,S)-2-(2-BOC-Aminothiazol-4-yl)-2-(2-BOC-methylaminoäthansulfonylamino)-essigsäure (Herstellung siehe Beispiel 79 c)) und 1,90 g 3-(1-Methyl-1H-tetrazol-5-ylthiomethyl)-3-cephem-4-carbonsäurepivaloyloxymethylester in Gegenwart von 0,32 g 1-Hydroxy-benztriazol und 1,90 g (2 x 0,95 g) N,N'-Dicyclohexylcarbodiimid in insgesamt 70 ml Tetrahydrofuran die Titelverbindung. Das Rohprodukt wird durch Chromatographieren an 45-facher Menge Kieselgel gereinigt, Eluiermittel: Methylenchlorid-Methylacetat (4:1), und erhält die Titelverbindung als Schaum. Rf: ca. 0,26 (Silicagel, UV 366, Doppelflecken, Diastereomerengemisch, Toluol-Aethylacetat 1:1).

Beispiel 132:

a) 7β-[(2R,S)-2-(2-Aminothiazol-4-yl)-2-(2-dimethylaminosulfonylaminoäthansulfonylamino)-acetamido]-3-cephem-4-carbonsäurenatriumsalz

Analog Beispiel 76 a) wird durch Umsetzung von 2,20 g 7β-[(2R,S)-2-(2-BOC-Aminothiazol-4-yl)-2-(2-dimethylaminosulfonylaminoäthansulfonylamino)-acetamido]-3-cephem-4-carbonsäurediphenylmethylester mit 40 ml Trifluoressigsäure in 0,90 ml Anisol und 4 ml Methylenchlorid und anschliessende Behandlung des Trifluoracetatsalzes mit 1N Natronla ge die Titelverbindung erhalten. F: ab 183° unter Zersetzung. Rf: ca. 0,63 (Silicagel, UV 366, n-Butanol-Pyridin-Eisessig-Wasser 42:24:4:30).

b) 7β-[(2R,S)-2-(2-BOC-Aminothiazol-4-yl)-2-(2-dimethylaminosulfonylaminoäthansulfonylamino)-acetamido]-3-cephem-4-carbonsäurediphenylmethylester

Analog Beispiel 128 b) erhält man durch Umsetzung von 4,0 g 7β-[(2R,S)-2-(2-BOC-Aminothiazol-4-yl)-2-(2-aminoäthansulfonylamino)-acetamido]-3-cephem-4-carbonsäurediphenylmethylester mit 1,80 ml N,N-Dimethylamidosulfonsäurechlorid in einem Gemisch von 100 ml Dioxan und 2 ml N-Methylmorpholin die Titelverbindung. Das Rohprodukt wird durch Chromatographieren an 30-facher Menge Kieselgel gereinigt. Eluiermittel: Methylenchlorid mit 15% Methylacetat. Rf: ca. 0,64 (Silicagel, UV 366, Doppelflecken, Diastereomerengemisch, Toluol-Aethylacetat 1:2).

Beispiel 133:

a) 3-(2,5-Dihydro-6-hydroxy-2-methyl-5-oxo-as-triazin-3-ylthiomethyl)-7β-[(2R,S)-2-(2-aminothiazol-4-yl)-2-methansulfonylaminoacetamido]-3-cephem-4-carbonsäuredinatriumsalz

1,5 g 3-(2,5-Dihydro-6-hydroxy-2-methyl-5-oxo-as-triazin-3-ylthiomethyl)-7β-[(2R,S)-2-(2-chloracetamidothiazol-4-yl)-2-methansulfonylaminoacetamido]-3-cephem-4-carbonsäuredinatriumsalz (Herstellung siehe Beispiel 133 b) werden unter $N_2$-Atmosphäre mit 0,7 g Thioharnstoff in 15 ml Wasser während 8 Stunden bei Zimmertemperatur gerührt, wobei das

pH der Reaktionsmischung durch Zugabe von 0,1 N wässriger NaOH mittels Titrator bei pH 6,8 konstant gehalten wird. Dann extrahiert man mit Essigester und wäscht 2 mal mit Wasser nach. Die vereinigten Wasserphasen engt man im Vakuum ein und chromatographiert an 150 g Opti-UPC-12 Kieselgel. Die mit einem $H_2O$-$CH_3CN$ 9:1-Gemisch eluierten, produkthaltigen Fraktionen werden vereinigt, eingeengt und das Hydrat der Titelverbindung durch Zugabe von Aethanol ausgefällt, abgenutscht und getrocknet; IR: 3700-2500 (breit), 1765, 1685, 1640 (Schulter), 1600, 1550 (Schulter), 1500 (Nujol).

b) 3-(2,5-Dihydro-6-hydroxy-2-methyl-5-oxo-as-triazin-3-ylthiomethyl)-7β-[(2R,S)-2-(2-chloracetamidothiazol-4-yl)-2-methansulfonylaminoacetamido-3-cephem-4-carbonsäuredinatriumsalz

200 mg (2R,S)-2-(2-Chloracetamidothiazol-4-yl)-2-methansulfonylaminoessigsäure werden in 1,82 ml Methylenchlorid mit 0,102 ml Triäthylamin versetzt. Dann kühlt man auf 0° ab, gibt 128 mg Phosphorpentachlorid zu und rührt 5 min bei 0° und 20 min bei Zimmertemperatur. Dann dampft man im Vakuum ein, digeriert zweimal mit Hexan und löst anschliessend in 1,8 ml Tetrahydrofuran. Darauf wird vom ausgefallenen Triäthylaminhydrochlorid abfiltriert. Die erhaltene Lösung, enthaltend (2R,S)-2-(2-Chloracetamidothiazol-4-yl)-2-methansulfonylaminoessigsäurechlorid, wird direkt für die nachfolgende Acylierung eingesetzt.
150 mg 3-(2,5-Dihydro-6-hydroxy-2-methyl-5-oxo-as-triazin-3-ylthiomethyl)-7β-amino-3-cephem-4-carbonsäure werden mit 0,4 ml N,O-Bis-(trimethylsilyl)-acetamid in 1,5 ml Tetrahydrofuran 1 Stunde am Rückflusskühler erhitzt. Dann kühlt man auf 20° ab, gibt 0,033 ml Pyridin und die gemäss obiger Vorschrift erhältliche Säurechloridlösung zu und rührt 3 Stunden bei Zimmertemperatur. Dann gibt man 2 ml Wasser zu, stellt mit 1N NaOH auf pH 7 und dampft im Vakuum zur Trockne ein. Anschliessend wird in Wasser an 20 g Opti-UPC-12 Kieselgel chromatographiert. Die mit Wasser eluierten, produkthaltigen Fraktionen werden vereinigt, eingeengt und das Hydrat der Titelverbindung durch Aethanolzugabe ausgefällt, abgenutscht und getrocknet. IR: 3700-2500 (breit),

1760, 1685, 1640 (Schulter), 1600, 1550 (Schulter), 1500 (Nujol).

Beispiel 134:

a) 3-Carbamoyloxymethyl-7β-[(2R,S)-2-(2-aminothiazol-4-yl)-2-methoxycarbonylmethansulfonylaminoacetamido]-3-cephem-4-carbonsäure-natriumsalz

1,2 g gemäss Beispiel 134 b) herstellbarer 3-Carbamoyloxymethyl-7β-[(2R,S)-2-(2-BOC-aminothiazol-4-yl)-2-methoxycarbonylmethansulfonylaminoacetamido]-3-cephem-4-carbonsäurediphenylmethylester werden in 2 ml $CH_2Cl_2$ und 0,38 ml Anisol mit 2,5 ml Trifluoressigsäure analog Beispiel 1 a) umgesetzt, aufgearbeitet, chromatographiert und umgefällt. Man erhält das Hydrat der Titelverbindung; IR: 3650-2500 (breit), 1775 (Schulter), 1744, 1677, 1604, 1520 (Nujol); UV: 253 (10200; $H_2O$).

b) 3-Carbamoyloxymethyl-7β-[(2R,S)-2-(2-BOC-aminothiazol-4-yl)-2-methoxycarbonylmethansulfonylaminoacetamido]-3-cephem-4-carbonsäurediphenylmethylester

10,2 g der gemäss Beispiel 134 c) erhältlichen (2R,S)-2-(2-BOC-Aminothiazol-4-yl)-2-methoxycarbonylmethansulfonylamino-essigsäure werden mit 10,9 g 3-Carbamoyloxymethyl-7β-amino-3-cephem-4-carbonsäurediphenylmethylester in 100 ml Tetrahydrofuran analog Beispiel 6 b) umgesetzt (3,3 g Hydroxybenztriazol; dreimal 1,73 g Dicyclohexylcarbodiimid in je 6,66 ml Tetrahydrofuran), aufgearbeitet und chromatographiert. Man erhält die Titelverbindung; IR: 3400, 3300, 1787, 1740, 1725, 1695, 1630, 1540, 1495 (Nujol).

c) (2R,S)-2-(2-BOC-Aminothiazol-4-yl)-2-methoxycarbonylmethansulfonylamino-essigsäure

6,8 g (2R,S)-2-(2-BOC-Aminothiazol-4-yl)-glycin werden in 60 ml Tetrahydrofuran mit 5,2 g Methoxycarbonylmethansulfochlorid analog

Beispiel 6 c) umgesetzt (20 ml N,O-Bis-(trimethylsilyl)-acetamid; 2 ml Pyridin) und aufgearbeitet. Man erhält die Titelverbindung, welche ohne Charakterisierung gemäss Beispiel 134 b) weiterverarbeitet wird.

Beispiel 135:

7β-[(2R,S)-2-(2-Aminothiazol-4-yl)-2-pivaloyloxymethoxycarbonylmethansulfonylaminoacetamido]-3-cephem-4-carbonsäurepivaloyloxymethylester-hydrochlorid

1,3 g 7β-[(2R,S)-2-(2-Aminothiazol-4-yl)-2-carboxymethansulfonylaminoacetamido]-3-cephem-4-carbonsäuredinatriumsalz (Herstellung siehe Beispiel 137) und 1,3 ml Jodmethylpivalat werden in 13 ml Dimethylformamid analog Beispiel 70 a) umgesetzt, aufgearbeitet und in das Hydrochlorid überführt. Man erhält die Titelverbindung; F: oberhalb 160° (unter Zersetzung); $[\alpha]_D^{20°}$ = +29°±1° (0,92% in DMSO); IR: 3650-2300 (breit), 1785 (Schulter), 1755, 1695, 1650 (Schulter), 1630, 1530 (Nujol); UV: 260 (9000; $CH_3OH$).

Beispiel 136:

7β-[(2R,S)-2-(2-Aminothiazol-4-yl)-2-methoxycarbonylmethansulfonylaminoacetamido]-3-cephem-4-carbonsäurepivaloyloxymethylesterhydrochlorid

1,0 g 7β-[(2R,S).2-(2-Aminothiazol-4-yl)-2-methoxycarbonylmethansulfonylaminoacetamido]-3-cephem-4-carbonsäurenatriumsalz (Herstellung siehe Beispiel 138 a) und 0,52 ml Jodmethylpivalat werden in 10 ml Dimethylformamid analog Beispiel 70 a) umgesetzt, aufgearbeitet und in das Hydrochlorid überführt. Man erhält die Titelverbindung; F: oberhalb 95° (unter Zersetzung); $[\alpha]_D^{20°}$ = +56°±1° (0,99% in DMSO); IR: 3660-2300 (breit), 1785, 1747, 1695, 1630, 1540 (Nujol); UV: 255 (9500; $CH_3OH$).

Beispiel 137:

7β-[(2R,S)-2-(2-Aminothiazol-4-yl)-2-carboxymethansulfonylaminoacetamido]-3-cephem-4-carbonsäuredinatriumsalz

2,7 g [(2R,S)-2-(2-Aminothiazol-4-yl)-2-methoxycarbonylmethansulfonylaminoacetamido]-3-cephem-4-carbonsäurenatriumsalz (Herstellung siehe Beispiel 138 a) werden in 135 ml Wasser mit 135 ml 0,1 N wässriger NaOH 20 min bei Zimmertemperatur gerührt. Dann stellt man mit 2N Salzsäure auf pH 7, engt im Vakuum ein und chromatographiert wie im Beispiel 1 a) beschrieben. Man erhält das Hydrat der Titelverbindung; F: oberhalb 220° (unter Zersetzung); $[\alpha]_D^{20°} = +61° \pm 1°$ (1,13% in $H_2O$); IR: 3700-2500 (breit), 1780 (Schulter), 1760, 1670 (Schulter), 1630-1565 (breit), 1520 (Nujol); UV: 253 (7000; $H_2O$).

Beispiel 138:

a) 7β-[(2R,S)-2-(2-Aminothiazol-4-yl)-2-methoxycarbonylmethansulfonylaminoacetamido]-3-cephem-4-carbonsäurenatriumsalz

12 g des gemäss Beispiel 138 b) herstellbaren 7β-[(2R,S)-2-(2-BOC-Aminothiazol-4-yl)-2-methoxycarbonylmethansulfonylaminoacetamido]-3-cephem-4-carbonsäurediphenylmethylesters werden in 20 ml $CH_2Cl_2$ und 3,8 ml Anisol mit 25 ml Trifluoressigsäure analog Beispiel 1 a) umgesetzt, aufgearbeitet, chromatographiert und umgefällt. Man erhält das Hydrat der Titelverbindung; F: oberhalb 190° (unter Zersetzung); $[\alpha]_D^{20°} = +98° \pm 1°$ (0,99% in $H_2O$); IR: 3650-2500 (breit), 1775 (Schulter), 1745, 1678, 1605, 1520 (Nujol); UV: 252 (9800; $H_2O$).

b) 7β-[(2R,S)-2-(2-BOC-Aminothiazol-4-yl)-2-methoxycarbonylmethansulfonylaminoacetamido]-3-cephem-4-carbonsäurediphenylmethylester

10,2 g der gemäss Beispiel 134 c) erhältlichen (2R,S)-2-(2-BOC-Aminothiazol-4-yl)-2-methoxycarbonylmethansulfonylamino-essigsäure werden mit 7,3 g 7β-Amino-3-cephem-4-carbonsäurediphenylmethylester in 100 ml Tetrahydrofuran analog Beispiel 6 b) umgesetzt, 3,3 g

Hydroxybenztriazol; dreimal 1,73 g Dicyclohexylcarbodiimid in je 6,66 ml Tetrahydrofuran), aufgearbeitet und chromatographiert. Man erhält die Titelverbindung; $[\alpha]_D^{20°} = +16°\pm1°$ (1,10% in EtOH); IR: 3400, 3300, 1789, 1740 (Schulter), 1725, 1696, 1635, 1542, 1497 ($CH_2Cl_2$); UV: 258 (14200; EtOH).

Beispiel 139:

a) 3-Carbamoyloxymethyl-7β-[(2R,S)-2-(5-amino-1,2,4-thiadiazol-3-yl)-2-methansulfonylaminoacetamido]-3-cephem-4-carbonsäurenatriumsalz

2,2 g (2,9 mMol) 3-Carbamoyloxymethyl-7β-[(2R,S)-2-(5-BOC-amino-1,2,4-thiadiazol-3-yl)-2-methansulfonylaminoacetamido]-3-cephem-4-carbonsäurediphenylmethylester werden in 4,4 ml $CH_2Cl_2$ und 1,5 ml Anisol mit 21 ml Trifluoressigsäure analog Beispiel 12 a) umgesetzt, aufearbeitet, chromatographiert und lyophilisiert. Man erhält die Titelverbindung mit einem Rf-Wert von 0,55 (Silicagel Opti UPC 12, Wasser: Acetonitril 6:1); IR (Nujol): charakteristische Absorptionsbande bei 3310, 1755, 1617, 1158.

b) 3-Carbamoyloxymethyl-7β-[(2R,S)-2-(5-BOC-amino-1,2,4-thiadiazol-3-yl)-2-methansulfonylaminoacetamido]-3-cephem-4-carbonsäurediphenylmethylester

1,8 g 5,0 mMol) (2R,S)-2-(5-BOC-Amino-1,2,4-thiadiazol-3-yl)-2-methansulfonylaminoessigsäure und 2,2 g (5,0 mMol) 3-Carbamoyloxymethyl-7β-amino-3-cephem-4-carbonsäurediphenylmethylester werden in 40 ml Tetrahydrofuran analog Beispiel 12 b) umgesetzt (0,55 g Hydroxybenztriazol), 1,25 g N,N'-Dicyclohexylcarbodiimid aufgearbeitet und aus Aether umgefällt. Man erhält die Titelverbindung; Rf-Wert: 0,50 (Silicagel, Aethylacetat); IR: (Nujol): charakteristische Absorptionsbaden bei 1765 und 1160.

Beispiel 140:

In analoger Weise zu den Beispielen 1 - 139 kann man folgende Verbindungen herstellen:

3-Methyl-7β-[(2R,S)-2-(2-aminothiazol-4-yl)-2-(2-aminoäthansulfonylamino)-acetamido]-3-cephem-4-carbonsäure; IR: u.a. 1765 (Nujol); UV: 250 (14100; 0,1 N HCl).

3-Chlor-7β-[(2R,S)-2-(2-aminothiazol-4-yl)-2-(2-aminoäthansulfonylamino)-acetamido]-3-cephem-4-carbonsäure; IR: u.a. 1766 (Nujol); UV:251 (15200; 0,1 N HCl).

3-(2-Methyl-5,6-dioxo-1,2,5,6-tetrahydro-as-triazin-3-ylthiomethyl)-7β-[(2R,S)-2-(2-aminothiazol-4-yl)-2-(2-aminoäthansulfonylamino)-acetamido]-3-cephem-4-carbonsäure; IR: u.a. 1762 (Nujol); UV: 240 (18600), 270 (22100; 0,1 N HCl).

3-Pyridiniomethyl-7β-[(2R,S)-2-(2-aminothiazol-4-yl)-2-(2-aminoäthansulfonylamino)-acetamido]-3-cephem-4-carboxylat, IR: u.a. 1761 (Nujol); UV: 249 (1400; 0,1 N HCl).

3-(4-Carbamoylpyridiniomethyl)-7β-[(2R,S)-2-(2-aminothiazol-4-yl)-2-(2-aminoäthansulfonylamino)-acetamido]-3-cephem-4-carboxylat; IR: u.a. 1764 (Nujol); UV: 250 (14800, 0,1 N HCl).

3-Methyl-7β-[(2R,S)-2-(2-aminothiazol-4-yl)-2-(2-methansulfonylaminoäthansulfonylamino)-acetamido]-3-cephem-4-carbonsäure: IR: u.a. 1770, 1748, 1730, 1692, 1610, 1530 (Nujol); UV: 253 (12500; $H_2O$).

3-Methoxy-7β-[(2R,S)-2-(2-aminothiazol-4-yl)-2-(2-methansulfonylaminoäthansulfonylamino)-acetamido]-3-cephem-4-carbonsäure; IR: u.a. 1769 (Nujol); UV: 252 (13300; $H_2O$).

3-Chlor-7β-[(2R,S)-2-(2-aminothiazol-4-yl)-2-(2-methansulfonylaminoäthansulfonylamino)-acetamido]-3-cephem-4-carbonsäure; IR: u.a. 1769 (Nujol); UV: 253 (14400; $H_2O$).

3-(1-Carboxymethyl-1H-tetrazol-5-ylthiomethyl)-7β-[(2R,S)-2-(2-aminothiazol-4-yl)-2-(2-methansulfonylaminoäthansulfonylamino)-acetamido]-3-cephem-4-carbonsäure; IR: u.a. 1765 (Nujol); UV: 252 (12900; $H_2O$).

3-(1-Sulfomethyl-1H-tetrazol-5-ylthiomethyl)-7β-[(2R,S)-2-(2-aminothiazol-4-yl)-2-(2-methansulfonylaminoäthansulfonylamino)-acetamido]-3-cephem-4-carbonsäure; IR: u.a. 1768 (Nujol); UV: 251 (13000; $H_2O$).

3-(2-Methyl-5,6-dioxo-1,2,5,6-tetrahydro-as-triazin-3-ylthiomethyl)-7β-[(2R,S)-2-(2-aminothiazol-4-yl)-2-(2-methansulfonylaminoäthansulfonylamino)-acetamido]-3-cephem-4-carbonsäure; IR: u.a. 1768 (Nujol); UV: 241 (19000), 271 (22500; $H_2O$).

3-Pyridiniomethyl-7β-[(2R,S)-2-(2-aminothiazol-4-yl)-2-(2-methansulfonylaminoäthansulfonylamino)-acetamido]-3-cephem-4-carboxylat; IR: u.a. 1765 (Nujol); UV: 252 (14800; $H_2O$).

3-(4-Carbamoylpyridiniomethyl)-7β-[(2R,S)-2-(2-aminothiazol-4-yl)-2-(2-methansulfonylaminoäthansulfonylamino)-acetamido]-3-cephem-4-carboxylat; IR: u.a. 1769 (Nujol); UV: 251 (15000; $H_2O$).

3-Methyl-7β-[(2R,S)-2-(2-aminothiazol-4-yl)-2-methansulfonylamino-acetamido]-3-cephem-4-carbonsäure; IR: u.a. 1768 (Nujol); UV: 252 (14400; $H_2O$).

3-Methoxy-7β-[(2R,S)-2-(2-aminothiazol-4-yl)-2-methansulfonylamino-acetamido]-3-cephem-4-carbonsäure; IR: u.a. 1770 (Nujol); UV: 250 (15000; $H_2O$).

3-Chlor-7β-[(2R,S)-2-(2-aminothiazol-4-yl)-2-methansulfonylamino-acetamido]-3-cephem-4-carbonsäure; IR: u.a. 1771 (Nujol); UV: 251 (14100; $H_2O$).

3-(1-Carboxymethyl-1H-tetrazol-5-ylthiomethyl)-7β-[(2R,S)-2-(2-aminothiazol-4-yl)-2-methansulfonylaminoacetamido]-3-cephem-4-carbonsäure; IR: u.a. 1770 (Nujol); UV: 252 (14900; $H_2O$).

3-Pyridiniomethyl-7β-[(2R,S)-2-(2-aminothiazol-4-yl)-2-methansulfonylaminoacetamido]-3-cephem-4-carboxylat; IR: u.a. 1769 (Nujol); UV: 251 (15200; $H_2O$)

3-Methyl-7β-[(2R,S)-2-(2-aminothiazol-4-yl)-2-äthansulfonylaminoacetamido]-3-cephem-4-carbonsäure; IR: u.a. 1770 (Nujol); UV: 251 (14200; $H_2O$).

3-Methoxy-7β-[(2R,S)-2-(2-aminothiazol-4-yl)-2-äthansulfonylaminoacetamido]-3-cephem-4-carbonsäure; IR: u.a. 1769 (Nujol); UV: 251 (13900; $H_2O$).

3-Chlor-7β-[(2R,S)-2-(2-aminothiazol-4-yl)-2-äthansulfonylaminoacetamido]-3-cephem-4-carbonsäure; IR: u.a. 1771 (Nujol); UV: 252 (15000; $H_2O$).

3-Acetoxymethyl-7β-[(2R,S)-2-(2-aminothiazol-4-yl)-2-äthansulfonylaminoacetamido]-3-cephem-4-carbonsäure; IR: u.a. 1669 (Nujol); UV: 253 (14000; $H_2O$).

3-(1-Methyl-1H-tetrazol-5-ylthiomethyl)-7β-[(2R,S)-2-(2-aminothiazol-4-yl)-2-äthansulfonylaminoacetamido]-3-cephem-4-carbonsäure; IR: u.a. 1770 (Nujol); UV: 251 (14200; $H_2O$).

3-(1-Carboxymethyl-1H-tetrazol-5-ylthiomethyl)-7β-[(2R,S)-2-(2-aminothiazol-4-yl)-2-äthansulfonylaminoacetamido]-3-cephem-4-carbonsäure; IR: u.a. 1770 (Nujol); UV: 250 (14500; $H_2O$).

3-(1-Sulfomethyl-1H-tetrazol-5-ylthiomethyl)-7β-[(2R,S)-2-(2-aminothiazol-4-yl)-2-äthansulfonylaminoacetamido]-3-cephem-4-carbonsäure; IR: u.a. 1768 (Nujol); UV: 251 (14700; $H_2O$).

3-[1-(2-Dimethylaminoäthyl)-1H-tetrazol-5-ylthiomethyl]-7β-[(2R,S)-2-(2-aminothiazol-4-yl)-2-äthansulfonylaminoacetamido]-3-cephem-4-carbonsäure; IR: u.a. 1768 (Nujol); UV: 252 (13900; $H_2O$).

3-(2-Methyl-5,6-dioxo-1,2,5,6-tetrahydro-as-triazin-3-ylthiomethyl)-7β-[(2R,S)-2-(2-aminothiazol-4-yl)-2-äthansulfonylaminoacetamido]-3-cephem-4-carbonsäure; IR: u.a. 1770 (Nujol); UV: 240 (18000), 270 (22000; $H_2O$).

3-Pyridiniomethyl-7β-[(2R,S)-2-(2-aminothiazol-4-yl)-2-äthansulfonylaminoacetamido]-3-cephem-4-carboxylat; IR: u.a. 1766 (Nujol); UV: 251 (14000; $H_2O$).

3-(4-Carbamoylpyridiniomethyl)-7β-[(2R,S)-2-(2-aminothiazol-4-yl)-2-äthansulfonylaminoacetamido]-3-cephem-4-carboxylat; IR: u.a. 1770 (Nujol); UV: 253 (15200; $H_2O$).

3-(2-Methyl-5,6-dioxo-1,2,5,6-tetrahydro-as-triazin-3-ylthiomethyl)-7β-[(2R,S)-2-(2-aminothiazol-4-yl)-2-(2-acetylaminoäthansulfonylamino)-acetamido]-3-cephem-4-carbonsäure; IR: u.a. 1768 (Nujol); UV: 240 (18300), 270 (21900).

3-Methyl-7β-[(2R,S)-2-(2-aminothiazol-4-yl)-2-(2-formylaminoäthansulfonylamino)-acetamido]-3-cephem-4-carbonsäure; IR: u.a. 1770 (Nujol); UV: 252 (14800; $H_2O$).

3-Methoxy-7β-[(2R,S)-2-(2-aminothiazol-4-yl)-2-(2-formylaminoäthansulfonylamino)-acetamido]-3-cephem-4-carbonsäure; IR: u.a. 1771 (Nujol); UV: 252 (14900; $H_2O$).

3-Chlor-7β-[(2R,S)-2-(2-aminothiazol-4-yl)-2-(2-formylaminoäthansulfonylamino)-acetamido]-3-cephem-4-carbonsäure; IR: u.a. 1769 (Nujol); UV: 253 (14700; $H_2O$).

3-(1-Carboxymethyl-1H-tetrazol-5-ylthiomethyl)-7β-[(2R,S)-2-(2-aminothiazol-4-yl)-2-(2-formylaminoäthansulfonylamino)-acetamido]-3-cephem-4-carbonsäure; IR: u.a. 1770 (Nujol); UV: 252 (15500; $H_2O$).

3-(1-Sulfomethyl-1H-tetrazol-5-ylthiomethyl)-7β-[(2R,S)-2-(2-aminothiazol-4-yl)-2-(2-formylaminoäthansulfonylamino)-acetamido]-3-cephem-4-carbonsäure; IR: u.a. 1771 (Nujol); UV: 253 (15300; $H_2O$).

3-[1-(2-Dimethylaminoäthyl)-1H-tetrazol-5-ylthiomethyl]-7β-[(2R,S)-2-(2-aminothiazol-4-yl)-2-(2-formylaminoäthansulfonylamino)-acetamido]-3-cephem-4-carbonsäure; IR: u.a. 1769 (Nujol); UV: 252 (15400; $H_2O$).

3-(2-Methyl-5,6-dioxo-1,2,5,6-tetrahydro-as-triazin-3-ylthiomethyl)-7β-[(2R,S)-2-(2-aminothiazol-4-yl)-2-(2-formylaminoäthansulfonylamino)-acetamido]-3-cephem-4-carbonsäure; IR: u.a. 1770 (Nujol); UV: 240 (17900), 270 (21800; $H_2O$).

3-Methyl-7β-[(2R,S)-2-(2-aminothiazol-4-yl)-2-(2-(2-aminothiazol-4-ylacetylamino)-äthansulfonylamino)-acetamido-3-cephem-4-carbonsäure; IR: u.a. 1768 (Nujol); UV: 249 (13200; $H_2O$).

3-Chlor-7β-[(2R,S)-2-(2-aminothiazol-4-yl)-2-(2-(2-aminothiazol-4-ylacetylamino)-äthansulfonylamino)-acetamido-3-cephem-4-carbonsäure; IR: u.a. 1770 (Nujol); UV: 249 (14100; $H_2O$).

3-Methoxy-7β-[(2R,S)-2-(2-aminothiazol-4-yl)-2-(2-(2-aminothiazol-4-ylacetylamino)-äthansulfonylamino)-acetamido-3-cephem-4-carbonsäure; IR: u.a. 1769 (Nujol); UV: 249 (14200; $H_2O$).

3-(1-Methyl-1H-tetrazol-5-ylthiomethyl)-7β-[(2R,S)-2-(2-aminothiazol-4-yl)-2-(2-(2-aminothiazol-4-ylacetylamino)-äthansulfonylamino)-acetamido-3-cephem-4-carbonsäure; IR: u.a. 1771 (Nujol); UV: 249 (15200; $H_2O$).

3-(1-Carboxymethyl-1H-tetrazol-5-ylthiomethyl)-7β-[(2R,S)-2-(2-aminothiazol-4-yl)-2-(2-(2-aminothiazol-4-ylacetylamino)-äthansulfonylamino)-acetamido-3-cephem-4-carbonsäure; IR: u.a. 1768 (Nujol); UV: 251 (14700; $H_2O$).

3-(1-Sulfomethyl-1H-tetrazol-5-ylthiomethyl)-7β-[(2R,S)-2-(2-aminothiazol-4-yl)-2-(2-(2-aminothiazol-4-ylacetylamino)-äthansulfonylamino)-acetamido]-3-cephem-4-carbonsäure; IR: u.a. 1767 (Nujol); UV: 251 (14900; $H_2O$).

3-[1-(2-Dimethylaminoäthyl)-1H-tetrazol-5-ylthiomethyl]-7β-[(2R,S)-2-(2-aminothiazol-4-yl)-2-(2-(2-aminothiazol-4-ylacetylamino)-äthansulfonylamino)-acetamido]-3-cephem-4-carbonsäure; IR: u.a. 1771 (Nujol); UV: 252 (15100; $H_2O$).

3-(2-Methyl-5,6-dioxo-1,2,5,6-tetrahydro-as-triazin-3-ylthiomethyl)-7β-[(2R,S)-2-(2-aminothiazol-4-yl)-2-(2-(2-aminothiazol-4-ylacetylamino)-äthansulfonylamino)-acetamido]-3-cephem-4-carbonsäure; IR: u.a. 1768 (Nujol); UV: 240 (19100), 270 (22500; $H_2O$).

3-Pyridiniomethyl-7β-[(2R,S)-2-(2-aminothiazol-4-yl)-2-(2-(2-aminothiazol-4-ylacetylamino)-äthansulfonylamino)-acetamido]-3-cephem-4-carboxylat; IR: u.a. 1768 (Nujol); UV: 251 (14400; $H_2O$).

3-(4-Carbamoylpyridiniomethyl)-7β-[(2R,S)-2-(2-aminothiazol-4-yl)-2-(2-(2-aminothiazol-4-ylacetylamino)-äthansulfonylamino)-acetamido]-3-cephem-4-carboxylat; IR: u.a. 1767 (Nujol); UV: 251 (14600; $H_2O$).

3-Methyl-7β-[(2R,S)-2-(2-aminothiazol-4-yl)-2-(2-p.nitrobenzolsulfonylaminoäthansulfonylamino)-acetamido]-3-cephem-4-carbonsäure; IR: u.a. 1767 (Nujol); UV: 251 (14900; $H_2O$).

3-Methoxy-7β-[(2R,S)-2-(2-aminothiazol-4-yl)-2-(2-p.nitrobenzolsulfonylaminoäthansulfonylamino)-acetamido]-3-cephem-4-carbonsäure; IR: u.a. 1770 (Nujol); UV: 251 (14800; $H_2O$).

3-Chlor-7β-[(2R,S)-2-(2-aminothiazol-4-yl)-2-(2-p.nitrobenzolsulfonylaminoäthansulfonylamino)-acetamido]-3-cephem-4-carbonsäure; IR: u.a. 1769 (Nujol); UV: 252 (15200; $H_2O$).

3-(2-Methyl-5,6-dioxo-1,2,5,6-tetrahydro-as-triazin-3-ylthiomethyl)-7β-[(2R,S)-2-(2-aminothiazol-4-yl)-2-(2-p.nitrobenzolsulfonylaminoäthansulfonylamino)-acetamido]-3-cephem-4-carbonsäure; IR: u.a. 1770 (Nujol); UV: 240 (18500), 270 (22000; $H_2O$).

3-Pyridiniomethyl-7β-[(2R,S)-2-(2-aminothiazol-4-yl)-2-(2-p.nitrobenzolsulfonylaminoäthansulfonylamino)-acetamido]-3-cephem-4-carboxylat; IR: u.a. 1769 (Nujol); UV: 252 (14800; $H_2O$).

3-(4-Carbamoylpyridiniomethyl)-7β-[(2R,S)-2-(2-aminothiazol-4-yl)-2-(2-p.nitrobenzolsulfonylaminoäthansulfonylamino)-acetamido]-3-cephem-4-carboxylat; IR: u.a. 1771 (Nujol); UV: 251 (14700; $H_2O$).

3-(2-Methyl-5,6-dioxo-1,2,5,6-tetrahydro-as-triazin-3-ylthiomethyl)-7β-[(2R,S)-2-(2-aminothiazol-4-yl)-2-(2-butyrylaminoäthansulfonylamino)-acetamido]-3-cephem-4-carbonsäure; IR: u.a. 1770 (Nujol); UV: 241 (17900); 271 (22500; $H_2O$).

3-(2-Methyl-5,6-dioxo-1,2,5,6-tetrahydro-as-triazin-3-ylthiomethyl)-7β-[(2R,S)-2-(2-aminothiazol-4-yl)-2-äthoxycarbonylaminoäthansulfonylamino)-acetamido]-3-cephem-4-carbonsäure; IR: u.a. 1769 (Nujol); UV: 240 (18000); 270 (22100; $H_2O$).

3-Methyl-7β-[(2R,S)-2-(2-aminothiazol-4-yl)-2-(2-methoxyäthansulfonylamino)-acetamido]-3-cephem-4-carbonsäure; IR: u.a. 1770 (Nujol); UV: 251 (14000; $H_2O$).

3-Methoxy-7β-[(2R,S)-2-(2-aminothiazol-4-yl)-2-(2-methoxyäthansulfonylamino)-acetamido]-3-cephem-4-carbonsäure; IR: u.a. 1769 (Nujol); UV 251 (14100; $H_2O$).

3-Chlor-7β-[(2R,S)-2-(2-aminothiazol-4-yl)-2-(2-methoxyäthansulfonylamino)-acetamido]-3-cephem-4-carbonsäure; IR: u.a. 1770 (Nujol); UV: 252 (15000; $H_2O$).

3-Acetoxymethyl-7β-[(2R,S)-2-(2-aminothiazol-4-yl)-2-(2-methoxyäthansulfonylamino)-acetamido]-3-cephem-4-carbonsäure; IR: u.a. 1771 (Nujol); UV: 251 (15100; $H_2O$).

3-(1-Carboxymethyl-1H-tetrazol-5-ylthiomethyl)-7β-[(2R,S)-2-(2-aminothiazol-4-yl)-2-(2-methoxyäthansulfonylamino)-acetamido]-3-cephem-4-carbonsäure; IR: u.a. 1769 (Nujol); UV: 250 (14700; $H_2O$).

3-(1-Sulfomethyl-1H-tetrazol-5-ylthiomethyl)-7β-[(2R,S)-2-(2-aminothiazol-4-yl)-2-(2-methoxyäthansulfonylamino)-acetamido]-3-cephem-4-carbonsäure; IR: u.a. 1771 (Nujol); UV: 251 (15100; $H_2O$).

3-[1-(2-Dimethylaminoäthyl)-1H-tetrazol-5-ylthiomethyl]-7β-[(2R,S)-2-(2-aminothiazol-4-yl)-2-(2-methoxyäthansulfonylamino)-acetamido]-3-cephem-4-carbonsäure; IR: u.A. 1770 (Nujol); UV: 252 (14800; $H_2O$).

3-(2-Methyl-5,6-dioxo-1,2,5,6-tetrahydro-as-triazin-3-ylthiomethyl)-7β-[(2R,S)-2-(2-aminothiazol-4-yl)-2-(2-methoxyäthansulfonylamino)-acetamido]-3-cephem-4-carbonsäure; IR: u.a. 1769 (Nujol); UV: 241 (17700), 272 (21800; $H_2O$).

3-Pyridiniomethyl-7β-[(2R,S)-2-(2-aminothiazol-4-yl)-2-(2-methoxyäthansulfonylamino)-acetamido]-3-cephem-4-carboxylat; IR: u.a. 1771 (Nujol); UV: 250 (15000; $H_2O$).

3-(4-Carbamoylpyridiniomethyl)-7β-[(2R,S)-2-(2-aminothiazol-4-yl)-2-(2-Methoxyäthansulfonylamino)-acetamido]-3-cephem-4-carboxylat; IR: u.a. 1771 (Nujol); 252 (14800; $H_2O$).

3-Methyl-7β-[(2R,S)-2-(2-aminothiazol-4-yl)-2-cyanomethansulfonylaminoacetamido]-3-cephem-4-carbonsäure; IR: u.a. 2260, 1770 (Nujol); UV: 250 (9400; $H_2O$).

3-Methoxy-7β-[(2R,S)-2-(2-aminothiazol-4-yl)-2-cyanomethansulfonylaminoacetamido]-3-cephem-4-carbonsäure; IR: u.a. 2260; 1769 (Nujol); UV: 251 (10100; $H_2O$).

3-Chlor-7β-[(2R,S)-2-(2-aminothiazol-4-yl)-2-cyanomethansulfonylaminoacetamido]-3-cephem-4-carbonsäure; IR: u.a. 2260, 1771 (Nujol); UV: 250 (9900; $H_2O$).

3-Acetoxymethyl-7β-[(2R,S)-2-(2-aminothiazol-4-yl)-2-cyanomethansulfonylaminoacetamido]-3-cephem-4-carbonsäure; IR: u.a. 2260; (Nujol); UV: 250 (9700; $H_2O$).

3-(2-Methyl-5,6-dioxo-1,2,5,6-tetrahydro-as-triazin-3-ylthiomethyl)-7β-[(2R,S)-2-(2-aminothiazol-4-yl)-2-cyanomethansulfonylaminoacetamido]-3-cephem-4-carbonsäure; IR: u.a. 2260, 1771 (Nujol); UV: 240 (19000), 270 (22000; $H_2O$).

3-Carbamoyloxymethyl-7β-[(2R,S)-2-(2-aminothiazol-4-yl)-2-cyanomethansulfonylaminoacetamido]-3-cephem-4-carbonsäure; IR: u.a. 2260, 1770 (Nujol); UV: 251 (9000; $H_2O$).

3-Pyridiniomethyl-7β-[(2R,S)-2-(2-aminothiazol-4-yl)-2-cyanomethansulfonylaminoacetamido]-3-cephem-4-carboxylat; IR: u.a. 2260, 1769 (Nujol); UV: 250 (10000; $H_2O$).

3-(4-Carbamoylpyridiniomethyl)-7β-[(2R,S)-2-(2-aminothiazol-4-yl)-2-cyanomethansulfonylaminoacetamido]-3-cephem-4-carboxylat; IR: u.a. 2260, 1771 (Nujol); UV: 250 (9800; $H_2O$).

3-Methyl-7β-[(2R,S)-2-(2-aminothiazol-4-yl)-2-(2-acryloylaminoäthansulfonylamino)-acetamido]-3-cephem-4-carbonsäure; IR: u.a. 1770 (Nujol); UV: 250 (14800; $H_2O$).

3-Methoxy-7β-[(2R,S)-2-(2-aminothiazol-4-yl)-2-(2-acryloylaminoäthansulfonylamino)-acetamido]-3-cephem-4-carbonsäure; IR: u.a. 1769 (Nujol); UV: 251 (14900; $H_2O$).

3-Chlor-7β-[(2R,S)-2-(2-aminothiazol-4-yl)-2-(2-acryloylaminoäthansulfonylamino)-acetamido]-3-cephem-4-carbonsäure; IR: u.a. 1771 (Nujol); UV: 252 (13800; $H_2O$).

3-Acetoxymethyl-7β-[(2R,S)-2-(2-aminothiazol-4-yl)-2-(2-acryloylaminoäthansulfonylamino)-acetamido]-3-cephem-4-carbonsäure; IR: u.a. 1770 (Nujol); UV: 251 (13300, $H_2C$).

3-(2-Methyl-5,6-dioxo-1,2,5,6-tetrahydro-as-triazin-3-ylthiomethyl)-7β-[(2R,S)-2-(2-aminothiazol-4-yl)-2-(2-acryloylaminoäthansulfonylamino)-acetamido]-3-cephem-4-carbonsäure; IR: u.a. 1769 (Nujol); UV: 240 (17800); 271 (22400; $H_2O$).

3-Pyridiniomethyl-7β-[(2R,S)-2-(2-aminothiazol-4-yl)-2-(2-acryloylaminoäthansulfonylamino)-acetamido]-3-cephem-4-carboxylat; IR: u.a. 1770 (Nujol); UV: 251 (14000; $H_2O$).

3-(4-Carbamoylpyridinomethyl)-7β-[(2R,S)-2-(2-aminothiazol-4-yl)-2-(2-acryloylaminoäthansulfonylamino)-acetamido]-3-cephem-4-carboxylat; IR: u.a. 1768 (Nujol); UV: 249 (16200; $H_2O$).

3-(1-Methyl-1H-tetrazol-5-ylthiomethyl)-7β-[(2R,S)-2-(2-aminothiazol-4-yl)-2-(2-cyclopropylcarbonylaminoäthansulfonylamino)-acetamido]-3-cephem-4-carbonsäure; IR: u.a. 1769 (Nujol); UV: 255 (14200; $H_2O$).

3-(2-Methyl-5,6-dioxo-1,2,5,6-tetrahydro-as-triazin-3-ylthiomethyl)-7β-[(2R,S)-2-(2-aminothiazol-4-yl)-2-(2-cyclopropylcarbonylaminoäthansulfonylamino)-acetamido]-3-cephem-4-carbonsäure; IR: u.a. 1770; UV: 241 (18000), 271 (22000; $H_2O$).

3-Carbamoyloxymethyl-7β-[(2R,S)-2-(2-aminothiazol-4-yl)-2-(2-cyclopropylcarbonylaminoäthansulfonylamino)-acetamido]-3-cephem-4-carbonsäure; IR: u.a. 1769 (Nujol); UV: 251 (15200; $H_2O$).

3-Methyl-7β-[(2R,S)-2-(2-aminothiazol-4-yl)-2-(2-cyanoacetylaminoäthansulfonylamino)-acetamido]-3-cephem-4-carbonsäure; IR: u.a. 1770 (Nujol); UV: 251 (14200; $H_2O$).

3-Methoxy-7β-[(2R,S)-2-(2-aminothiazol-4-yl)-2-(2-cyanoacetylaminoäthansulfonylamino)-acetamido]-3-cephem-4-carbonsäure; IR: u.a. 1769 (Nujol); UV: 252 (14100, $H_2O$).

3-Chlor-7β-[(2R,S)-2-(2-aminothiazol-4-yl)-2-(2-cyanoacetylaminoäthansulfonylamino)-acetamido]-3-cephem-4-carbonsäure; IR: u.a. 1771 (Nujol); UV: 252 (15100; $H_2O$).

3-Acetoxymethyl-7β-[(2R,S)-2-(2-aminothiazol-4-yl)-2-(2-cyanoacetylaminoäthansulfonylamino)-acetamido]-3-cephem-4-carbonsäure; IR: u.a. 1770 (Nujol); UV: 253 (14800; $H_2O$).

3-(1-Methyl-1H-tetrazol-5-ylthiomethyl)-7β-[(2R,S)-2-(2-aminothiazol-4-yl)-2-(2-cyanoacetylaminoäthansulfonylamino)-acetamido]-3-cephem-4-carbonsäure; IR: u.a. 1770 (Nujol); UV: 253 (14400; $H_2O$).

3-(2-Methyl-5,6-dioxo-1,2,5,6-tetrahydro-as-triazin-3-ylthiomethyl)-7β-[(2R,S)-2-(2-aminothiazol-4-yl)-2-(2-cyanoacetylaminoäthansulfonylamino)-acetamido]-3-cephem-4-carbonsäure; IR: u.a. 1770 (Nujol); UV: 241 (19000), 272 (21000; $H_2O$).

3-Carbamoyloxymethyl-7β-(2R,S)-2-(2-aminothiazol-4-yl)-2-(2-cyanoacetylaminoäthansulfonylamino)-acetamido]-3-cephem-4-carbonsäure; IR: u.a. 1769 (Nujol); UV: 251 (11000; $H_2O$).

3-Pyridiniomethyl-7β-[(2R,S)-2-(2-aminothiazol-4-yl)-2-(2-cyanoacetylaminoäthansulfonylamino)-acetamido]-3-cephem-4-carboxylat; IR: u.a. 1776 (Nujol); UV: 250 (12100; $H_2O$).

3-(4-Carbamoylpyridiniomethyl)-7β-[(2R,S)-2-(2-aminothiazol-4-yl)-2-(2-cyanoacetylaminoäthansulfonylamino)-acetamido]-3-cephem-4-carboxylat; IR: u.a. 1771 (Nujol); UV: 253 (16100; $H_2O$).

3-Methyl-7β-[(2R,S)-2-(2-aminothiazol-4-yl)-2-(2-methoxyacetylaminoäthansulfonylamino)-acetamido]-3-cephem-4-carbonsäure; IR: u.a. 1769 (Nujol); UV: 251 (14200; $H_2O$).

3-Methoxy-7β-[(2R,S)-2-(2-aminothiazol-4-yl)-2-(2-methoxyacetylaminoäthansulfonylamino)-acetamido]-3-cephem-4-carbonsäure; IR: u.a. 1770 (Nujol); UV: 250 (14300; $H_2O$).

3-Chlor-7β-[(2R,S)-2-(2-aminothiazol-4-yl)-2-(2-methoxyacetylaminoäthansulfonylamino)-acetamido]-3-cephem-4-carbonsäure; IR: u.a. 1771 (Nujol); UV: 251 (13900; $H_2O$).

3-Acetoxymethyl-7β-[(2R,S)-2-(2-aminothiazol-4-yl)-2-(2-methoxyacetylaminoäthansulfonylamino)-acetamido]-3-cephem-4-carbonsäure; IR: u.a. 1770 (Nujol); UV: 251 (14800; $H_2O$).

3-(2-Methyl-5,6-dioxo-1,2,5,6-tetrahydro-as-triazin-3-ylthiomethyl)-7β-[(2R,S)-2-(2-aminothiazol-4-yl)-2-(2-methoxyacetylaminoäthansulfonylamino)-acetamido]-3-cephem-4-carbonsäure; IR: u.a. 1771 (Nujol); UV: 240 (17700), 270 (20900; $H_2O$).

3-Pyridiniomethyl-7β-[(2R,S)-2-(2-aminothiazol-4-yl)-2-(2-methoxyacetylaminoäthansulfonylamino)-acetamido]-3-cephem-4-carboxylat; IR: u.a. 1770 (Nujol); UV: 251 (14800; $H_2O$).

3-(4-Carbamoylpyridiniomethyl)-7β-[(2R,S)-2-(2-aminothiazol-4-yl)-2-(2-methoxyacetylaminoäthansulfonylamino)-acetamido]-3-cephem-4-carboxylat; IR: u.a. 1771 (Nujol); UV: 251 (14800; $H_2O$).

3-(2-Methyl-5,6-dioxo-1,2,5,6-tetrahydro-as-triazin-3-ylthiomethyl)-7β-[(2R,S)-2-(2-aminothiazol-4-yl)-2-(2-propiolylaminoäthansulfonylamino)-acetamido]-3-cephem-4-carbonsäure; IR: u.a. 2120, 1760 (Nujol); UV: 241 (17300), 271 (22400; $H_2O$).

3-Carbamoyloxymethyl-7β-[(2R,S)-2-(2-aminothiazol-4-yl)-2-(2-propioloylaminoäthansulfonylamino)-acetamido]-3-cephem-4-carbonsäure; IR: u.a. 2120, 1764 (Nujol); UV: 249 (9900; $H_2O$).

3-Methyl-7β-[(2R,S)-2-(2-aminothiazol-4-yl)-2-(2-methylaminoäthansulfonylamino)-acetamido]-3-cephem-4-carbonsäure; IR: u.a. 1768 (Nujol): UV: 249 (11100; $H_2O$).

3-Methoxy-7β-[(2R,S)-2-(2-aminothiazol-4-yl)-2-(2-methylaminoäthansulfonylamino)-acetamido]-3-cephem-4-carbonsäure; IR: u.a. 1767 (Nujol); UV: 248 (12100; $H_2O$).

3-Chlor-7β-[(2R,S)-2-(2-aminothiazol-4-yl)-2-(2-methylaminoäthansulfonylamino)-acetamido]-3-cephem-4-carbonsäure; IR: u.a. 1768 (Nujol); UV: 249 (12400; $H_2O$).

3-Acetoxymethyl-7β-[(2R,S)-2-(2-aminothiazol-4-yl)-2-(2-methylaminoäthansulfonylamino)-acetamido]-3-cephem-4-carbonsäure; IR: u.a. 1765 (Nujol); UV: 249 (14200; $H_2O$).

3-(1-Carboxymethyl-1H-tetrazol-5-ylthiomethyl)-7β-[(2R,S)-2-(2-aminothiazol-4-yl)-2-(2-methylaminoäthansulfonylamino)-acetamido]-3-cephem-4-carbonsäure; IR: u.a. 1770 (Nujol); UV: 251 (14200; $H_2O$).

3-(1-Sulfomethyl-1H-tetrazol-5-ylthiomethyl)-7β-[(2R,S)-2-(2-aminothiazol-4-yl)-2-(2-methylaminoäthansulfonylamino)-acetamido]-3-cephem-4-carbonsäure; IR: u.a. 1770 (Nujol); UV: 249 (9900; $H_2O$).

3-[1-(2-Dimethylaminoäthyl)-1H-tetrazol-5-ylthiomethyl]-7β-[(2R,S)-2-(2-aminothiazol-4-yl)-2-(2-methylaminoäthansulfonylamino)-acetamido]-3-cephem-4-carbonsäure; IR: u.a. 1769 (Nujol); UV: 248 (10300; $H_2O$).

3-(2-Methyl-5,6-dioxo-1,2,5,6-Tetrahydro-as-triazin-3-ylthiomethyl)-7β-[(2R,S)-2-(2-aminothiazol-4-yl)-2-(2-methylaminoäthansulfonylamino)-acetamido]-3-cephem-4-carbonsäure; IR: u.a. 1770 (Nujol); UV: 240 (17700), 271 (21900; $H_2O$).

3-Pyridiniomethyl-7β-[(2R,S)-2-(2-aminothiazol-4-yl)-2-(2-methylaminoäthansulfonylamino)-acetamido]-3-cephem-4-carboxylat; IR: u.a. 1770 (Nujol); UV: 249 (14000; $H_2O$).

3-(4-Carbamoylpyridiniomethyl)-7β-[(2R,S)-2-(2-aminothiazol-4-yl)-2-(2-methylaminoäthansulfonylamino)-acetamido]-3-cephem-4-carboxylat; IR: u.a. 1769 (Nujol); UV: 250 (14300; $H_2O$).

3-Methyl-7β-[(2R,S)-2-(2-aminothiazol-4-yl)-2-(2-(2,4-dinitrobenzolsulfonylamino)-äthansulfonylamino)-acetamido]-3-cephem-4-carbonsäure; IR: u.a. 1768 (Nujol); UV: 251 (14100; $H_2O$).

3-Methoxy-7β-[(2R,S)-2-(2-aminothiazol-4-yl)-2-(2-(2,4-dinitrobenzolsulfonylamino)-äthansulfonylamino)-acetamido]-3-cephem-4-carbonsäure; IR; u.a. 1765 (Nujol); UV: 251 (13800; $H_2O$).

3-Chlor-7β-[(2R,S)-2-(2-aminothiazol-4-yl)-2-(2-(2,4-dinitrobenzolsulfonylamino)-äthansulfonylamino)-acetamido]-3-cephem-4-carbonsäure; IR: u.a. 1765 (Nujol); UV: 249 (13900; $H_2O$).

3-Acetoxymethyl-7β-[(2R,S)-2-(2-aminothiazol-4-yl)-2-(2-(2,4-dinitrobenzolsulfonylamino)-äthansulfonylamino)-acetamido]-3-cephem-4-carbonsäure; IR; u.a. 1765 (Nujol); UV: 251 (14600; $H_2O$).

3-(2-Methyl-5,6-dioxo-1,2,5,6-tetrahydro-as-triazin-3-ylthiomethyl)-7β-[(2R,S)-2-(2-aminothiazol-4-yl)-2-(2-(2,4-dinitrobenzolsulfonylamino)-äthansulfonylamino)-acetamido]-3-cephem-4-carbonsäure; IR: u.a. 1768 (Nujol); UV: 241 (16900); 270 (20100; $H_2O$).

3-Pyridiniomethyl-7β-[(2R,S)-2-(2-aminothiazol-4-yl)-2-(2-(2,4-dinitrobenzolsulfonylamino)-äthansulfonylamino)-acetamido]-3-cephem-4-carboxylat; IR: u.a. 1768 (Nujol); UV: 250 (14900; $H_2O$).

3-(4-Carbamoylpyridiniomethyl)-7β-[(2R,S)-2-(2-aminothiazol-4-yl)-2-(2-(2,4-dinitrobenzolsulfonylamino)-äthansulfonylamino)-acetamido]-3-cephem-4-carboxylat; IR: u.a. 1768 (Nujol); UV 250 (15200; ($H_2O$).

3-(1-Methyl-1H-tetrazol-5-ylthiomethyl)-7β-[(2R,S)-2-(2-aminothiazol-4-yl)-2-(2-(2-cyanomethansulfonylamino)-äthansulfonylamino)-acetamido]-3-cephem-4-carbonsäure; IR: u.a. 1768 (Nujol); UV: 251 (14300, $H_2O$).

3-(2-Methyl-5,6-dioxo-1,2,5,6-tetrahydro-as-triazin-3-ylthiomethyl)-7β-[(2R,S)-2-(2-aminothiazol-4-yl)-2-(2-(2-cyanomethansulfonylamino)-äthansulfonylamino)-acetamido]-3-cephem-4-carbonsäure; IR: u.a. 1770 (Nujol); UV 240 (19000), 280 (21100; $H_2O$).

3-Carbamoyloxymethyl-7β-[(2R,S)-2-(2-aminothiazol-4-yl)-2-(2-(2-cyanomethansulfonylamino)-äthansulfonylamino)-acetamido]-3-cephem-4-carbonsäure; IR: u.a. 1768 (Nujol); UV: 249 (14800; $H_2O$).

3-Methyl-7β-[(2R,S)-2-(2-aminothiazol-4-yl)-2-vinylsulfonylamino-acetamido]-3-cephem-4-carbonsäure; IR: u.a. 1768 (Nujol); UV: 251 (14200; $H_2O$).

3-Methoxy-7β-[(2R,S)-2-(2-aminothiazol-4-yl)-2-vinylsulfonylamino-acetamido]-3-cephem-4-carbonsäure; IR: u.a. 1768 (Nujol); UV: 250 (14900; $H_2O$).

3-Chlor-7β-[(2R,S)-2-(2-aminothiazol-4-yl)-2-vinylsulfonylamino-acetamido]-3-cephem-4-carbonsäure; IR: u.a. 1767 (Nujol); UV: 251 (13900; $H_2O$).

3-Acetoxymethyl-7β-[(2R,S)-2-(2-aminothiazol-4-yl)-2-vinylsulfonyl-aminoacetamido]-3-cephem-4-carbonsäure; IR: u.a. 1768 (Nujol); UV: 252 (14100; $H_2O$).

3-(1-Methyl-1H-tetrazol-5-ylthiomethyl)-7β-[(2R,S)-2-(2-aminothiazol-4-yl)-2-vinylsulfonylaminoacetamido]-3-cephem-4-carbonsäure; IR: u.a. 1768 (Nujol); UV; 251 (14400; $H_2O$).

3-(2-Methyl-5,6-dioxo-1,2,5,6-tetrahydro-as-triazin-3-ylthiomethyl)-7β-[(2R,S)-2-(2-aminothiazol-4-yl)-2-vinyl-sulfonylaminoacetamido]-3-cephem-4-carbonsäure; IR: u.a. 1768 (Nujol); UV: 241 (18900), 280 (22200; $H_2O$).

3-Carbamoyloxymethyl-7β-[(2R,S)-2-(2-aminothiazol-4-yl)-2-vinylsulfonylaminoacetamido]-3-cephem-4-carbonsäure; IR: u.a. 1765 (Nujol); UV: 251 (14000; $H_2O$).

3-Pyridiniomethyl-7β-[(2R,S)-2-(2-aminothiazol-4-yl)-2-vinylsulfonylaminoacetamido]-3-cephem-4-carbonsäure; IR: u.a. 1766 (Nujol); UV: 252 (14100; $H_2O$).

3-(4-Carbamoylpyridiniomethyl)-7β-[(2R,S)-2-(2-aminothiazol-4-yl)-2-vinylsulfonylaminoacetamido]-3-cephem-4-carbonsäure; IR: u.a. 1767 (Nujol); UV: 251 (15000; $H_2O$).

3-Methyl-7β-[(2R,S)-2-(2-aminothiazol-4-yl)-2-(2-(3-pyridylsulfonylamino)-äthansulfonylamino)-acetamido]-3-cephem-4-carbonsäure; IR: u.a. 1770 (Nujol); UV: 251 (13800; $H_2O$).

3-Methoxy-7β-[(2R,S)-2-(2-aminothiazol-4-yl)-2-(2-(3-pyridylsulfonylamino)-äthansulfonylamino)-acetamido]-3-cephem-4-carbonsäure; IR: u.a. 1769 (Nujol); UV: 252 (14100; $H_2O$).

3-Chlor-7β-[(2R,S)-2-(2-aminothiazol-4-yl)-2-(2-(3-pyridylsulfonylamino)-äthansulfonylamino)-acetamido]-3-cephem-4-carbonsäure; IR: u.a. 1770 (Nujol); UV: 251 (13800; $H_2O$).

3-Acetoxymethyl-7β-[(2R,S)-2-(2-aminothiazol-4-yl)-2-(2-(3-pyridylsulfonylamino)-äthansulfonylamino)-acetamido]-3-cephem-4-carbonsäure; IR: u.a. 1770 (Nujol); UV: 250 (14800; $H_2O$).

3-(1-Methyl-1H-tetrazol-5-ylthiomethyl)-7β-[(2R,S)-2-(2-aminothiazol-4-yl)-2-(2-(3-pyridylsulfonylamino)-äthansulfonylamino)-acetamido]-3-cephem-4-carbonsäure; IR: u.a. 1769 (Nujol); UV: 251 (15100; $H_2O$).

3-(2-Methyl-5,6-dioxo-1,2,5,6-tetrahydro-as-triazin-3-ylthiomethyl)-7β-[(2R,S)-2-(2-aminothiazol-4-yl)-2-(2-(3-pyridylsulfonylamino)-äthansulfonylamino)-acetamido]-3-cephem-4-carbonsäure; IR: u.a. 1768 (Nujol); UV: 241 (18900); 270 (21500; $H_2O$).

3-Carbamoyloxymethyl-7β-[(2R,S)-2-(2-aminothiazol-4-yl)-2-(2-(3-pyridylsulfonylamino)-äthansulfonylamino)-acetamido]-3-cephem-4-carbonsäure; IR: u.a. 1768 (Nujol); UV: 251 (13800; $H_2O$).

3-(4-Carbamoylpyridiniomethyl)-7β-[(2R,S)-2-(2-aminothiazol-4-yl)-2-(2-(3-pyridylsulfonylamino)-äthansulfonylamino)-acetamido]-3-cephem-4-carbonsäure; IR: 1768 (Nujol); UV: 251 (13800; $H_2O$).

3-(1-Methyl-1H-tetrazol-5-ylthiomethyl)-7β-[(2R,S)-2-(2-aminothiazol-4-yl)-2-(2-methylcarbamoylaminoäthansulfonylamino)-acetamido]-3-cephem-4-carbonsäure; IR: u.a. 1770 (Nujol); UV: 251 (15200; $H_2O$)

7β-[(2R,S)-2-(2-Aminothiazol-4-yl)-2-(2-dimethylaminoäthansulfonylamino)-acetamido]-3-cephem-4-carbonsäure; IR: u.a. 1770 (Nujol); UV: 251 (14000; $H_2O$).

3-Carbamoyloxymethyl-7β-[(2R,S)-2-(2-aminothiazol-4-yl)-2-(2-acetylmethylaminoäthansulfonylamino)-acetamido]-3-cephem-4-carbonsäure; IR: u.a. 1770 (Nujol); UV: 252 (14300; $H_2O$).

3-Carbamoyloxymethyl-7β-[(2R,S)-2-(2-aminothiazol-4-yl)-2-vinylsulfonylaminoacetamido]-3-cephem-4-carbonsäurepivaloyloxymethylester.

7β-[(2R,S)-2-(2-Aminothiazol-4-yl)-2-(2-dimethylaminoäthansulfonylamino)-acetamido]-3-cephem-4-carbonsäure-natriumsalz.

7β-[(2R,S)-2-(2-Aminothiazol-4-yl)-2-(2-guanidinoäthansulfonyl-amino)-acetamido]-3-cephem-4-carbonsäure.

7β-[(2R,S)-2-(2-Aminothiazol-4-yl)-2-(2-ureidoäthansulfonylamino)-acetamido]-3-cephem-4-carbonsäurenatriumsalz.

7β-[(2R,S)-2-(2-Aminothiazol-4-yl)-2-(2-thioureidoäthansulfonyl-amino)-acetamido]-3-cephem-4-carbonsäurenatriumsalz.

7β-[(2R,S)-2-(2-Aminothiazol-4-yl)-2-(2-(N-(methoxycarbonylimino-methoxycarbonylamino)-methylamino)-äthansulfonylamino)-acetamido]-3-cephem-4-carbonsäurenatriumsalz.

7β-[(2R,S)-2-(2-Aminothiazol-4-yl)-2-(2-(4-pyridyl)-äthansulfonyl-amino)-acetamido]-3-cephem-4-carbonsäurenatriumsalz,

sowie die R- und die S-Derivate, die Salze, z.B. die Natriumsalze, und die unter physiologischen Bedingungen spaltbaren Ester, z.B. die Pivaloyloxymethyl-, 2-Propionyloxyäthyl-, Aethoxycarbonyloxy-äthyl- oder tert.-Butyloxycarbonyloxymethylester.

Beispiel 141: Trockenampullen oder Vialen enthaltend 0,5 g 3-(1-Methyl-1H-tetrazol-5-ylthiomethyl)-7β-[(2R,S)-2-(2-aminothiazol-4-yl)-2-(2-aminoäthansulfonylamino)-acetamido]-3-cephem-4-carbonsäure-natriumsalz werden folgendermassen hergestellt:

| | |
|---|---|
| 3-(1-Methyl-1H-tetrazol-5-ylthiomethyl)-7β-[(2R,S)-2-(2-aminothiazol-4-yl)-2-(2-amino-äthansulfonylamino)-acetamido]-3-cephem-4-carbonsäurenatriumsalz | 0,5 g |
| Mannit | 0,05 g |

Eine sterile wässrige Lösung von 3-(1-Methyl-1H-tetrazol-5-yl-thiomethyl)-7β-[(2R,S)-2-(2-aminothiazol-4-yl)-2-(2-aminoäthansulfonylamino)-acetamido]-3-cephem-4-carbonsäurenatriumsalz wird unter aseptischen Bedingungen in 5 ml Ampullen oder Vialen verschlossen und geprüft.

Auf gleicher Weise können die in den anderen Beispielen genannten Verbindungen in Trockenampullen oder Vialen abgefüllt werden.

Beispiel 142: 7β-[(2R,S)-2-(2-Aminothiazol-4-yl)-2-pivaloyloxymethoxycarbonylmethansulfonylaminoacetamido]-3-cephem-4-carbonsäurepivaloyloxymethylester

1,3 g 7β-[(2R,S)-2-(Aminothiazol-4-yl)-2-carboxymethansulfonylaminoacetamido]-3-cephem-4-carbonsäuredinatriumsalz (Herstellung siehe Beispiel 137) und 1,3 ml Jodmethylpivalat werden in 13 ml Dimethylformamid und 5 ml Methanol analog Beispiel 70a) umgesetzt, aufgearbeitet und in das Hydrochlorid überführt. Man erhält die Titelverbindung; F: oberhalb 160° (unter Zersetzung); $[\alpha]_D^{20°} = +29° \pm 1°$ (0,92 % in DMSO); IR: 3650 - 2400 (breit), 1785 (Schulter), 1755, 1695, 1650 (Schulter), 1630, 1530 (Nujol); UV: 260 (9000; $CH_3OH$).

Beispiel 143:

a) 3-Carbamoyloxymethyl-7β-[(2R,S)-2-(2-aminothiazol-4-yl)-2-methoxycarbonylmethansulfonylaminoacetamido]-3-cephem-4-carbonsäurenatriumsalz

3,4 g des gemäss Beispiel 143 b) herstellbaren 3-Carbamoyloxymethyl-7β-[(2R,S)-2-(2-BOC-aminothiazol-4-yl)-2-methoxycarbonylmethansulfonylaminoacetamido]-3-cephem-4-carbonsäurediphenylmethylesters werden in 6 ml $CH_2Cl_2$ und 0,98 ml Anisol mit 10 ml Trifluoressigsäure analog Beispiel 1a) umgesetzt, aufgearbeitet, chromatographiert und umgefällt. Man erhält das Hydrat der Titelverbindung; F: oberhalb 210° (unter

Zersetzung); $[\alpha]_D^{20°} = 158° \pm 1°$ (0,89 % in $H_2O$); IR: 3650 - 2500 (breit), 1750, 1710 (Schulter), 1677, 1610, 1520 (Nujol); UV: 258 (12800; $H_2O$).

b) 3-Carbamoyloxymethyl-7β-[(2R,S)-2-(2-BOC-aminothiazol-4-yl)-2-methoxycarbonylmethansulfonylaminoacetamido]-3-cephem-4-carbonsäure-diphenylmethylester

10,2 g der gemäss Beispiel 134 c) erhältlichen (2R,S)-2-(2-BOC-Aminothiazol-4-yl)-2-methoxycarbonylmethansulfonylamino-essigsäure werden mit 10,9 g 3-Carbamoyloxymethyl-7β-amino-3-cephem-4-carbonsäurediphenylmethylester in 100 ml Tetrahydrofuran analog Beispiel 6b) umgesetzt (3,3 g Hydroxybenztriazol; dreimal 1,74 g Dicyclohexylcarbodiimid in je 6,66 ml Tetrahydrofuran), aufgearbeitet und chromatographiert. Man erhält die Titelverbindung; $[\alpha]_D^{20°} = 0° \pm 1°$ (0,94 % in $CHCl_3$); IR: 3525, 3420, 3400 (Schulter), 3300, 1787, 1730, 1700 (Schulter), 1581, 1542 ($CH_2Cl_2$); UV: 259 (15400; $CHCl_3$).

Beispiel 144: 3-(1,2,3-Triazol-5-ylthiomethyl)-7β-[(2R,S)-2-(2-aminothiazol-4-yl)-2-(2-methansulfonylamino)-äthansulfonylaminoacetamido]-3-cephem-4-carbonsäurenatriumsalz

Eine Aufschlämmung von 1,52 g (2,4 mMol) 3-Acetoxymethyl)-7β-[(2R,S)-2-(2-aminothiazol-4-yl)-2-(2-methansulfonylamino)-äthansulfonylaminoacetamido]-3-cephem-4-carbonsäurenatriumsalz und 0,590 g (4,8 mMol) 5-Mercapto-1,2,3-triazol in 7 ml Wasser wird auf pH 7 gestellt, während 5 Stunden bei 70° gerührt, anschliessend auf 0° abgekühlt und in 300 ml Aethanol eingetragen. Der gebildete Niederschlag wird abfiltriert, in 10 ml Wasser gelöst und an 25 g silyliertem Kieselgel (Antec Opti- $UPC_{12}$) mit Wasser als Eluiermittel gereinigt, woraus die Titelverbindung in Form des Dihydrats erhalten wird. Smp. ab 131° (unter Zersetzung); Rf: ca. 0,75 (Silicagel Opti UP $C_{12}$, UV 366, Acetonitril-Wasser 1:4).

Patentansprüche

(für alle benannten Länder ausser Oesterreich)

1. 7β-Acylamido-3-cephem-4-carbonsäure-Verbindungen der Formel

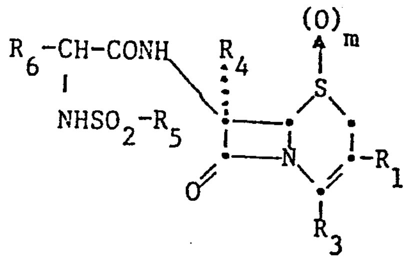

(I),

worin m eine ganze Zahl von 0 bis 2,

$R_1$ Wasserstoff, Niederalkyl, Niederalkenyl, Niederalkoxy, Halogen, eine Gruppe der Formel $-CH_2-R_2$, worin $R_2$ eine freie, veresterte oder verätherte Hydroxy- oder Mercaptogruppe oder eine Ammoniogruppe darstellt, oder eine Gruppe der Formel $-CH=CHR_2$, worin $R_2$ eine verätherte Mercaptogruppe darstellt,

$R_3$ Carboxy oder geschütztes Carboxy,

$R_4$ Wasserstoff,

$R_5$ einen organischen Rest, welcher mit einem Kohlenstoffatom an die Sulfonylgruppe gebunden ist, und

$R_6$ einen heterocyclischen Rest darstellen,

Stereoisomere, Mischungen von diesen Stereoisomeren, Hydrate und Salze von Verbindungen der Formel I.

2. 7β-Acylamido-3-cephem-4-carbonsäure-Verbindungen der Formel I, worin m eine ganze Zahl von 0 bis 2, $R_1$ Wasserstoff, Niederalkyl, Halogen oder eine Gruppe der Formel $-CH_2-R_2$, worin eine freie, veresterte oder verätherte Hydroxy- oder Mercaptogruppe oder eine Ammoniogruppe darstellt, $R_3$ Carboxy oder geschütztes Carboxy, $R_4$ Wasserstoff, $R_5$ einen organischen Rest, welcher mit einem Kohlenstoffatom an die Sulfonylgruppe gebunden ist, und $R_6$ einen heterocyclischen Rest darstellen, Stereoisomere, Mischungen von Stereoisomeren, Hydrate und

Salze von Verbindungen der Formel I.

3. 7β—Acylamido-3-cephem-4-carbonsäure-Verbindungen der Formel I, worin m eine ganze Zahl von 0 bis 2, $R_1$ Wasserstoff, Niederalkyl, Halogen oder eine Gruppe der Formel $-CH_2-R_2$, worin $R_2$ eine freie, veresterte oder verätherte Hydroxy- oder Mercaptogruppe oder eine Ammoniogruppe darstellt, $R_3$ Carboxy oder geschütztes Carboxy, $R_4$ Wasserstoff, $R_5$ einen organischen Rest, welcher mit einem Kohlenstoffatom an die Sulfonylgruppe gebunden ist, und $R_6$ einen heterocyclischen Rest darstellen, Stereoisomere, Mischungen von Stereoisomeren, Hydrate und Salze von Verbindungen der Formel I.

4. Verbindungen nach Anspruch 1 der Formel I, worin m eine ganze Zahl von 0 bis 2, $R_1$ Wasserstoff, Niederalkyl, z.B. Methyl, Niederalkoxy, z.B. Methoxy oder Aethoxy, Halogen, z.B. Chlor, oder eine Gruppe der Formel $-CH_2-R_2$, worin $R_2$ Niederalkanoyloxy, z.B. Acetoxy, Carbamoyloxy, Niederalkylcarbamoyloxy, aromatisches. monocyclisches, fünf- oder sechsgliedriges Heterocyclylthio, z.B. Diaza-, Triaza-, Tetraaza-, Thiaza, Thiadiaza-, Oxaza- oder Oxadiazacyclylthio, z.B. Imidazolylthio, Triazolylthio, z.B. 1H-1,2,3-Triazol-5-ylthio, Tetrazolylthio, z.B. 1H-Tetrazol-5-ylthio, Thiazolylthio, Thiadiazolylthio, z.B. 1,3,4-Thiadiazol-5-ylthio, Oxazolylthio, Oxadiazolylthio oder 5,6-Dioxotetrahydro-as-triazin-3-ylthio, z.B. 5,6-Dioxo-1,2,5,6-tetrahydro-as-triazin-3-ylthio oder 5,6-Dioxo-1,4,5,6-tetrahydro-as-triazin-3-ylthio, welche durch Niederalkyl, z.B. Methyl, Diniederalkylaminoniederalkyl, z.B. Dimethylaminomethyl oder 2-Dimethylaminoäthyl, Sulfoniederalkyl, z.B. Sulfomethyl oder Sulfoäthyl, Carboxyniederalkyl, z.B. Carboxymethyl, Amino, Carboxyniederalkylamino, z.B. 2-Carboxyäthylamino, Carbamoyl oder durch Tetrazolylniederalkyl, z.B. Tetrazol-1H-5-ylmethyl, substituiert sein können, oder eine Ammoniogruppe, z.B. 2-Niederalkyl-1-pyrazolio, z.B. 2-Methyl-1-pyrazolio, 2-Carboxyniederalkyl-1-pyrazolio, z.B. 2-Carboxymethyl-1-pyrazolio, 3-Niederalkyl-1-triazolio, z.B. 3-Methyl-1-triazolio, Pyridinio, durch Hydroxyniederalkyl, z.B. Hydroxymethyl,

Carboxy, Carboxyniederalkyl, z.B. Carboxymethyl, Halogen, z.B. Chlor oder Brom, oder Carbamoyl substituiertes Pyridinio, z.B. 3- oder 4-Hydroxymethylpyridinio, 4-Carboxypyridinio, 3- oder 4-Carboxymethylpyridinio, 3- oder 4-Chlorpyridinio, 3- oder 4-Brompyridinio oder 3- oder 4-Carbamoylpyridinio, $R_3$ Carboxy oder unter physiologischen Bedingungen spaltbares Carboxy, z.B. Acyloxyniederalkoxycarbonyl, z.B. Niederalkanoyloxyniederalkoxycarbonyl, z.B. Niederalkanoyloxymethoxycarbonyl oder Niederalkanoyloxyäthoxycarbonyl, z.B. Pivaloyloxymethoxycarbonyl oder 2-Propionyloxyäthoxycarbonyl oder Niederalkoxycarbonyloxyniederalkoxycarbonyl, z.B. 1-Aethoxycarbonyloxyäthoxycarbonyl oder tert.-Butyloxycarbonyloxymethoxycarbonyl, $R_4$ Wasserstoff, $R_5$ Niederalkyl, z.B. Methyl oder Aethyl, Hydroxyniederalkyl, z.B. Hydroxymethyl oder 2-Hydroxyäthyl, Niederalkoxyniederalkyl, z.B. Methoxymethyl, 2-Methoxyäthyl oder 2-Aethoxyäthyl, Niederalkenyloxyniederalkyl, z.B. 2-Vinyloxyäthyl, Niederalkanoyloxyniederalkyl, z.B. 2-Acetoxyäthyl, Halogenniederalkyl, z.B. Chloromethyl, 2-Chloroäthyl, 3-Chloropropyl, 4-Chlorobutyl oder 2-Bromäthyl, Niederalkylthioniederalkyl, z.B. 2-Methylthioäthyl oder 2-Aethylthioäthyl, Aminocarboxyniederalkylthioniederalkyl, z.B. 2-(2-Amino-2-carboxyäthylthio)-äthyl, Benzoylniederalkyl, z.B. Benzoylmethyl, Carboxyniederalkyl, z.B. Carboxymethyl oder 2-Carboxyäthyl, Niederalkoxycarbonylniederalkyl, z.B. Aethoxycarbonylmethyl oder 2-Aethoxycarbonyläthyl, Carbamoylniederalkyl, z.B. Carbamoylmethyl, Cyanoniederalkyl, z.B. Cyanomethyl oder 1-Cyano- oder 2-Cyanoäthyl, Sulfoniederalkyl, z.B. Sulfomethyl oder 2-Sulfoäthyl, Sulfamoylniederalkyl, z.B. Sulfamoylmethyl oder 2-Sulfamoyläthyl, Aminocarboxyniederalkyl, z.B. 2-Amino-2-carboxyäthyl oder eine Gruppe der Teilformel A, worin die Gruppe $-(C_nH_{2n})-$ Aethylen oder Propylen, $R_o$ Wasserstoff oder Niederalkyl, z.B. Methyl, und R Wasserstoff, Niederalkyl, z.B. Methyl oder Aethyl, Niederalkanoyl, z.B. Formyl oder Acetyl, Niederalkanoyl substituiert durch Hydroxy, Niederalkoxy, z.B. Methoxy, Halogen, z.B. Brom, Carboxy, Cyano oder Amino, z.B. α-Hydroxypropionyl, Methoxyacetyl, Bromacetyl, Carboxyacetyl, Cyanacetyl oder Glycyl, Niederalkenoyl,

z.B. Acryloyl, Niederalkinoyl, z.B. Propioloyl, Cycloalkylcarbonyl, z.B. Cyclopropylcarbonyl, Benzoyl, 4-Aminobenzoyl, 4-Niederalkanoylaminobenzoyl, z.B. 4-Acetylaminobenzoyl, 4-Cyanobenzoyl, 4-Nitrobenzoyl oder 2,4-Dinitrobenzoyl, Pyridylcarbonyl, z.B. Nicotinoyl oder Isonicotinoyl, Furoyl, z.B. 2-Furoyl, Thienylcarbonyl, z.B. 2-Thienylcarbonyl, Hydroxypyrimidylcarbonyl, z.B. 2,6-Dihydroxy-1,3-pyrimid-4-ylcarbonyl, Hydroxythiadiazolylcarbonyl, z.B. 4-Hydroxy-1,2,5-thiadiazol-3-ylcarbonyl, Tetrazolylniederalkanoyl, z.B. 2-Tetrazol-5-ylcetyl oder Aminothiazolylniederalkanoyl, z.B. 2-(2-Amino-1,3-thiazol-4-yl)-acetyl, die Acylgruppe eines Halbesters der Kohlensäure, beispielsweise Niederalkoxycarbonyl, z.B. Methoxycarbonyl oder Isopropoxycarbonyl, Niederalkanoyloxy substituiert durch Carboxy und Amino, z.B. 2-Amino-2-carboxyäthoxycarbonyl, oder Benzoyloxycarbonyl, die Acylgruppe einer substituierten Carbaminsäure, beispielsweise Niederalkylcarbamoyl, z.B. Methylcarbamoyl oder Anilinocarbonyl, die Acylgruppe einer substituierten Thiocarbaminsäure, beispielsweise Niederalkylthiocarbamoyl, z.B. Methylthiocarbamoyl, die Acylgruppe einer substituierten Sulfonsäure, beispielsweise Niederalkansulfonyl, z.B. Methansulfonyl, Benzolsulfonyl, 4-Nitrobenzolsulfonyl, 2,4-Dinitrobenzolsulfonyl, Aminobenzolsulfonyl, z.B. 4-Aminobenzolsulfonyl, eine Acylcarbamoylgruppe, beispielsweise Benzoylcarbamoyl oder Furoylcarbamoyl, eine Acylthiocarbamoylgruppe, beispielsweise Benzoylthiocarbamoyl oder Furoylthiocarbamoyl, 2-Oxo-1-imidazolidinocarbonyl, 4-Niederalkyl-2,3-dioxo-1-piperazinocarbonyl, z.B. 4-Aethyl-2,3-dioxo-1-piperazinocarbonyl, und 4-Niederalkansulfonyl-1-piperazinocarbonyl, z.B. 4-Methansulfonyl-1-piperazinocarbonyl, bedeuten, und $R_6$ Pyridyl, z.B. 3- oder 4-Pyridyl, Thienyl, z.B. 2- oder 3-Thienyl, Furyl, z.B. 2- oder 3-Furyl, Aminothiazolyl, z.B. 2-Amino-4-thiazolyl, Hydroxypyrimidyl, z.B. 2,6-Dihydroxy-1,3-pyrimid-4-yl, Aminothiadiazolyl, z.B. 5-Amino-1,2,4-thiadiazol-3-yl, Hydroxythiadiazolyl, z.B. 4-Hydroxy-1,2,5-thiadiazol-3-yl, oder Aminotriazolyl, z.B. 5-Amino-1,2,4-triazol-3-yl bedeuten, Stereoisomere, Mischungen von diesen Stereoisomeren, Hydrate und pharmazeutisch verwendbare Salze von solchen Verbindungen.

5. Verbindungen nach Anspruch 2 der Formel I, worin m, $R_1$, $R_2$, $R_3$, $R_4$, $R_5$ und $R_6$ die im Anspruch 4 unter Formel I genannten Bedeutungen haben, Stereoisomere, Mischungen von diesen Stereoisomeren, Hydrate und pharmazeutisch verwendbare Salze von solchen Verbindungen.

6. Verbindungen nach Anspruch 3 der Formel I, worin m, $R_1$, $R_2$, $R_3$, $R_4$, $R_5$ und $R_6$ die im Anspruch 4 unter Formel I genannten Bedeutungen haben, Stereoisomere, Mischungen von diesen Stereoisomeren, Hydrate und pharmazeutisch verwendbare Salze von solchen Verbindungen.

7. Verbindungen nach Anspruch 1 der Formel I, worin m Null, $R_1$ Wasserstoff, Niederalkyl, z.B. Methyl, Niederalkoxy, z.B. Methoxy, Halogen, z.B. Chlor, oder eine Gruppe $-CH_2-R_2$, worin $R_2$ Niederalkanoyloxy, z.B. Acetoxy, Carbamoyloxy, Triazolylthio, z.B. 1H-1,2,3-Triazol-5-ylthio, Tetrazolylthio, z.B. 1H-Tetrazol-5-ylthio, durch Niederalkyl, z.B. Methyl, Diniederalkylaminoniederalkyl, z.B. 2-Dimethylaminoäthyl, Sulfoniederalkyl, z.B. Sulfomethyl, Carboxyniederalkyl, z.B. Carboxymethyl, oder durch Carbamoyl substituiertes Tetrazolylthio, z.B. 1-Methyl-1H-tetrazol-5-ylthio, 1-Sulfomethyl-1H-tetrazol-5-ylthio, 1-Carboxymethyl-1H-tetrazol-5-ylthio oder 1-(2-Dimethylaminoäthyl)-1H-tetrazol-5-ylthio, Thiadiazolylthio, z.B. 1,3,4-Thiadiazol-5-ylthio, durch Niederalkyl, z.B. Methyl, substituiertes Thiadiazolylthio, z.B. 2-Methyl-1,3,4-thiadiazol-5-ylthio, durch Niederalkyl, z.B. Methyl, substituiertes 5,6-Dioxotetrahydro-as-triazin-3-ylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, Pyridinio oder durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinio, z.B. 3- oder 4-Hydroxymethylpyridinio, 4-Carboxypyridinio, 3- oder 4-Carboxymethylpyridinio, 3- oder 4-Chlorpyridinio, 3- oder 4-Brompyridinio oder 3- oder 4-Carbamoylpyridinio bedeutet, $R_3$ Carboxyl, oder unter physiologischen Bedingungen spaltbares Carboxyl, z.B. Acyloxyniederalkoxycarbonyl, z.B. Niederalkanoyloxyniederalkoxycarbonyl, z.B. Niederalkanoyloxymethoxycarbonyl oder Niederalkanoyloxyäthoxycarbonyl, z.B. Pivaloyloxymethoxycarbonyl oder 2-Propionyloxyäthoxycarbonyl, oder Niederalkoxycarbonyloxyniederalkoxycarbonyl, z.B. 1-Aethoxycarbonyloxyäthoxycarbonyl oder tert.-Butoxycarbonyloxymethoxycarbonyl, $R_4$ Wasserstoff, $R_5$ Niederalkyl, z.B. Methyl oder Aethyl, Hydroxyniederalkyl, z.B. Hydroxymethyl oder Hydroxyäthyl, Niederalkoxyniederalkyl, z.B. Methoxymethyl, 2-Methoxyäthyl oder 2-Aethoxyäthyl, Niederalkenyloxyniederalkyl, z.B. 2-Vinyloxyäthyl, Halogenniederalkyl, z.B. Chlormethyl oder 2-Chloräthyl, Niederalkylthioniederalkyl, z.B. 2-Methylthioäthyl oder 2-Aethylthioäthyl, Carboxyniederalkyl, z.B. Carboxymethyl oder 2-Carboxyäthyl, Carbamoylniederalkyl, z.B. Carbamoylmethyl, Cyanoniederalkyl, z.B. Cyanomethyl oder 1-Cyano- oder 2-Cyanoäthyl, oder eine Gruppe der Teilformel A, welche 2-Aminoäthyl, 2-Niederalkylaminoäthyl, z.B. 2-Methylaminoäthyl oder 2-n-Hexylaminoäthyl, 2-Diniederalkylaminoäthyl, z.B. 2-Dimethylaminoäthyl oder 2-Di-n-hexylaminoäthyl, 2-Sulfoaminoäthyl, Niederalkanoylaminoäthyl, z.B. 2-Formylaminoäthyl oder 2-Acetylaminoäthyl, 2-Niederalkoxyniederalkanoylaminoäthyl, z.B. 2-Methoxyacetylaminoäthyl, 2-Halogenniederalkanoylaminoäthyl, z.B. 2-Bromacetylaminoäthyl, 2-(α-Hydroxypropionylamino)-äthyl, 2-Glycylaminoäthyl, 2-(3-Amino-3-carboxypropionylamino)-äthyl, 2-Acryloylaminoäthyl, 2-Propioloylaminoäthyl, 2-Cyclopropylcarbonylaminoäthyl, 2-Benzoylaminoäthyl, 2-(4-Aminobenzoylamino)-äthyl, 2-(4-Acetylaminobenzoylamino)-äthyl, 2-(4-Cyanobenzoylamino)-äthyl, 2-(4-Nitrobenzoylamino)-äthyl, 2-(3,4-Dinitrobenzoylamino)-äthyl, 2-Mandeloylaminoäthyl, 2-Phenylglycylaminoäthyl, 2-Nicotinoylaminoäthyl, 2-Isonicotinoylaminoäthyl, 2-(2-Furoylamino)-äthyl, 2-(2-Thienylcarbonylamino)-äthyl, 2-(2,6-Dihydroxy-1,3-pyrimid-4-ylcarbonylamino)-äthyl, 2-(4-Hydroxy-1,2,5-thiadiazol-3-ylcarbonylamino)-äthyl, 2-(2-Tetrazol-1-ylacetylamino)-äthyl, 2-[2-(2-Amino-1,3-thiazol-4-yl)-acetylamino]-äthyl, 2-Niederalkoxycarbonylaminoäthyl, z.B. 2-Methoxycarbonylaminoäthyl oder 2-Isopropoxycarbonylaminoäthyl, 2-(2-Amino-2-carboxyäthoxycarbonylamino)-äthyl, 2-Benzoyloxycarbonylaminoäthyl, 2-Niederalkylcarbamoylaminoäthyl, z.B. 2-Methylcarbamoylaminoäthyl, 2-Anilinocarbonylaminoäthyl, 2-Niederalkylthiocarbamoylaminoäthyl, z.B. 2-Methylthiocarbamoylaminoäthyl, 2.Niederalkansulfonylaminoäthyl, z.B. 2-Methansulfonylaminoäthyl, 2-Halogenmethansulfonylaminoäthyl, z.B. 2-Difluormethansulfonylaminoäthyl, 2-Cyanomethansulfonylaminoäthyl, 2-Benzolsulfonylaminoäthyl, 2-(4-Nitrobenzolsulfonylamino)-äthyl, 2-(2,4-Dinitrobenzolsulfonylaminoäthyl, 2-Benzoylcarbamoylaminoäthyl, 2-(2-Furoylcarbamoylamino)-äthyl, 2-(2-Oxo-1-imidazolidinocarbonylamino)-äthyl, 2-(4-Aethyl-2,3-dioxo-1-piperazinocarbonylamino)-äthyl und 2-(4-Methansulfonyl-1-piperazinocarbonylamino)-äthyl bedeutet, und $R_6$ Aminothiazolyl, z.B. 2-Amino-4-thiazolyl, Aminothiadiazolyl, z.B. 5-Amino-1,2,4-thiadiazolyl-3-yl, oder Aminotriazolyl, z.B. 5-Amino-1,2,4-triazol-3-yl, bedeutet, Stereoisomere, Mischungen von diesen Stereoisomeren, Hydrate und pharmazeutisch verwendbare Salze von solchen Verbindungen.

8. Verbindungen nach Anspruch 2 der Formel I, worin m, $R_1$, $R_2$, $R_3$, $R_4$, $R_5$ und $R_6$ die in Anspruch 7 unter Formel I genannten Bedeutungen haben, Stereoisomere, Mischungen von diesen Stereoisomeren, Hydrate und pharmazeutisch verwendbare Salze von solchen Verbindungen.

9. Verbindungen nach Anspruch 3 der Formel I, worin m, $R_1$, $R_2$, $R_3$, $R_4$, $R_5$ und $R_6$ die im Anspruch 7 unter Formel I genannten Bedeutungen haben, Stereoisomere, Mischungen von diesen Stereoisomeren, Hydrate und pharmazeutisch verwendbare Salze von solchen Verbindungen.

10. Verbindungen nach Anspruch 1 der Formel I, worin m Null, $R_1$ Wasserstoff, Niederalkoxy, z.B. Methoxy, Halogen, z.B. Chlor, oder eine Gruppe der Formel $-CH_2-R_2$, worin $R_2$

Niederalkanoyloxy, z.B. Acetoxy, Carbamoyloxy, Tetrazolylthio, z.B. 1H-Tetrazol-5-ylthio, durch Niederalkyl, z.B. Methyl, Diniederalkylaminoniederalkyl, z.B. 2-Dimethylaminoäthyl, Sulfoniederalkyl, z.B. Sulfomethyl, oder Carboxyniederalkyl, z.B. Carboxymethyl, substituiertes Tetrazolylthio, z.B. 1-Methyl-1H-tetrazol-5-ylthio, 1-(2-Dimethylaminoäthyl)-1H-tetrazol-5-ylthio, 1-Carboxymethyl-1H-tetrazol-5-ylthio, 1-Sulfomethyl-1H-tetrazol-5-ylthio oder 1-Carboxymethyl-1H-tetrazol-5-ylthio, durch Niederalkyl, z.B. Methyl, substituiertes 5,6-Dioxotetrahydro-as-triazin-3-ylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, Pyridinio oder durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinio, z.B. 3- oder 4-Hydroxymethylpyridinio, 4-Carboxypyridinio, 3- oder 4-Carboxymethylpyridinio, 3- oder 4-Chlorpyridinio, 3- oder 4-Brompyridinio oder 3- oder 4-Carbamoylpyridinio bedeutet, $R_3$ Carboxy, Niederalkanoyloxyniederalkoxycarbonyl, z.B. Niederalkanoyloxymethoxycarbonyl oder Niederalkanoyloxyäthoxycarbonyl, z.B. Pivaloyloxymethoxycarbonyl oder 2-Propionyloxyäthoxycarbonyl oder Niederalkoxycarbonyloxyniederalkoxycarbonyl, z.B. 1-Aethoxycarbonyloxyäthoxycarbonyl oder tert.-Butoxycarbonyloxymethoxycarbonyl, $R_4$ Wasserstoff, $R_5$ Niederalkyl, z.B. Methyl oder Aethyl, Niederalkoxyniederalkyl, z.B. Methoxymethyl, 2-Methoxyäthyl oder 2-Aethoxyäthyl, Niederalkenyloxyniederalkyl, z.B. 2-Vinyloxyäthyl, Halogenniederalkyl, z.B. Chlormethyl oder 2-Chloräthyl, Carboxyniederalkyl, z.B. Carboxymethyl oder 2-Carboxyäthyl, Cyanoniederalkyl, z.B. Cyanomethyl oder 1-Cyano- oder 2-Cyanoäthyl, oder eine Gruppe der Teilformel A, welche 2-Aminoäthyl, 2-Niederalkylaminoäthyl, z.B. 2-Methylaminoäthyl oder 2-Aethylaminoäthyl, 2-Diniederalkylaminoäthyl, z.B. 2-Dimethylaminoäthyl, 2-Sulfoaminoäthyl, Niederalkanoylaminoäthyl, z.B. 2-Formylaminoäthyl oder 2-Acetylaminoäthyl, Niederalkoxyniederalkanoylaminoäthyl, z.B. 2-Methoxyacetylaminoäthyl, Cyanoniederalkanoylaminoäthyl, z.B. 2-Cyanoacetylaminoäthyl, Niederalkanoylaminoäthyl, z.B. 2-Acryloylaminoäthyl, Niederalkinoylaminoäthyl, z.B. 2-Propionylaminoäthyl, Cycloalkanoylaminoäthyl, z.B. 2-Cyclopropanoylaminoäthyl, 2-(4-Hydroxy-1,2,5-thiadiazol-3-ylcarbonylamino)-äthyl, 2-(2-Tetrazol-5-ylacetylamino)-äthyl, 2-[2-(2-Amino-1,3-thiazol-4-yl)-acetylamino]-äthyl, 2-Niederalkoxycarbonylaminoäthyl, z.B. 2-Methoxycarbonylaminoäthyl, 2-Niederalkansulfonylaminoäthyl, z.B. 2-Methansulfonylaminoäthyl, 2-Benzolsulfonylaminoäthyl, 2-Benzolsulfonylaminoäthyl, worin Benzol durch Nitro oder Amino substituiert ist, z.B. 2-(4-Nitrobenzolsulfonylamino)-äthyl, 2-(2,4-Dinitrobenzolsulfonylamino)-äthyl, 2-(2-Oxo-1-imidazolidinocarbonylamino)-äthyl, 2-(4-Aethyl-2,3-dioxo-1-piperazinocarbonylamino)-äthyl, oder 2-(4-Methylsulfonyl-1-piperazino-carbonylamino)-äthyl, bedeutet, und $R_6$ Aminothiazolyl, z.B. 2-Amino-4-thiazolyl, bedeutet, Stereoisomere, Mischungen von diesen Stereoisomeren, Hydrate und pharmazeutisch verwendbare Salze von solchen Verbindungen, dadurch gekennzeichnet, dass man die in Anspruch 1 genannten Massnahmen durchführt.

11. Verbindungen nach Anspruch 2 der Formel I, worin m, $R_1$, $R_2$, $R_3$, $R_4$, $R_5$ und $R_6$ die im Anspruch 10 genannten Bedeutungen haben, Stereoisomere, Mischungen von diesen Stereoisomeren, Hydrate und pharmazeutisch verwendbare Salze von solchen Verbindungen.

12. Verbindungen nach Anspruch 3 der Formel I, worin m, $R_1$, $R_2$, $R_3$, $R_4$, $R_5$ und $R_6$ die im Anspruch 10 genannten Bedeutungen haben, Stereoisomere, Mischungen von diesen Stereoisomeren, Hydrate und pharmazeutisch verwendbare Salze von solchen Verbindungen.

13. 3-(1-Methyl-1H-tetrazol-5-ylthiomethyl)-7β-[(2R)-2-(2-aminothiazol-4-yl)-2-(3-formylaminoäthansulfonylamino)-acetamido]-3-cephem-4-carbonsäure, gemäss Anspruch 2.

14. 3-(1-Methyl-1H-tetrazol-5-ylthiomethyl)-7β-[(2R,S)-2-(2-aminothiazol-4-yl)-2-methansulfonylaminoacetamido]-3-cephem-4-carbonsäure, gemäss Anspruch 2.

15. 3-(4-Carbamoylpyridiniomethyl)-7β-[(2R,S)-2-(2-aminothiazol-4-yl)-2-methansulfonylaminoacetamido]—3-cephem-4-carbonsäure, gemäss Anspruch 2.

16. 3-Acetoxymethyl-7β-[(2R,S)-2-(2-aminothiazol-4-yl)-2-(2-formylaminoäthansulfonylamino)-acetamido]-3-cephem-4-carbonsäure, gemäss Anspruch 2.

17. 3-(1-Methyl-1H-tetrazol-5-ylthiomethyl)-7β-[(2R,S)-2-(2-aminothiazol-4-yl)-2-cyanomethansulfonylaminoacetamido]-3-cephem-4-carbonsäure, gemäss Anspruch 2.

18. 3-Carbamoyloxymethyl-7β-[(2R,S)-2-(2-aminothiazol-4-yl)-2-(2-formylaminoäthansulfonylamino)-acetamido]-3-cephem-4-carbonsäure, gemäss Anspruch 2.

19. 3-Carbamoyloxymethyl-7β-[(2S)-2-(2-aminothiazol-4-yl)-2-methansulfonylaminoacetamido]-3-cephem-4-carbonsäure, gemäss Anspruch 2.

20. 3-Carbamoyloxymethyl-7β-[(2S)-2-(2-aminothiazol-4-yl)-2-(2-(4-nitrobenzolsulfonylamino)-äthansulfonylamino)-acetamido]-3-cephem-4-carbonsäure, gemäss Anspruch 3.

21. 3-Carbamoyloxymethyl-7β-[(2R,S)-2-(2-aminothiazol-4-yl)-2-(2-methansulfonylaminoäthansulfonylamino)-acetamido]-3-cephem-4-carbonsäure, gemäss Anspruch 3.

22. 3-Carbamoyloxymethyl-7β-[(2S)-2-(2-aminothiazol-4-yl)-2-(2-acryloylaminoäthansulfonylamino)-acetamido]-3-cephem-4-carbonsäure, gemäss Anspruch 3.

23. 3-Carbamoyloxymethyl-7β-[(2S)-2-(2-aminothiazol-4-yl)-2-(2-(4-aminobenzolsulfonylamino)-äthansulfonylamino)-acetamido]-3-cephem-4-carbonsäure, gemäss Anspruch 3.

24. 3-Carbamoyloxymethyl-7β-[(2S)-2-(2-aminothiazol-4-yl)-2-(2-methoxy-acetylaminoäthansulfonylamino)-acetamido]-3-cephem-4-carbonsäure, gemäss Anspruch 3.

25. 3-Acetoxymethyl-7β-[(2S)-2-(2-(2-aminothiazol-4-ylacetamido)-äthansulfonylamino)-2-(2-aminothiazol-4-yl)-acetamido]-3-cephem-4-carbonsäure,gemäss Anspruch 3.

26. 3-Carbamoyloxymethyl-7β-[(2S)-2-(2-(2-aminothiazol-4-ylacetamido)-äthansulfonylamino)-2-(2-aminothiazol-4-yl)acetamido]-3-cephem-4-carbonsäure, gemäss Anspruch 3.

27. 3-Carbamoyloxymethyl-7β-[(2S)-2-(2-aminothiazol-4-yl)-2-äthan-sulfonylaminoacetamido]-3-cephem-4-carbonsäure, gemäss Anspruch 3.

28. 3-(2,5-Dihydro-6-hydroxy-2-methyl-5-oxo-as-triazin-3-ylthiomethyl)-7β-[(2R,S)-2-(2-aminothiazol-4-yl)-2-methansulfonylaminoacetamido]-3-cephem-4-carbonsäure, gemäss Anspruch 3.

29. Die Natriumsalze von Verbindungen gemäss Anspruch 13 - 28.

30. Verfahren zur Herstellung von 7β-Acylamido-3-cephem-4-carbonsäure-Verbindungen der Formel I, gemäss Anspruch 1, worin m, $R_1$, $R_2$, $R_3$, $R_4$, $R_5$ und $R_6$ die in Anspruch 1 genannten Bedeutungen haben, Stereoisomeren, Mischungen von diesen stereoisomeren, Hydraten und Salze von Verbindungen der Formel I, dadurch gekennzeichnet, dass man

a) in einer Verbindung der Formel

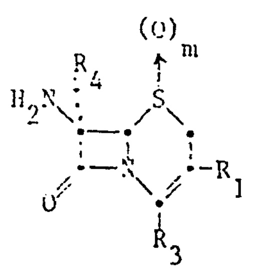

(II),

worin m, $R_1$, $R_3$ und $R_4$ die unter Formel I genannten Bedeutungen haben, und worin eine in $R_1$ vorhandene funktionelle Gruppe geschützt ist und die 7β-Aminogruppe gegebenenfalls durch eine die Acylierungsreaktion erlaubende Gruppe geschützt ist, die 7β-Aminogruppe durch Umsetzung mit einem den Acylrest einer Carbonsäure der Formel

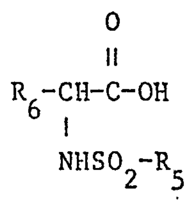 (III),

einführenden Acylierungsmittel, worin $R_5$ und $R_6$ die unter Formel I genannten Bedeutungen haben, und worin eine in $R_5$ und/oder $R_6$ vorhandene funktionelle Gruppe in geschützter Form vorliegt, acyliert oder

b) in einer Verbindung der Formel

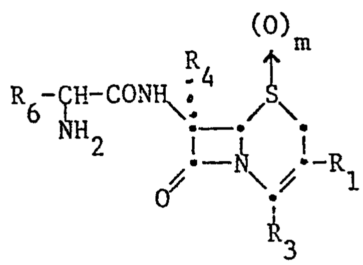 (IV) ,

worin m, $R_1$, $R_3$, $R_4$ und $R_6$ die unter Formel I genannten Bedeutungen haben, und worin eine in $R_1$ und/oder $R_6$ vorhandene funktionelle Gruppe geschützt ist und die 2-Aminogruppe gegebenenfalls durch eine die Sulfonylierungsreaktion erlaubende Gruppe geschützt ist, die 2-Aminogruppe durch Umsetzung mit einem den $R_5$-Sulfonylrest einer Sulfonsäure der Formel

$$R_5 - SO_2 - OH \qquad (V) ,$$

einführenden Sulfonylierungsmittel, worin $R_5$ die unter Formel I genannte Bedeutung hat und eine in $R_5$ vorhandene funktionelle Gruppe in geschützter Form vorliegt, oder mit einem reaktionsfähigen, funktionellen Säurederivat oder einem Salz davon sulfonyliert oder

c) eine 2-Cephem-Verbindung der Formel

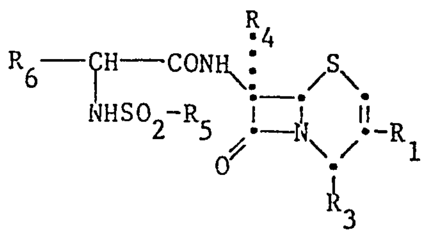

(VI),

worin $R_1$, $R_3$, $R_4$, $R_5$ und $R_6$ die unter Formel I genannten Bedeutungen haben und eine in $R_1$, $R_5$ und/oder $R_6$ vorhandene funktionelle Gruppe gegebenenfalls in geschützter Form vorliegt, zur entsprechenden 3-Cephem-Verbindung der Formel I isomerisiert und, wenn erwünscht, eine erfindungsgemäss erhältliche Verbindung der Formel I in eine andere definitionsgemässe Verbindung der Formel I umwandelt und/oder eine erfindungsgemäss erhältliche Verbindung der Formel I, worin m 0 bedeutet, in eine Verbindung der Formel I überführt, worin m 1 oder 2 bedeutet, und/oder eine Verbindung der Formel I, worin m 1 oder 2 bedeutet, in eine Verbindung der Formel I überführt, worin m 0 bedeutet, und/oder in einer Verbindung der Formel I in geschützter Form vorliegende funktionelle Gruppen in freie funktionelle Gruppen überführt, und/oder ein erhältliches Salz in die freie Verbindung oder in ein anderes Salz überführt, und/oder eine erhältliche freie Verbindung mit einer salzbildenden Gruppe in ein Salz überführt und/oder ein erhältliches Gemisch von isomeren Verbindungen der Formel I in die einzelnen Isomere auftrennt.

31. Die nach dem Verfahren gemäss Anspruch 30 erhältlichen Verbindungen.

32. Pharmazeutische Präparate enthaltend Verbindungen der Formel I gemäss Anspruch 1, Hydrate oder pharmazeutisch verwendbare Salze von solchen Verbindungen.

33. Verbindungen der Formel I gemäss Anspruch 1 zur Anwendung bei der Behandlung des menschlichen oder tierischen Körpers von bakteriellen Infektionen.

34. Verbindungen der Formel III, worin $R_5$ und $R_6$ die in Anspruch 1 unter Formel I genannten Bedeutungen haben, und worin eine in $R_5$ und/oder $R_6$ vorhandene funktionelle Gruppe in freier oder geschützter Form vorliegt.

35. Verfahren zur Herstellung von Verbindungen der Formel III, worin $R_5$ und $R_6$ die in Anspruch 1 unter Formel I genannten Bedeutungen haben, und worin eine in $R_5$ und/oder $R_6$ vorhandene funktionelle Gruppe in freier oder geschützter Form vorliegt, dadurch gekennzeichnet, dass man in einer Verbindung der Formel

$$R_6 - \underset{NH_2}{\underset{|}{CH}} - \overset{O}{\overset{\|}{C}} - OH \qquad \text{(VII)},$$

worin $R_6$ die unter Formel I genannten Bedeutungen hat und eine in $R_6$ vorhandene funktionelle Gruppe in geschützter Form vorliegt, die 2-Aminogruppe mit einer Sulfonsäure der Formel

$$R_5 - SO_2 - OH \qquad \text{(V)},$$

worin $R_5$ die unter Formel I genannte Bedeutung hat und eine in $R_5$ vorhandene funktionelle Gruppe in geschützter Form vorliegt, oder mit einem reaktionsfähigen, funktionellen Säurederivat oder einem Salz

davon acyliert und, wenn erwünscht, in einer erhältlichen Verbindung vorhandene Schutzgruppen abspaltet und/oder eine erhältliche Verbindung der Formel III in eine andere Verbindung der Formel III umwandelt.

<u>Ansprüche</u> (für Oesterreich)

1. Verfahren zur Herstellung von 7β-Acylamido-3-cephem-4-carbonsäureverbindungen der Formel

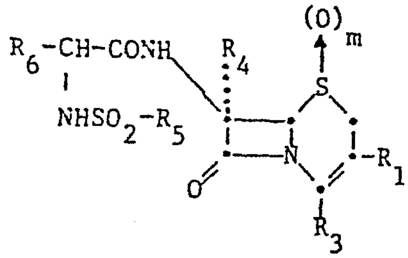

(I),

worin m eine ganze Zahl von 0 bis 2

$R_1$ Wasserstoff, Niederalkyl, Niederalkenyl, Niederalkoxy, Halogen, eine Gruppe der Formel $-CH_2-R_2$, worin $R_2$ eine freie, veresterte oder verätherte Hydroxy- oder Mercaptogruppe oder eine Ammoniogruppe darstellt, oder eine Gruppe der Formel $-CH=CHR_2$, worin $R_2$ eine verätherte Mercaptogruppe darstellt,

$R_3$ Carboxy oder geschütztes Carboxy,

$R_4$ Wasserstoff,

$R_5$ einen organischen Rest, welcher mit einem Kohlenstoffatom an die Sulfonylgruppe gebunden ist, und

$R_6$ einen heterocyclischen Rest darstellen,

Stereoisomeren, Mischungen dieser Stereoisomeren, Hydraten und Salzen von Verbindungen der Formel I, dadurch gekennzeichnet, dass man

a) in einer Verbindung der Formel

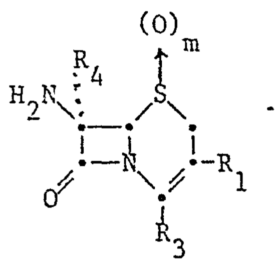

(II),

worin m, $R_1$, $R_3$ und $R_4$ die unter Formel I genannten Bedeutungen haben, und worin eine in $R_1$ vorhandene funktionelle Gruppe geschützt ist und die 7β-Aminogruppe gegebenenfalls durch eine die Acylierungsreaktion

erlaubende Gruppe geschützt ist, die 7β-Aminogruppe durch Umsetzung mit einem den Acylrest einer Carbonsäure der Formel

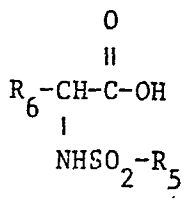

(III),

einführenden Acylierungsmittel, worin $R_5$ und $R_6$ die unter Formel I genannten Bedeutungen haben, und worin eine in $R_5$ und/oder $R_6$ vorhandene funktionelle Gruppe in geschützter Form vorliegt, acyliert oder

b) in einer Verbindung der Formel

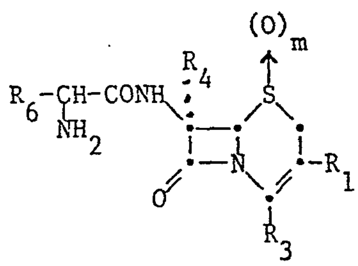

(IV) ,

worin m, $R_1$, $R_3$, $R_4$ und $R_6$ die unter Formel I genannten Bedeutungen haben, und worin eine in $R_1$ und/oder $R_6$ vorhandene funktionelle Gruppe geschützt ist und die 2-Aminogruppe gegebenenfalls durch eine die Sulfonylierungsreaktion erlaubende Gruppe geschützt ist, die 2-Aminogruppe durch Umsetzung mit einem den $R_5$-Sulfonylrest einer Sulfonsäure der Formel

$$R_5 - SO_2 - OH \qquad (V) ,$$

einführenden Sulfonylierungsmittel, worin $R_5$ die unter Formel I genannte Bedeutung hat und eine in $R_5$ vorhandene funktionelle Gruppe in geschützter Form vorliegt, oder mit einem reaktionsfähigen, funktionellen Säurederivat oder einem Salz davon sulfonyliert oder

c) eine 2-Cephem-Verbindung der Formel

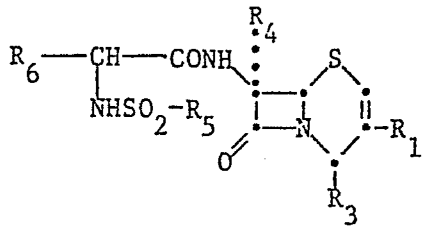

(VI),

worin $R_1$, $R_3$, $R_4$, $R_5$ und $R_6$ die unter Formel I genannten Bedeutungen haben und eine in $R_1$, $R_5$ und/oder $R_6$ vorhandene funktionelle Gruppe gegebenenfalls in geschützter Form vorliegt, zur entsprechenden 3-Cephem-Verbindung der Formel I isomerisiert und, wenn erwünscht, eine erfindungsgemäss erhältliche Verbindung der Formel I in eine andere definitionsgemässe Verbindung der Formel I umwandelt und/oder eine erfindungsgemäss erhältliche Verbindung der Formel I, worin m 0 bedeutet, in eine Verbindung der Formel I überführt, worin m 1 oder 2 bedeutet, und/oder eine Verbindung der Formel I, worin m 1 oder 2 bedeutet, in eine Verbindung der Formel I überführt, worin m 0 bedeutet, und/oder in einer Verbindung der Formel I in geschützter Form vorliegende funktionelle Gruppen in freie funktionelle Gruppen überführt, und/oder ein erhältliches Salz in die freie Verbindung oder in ein anderes Salz überführt, und/oder eine erhältliche freie Verbindung mit einer salzbildenden Gruppe in ein Salz überführt und/oder ein erhältliches Gemisch von isomeren Verbindungen der Formel I in die einzelnen Isomere auftrennt.

2. Verfahren nach Anspruch 1 zur Herstellung von Verbindungen der Formel I, worin

m eine ganze Zahl von 0 bis 2, $R_1$ Wasserstoff, Niederalkyl, z.B. Methyl, Niederalkoxy, z.B. Methoxy oder Aethoxy, Halogen, z.B. Chlor, oder eine Gruppe der Formel $-CH_2-R_2$, worin $R_2$ Niederalkanoyloxy, z.B. Acetoxy, Carbamoyloxy, Niederalkylcarbamoyloxy, aromatisches. monocyclisches, fünf- oder sechsgliedriges Heterocyclylthio, z.B. Diaza-, Triaza-, Tetraaza-, Thiaza, Thiadiaza-, Oxaza- oder

Oxadiazacyclylthio, z.B. Imidazolylthio, Triazolylthio, z.B. 1H-1,2,3-Triazol-5-ylthio, Tetrazolylthio, z.B. 1H-Tetrazol-5-ylthio, Thiazolylthio, Thiadiazolylthio, z.B. 1,3,4-Thiadiazol-5-ylthio, Oxazolylthio, Oxadiazolylthio oder 5,6-Dioxotetrahydro-as-triazin-3-ylthio, z.B. 5,6-Dioxo-1,2,5,6-tetrahydro-as-triazin-3-ylthio oder 5,6-Dioxo-1,4,5,6-tetrahydro-as-triazin-3-ylthio, welche durch Niederalkyl, z.B. Methyl, Diniederalkylaminoniederalkyl, z.B. Dimethylaminomethyl oder 2-Dimethylaminoäthyl, Sulfoniederalkyl, z.B. Sulfomethyl oder Sulfoäthyl, Carboxyniederalkyl, z.B. Carboxymethyl, Amino, Carboxyniederalkylamino, z.B. 2-Carboxyäthylamino, Carbamoyl oder durch Tetrazolylniederalkyl, z.B. Tetrazol-1H-5-ylmethyl, substituiert sein können, oder eine Ammoniogruppe, z.B. 2-Niederalkyl-1-pyrazolio, z.B. 2-Methyl-1-pyrazolio, 2-Carboxyniederalkyl-1-pyrazolio, z.B. 2-Carboxymethyl-1-pyrazolio, 3-Niederalkyl-1-triazolio, z.B. 3-Methyl-1-triazolio, Pyridinio, durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinio, z.B. 3- oder 4-Hydroxymethylpyridinio, 4-Carboxypyridinio, 3- oder 4-Carboxymethylpyridinio, 3- oder 4-Chlorpyridinio, 3- oder 4-Brompyridinio oder 3- oder 4-Carbamoylpyridinio, $R_3$ Carboxy oder unter physiologischen Bedingungen spaltbares Carboxy, z.B. Acyloxyniederalkoxycarbonyl, z.B. Niederalkanoyloxyniederalkoxycarbonyl, z.B. Niederalkanoyloxymethoxycarbonyl oder Niederalkanoyloxyäthoxycarbonyl, z.B. Pivaloyloxymethoxycarbonyl oder 2-Propionyloxyäthoxycarbonyl oder Niederalkoxycarbonyloxyniederalkoxycarbonyl, z.B. 1-Aethoxycarbonyloxyäthoxycarbonyl oder tert.-Butyloxycarbonyloxymethoxycarbonyl, $R_4$ Wasserstoff, $R_5$ Niederalkyl, z.B. Methyl oder Aethyl, Hydroxyniederalkyl, z.B. Hydroxymethyl oder 2-Hydroxyäthyl, Niederalkoxyniederalkyl, z.B. Methoxymethyl, 2-Methoxyäthyl oder 2-Aethoxyäthyl, Niederalkenyloxyniederalkyl, z.B. 2-Vinyloxyäthyl, Niederalkanoyloxyniederalkyl, z.B. 2-Acetoxyäthyl, Halogenniederalkyl, z.B. Chloromethyl, 2-Chloroäthyl, 3-Chloropropyl, 4-Chlorobutyl oder 2-Bromäthyl, Niederalkylthioniederalkyl, z.B. 2-Methylthioäthyl oder 2-Aethylthioäthyl, Aminocarboxyniederalkylthioniederalkyl, z.B. 2-(2-Amino-2-carboxyäthylthio)-äthyl, Benzoylniederalkyl, z.B. Benzoylmethyl, Carboxyniederalkyl, z.B. Carboxymethyl oder 2-Carboxyäthyl, Niederalkoxycarbonylniederalkyl, z.B. Aethoxycarbonylmethyl oder 2-Aethoxycarbonyläthyl, Carbamoylniederalkyl, z.B. Carbamoylmethyl, Cyanoniederalkyl, z.B. Cyanomethyl oder 1-Cyano- oder 2-Cyanoäthyl, Sulfoniederalkyl, z.B. Sulfomethyl oder 2-Sulfoäthyl, Sulfamoylniederalkyl, z.B. Sulfamoylmethyl oder 2-Sulfamoyläthyl, Aminocarboxyniederalkyl, z.B. 2-Amino-2-carboxyäthyl oder eine Gruppe der Teilformel A, worin die Gruppe $-(C_nH_{2n})-$ Aethylen oder Propylen, $R_o$ Wasserstoff oder Niederalkyl, z.B. Methyl, und R Wasserstoff, Niederalkyl, z.B. Methyl oder Aethyl, Niederalkanoyl, z.B. Formyl oder Acetyl, Niederalkanoyl substituiert durch Hydroxy, Niederalkoxy, z.B. Methoxy, Halogen, z.B. Brom, Carboxy, Cyano oder Amino, z.B. α-Hydroxypropionyl, Methoxyacetyl, Bromacetyl, Carboxyacetyl, Cyanacetyl oder Glycyl, Niederalkenoyl, z.B. Acryloyl, Niederalkinoyl, z.B. Propioloyl, Cycloalkylcarbonyl, z.B. Cyclopropylcarbonyl, Benzoyl, 4-Aminobenzoyl, 4-Niederalkanoylaminobenzoyl, z.B. 4-Acetylaminobenzoyl, 4-Cyanobenzoyl, 4-Nitrobenzoyl oder 2,4-Dinitrobenzoyl, Pyridylcarbonyl, z.B. Nicotinoyl oder Isonicotinoyl, Furoyl, z.B. 2-Furoyl, Thienylcarbonyl, z.B. 2-Thienylcarbonyl, Hydroxypyrimidylcarbonyl, z.B. 2,6-Dihydroxy-1,3-pyrimid-4-ylcarbonyl, Hydroxythiadiazolylcarbonyl, z.B. 4-Hydroxy-1,2,5-thiadiazol-3-ylcarbonyl, Tetrazolylniederalkanoyl, z.B. 2-Tetrazol-5-ylcetyl oder Aminothiazolylniederalkanoyl, z.B. 2-(2-Amino-1,3-thiazol-4-yl)-acetyl, die Acylgruppe eines Halbesters der Kohlensäure, beispielsweise Niederalkoxycarbonyl, z.B. Methoxycarbonyl oder Isopropoxycarbonyl, Niederalkanoyloxy substituiert durch Carboxy und Amino, z.B. 2-Amino-2-carboxyäthoxycarbonyl, oder Benzoyloxycarbonyl, die Acylgruppe einer substituierten Carbaminsäure, beispielsweise Niederalkylcarbamoyl, z.B. Methylcarbamoyl oder Anilinocarbonyl, die Acylgruppe einer substituierten Thiocarbaminsäure, beispielsweise Niederalkylthiocarbamoyl, z.B. Methylthiocarbamoyl, die Acylgruppe

einer substituierten Sulfonsäure, beispielsweise Niederalkansulfonyl, z.B. Methansulfonyl, Benzolsulfonyl, 4-Nitrobenzolsulfonyl, 2,4-Dinitrobenzolsulfonyl, Aminobenzolsulfonyl, z.B. 4-Aminobenzolsulfonyl, eine Acylcarbamoylgruppe, beispielsweise Benzoylcarbamoyl oder Furoylcarbamoyl, eine Acylthiocarbamoylgruppe, beispielsweise Benzoylthiocarbamoyl oder Furoylthiocarbamoyl, 2-Oxo-1-imidazolidinocarbonyl, 4-Niederalkyl-2,3-dioxo-1-piperazinocarbonyl, z.B. 4-Aethyl-2,3-dioxo-1-piperazinocarbonyl, und 4-Niederalkansulfonyl-1-piperazinocarbonyl, z.B. 4-Methansulfonyl-1-piperazinocarbonyl, bedeuten, und $R_6$ Pyridyl, z.B. 3- oder 4-Pyridyl, Thienyl, z.B. 2- oder 3-Thienyl, Furyl, z.B. 2- oder 3-Furyl, Aminothiazolyl, z.B. 2-Amino-4-thiazolyl, Hydroxypyrimidyl, z.B. 2,6-Dihydroxy-1,3-pyrimid-4-yl, Aminothiadiazolyl, z.B. 5-Amino-1,2,4-thiadiazol-3-yl, Hydroxythiadiazolyl, z.B. 4-Hydroxy-1,2,5-thiadiazol-3-yl, oder Aminotriazolyl, z.B. 5-Amino-1,2,4-triazol-3-yl bedeuten, Stereoisomere, Mischungen von diesen Stereoisomeren, Hydrate und pharmazeutisch verwendbare Salze von solchen Verbindungen, dadurch gekennzeichnet, dass man die in Anspruch 1 genannten Massnahmen durchführt.

3. Verfahren nach Anspruch 1 zur Herstellung von Verbindungen der Formel I, worin m Null, $R_1$ Wasserstoff, Niederalkyl, z.B. Methyl, Niederalkoxy, z.B. Methoxy, Halogen, z.B. Chlor, oder eine Gruppe $-CH_2-R_2$, worin $R_2$ Niederalkanoyloxy, z.B. Acetoxy, Carbamoyloxy, Triazolylthio, z.B. 1H-1,2,3-Triazol-5-ylthio, Tetrazolylthio, z.B. 1H-Tetrazol-5-ylthio, durch Niederalkyl, z.B. Methyl, Diniederalkylaminoniederalkyl, z.B. 2-Dimethylaminoäthyl, Sulfoniederalkyl, z.B. Sulfomethyl, Carboxyniederalkyl, z.B. Carboxymethyl, oder durch Carbamoyl substituiertes Tetrazolylthio, z.B. 1-Methyl-1H-tetrazol-5-ylthio, 1-Sulfomethyl-1H-tetrazol-5-ylthio, 1-Carboxymethyl-1H-tetrazol-5-ylthio oder 1-(2-Dimethylaminoäthyl)-1H-tetrazol-5-ylthio, Thiadiazolylthio, z.B. 1,3,4-Thiadiazol-5-ylthio, durch Niederalkyl,

z.B. Methyl, substituiertes Thiadiazolylthio, z.B. 2-Methyl-1,3,4-thiadiazol-5-ylthio, durch Niederalkyl, z.B. Methyl, substituiertes 5,6-Dioxotetrahydrotriazin-3-ylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, Pyridinio oder durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinio, z.B. 3- oder 4-Hydroxymethylpyridinio, 4-Carboxypyridinio, 3- oder 4-Carboxymethylpyridinio, 3- oder 4-Chlorpyridinio, 3- oder 4-Brompyridinio oder 3- oder 4-Carbamoylpyridinio bedeutet, $R_3$ Carboxyl, oder unter physiologischen Bedingungen spaltbares Carboxyl, z.B. Acyloxyniederalkoxycarbonyl, z.B. Niederalkanoyloxyniederalkoxycarbonyl, z.B. Niederalkanoyloxymethoxycarbonyl oder Niederalkanoyloxyäthoxycarbonyl, z.B. Pivaloyloxymethoxycarbonyl oder 2-Propionyloxyäthoxycarbonyl, oder Niederalkoxycarbonyloxyniederalkoxycarbonyl, z.B. 1-Aethoxycarbonyloxyäthoxycarbonyl oder tert.-Butoxycarbonyloxymethoxycarbonyl, $R_4$ Wasserstoff, $R_5$ Niederalkyl, z.B. Methyl oder Aethyl, Hydroxyniederalkyl, z.B. Hydroxymethyl oder Hydroxyäthyl, Niederalkoxyniederalkyl, z.B. Methoxymethyl, 2-Methoxyäthyl oder 2-Aethoxyäthyl, Niederalkenyloxyniederalkyl, z.B. 2-Vinyloxyäthyl, Halogenniederalkyl, z.B. Chlormethyl oder 2-Chloräthyl, Niederalkylthioniederalkyl, z.B. 2-Methylthioäthyl oder 2-Aethylthioäthyl, Carboxyniederalkyl, z.B. Carboxymethyl oder 2-Carboxyäthyl, Carbamoylniederalkyl, z.B. Carbamoylmethyl, Cyanoniederalkyl, z.B. Cyanomethyl oder 1-Cyano- oder 2-Cyanoäthyl, oder eine Gruppe der Teilformel A, welche 2-Aminoäthyl, 2-Niederalkylaminoäthyl, z.B. 2-Methylaminoäthyl oder 2-n-Hexylaminoäthyl, 2-Diniederalkylaminoäthyl, z.B. 2-Dimethylaminoäthyl oder 2-Di-n-hexylaminoäthyl, 2-Sulfoaminoäthyl, Niederalkanoylaminoäthyl, z.B. 2-Formylaminoäthyl oder 2-Acetylaminoäthyl, 2-Niederalkoxyniederalkanoylaminoäthyl, z.B. 2-Methoxyacetylaminoäthyl, 2-Halogenniederalkanoylaminoäthyl, z.B. 2-Bromacetylaminoäthyl, 2-(α-Hydroxypropionylamino)-äthyl, 2-Glycylaminoäthyl, 2-(3-Amino-3-carboxypropionylamino)-äthyl,

2-Acryloylaminoäthyl, 2-Propioloylaminoäthyl, 2-Cyclopropylcarbonylaminoäthyl, 2-Benzoylaminoäthyl, 2-(4-Aminobenzoylamino)-äthyl, 2-(4-Acetylaminobenzoylamino)-äthyl, 2-(4-Cyanobenzoylamino)-äthyl, 2-(4-Nitrobenzoylamino)-äthyl, 2-(3,4-Dinitrobenzoylamino)-äthyl, 2-Mandeloylaminoäthyl, 2-Phenylglycylaminoäthyl, 2-Nicotinoylaminoäthyl,2-Isonicotinoylaminoäthyl, 2-(2-Furoylamino)-äthyl, 2-(2-Thienylcarbonylamino)-äthyl, 2-(2,6-Dihydroxy-1,3-pyrimid-4-ylcarbonylamino)-äthyl, 2-(4-Hydroxy-1,2,5-thiadiazol-3-ylcarbonylamino)-äthyl, 2-(2-Tetrazol-1-ylacetylamino)-äthyl, 2-[2-(2-Amino-1,3-thiazol-4-yl)-acetylamino]-äthyl, 2-Niederalkoxycarbonylaminoäthyl, z.B. 2-Methoxycarbonylaminoäthyl oder 2-Isopropoxycarbonylaminoäthyl, 2-(2-Amino-2-carboxyäthoxycarbonylamino)-äthyl, 2-Benzoyloxycarbonylaminoäthyl, 2-Niederalkylcarbamoylaminoäthyl, z.B. 2-Methylcarbamoylaminoäthyl, 2-Anilinocarbonylaminoäthyl, 2-Niederalkylthiocarbamoylaminoäthyl, z.B. 2-Methylthiocarbamoylaminoäthyl, 2.Niederalkansulfonylaminoäthyl, z.B. 2-Methansulfonylaminoäthyl, 2-Halogenmethansulfonylaminoäthyl, z.B. 2-Difluormethansulfonylaminoäthyl, 2-Cyanomethansulfonylaminoäthyl, 2-Benzolsulfonylaminoäthyl, 2-(4-Nitrobenzolsulfonylamino)-äthyl, 2-(2,4-Dinitrobenzolsulfonylaminoäthyl, 2-Benzoylcarbamoylaminoäthyl, 2-(2-Furoylcarbamoylamino)-äthyl, 2-(2-Oxo-1-imidazolidinocarbonylamino)-äthyl, 2-(4-Aethyl-2,3-dioxo-1-piperazinocarbonylamino)-äthyl und 2-(4-Methansulfonyl-1-piperazinocarbonylamino)-äthyl bedeutet, und $R_6$ Aminothiazolyl, z.B. 2-Amino-4-thiazolyl, Aminothiadiazolyl, z.B. 5-Amino-1,2,4-thiadiazolyl-3-yl, oder Aminotriazolyl, z.B. 5-Amino-1,2,4-triazol-3-yl, bedeutet, Stereoisomere, Mischungen von diesen Stereoisomeren, Hydrate und pharmazeutisch verwendbare Salze von solchen Verbindungen, dadurch gekennzeichnet, dass man die in Anspruch 1 genannten Massnahmen durchführt.

4. Verfahren nach Anspruch 1 zur Herstellung von Verbindungen der Formel I, worin m Null, $R_1$ Wasserstoff, Niederalkoxy, z.B. Methoxy, Halogen, z.B. Chlor, oder eine Gruppe der Formel $-CH_2-R_2$, worin $R_2$

Niederalkanoyloxy, z.B. Acetoxy, Carbamoyloxy, Tetrazolylthio, z.B. 1H-Tetrazol-5-ylthio, durch Niederalkyl, z.B. Methyl, Diniederalkylaminoniederalkyl, z.B. 2-Dimethylaminoäthyl, Sulfoniederalkyl, z.B. Sulfomethyl, oder Carboxyniederalkyl, z.B. Carboxymethyl, substituiertes Tetrazolylthio, z.B. 1-Methyl-1H-tetrazol-5-ylthio, 1-(2-Dimethylaminoäthyl)-1H-tetrazol-5-ylthio, 1-Carboxymethyl-1H-tetrazol-5-ylthio, 1-Sulfomethyl-1H-tetrazol-5-ylthio oder 1-Carboxymethyl-1H-tetrazol-5-ylthio, durch Niederalkyl, z.B. Methyl, substituiertes 5,6-Dioxotetrahydro-as-triazin-3-ylthio, z.B. 2-Methyl-5,6-dioxo-1,2,5,6-tetrahydro-as-triazin-3-ylthio oder 4-Methyl-5,6-dioxo-1,4,5,6-tetrahydro-as-triazin-3-ylthio, Pyridinio oder durch Hydroxyniederalkyl, z.B. Hydroxymethyl, Carboxy, Carboxyniederalkyl, z.B. Carboxymethyl, Halogen, z.B. Chlor oder Brom, oder Carbamoyl substituiertes Pyridinio, z.B. 3- oder 4-Hydroxymethylpyridinio, 4-Carboxypyridinio, 3- oder 4-Carboxymethylpyridinio, 3- oder 4-Chlorpyridinio, 3- oder 4-Brompyridinio oder 3- oder 4-Carbamoylpyridinio bedeutet, $R_3$ Carboxy, Niederalkanoyloxyniederalkoxycarbonyl, z.B. Niederalkanoyloxymethoxycarbonyl oder Niederalkanoyloxyäthoxycarbonyl, z.B. Pivaloyloxymethoxycarbonyl oder 2-Propionyloxyäthoxycarbonyl oder Niederalkoxycarbonyloxyniederalkoxycarbonyl, z.B. 1-Aethoxycarbonyloxyäthoxycarbonyl oder tert.-Butoxycarbonyloxymethoxycarbonyl, $R_4$ Wasserstoff, $R_5$ Niederalkyl, z.B. Methyl oder Aethyl, Niederalkoxyniederalkyl, z.B. Methoxymethyl, 2-Methoxyäthyl oder 2-Aethoxyäthyl, Niederalkenyloxyniederalkyl, z.B. 2-Vinyloxyäthyl, Halogenniederalkyl, z.B. Chlormethyl oder 2-Chloräthyl, Carboxyniederalkyl, z.B. Carboxymethyl oder 2-Carboxyäthyl, Cyanoniederalkyl, z.B. Cyanomethyl oder 1-Cyano- oder 2-Cyanoäthyl, oder eine Gruppe der Teilformel A, welche 2-Aminoäthyl, 2-Niederalkylaminoäthyl, z.B. 2-Methylaminoäthyl oder 2-Aethylaminoäthyl, 2-Diniederalkylaminoäthyl, z.B. 2-Dimethylaminoäthyl, 2-Sulfoaminoäthyl, Niederalkanoylaminoäthyl, z.B. 2-Formylaminoäthyl oder 2-Acetylaminoäthyl, Niederalkoxyniederalkanoylaminoäthyl, z.B. 2-Methoxyacetylaminoäthyl, Cyanoniederalkanoylaminoäthyl, z.B. 2-Cyanoacetylaminoäthyl, Niederalkanoylaminoäthyl, z.B. 2-Acryloylaminoäthyl, Niederalkinoylaminoäthyl, z.B. 2-Propionylaminoäthyl, Cycloalkanoylaminoäthyl, z.B. 2-Cyclopropanoylaminoäthyl, 2-(4-Hydroxy-1,2,5-thiadiazol-3-ylcarbonylamino)-äthyl, 2-(2-Tetrazol-5-ylacetylamino)-äthyl, 2-[2-(2-Amino-1,3-thiazol-4-yl)-acetylamino]-äthyl, 2-Niederalkoxycarbonylaminoäthyl, z.B. 2-Methoxycarbonylaminoäthyl, 2-Niederalkansulfonylaminoäthyl, z.B. 2-Methansulfonylaminoäthyl, 2-Benzolsulfonylaminoäthyl, 2-Benzolsulfonylaminoäthyl, worin Benzol durch Nitro oder Amino substituiert ist, z.B. 2-(4-Nitrobenzolsulfonylamino)-äthyl, 2-(2,4-Dinitrobenzolsulfonylamino)-äthyl, 2-(2-Oxo-1-imidazolidinocarbonylamino)-äthyl, 2-(4-Aethyl-2,3-dioxo-1-piperazinocarbonylamino)-äthyl, oder 2-(4-Methylsulfonyl-1-piperazino-carbonylamino)-äthyl, bedeutet, und $R_6$ Aminothiazolyl, z.B. 2-Amino-4-thiazolyl, bedeutet, Stereoisomere, Mischungen von diesen Stereoisomeren, Hydrate und pharmazeutisch verwendbare Salze von solchen Verbindungen, dadurch gekennzeichnet, dass man die in Anspruch 1 genannten Massnahmen durchführt.

5. Verfahren zur Herstellung von 3-(1-Methyl-1H-tetrazol-5-ylthiomethyl)-7β-[(2R)-2-(2-aminothiazol-4-yl)-2-(2-formylaminoäthansulfonylamino)-acetamido]-3-cephem-4-carbonsäure, gemäss Anspruch 1.

6. Verfahren zur Herstellung von 3-(1-Methyl-1H-tetrazol-5-ylthiomethyl)-7β-[(2R,S)-2-(2-aminothiazol-4-yl)-2-methansulfonylaminoacetamido]-3-cpehem-4-carbonsäure gemäss Anspruch 1.

7. Verfahren zur Herstellung von 3-(4-Carbamoylpyridiniomethyl)-7β-[(2R,S)-2-(2-aminothiazol-4-yl)-2-methansulfonylaminoacetamido]-3-cephem-4-carbonsäure, gemäss Anspruch 1.

8. Verfahren zur Herstellung von 3-Acetoxymethyl-7β-[(2R,S)-2-(2-aminothiazol-4-yl)-2-(2-formylaminoäthansulfonylamino)-acetamido]-3-cephem-4-carbonsäure, gemäss Anspruch 1.

9. Verfahren zur Herstellung von 3-(1-Methyl-1H-tetrazol-5-ylthiomethyl)-7β-[(2R,S)-2-(2-aminothiazol-4-yl)-2-cyanomethansulfonylaminoacetamido]-3-cephem-4-carbonsäure, gemäss Anspruch 1.

10. Verfahren zur Herstellung von 3-Carbamoyloxymethyl-7β-[(2R,S)-2-(2-aminothiazol-4-yl)-2-(2-formylaminoäthansulfonylamino)-acetamido]-3-cephem-4-carbonsäure, gemäss Anspruch 1.

11. Verfahren zur Herstellung von 3-Carbamoyloxymethyl-7β-[(2S)-2-(2-aminothiazol-4-yl)-2-methansulfonylaminoacetamido]-3-cephem-4-carbonsäure, gemäss Anspruch 1.

12. Verfahren zur Herstellung von 3-Carbamoyloxymethyl-7β-[(2S)-2-(2-aminothiazol-4-yl)-2-(2-(4-nitrobenzolsulfonylamino)-äthansulfonylamino)-acetamido]-3-cephem-4-carbonsäure, gemäss Anspruch 1.

13. Verfahren zur Herstellung von 3-Carbamoyloxymethyl)-7β-[(2R,S)-2-(2-aminothiazol-4-yl)-2-(2-methansulfonylaminoäthansulfonylamino)-acetamido]-3-cephem-4-carbonsäure, gemäss Anspruch 1.

14. Verfahren zur Herstellung von 3-Carbamoyloxymethyl-7β-[(2S)-2-(2-aminothiazol-4-yl)-2-(2-acryloylaminoäthansulfonylamino)-acetamido]-3-cephem-4-carbonsäure, gemäss Anspruch 1.

15. Verfahren zur Herstellung von 3-Carbamoyloxymethyl-7β-[(2S)-2-(2-aminothiazol-4-yl)-2-(2-(4-aminobenzolsulfonylamino)-äthansulfonylamino)-acetamido]-3-cephem-4-carbonsäure, gemäss Anspruch 1.

16. Verfahren zur Herstellung von 3-Carbamoyloxymethyl-7β-[(2S)-2-(2-aminothiazol-4-yl)-2-(2-methoxyacetylaminoäthansulfonylamino)-acetamido]-3-cephem-4-carbonsäure, gemäss Anspruch 1.

17. Verfahren zur Herstellung von 3-Acetoxymethyl-7β-[(2S)-2-(2-(2-aminothiazol-4-ylacetamido)-äthansulfonylamino)-2-(2-aminothiazol-4-yl)-acetamido]-3-cephem-4-carbonsäure, gemäss Anspruch 1.

18. Verfahren zur Herstellung von 3-Carbamoyloxymethyl-7β-[(2S)-2-(2-(2-aminothiazol-4-ylacetamido)-äthansulfonylamino)-2-(2-aminothiazol-4-yl)acetamido]-3-cephem-4-carbonsäure, gemäss Anspruch 1.

19. Verfahren zur Herstellung von 3-Carbamoyloxymethyl-7β-[(2S)-2-(2-aminothiazol-4-yl)-2-äthansulfonylaminoacetamido]-3-cephem-4-carbonsäure, gemäss Anspruch 1.

20. Verfahren zur Herstellung von 3-(2,5-Dihydro-6-hydroxy-2-methyl-5-oxo-as-triazin-3-ylthiomethyl)-7β-[(2R,S)-2-(2-aminothiazol-4-yl)-2-methansulfonylaminoacetamido]-3-cephem-4-carbonsäure, gemäss Anspruch 1.

21. Verfahren zur Herstellung von pharmazeutischen Präparaten, dadurch gekennzeichnet, dass man 7β-Acylamido-3-cephem-4-carbonsäure-Verbindungen der Formel I, Stereoisomere, Mischungen von diesen Stereoisomeren, Hydrate und Salze nach dem Verfahren gemäss Anspruch 1 herstellt und diese mit einem pharmazeutischen Trägermaterial mischt.

22. Verfahren zur Herstellung von pharmazeutischen Präparaten, gekennzeichnet durch die Verarbeitung eines Wirkstoffs der Formel I, sowie Stereoisomeren, Mischungen von diesen Stereoisomeren, Hydraten und Salzen davon mit einem pharmazeutischen Trägermaterial.

23. Verfahren zur Herstellung von Verbindungen der Formel III, worin $R_5$ und $R_6$ die in Anspruch 1 unter Formel I genannten Bedeutungen haben, und worin eine in $R_5$ und $R_6$ vorhandene

funktionelle Gruppe in freier oder geschützter Form vorliegt, dadurch gekennzeichnet, dass man in eine Verbindung der Formel

$$R_6 - \underset{NH_2}{\underset{|}{CH}} - \overset{O}{\overset{\|}{C}} - OH \qquad (VII),$$

worin $R_6$ die unter Formel I genannten Bedeutungen hat und eine in $R_6$ vorhandene funktionelle Gruppe in geschützter Form vorliegt, die 2-Aminogruppe mit einer Sulfonsäure der Formel

$$R_5 - SO_2 - OH \qquad (V),$$

worin $R_5$ die unter Formel I genannte Bedeutung hat und eine in $R_5$ vorhandene funktionelle Gruppe in geschützter Form vorliegt, oder mit einem reaktionsfähigen, funktionellen Säurederivat oder einem Salz davon acyliert und, wenn erwünscht, in einer erhältlichen Verbindung vorhandene Schutzgruppen abspaltet und/oder eine erhältliche Verbindung der Formel III in eine andere Verbindung der Formel III umwandelt.